# EUROPEAN PATENT APPLICATION

(11) **EP 1 229 025 A1**
(43) Date of publication of application: **07.08.2002**
(21) Application number: 00962890.0
(22) Date of filing: 27.09.2000
(51) Int. Cl.: C07D 239/94, A61K 31/517, C07D 401/04, C07D 401/12, C07D 405/12, C07D 409/12, A61P 37/06

(54) **NOVEL QUINAZOLINE DERIVATIVES**

(30) Priority: 01.10.1999 JP 28207899
(71) Applicant: Japan Energy Corporation, Tokyo-to 105-8407 (JP)
(72) Inventor: TOBE, Masanori Japan Energy Corporation, Toda-shi, Saitama 335-8502 (JP); ISOBE, Yoshiaki Japan Energy Corporation, Toda-shi, Saitama 335-8502 (JP); TOMIZAWA, Hideyuki Japan Energy Corporation, Toda-shi, Saitama 335-8502 (JP); MATSUMOTO, Mitsuhiro Japan Energy Corporation, Toda-shi, Saitama 335-8502 (JP); NAGASAKI, Takahiro Japan Energy Corporation, Toda-shi, Saitama 335-8502 (JP); OBARA, Fumihiro Japan Energy Corporation, Toda-shi, Saitama 335-8502 (JP)
(74) Representative: Ziebig, Marlene, Dr. Dipl.-Chem.
(86) International application number: JP0006666
(87) International publication number: WO0125218

(57) **Abstract**

Quinazoline derivatives represented by the general formula (1) or a pharmaceutically acceptable salt thereof in said formula R¹ is nitro or halogen; R² and R⁴ are each hydrogen, C₁₋₄ alkyl, carboxyl, or C₂₋₅ alkoxycarbonyl; R³ is hydrogen, amino, optionally substituted C₁₋₄ alkyl, C₁₋₄ alkanoyl, or C₂₋₅ alkoxycarbonyl; W is carbon or nitrogen; and m is 0 to 2.

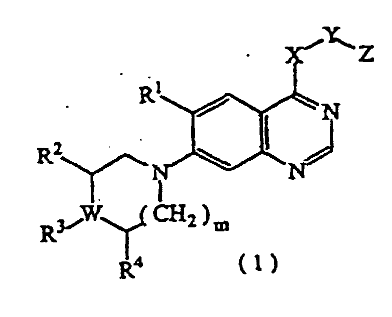

## Description

### Technical Field

The present invention relates to novel quinazoline derivatives useful as medicament, more particularly to novel quinazoline derivatives having an action inhibiting the production of TNF-α, IL-4 and IL-5 and the pharmaceutically acceptable salts thereof.

### Background Art

TNF-α is a peptide consisting of 157 amino acids and having a molecular weight of about 17,000, and one of cytokines produced from various cells including macrophage. TNF-α was found at first as a cytokine which induced hemorrhagic necrosis in tumor portions, but through subsequent studies it has been made clear that its actions range not only over tumor cells but also over many normal cells and it shows a variety of activities. Examples thereof are antitumor activity through cytostatic and cytopathic effects against tumor cells, regulation of differentiation and multiplication through the induction of apoptosis, inhibition of the lipoprotein lipase activity of fat globules, development of HLA antigen of vascular endothelial cells and fibroblast, activation of neutrophile and generation of superoxide and so forth.
Of these functions, for example regarding inflammation, TNF-α acts on all the cells which are involved in inflammation, produces or induces cytokines, such as IL-6, IL-8 and GM-CSF, and cell adhesive molecules, such as ICAM-1, and plays a part in many phenomena observed at the time of inflammation, such as production and induction of, for example, prostagladin E2 and protease, cell differentiation and activation, bone resorption, and proliferation of synovial cells. And, it is understood as a cytokine which extensively participates in the biophylaxis through the immune reaction against inflammation (Masatoshi Yamazaki: Cytokine 94-From the basic to latest information-; Nippon Igaku Kan, 109-117(1994), Vassalli. P., Ann. Rev. Immunol., 10, 411(1992)).

On the other hand, it has come clear that a continuous or excess production of TNF-α, in contrast with the above, acts on normal cells and causes various diseases. For example, it is reported that TNF-α is increased in the joint synovia and blood of rheumatoid arthritis patients (Tetta, C., Camussi, G., Modena, V., Vittorio, C. D., Baglioni, C., Ann. Rheum. Dis. 49, 665(1990)) and that in the clinical test using anti-TNF-α antibody this shows a remarkable effect (Elliott, M. J., Maini, R. N., Feldman, M., Kalden, J. R. et al., Lancet, 344, 1105(1994), Elliot, M. J., Maini, R. N., Feldman, M. et al., Lancet, 344, 1125(1994), E. C. C. Rankin, E. H. S. Choy, et al., British J. Rheum., 34, 334(1995)). Concerning the septic shock, too, TNF-α is one of causes thereof and it is reported that in the experiment using anti-TNF-α antibody this has a inhibitory effect (Starnes, H. F., Jr., Pearce, M. K., Tewari, A. et al., J. Immunol., 145, 4185(1990), Beutler, B., Milsark, I. W., Cerami, A. C., Science, 229, 869(1985), Hinshaw, L. B., Tekamp-olson, P., Chang, A. C. K. et al., Circ. Shock, 30, 279(1990)). Further, the participation of TNF-α is suggested also regarding inflammatory bowel diseases such as ulserative colitis and Crohn's disease (Murch, S., Walker-Smith, J. A., Arch. Dis. Child, 66, 561(1991), Masahiro Maeda, Digestive Organ and Immunity, 22, 111(1989)), and it is reported that in the clinical test in which anti-TNF-α antibody is used the effect is demonstrated (Hendrik, M. VanDullemen, Sander, J. H. VanDeventer, et al., Gastroenterology, 109, 129(1995)).

It is also suggested that the excess production of TNF-α plays a part in diseases such as osteoarthritis (Venn, G., Nietfeld, J. J., Duits, A. J., et al., Arthritis Rheum., 36, 819(1993)), multiple sclerosis (Sharief, M. K., Hentges, R., N. Engl. J. Med., 325, 467(1991)), Behcet's disease (Akoglu, T., Direskeneli, H., Yazici, H., Lawrence, R., J. Rheumatol., 17, 1107(1990)), systemic lupus erythematodes (SLE)(Maury, C. P. J., Teppo, A-M., Sharief, M. K., Arthritis Rheum., 32, 146(1989)), rejection at the time of the bone marrow transplantation (Nestel, F. P., Price, K. S., Seemeyer, T. A., Lapp, W. S., J. Exp., Med., 175, 405(1992)), hepatitis (Kozo Kanno, Liver, 33, 213(1992) and type II diabetes (Hotamisligil, G. S., Shargill, N. S., Spiegelman, B. M., Science, 259, 87(1993)).

Further, it is reported that, also in allergic diseases such as asthma, allergic dermatitis and allergic rhinitis, the excess production of TNF-α participates in the pathologic aggravation of the diseases (Shah, A., Church, M. K., Holgate, S. T., Clin. Exp. Allergy., 25, 1038(1995)). Particularly, in asthma, not only the excess production of TNF-α but also the excess production of IL-4 and I1-5 as cytokines plays a part of the worsening of the disease, and the presence of them is actually confirmed in the affected part of a patient (Hamid, Q. M., Azzawi, M., Sun Ying, et al., J. Clin. Invest., 87, 1561(1991), Bradding, P., Roberts, J. A., Britten, K. M., et al., Am. J. Respir. Cell. Mol. Biol., 10, 471(1994)). IL-4 induces B cells to produce the IgE antibody and besides has a function of differentiating and multiplying the type 2 helper T-cell. The IL-5 has actions such as activation, differentiation and multiplication, and extension of life of eosinophilic leukocyte and plays an important role in allergic inflammation. Accordingly, it is considered that a therapeutic medicament which inhibits the excess production of not only TNF-α but also IL-4 and IL-5 is more desirable for improving the pathosis of asthma.

As mentioned above, it has become clear that the excess production of TNF-α, IL-4 or IL-5 adversely affects the diseases above-mentioned, and there is desired research and development on a compound inhibiting the production of TNF-α, IL-4 and IL-5 as a therapeutic medicament for such diseases.

As the compounds which show an action of inhibiting the production of TNF-α, the followings are enumerated.

JP-A-10-130149 discloses that quinolone derivatives are useful as a TNF-α production inhibitor.

JP-A-11-1481 discloses that piperidinylphthalazine derivatives are useful as a TNF-α production inhibitor.

Pentoxifylline having the 7-methylxanthine skeleton is known as a compound showing the production inhibitory action on TNF-α (Ward, A., Clissold, S. P., Drugs, 34, 50(1987), Semmler, J., Gebert, U., Eisenhut, T., Biochem. Biophys. Res. Commun., 155, 1230(1988), Zabel, P., Schade, F. U., Schlaak, M., Immunobiology, 187, 447(1993)).

Besides, as compounds or factors showing the action inhibiting the TNF-α production, there are known hitherto glucocorticoid, protease inhibitor, phospholipase A2 inhibitor, lipoxygenase inhibitor, PAF (platelet aggregating factor) antagonist and anti-TNF-α antibody. These compounds, however, may have side effects due to their various pharmacological actions, raising the problem regarding their use, and development of a safe drug having a new mechanism is longed for.

Meanwhile, many compounds are known which have the quinazoline skeleton. For example, there are mentioned the followings as quinazoline derivatives in which the 6-position has a nitro group or halogen atom and the 4-and 7-position have N-containing substituents.

Journal of Medicinal Chemistry (J. Med. Chem.), 39, 267(1996) describes that a 4-anilinoquinazoline derivative has an inhibitory action on phosphorylation of EGF receptors.

WO98/14431 discloses that quinazoline derivatives are useful as an inhibitor of phosphorylation of PDGF receptors.

WO98/08848 discloses that quinazoline derivatives are useful as synthetic intermediates for imidazoquinazolines derivatives which are useful as an inhibitor of cGMP-specific phosphodiesterases.

### Disclosure of the Invention

The present invention provides a novel compound which has an inhibitory action on the production of TNF-α, IL-4 or IL-5 and which is useful for the treatment of diseases in which the continuous or excess production of TNF-α, IL-4 or IL-5 is considered to play a part in the start of the diseases, such as rheumatoid arthritis, septic shock, inflammatory bowel diseases, osteoarthritis, multiple sclerosis, Behcet's disease, systemic lupus erythematodes (SLE), rejection at the time of the bone marrow transplantation, hepatitis, type II diabetes, asthma, allergic dermatitis and allergic rhinitis, or provides a pharmaceutically acceptable salt thereof.

The inventors studied hard to solve the above-mentioned problem and found, as a result, that the quinazoline derivatives represented by the following general formula (1) and the salts thereof have an inhibitory action on the production of TNF-α, IL-4 and IL-5, thus accomplishing the present invention. The compounds of the present invention indicated by the general formula (1) are novel compounds which are not included in any of the related art. Further, none of the related art describes that the compounds of the present invention indicated by the general formula (1) possesses an action inhibiting the production of TNF-α, IL-4 and IL-5.

That is, the present invention includes the following inventions.
(i) A quinazoline derivative of the general formula (1) or a pharmaceutically acceptable salt thereof: 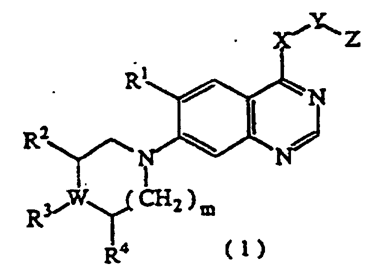 [wherein R¹ represents nitro group or halogen atom; R² and R⁴ represent hydrogen atom, alkyl group having 1 to 4 carbon atoms, carboxyl group, or alkoxycarbonyl group having 2 to 5 carbon atoms; R³ represents hydrogen atom, amino group, unsubstituted or substituted alkyl group having 1 to 4 carbon atoms, alkanoyl group having 1 to 4 carbon atoms or alkoxycarbonyl group having 2 to 5 carbon atoms; W represents carbon atom or nitrogen atom; m represents 0 to 2; X represents a group represented by the following formula (a), (b) or (c): 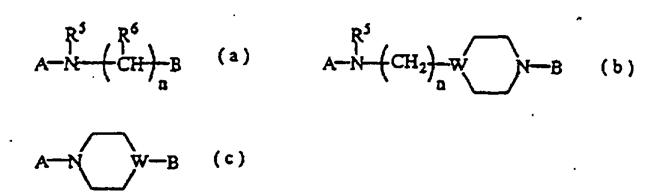 [wherein R⁵ represents hydrogen atom, or unsubstituted or substituted alkyl group having I to 4 carbon atoms; R⁶ represents hydrogen atom, unsubstituted or substituted alkyl group having 1 to 4 carbon atoms, hydroxyl group, unsubstituted or substituted aryl group, carboxyl group, or alkoxycarbonyl group having 2 to 5 carbon atoms (if n is two or more, each R⁶ may be the same or different); n represents 0 to 3; W represents carbon atom or nitrogen atom; A represents a single bond to quinazoline ring; and B represents a single bond to Y]; Y represents, if X is represented by the formula (a), single bond or CH₂, if X is represented by the formula (b), single bond, CO, CH₂, CONH, CSNH or SO₂, if X is represented by the formula (c), single bond, CO, CH₂ or SO₂; and Z represents unsubstituted or substituted aryl group, or unsubstituted or substituted heteroaryl group.]
(ii) A compound according to the above (i), wherein R¹ in the general formula (1) is nitro group or fluorine atom.
(iii) A compound according to the above (i) or (ii), wherein R² and R⁴ in the general formula (1) are hydrogen atom or alkyl group having 1 to 4 carbon atoms.
(iv) A compound according to any one of the above (i) to (iii), wherein R³ in the general formula (1) R³ is hydrogen atom.
(v) A compound according to any one of the above (i) to (iv), wherein W in the general formula (1) is nitrogen atom.
(vi) A compound according to any one of the above (i) to (v), wherein X in the general formula (1) is represented by the formula (a), and R⁵ and R⁶ in the general formula (1) are hydrogen atom.
(vii) A compound according to any one of the above (i) to (vi), wherein n in the formula (a) is 1 or 2.
(viii) A compound according to any one of the above (i) to (v), wherein X in the general formula (1) is represented by the formula (c).
(ix) A compound according to any one of the above (i) to (vii), wherein Z in the general formula (1) is phenyl, substituted phenyl, thienyl, furyl or pyridyl.
(x) A medicament comprising a quinazoline derivative according to any one of the above (i) to (ix) or a pharmaceutically acceptable salt thereof as effective ingredient.
(xi) A medicament for treating the diseases caused by the excess production of TNF-α, comprising a quinazoline derivative according to any one of the above (i) to (ix) or a pharmaceutically acceptable salt thereof as effective ingredient.
(xii) A medicament for treating the diseases caused by the excess production of IL-4, comprising a quinazoline derivative according to any one of the above (i) to (ix) or a pharmaceutically acceptable salt thereof as effective ingredient.
(xiii) A medicament for treating the diseases caused by the excess production of IL-5, comprising a quinazoline derivative according to any one of the above (i) to (ix) or a pharmaceutically acceptable salt thereof as effective ingredient.

Concretely, the present invention relates to the quinazoline derivatives of the formula (1) or the salts thereof and relates to inhibitors of TNF-α, IL-4 or IL-5 production which contain them as an effective ingredient. More concretely, the present invention relates to therapeutic medicaments which contain a quinazolone derivative of the formula (1) or a salt thereof as an effective ingredient, for rheumatoid arthritis, septic shock, inflammatory bowel diseases, osteoarthritis, multiple sclerosis, Behcet's disease, systemic lupus erythematodes (SLE), rejection at the time of the bone marrow transplantation, hepatitis, type II diabetes, asthma, allergic dermatitis and allergic rhinitis and the like.

The compounds of the present invention will be explained more in detail.

Each substituent in the general formula (1) will hereinunder be explained concretely.

In the general formula (1), R¹ represents nitro group or halogen atom, in which nitro group, chlorine atom or fluorine atom is preferable, and nitro group is most preferable.

In the general formula (1), R² and R⁴ represent hydrogen atom, alkyl group having 1 to 4 carbon atoms, carboxyl group or alkoxycarbonyl group having 2 to 5 carbon atoms. The alkyl group having 1 to 4 carbon atoms includes, for example, methyl, ethyl, propyl, butyl and the like. The alkoxycarbonyl group having 2 to 5 carbon atoms includes, for example, methoxycarbonyl, ethoxycarbonyl, propoxycarbonyl, butoxycarbonyl and the like. Of these, hydrogen atom or alkyl group having 1 to 4 carbon atoms is preferred, and hydrogen atom is most preferred.

In the general formula (1), R³ represents hydrogen atom, amino group, unsubstituted or substituted alkyl group having 1 to 4 carbon atoms, alkanoyl group having 1 to 4 carbon atoms or alkoxycarbonyl group having 2 to 5 carbon atoms. The unsubstituted alkyl group having 1 to 4 carbon atoms includes, for example, methyl, ethyl, propyl, isopropyl, butyl and the like.

The substituted alkyl group in R³ includes alkyl group having 1 to 4 carbon atoms (for example, methyl, ethyl, propyl, isopropyl, butyl and the like), which is substituted with a substituent. Said substituent includes hydroxyl group, amino group, alkoxy group having 1 to 4 carbon atoms (for example, methoxy, ethoxy, propoxy, isopropoxy, butoxy and the like).

The alkanoyl group having 1 to 4 carbon atoms in R³ includes, for example, formyl, acetyl, propionyl, butyryl and the like.

The alkoxycarbonyl group having 2 to 5 carbon atoms in R³ includes, for example, methoxycarbonyl, ethoxycarbonyl, propoxycarbonyl, isopropoxycarbonyl, butoxycarbonyl and the like.

The substituent as R³ is preferably hydrogen atom, methyl, ethyl, propyl, isopropyl, butyl, aminomethyl, aminoethyl or hydroxyethyl, and hydrogen atom is particulary preferable.

In the general formula (1), W represents carbon atom or nitrogen atom, and nitrogen atom is particularly preferable.

In the general formula (1), m represents 0 to 2, and 1 or 2 is preferable, 1 being particularly preferable.

In the general formula (1), X is a group represented by any one of the above formula (a), (b) or (c). In the above formulae, R⁵ represents hydrogen atom, unsubstituted or substituted alkyl group having 1 to 4 carbon atoms, R⁶ represents hydrogen atom, unsubstituted or substituted alkyl group having 1 to 4 carbon atoms, hydroxyl group, unsubstituted or substituted aryl group, carboxyl group or alkoxycarbonyl group having 2 to 5 carbon atoms, n represents 0 to3, and W represents carbon atom or nitrogen atom. If n is 2 or more, each R⁶ may be the same or different.

The unsubstituted alkyl group having 1 to 4 carbon atoms in R⁵ and R⁶ in the formula (a) or (b) includes, for example, methyl, ethyl, propyl, isopropyl, butyl and the like.

The substituted alkyl group in R⁵ and R⁶ in the formula (a) or (b) includes, for example, alkyl group having 1 to 4 carbon atoms (for example, methyl, ethyl, propyl, isopropyl, butyl and the like), which is substituted with a substituent. Said substituent includes, for example, hydroxyl group, amino group, alkoxy group having 1 to 4 carbon atoms (for example, methoxy, ethoxy, propoxy, isopropoxy, butoxy and the like.)

The unsubstituted aryl group in R⁶ in the formula (a) is limited in no way as long as it is a monocyclic or condensed aromatic compound, and includes, concretely, phenyl, 1-naphthyl, 2-naphthyl and the like.

The substituted aryl group in R⁶ in the formula (a) includes a monocyclic or condensed aromatic compound having substituent and is not limited in particular as long as it is a group derived from various aromatic hydrocarbon compounds. The number of the substituent on the aryl group may be single or plural, and in the case of plural substituents, these substituents may be the same or different each other. Further, the substituent can be present in any position on the aryl group. The substituent on the substituted aryl group includes halogen atom such as fluorine, chlorine and bromine atom; alkyl group having 1 to 4 carbon atoms such as methyl, ethyl, propyl, isopropyl and butyl; alkoxy group having 1 to 4 carbon atoms such as methoxy, ethoxy, propoxy and butoxy; hydroxyl group; amino group; nitro group; cyano group; alkylamino group having 1 to 4 carbon atoms such as methylamino, ethylamino, propylamino and butylamino; dialkylamino group having 2 to 8 carbon atoms such as dimethylamino and diethylamino; (the two alkyl groups may be the same or different); alkylcarbamoyl group having 2 to 5 carbon atoms such as carbamoyl, methylcarbamoyl, ethylcarbamoyl, propylcarbamoyl and butylcarbamoyl; alkanoyloxy group having 2 to 5 carbon atoms such as acetyloxy, propionyloxy and butyryloxy; alkoxycarbonyl group having 2 to 5 carbon atoms such as methoxycarbonyl, ethoxycarbonyl, propoxycarbonyl and butoxycarbonyl; trifluoromethyl group; and methylenedioxy or ethylenedioxy group with adjacent two groups together.

The alkoxycarbonyl group having 2 to 5 carbon atoms in R⁶ of the formula (a) includes, for example, methoxycarbonyl, ethoxycarbonyl, propoxycarbonyl, isopropoxycarbonyl butoxycarbonyl and the like.

The R⁵ in the formula (a) or (b) is preferably hydrogen atom or alkyl group having 1 to 4 carbon atoms, hydrogen atom being particularly preferable.

The R⁶ in the formula (a) is preferably hydrogen atom, alkyl group having 1 to 4 carbon atoms or alkoxycarbonyl group having 2 to 5 carbon atoms, hydrogen atom being particularly preferable.

The n in the formula (a) represents 0 to 3, 1 to 3 is preferable, and 1 or 2 is particularly preferable.

The n in the formula (b) represents 0 to 3, 1 or 2 is preferable, and 1 is particularly preferable.

The W in the formula (b) represents carbon atom or nitrogen atom, and carbon atom is particularly preferable.

The W in the formula (c) represents carbon atom or nitrogen atom, and nitrogen atom is particularly preferable.

In the formula (1), Y represents, if X is represented by the formula (a), single bond or CH₂, if X is represented by the formula (b), single bond, CO, CH₂, CONH, CSNH or SO₂, if X is represented by the formula (c), single bond, CO, CH₂ or SO₂, wherein preference is given to single bond or CH₂, particularly to single bond.

In the formula (1), Z represents unsubstituted or substituted aryl group or unsubstituted or substituted heteroaryl group.

The unsubstituted aryl group of Z is limited in no way as long as it is a monocyclic or condensed aromatic compound, and includes, concretely, phenyl, 1-naphthyl, 2-naphthyl and the like.

The substituted aryl group as Z includes a monocyclic or condensed aromatic compound having substituent and is not limited in particular as long as it is a group derived from various aromatic hydrocarbon compounds. The number of the substituent on the aryl group may be single or plural, and in the case of plural substituents, these substituents may be the same or different each other. Further, the substituent can be present in any position on the aryl group. Said substituent on the substituted aryl group includes halogen atom such as fluorine, chlorine and bromine atom; alkyl group having 1 to 5 carbon atoms or cycloalkyl group such as methyl, ethyl, propyl, isopropyl, butyl and pentyl; alkoxy group having 1 to 5 carbon atoms or cycloalkyloxy group such as methoxy, ethoxy, propoxy and butoxy, pentyloxy, cyclopropyloxy, cyclobutyloxy, cyclopentyloxy; aralkyloxy group such as benzyloxy; acetamide group; hydroxyl group; amino group; nitro group; cyano group; alkylamino group having 1 to 5 carbon atoms such as methylamino, ethylamino, propylamino, butylamino and pentylamino; dialkylamino group having 2 to 8 carbon atoms such as dimethylamino and diethylamino (the two alkyl groups may be the same or different); alkylcarbamoyl group having 1 to 4 carbon atoms such as carbamoyl, methylcarbamoyl, ethylcarbamoyl, propylcarbamoyl and butylcarbamoyl; alkanoyloxy group having 2 to 5 carbon atoms such as acetyloxy, propionyloxy and butyryloxy; alkoxycarbonyl group having 2 to 5 carbon atoms such as methoxycarbonyl, ethoxycarbonyl, propoxycarbonyl and butoxycarbonyl; trifluoromethyl group; and methylenedioxy or ethylenedioxy group with adjacent two groups together.

The unsubstituted heteroaryl group in Z includes a 5- or 6-membered ring containing one or two hetero atoms (two hetero atoms may be the same or different) selected from the group consisting of nitrogen, oxygen and sulfur atom, or a 5- or 6-membered ring, which is condensed with phenyl ring, containing one or two hetero atoms (two hetero atoms may be the same or different) selected from the group consisting of nitrogen, oxygen and sulfur atom. Concretely, it includes thienyl, furyl, pyrrolyl, pyrazolyl, oxazolyl, isoxazolyl, isothiazolyl, benzoxazolyl, benzothiazolyl, benzoimidazolyl, pyridyl, pyradinyl, pyrimidyl, pyridazinyl and the like.

The substituted heteroaryl group in Z includes a 5- or 6-membered ring substituted with substituent and containing one or two hetero atoms selected from the group consisting of nitrogen, oxygen and sulfur atom, or a 5- or 6-membered ring, which is condensed with phenyl ring, containing one or two hetero atoms selected from the group consisting of nitrogen, oxygen and sulfur atom. The substituent includes, for example, halogen atom such as fluorine, chlorine and bromine atom, alkyl group having 1 to 4 carbon atoms such as methyl, ethyl, propyl, isopropyl and butyl group, alkoxy group having 1 to 4 carbon atoms such as methoxy, ethoxy, propoxy and butoxy, hydroxyl group, amino group, alkanoyloxy group having 2 to 5 carbon atoms such as acetyloxy, propionyloxy and butyryloxy, and alkoxycarbonyl group having 2 to 5 carbon atoms such as methoxycarbonyl, ethoxycarbonyl, propoxycarbonyl and butoxycarbonyl.

As the substituent in Z, unsubstituted or substituted aryl group or heteroaryl group is preferable and unsubstituted or substituted phenyl, thienyl, furyl or pyridyl is particularly preferable.

The compound of the present invention can form a salt with an acid. The preferable acid is a pharmaceutically acceptable acid and includes, concretely, an inorganic acid such as hydrochloric acid, sulfuric acid, hydrobromic acid and phosphoric acid and an organic acid such as acetic acid, oxalic acid, citric acid, malic acid, tartaric acid, fumaric acid, maleic acid and methanesulfonic acid. Further, in case that the compound of the present invention has an acidic substituent, it can form a salt with a base. The preferable base is a pharmaceutically acceptable base and includes, concretely, a hydroxide of alkali metal such as sodium and potassium or of alkaline-earth metal such as magnesium and calcium, an inorganic base such as carbonate, and an organic base such as triethylamine and pyridine. In case that the compound of the present invention has one, or two or more asymmetric carbon atoms, it includes stereoisomers in any pure form thereof such as optical isomer and diastereomer, arbitrary any mixtures thereof or racemic compounds, and further includes all the hydrates, solvates and crystals.

The compounds of the present invention concretely includes, for example, the compounds shown in Tables 1 to 20. The quinazoline derivateives used as an effective ingredient of the medicament according to the present invention, however, are not limited to these.

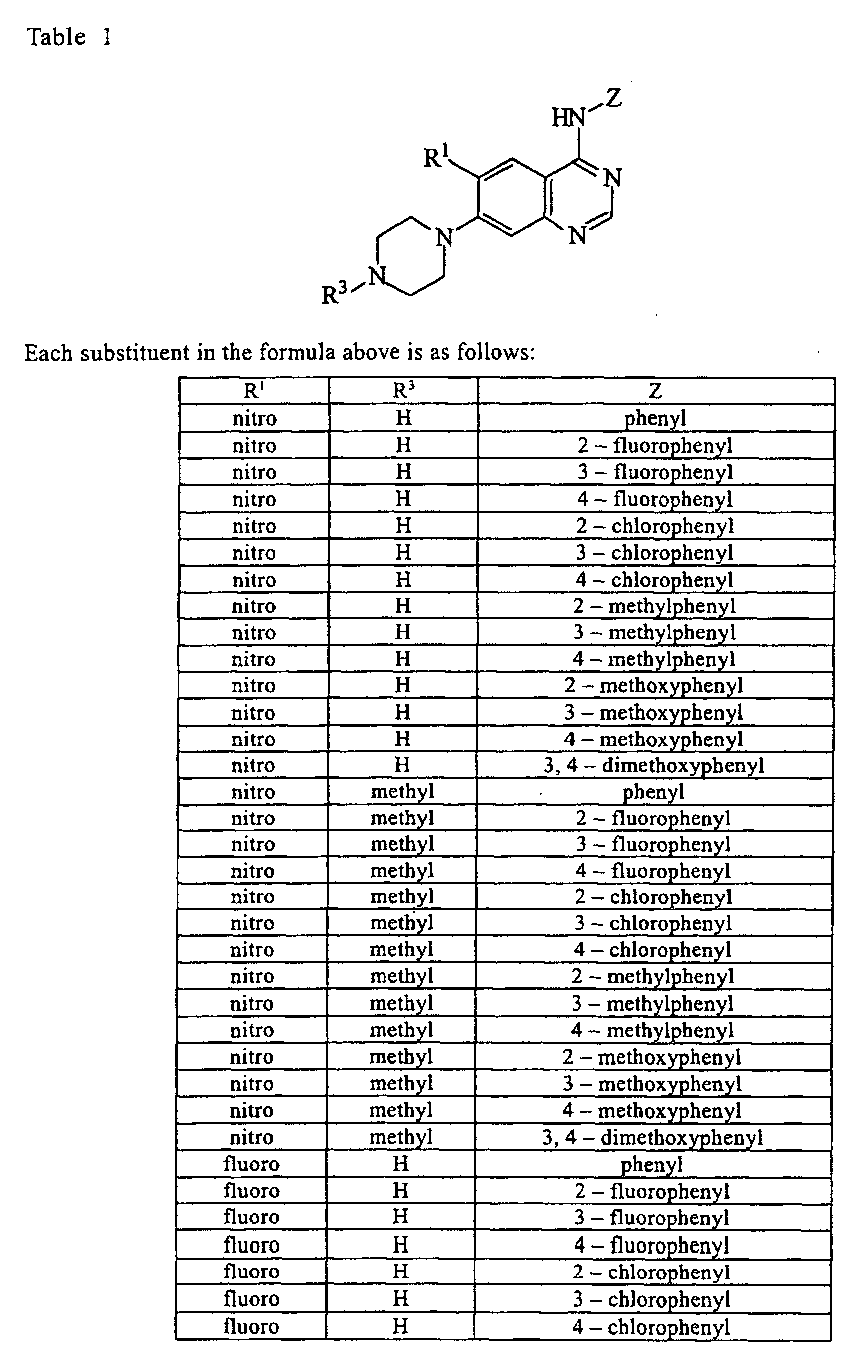

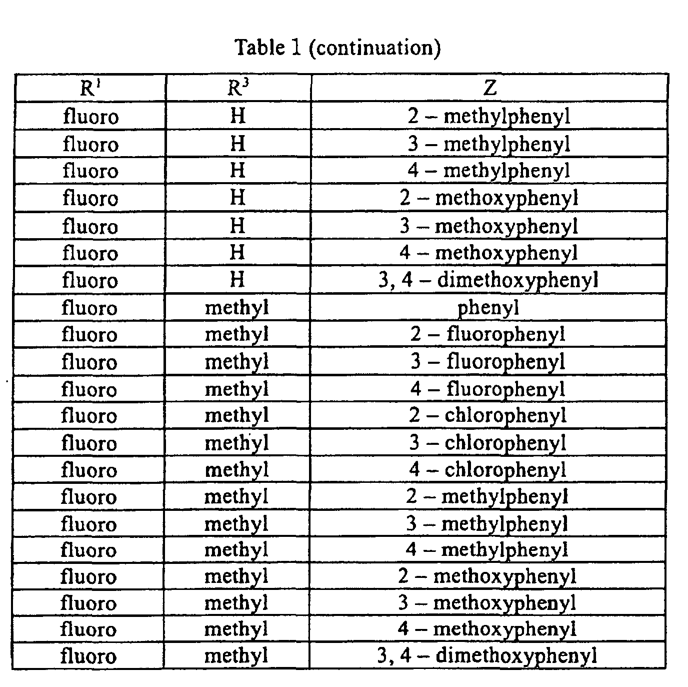

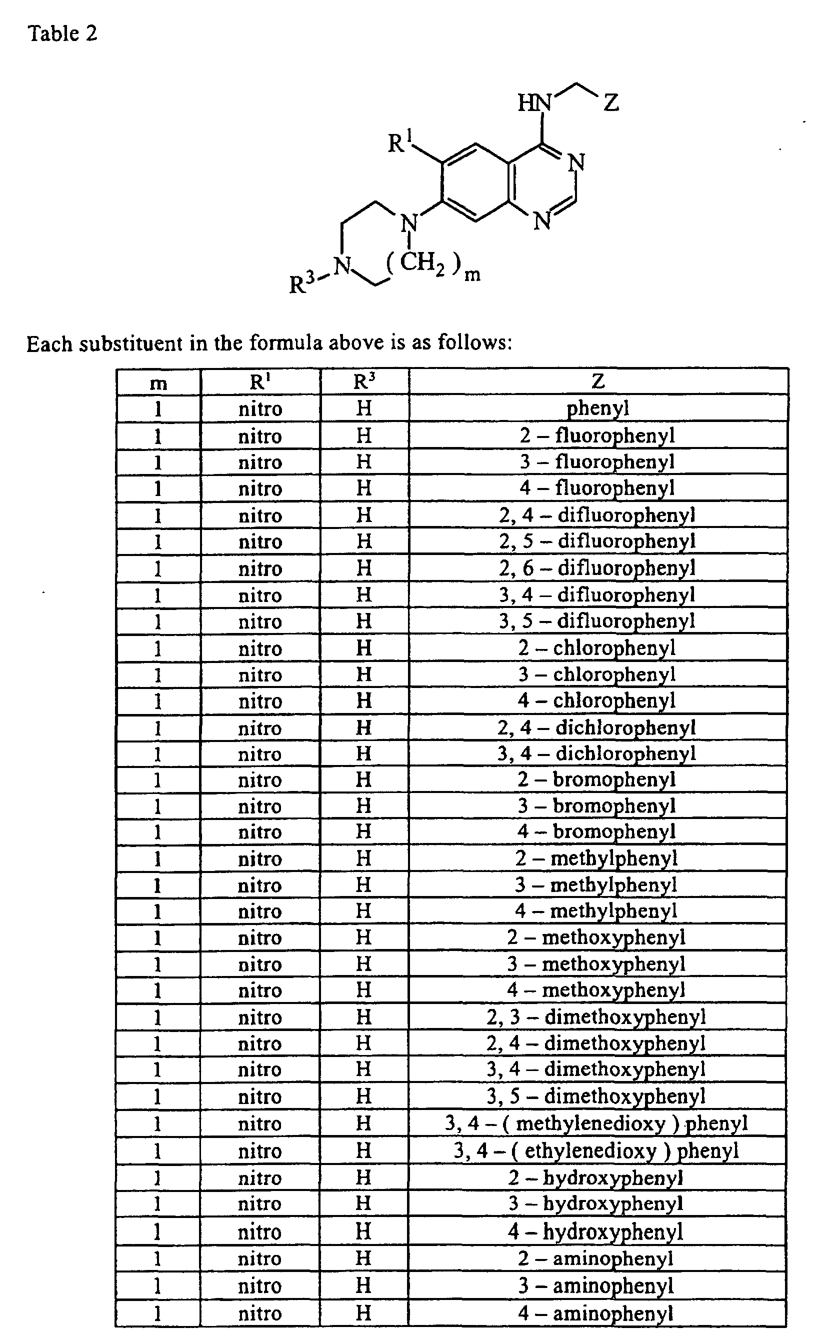

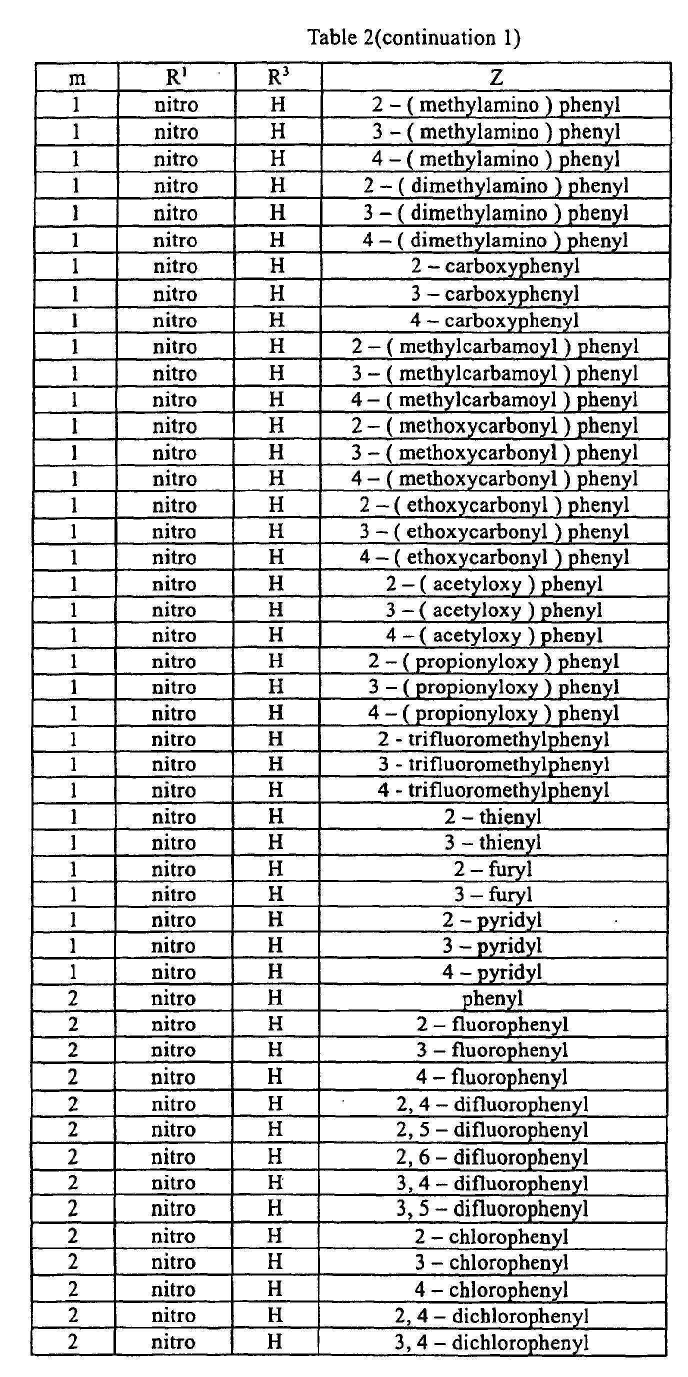

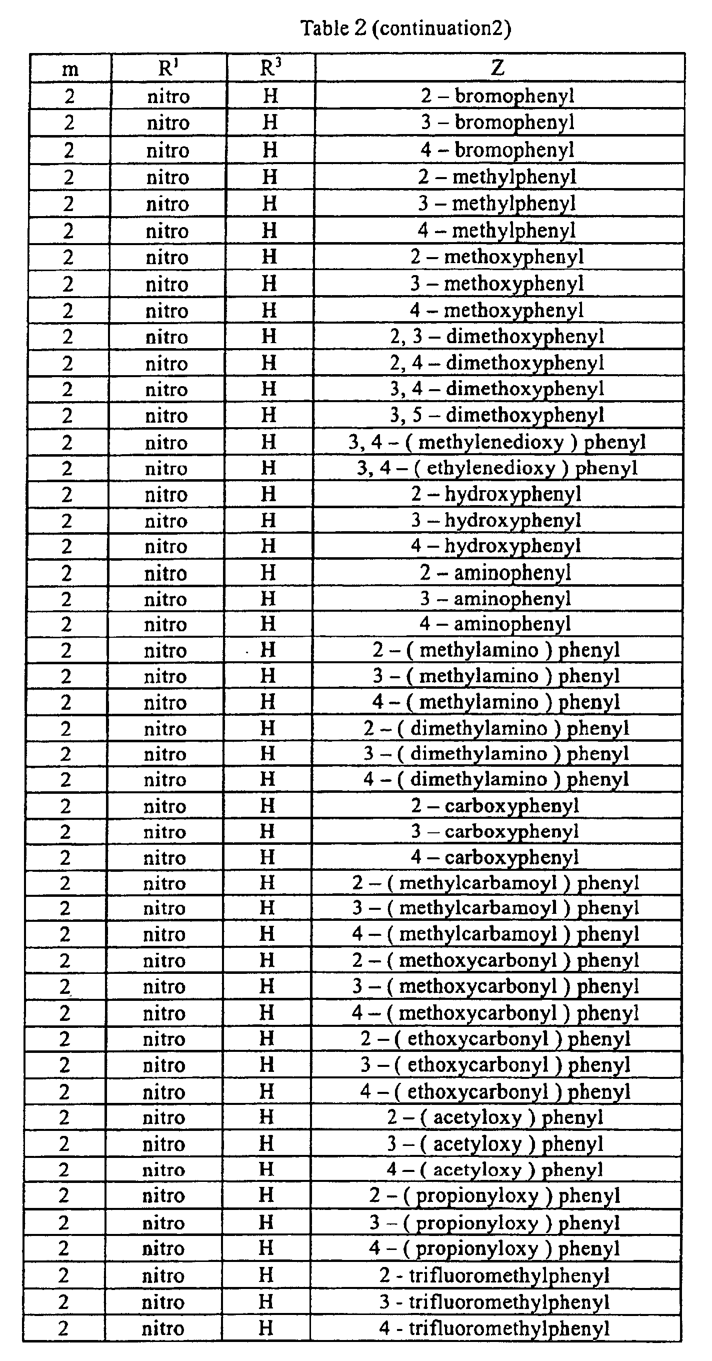

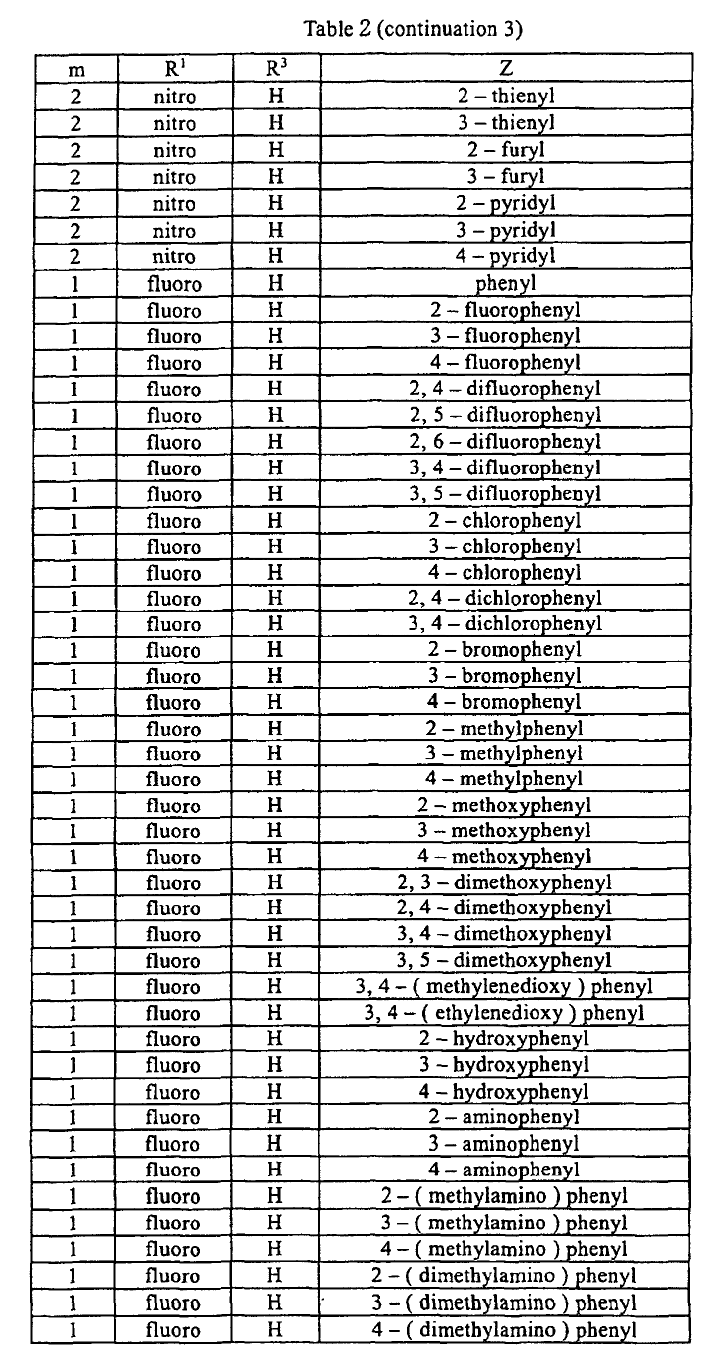

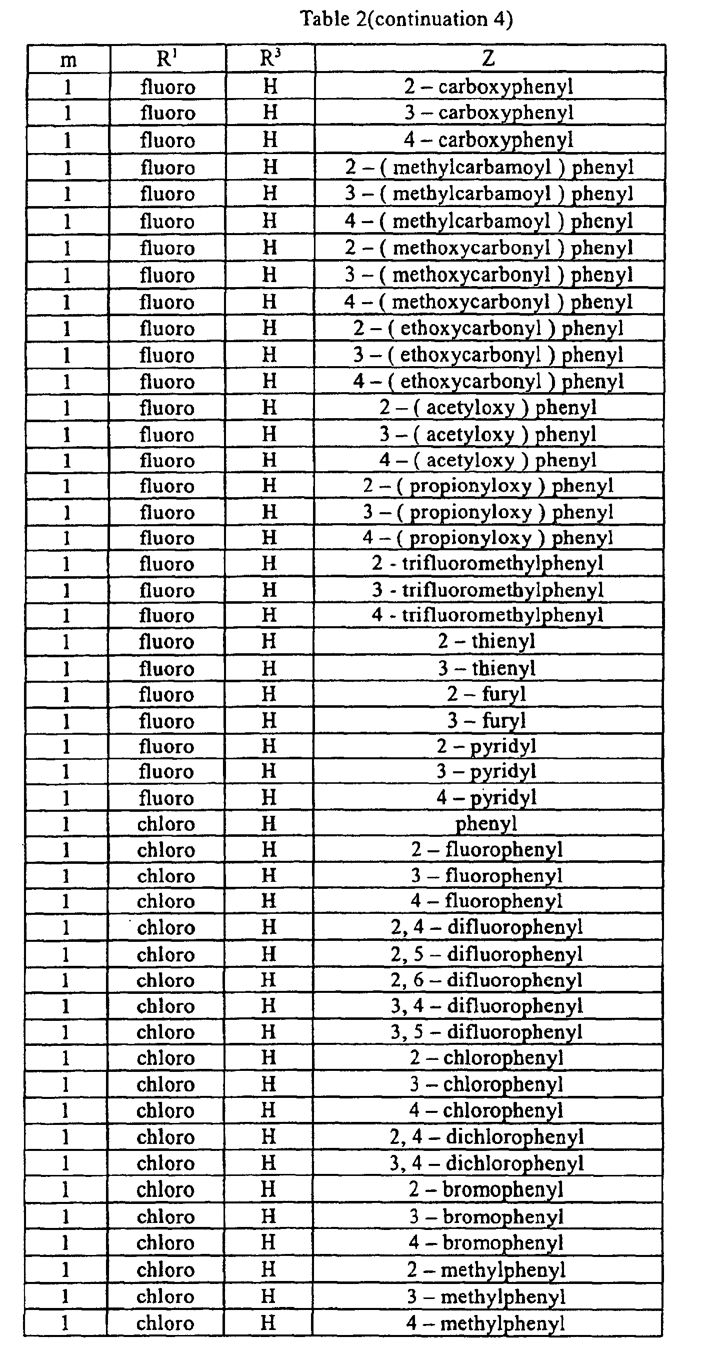

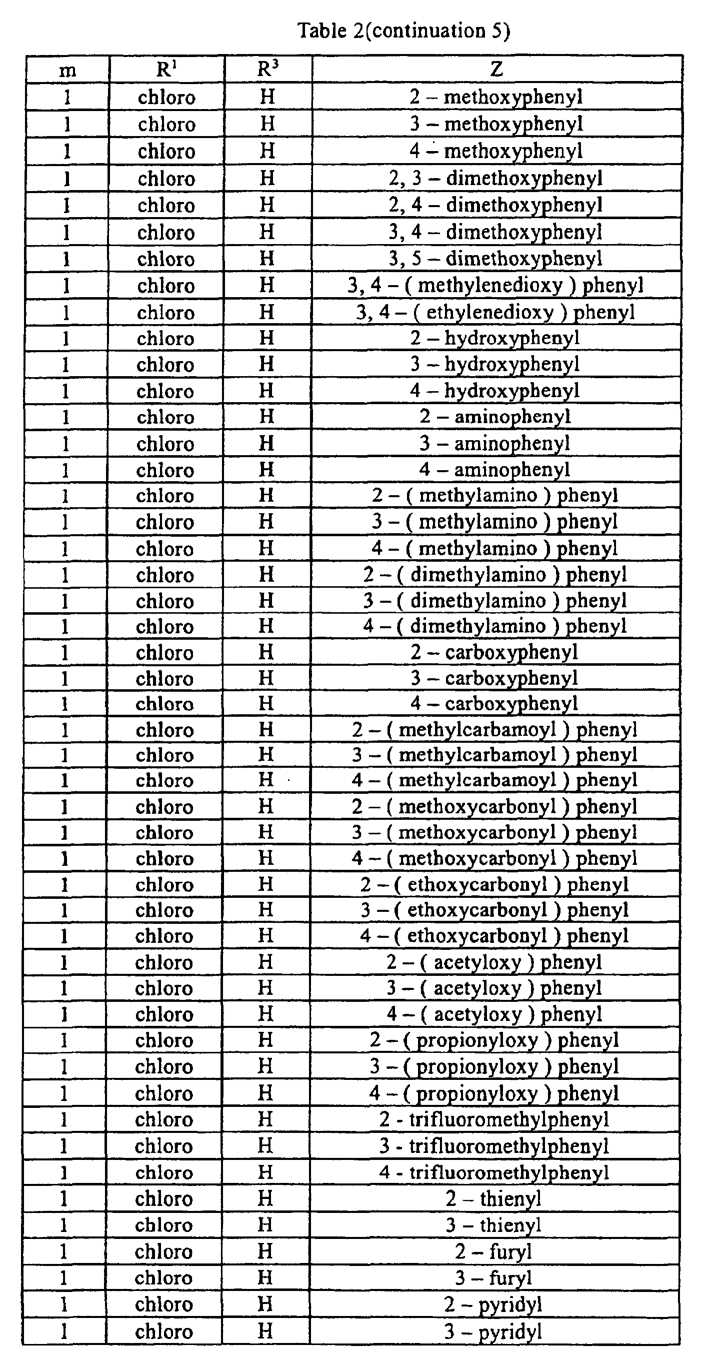

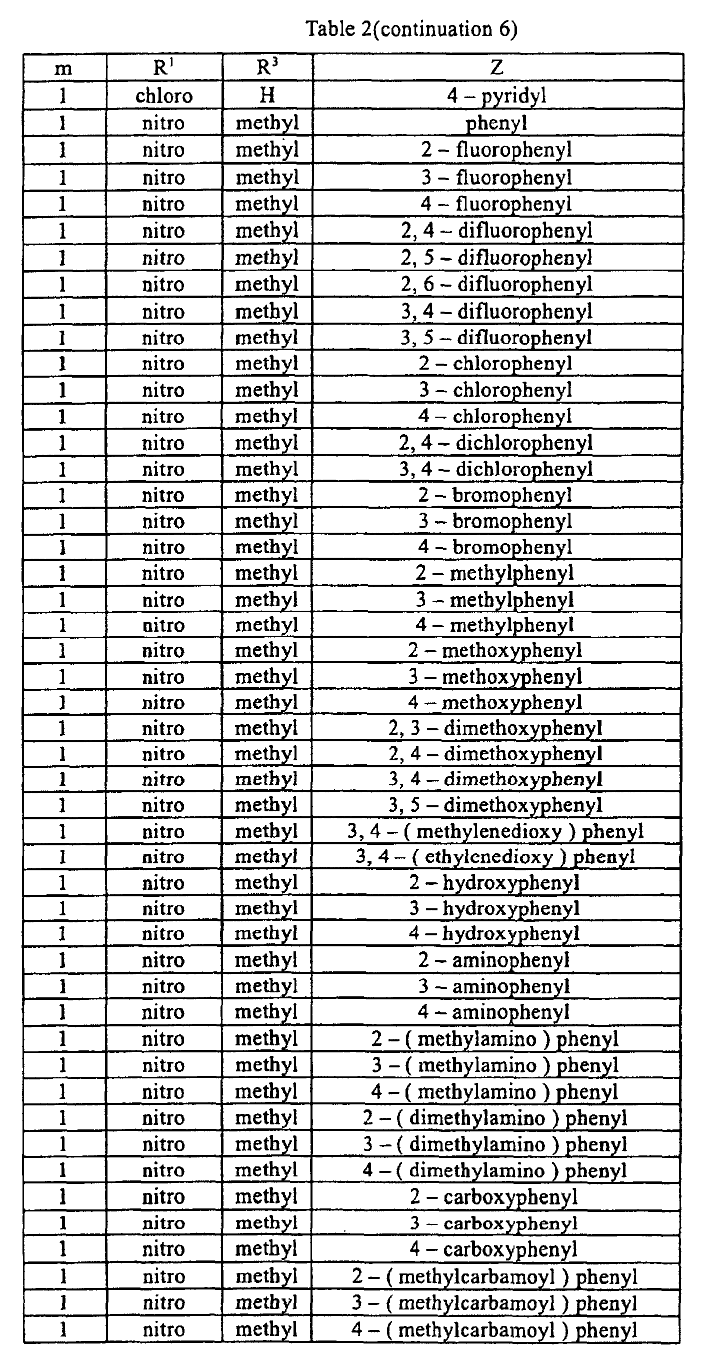

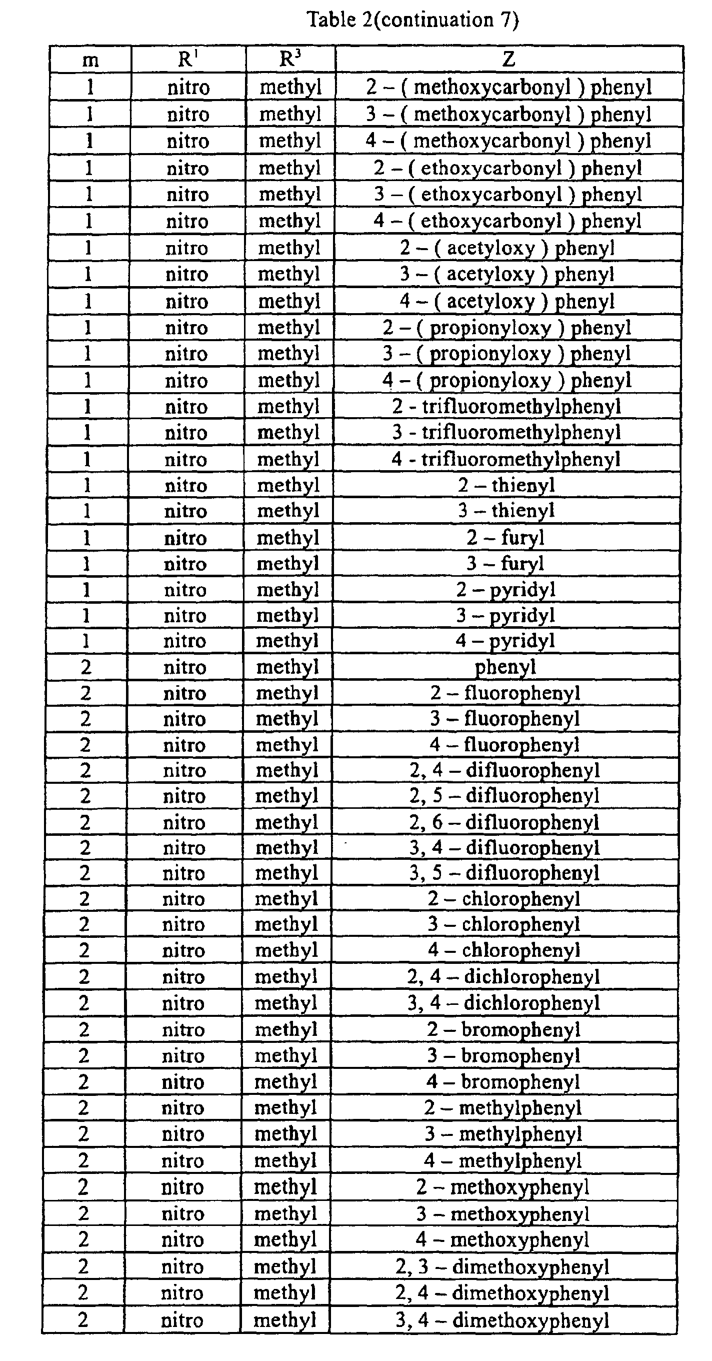

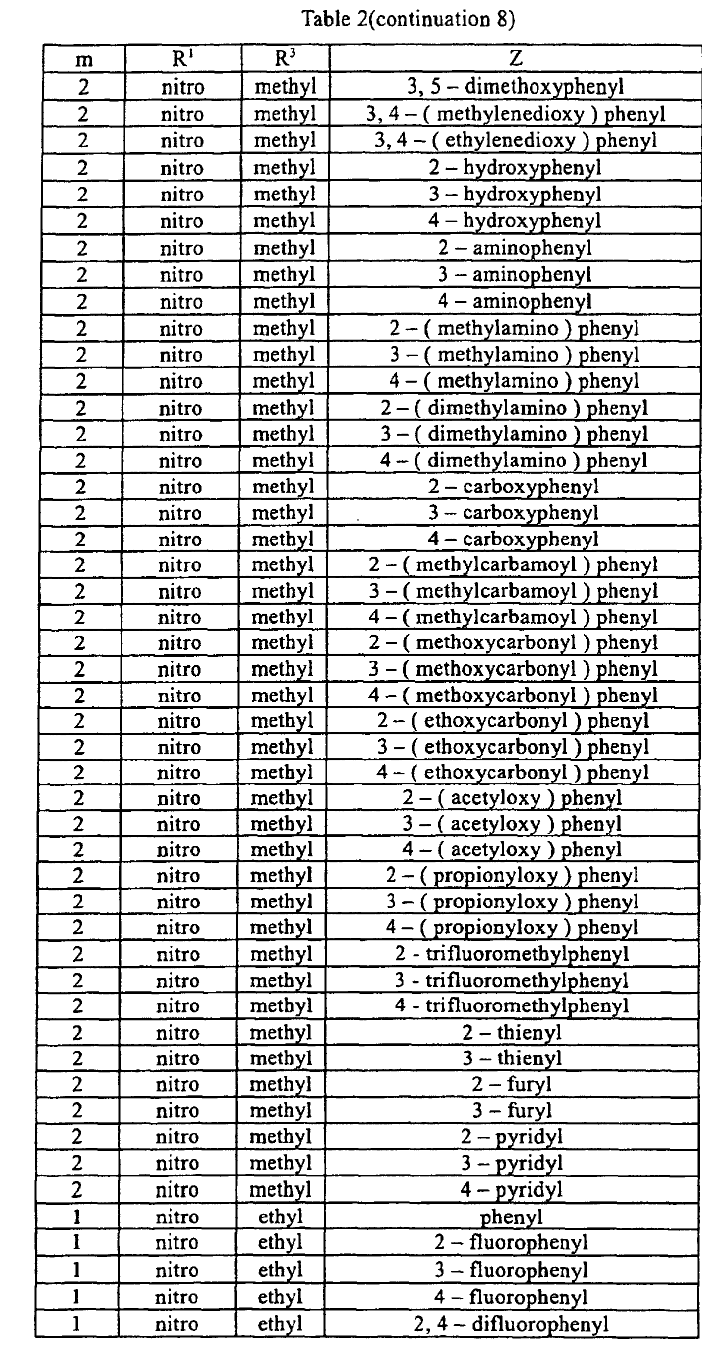

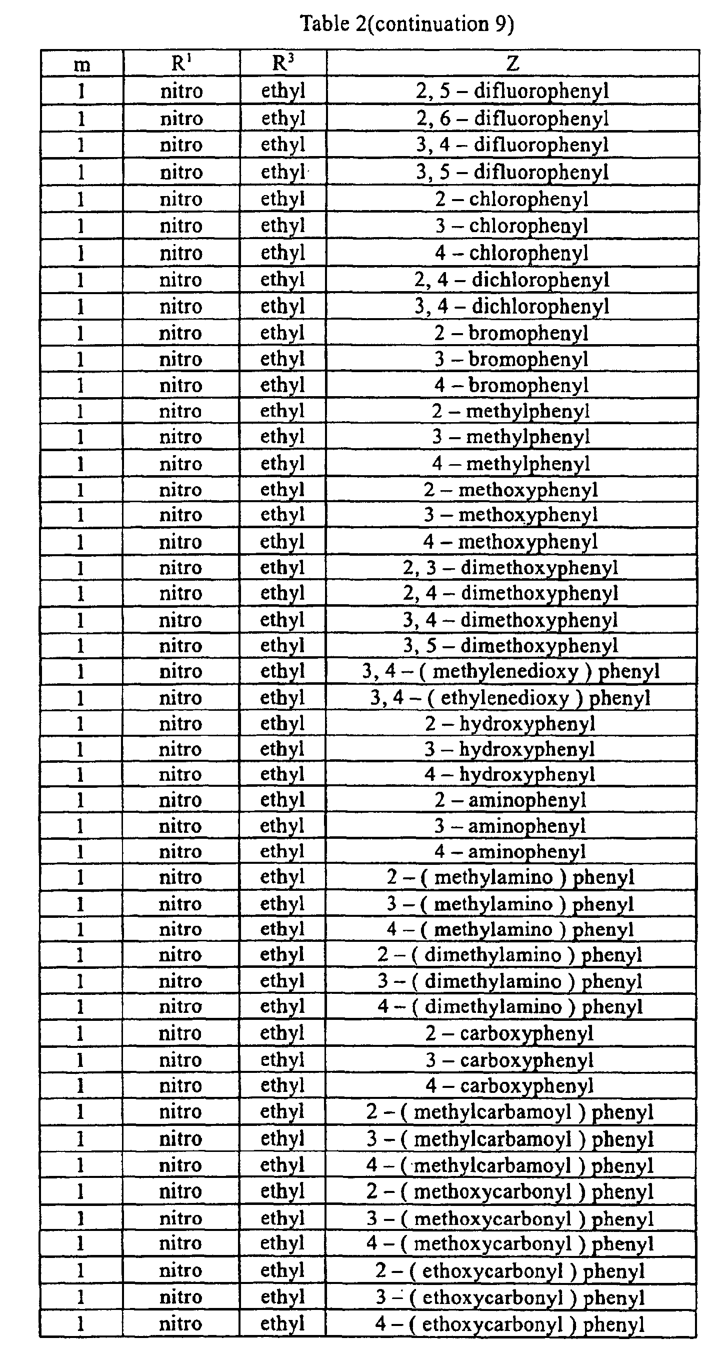

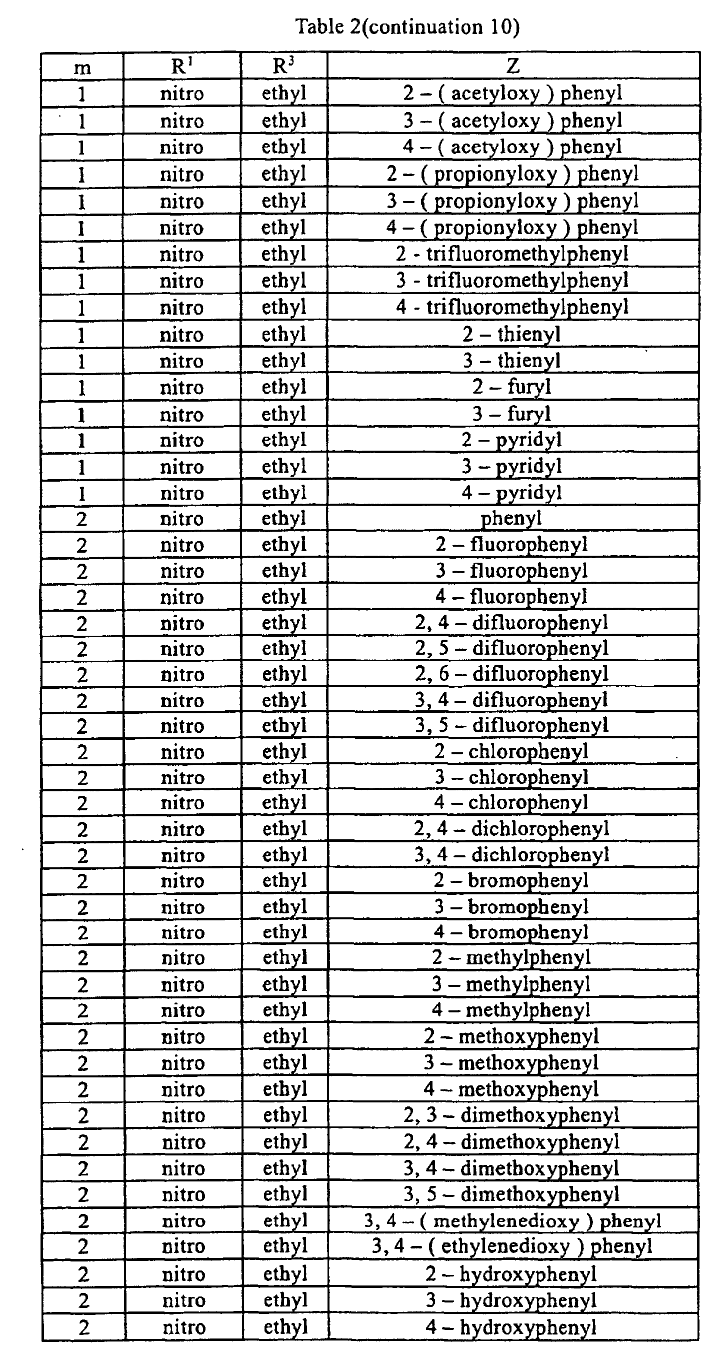

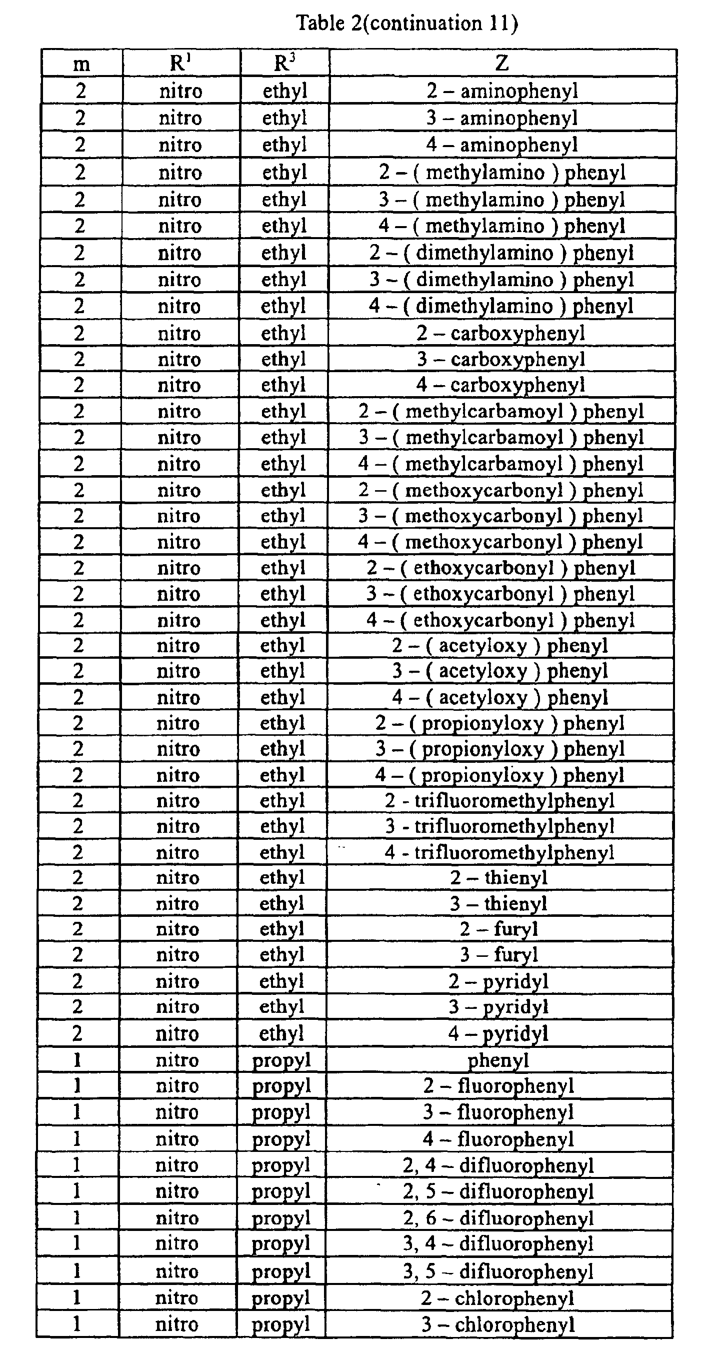

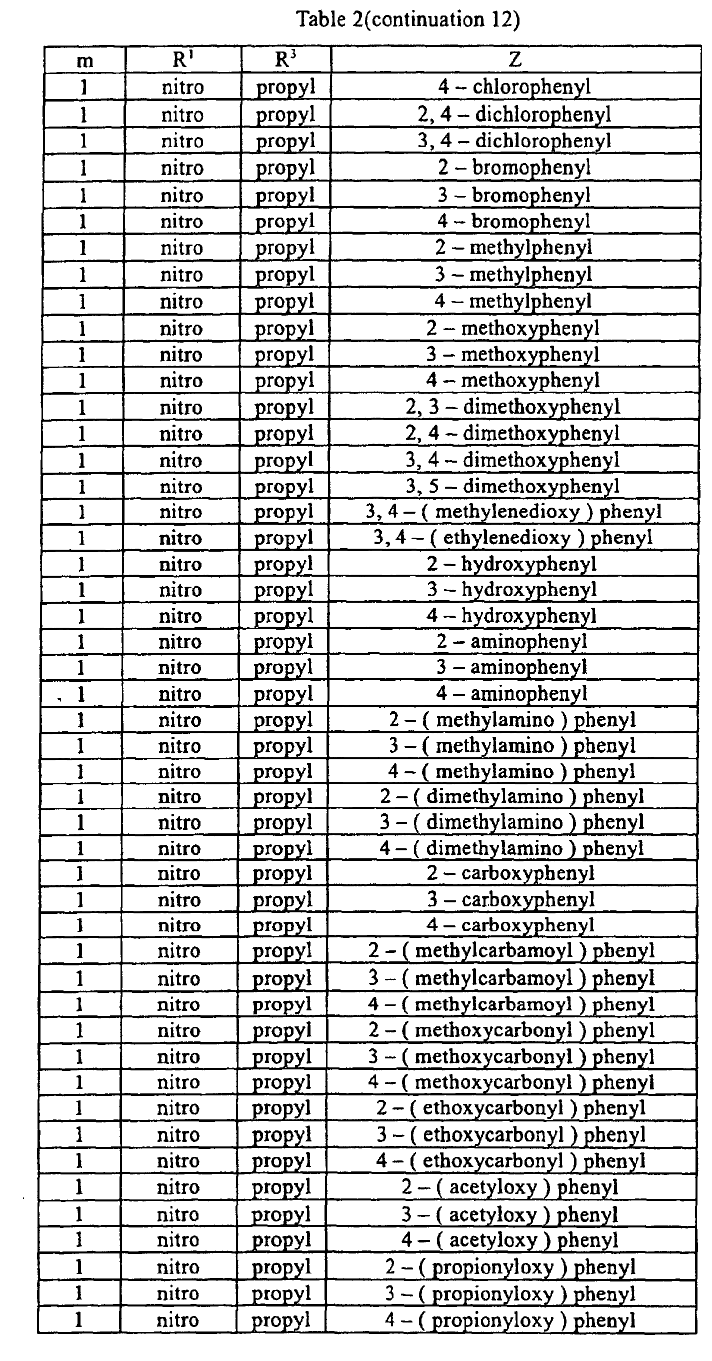

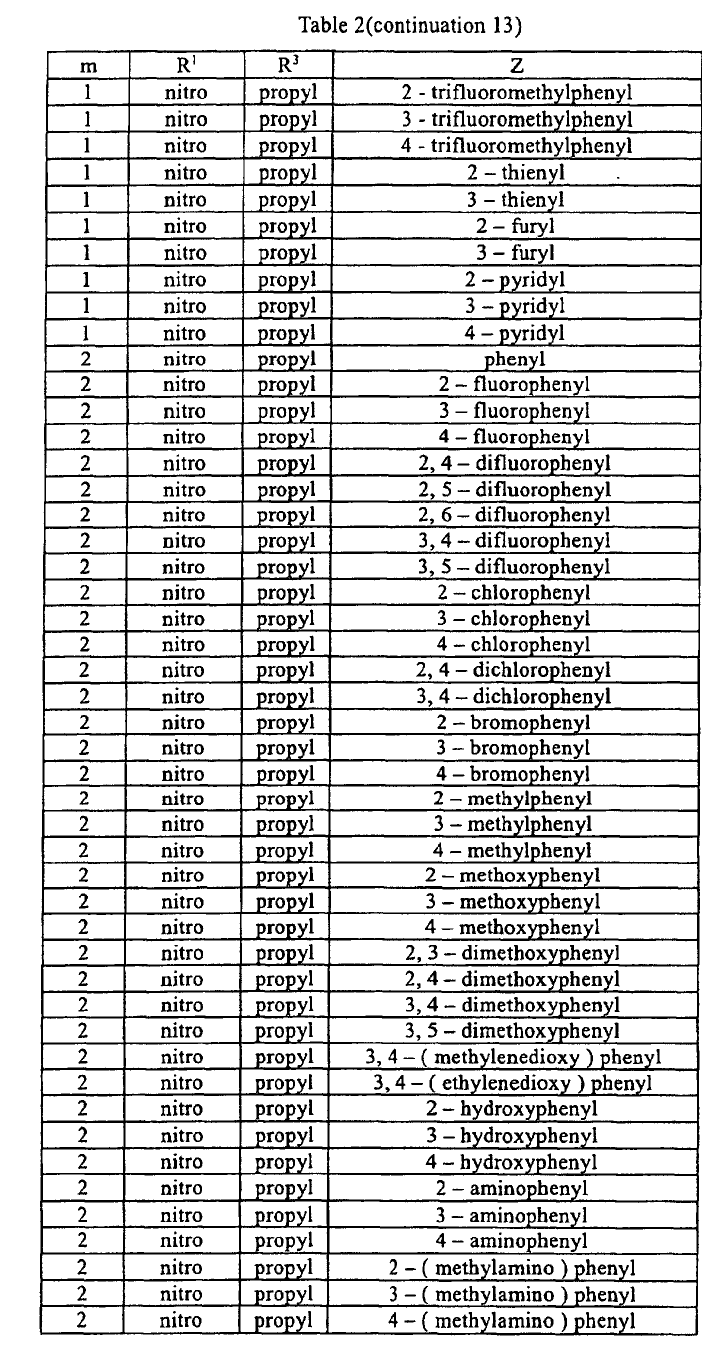

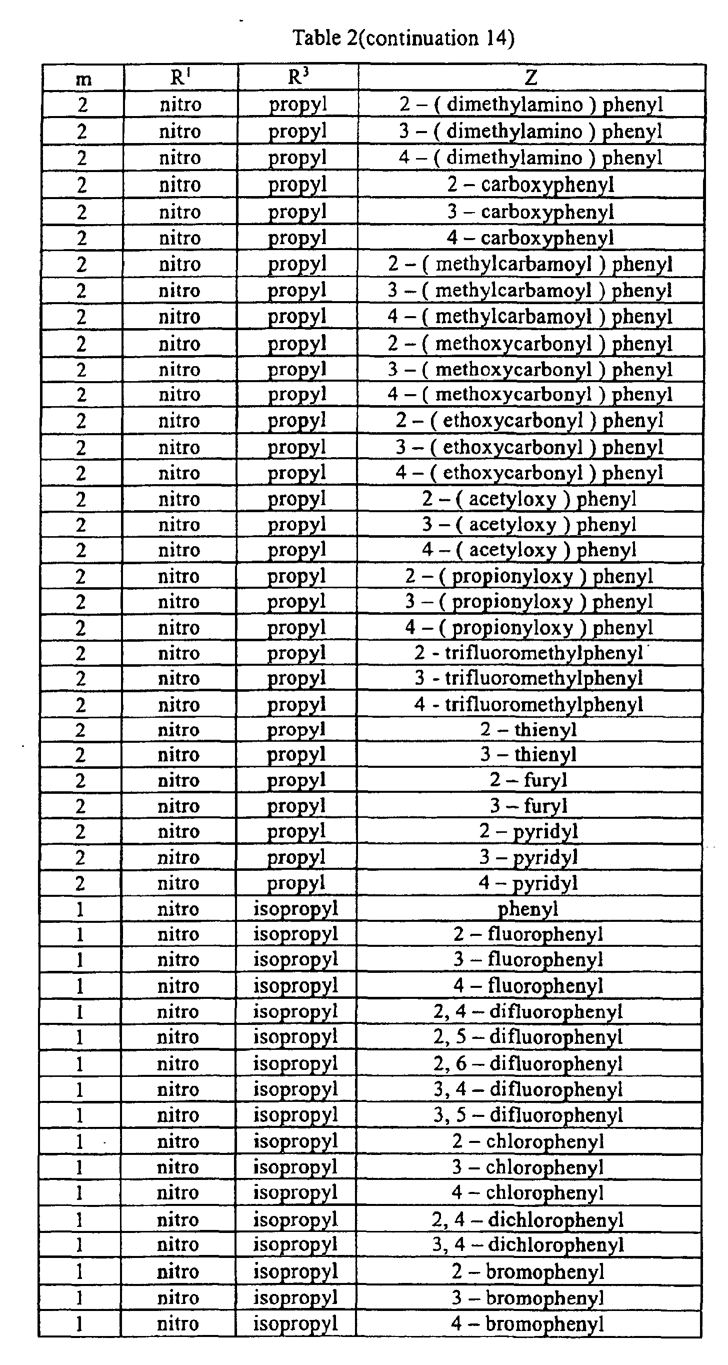

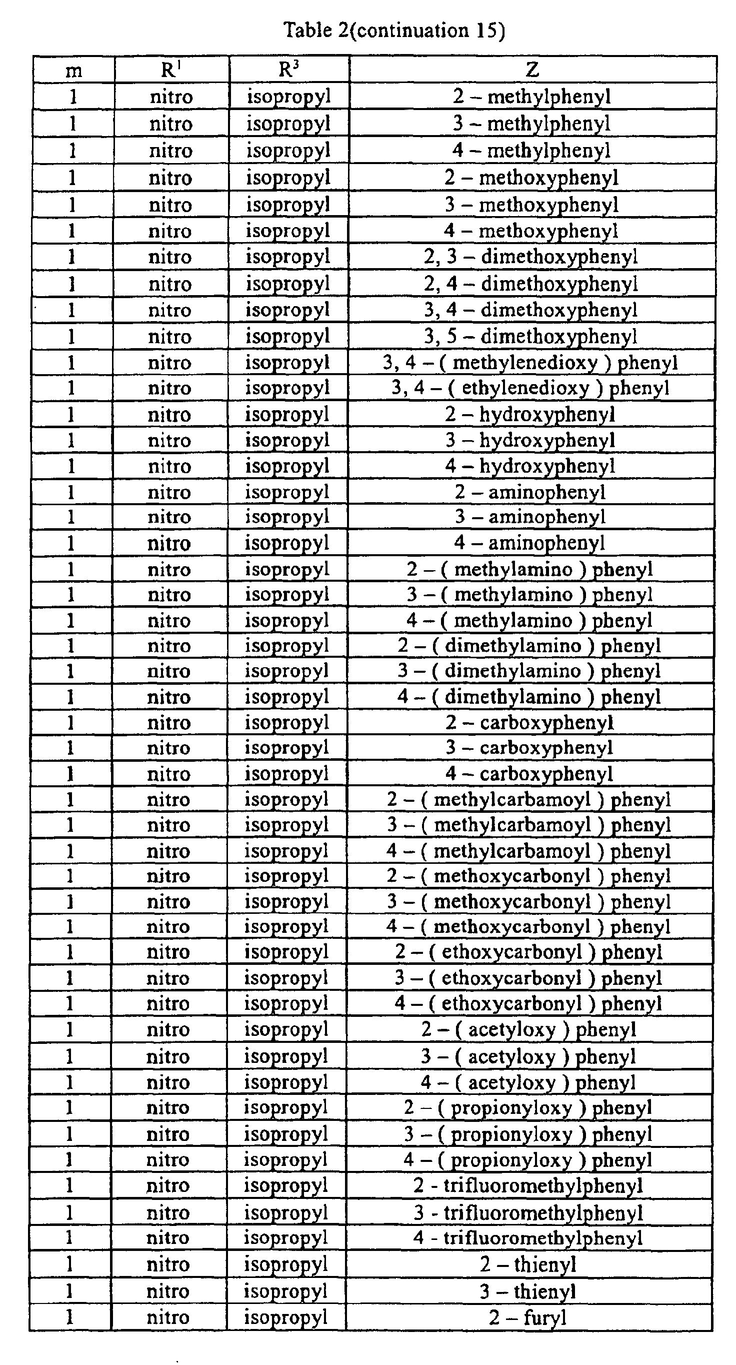

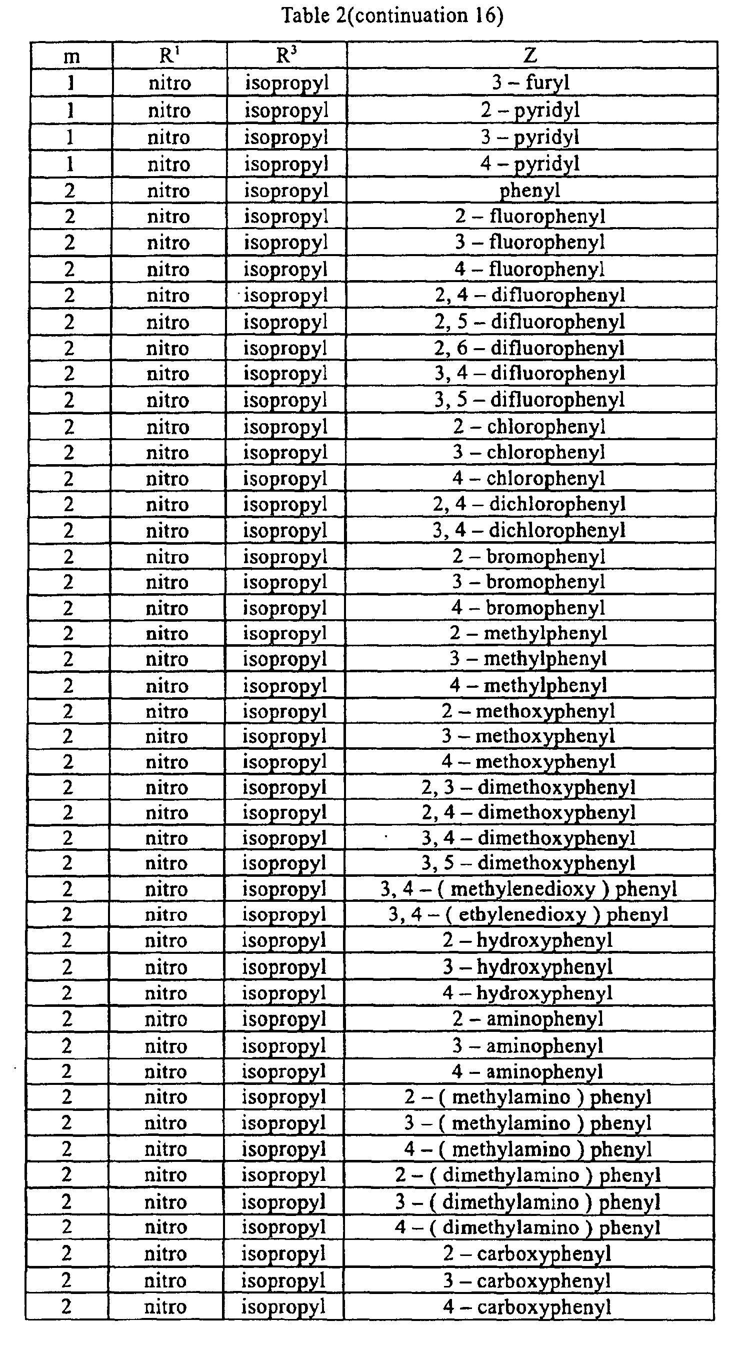

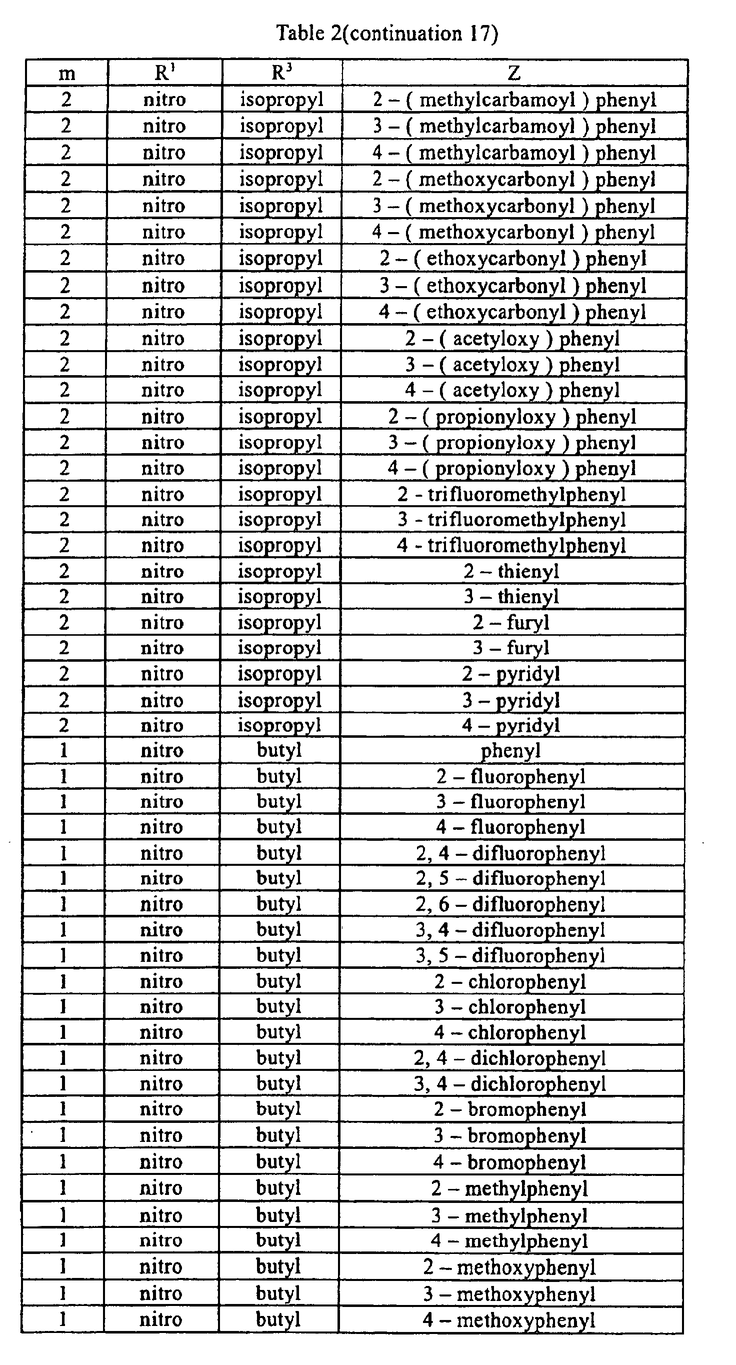

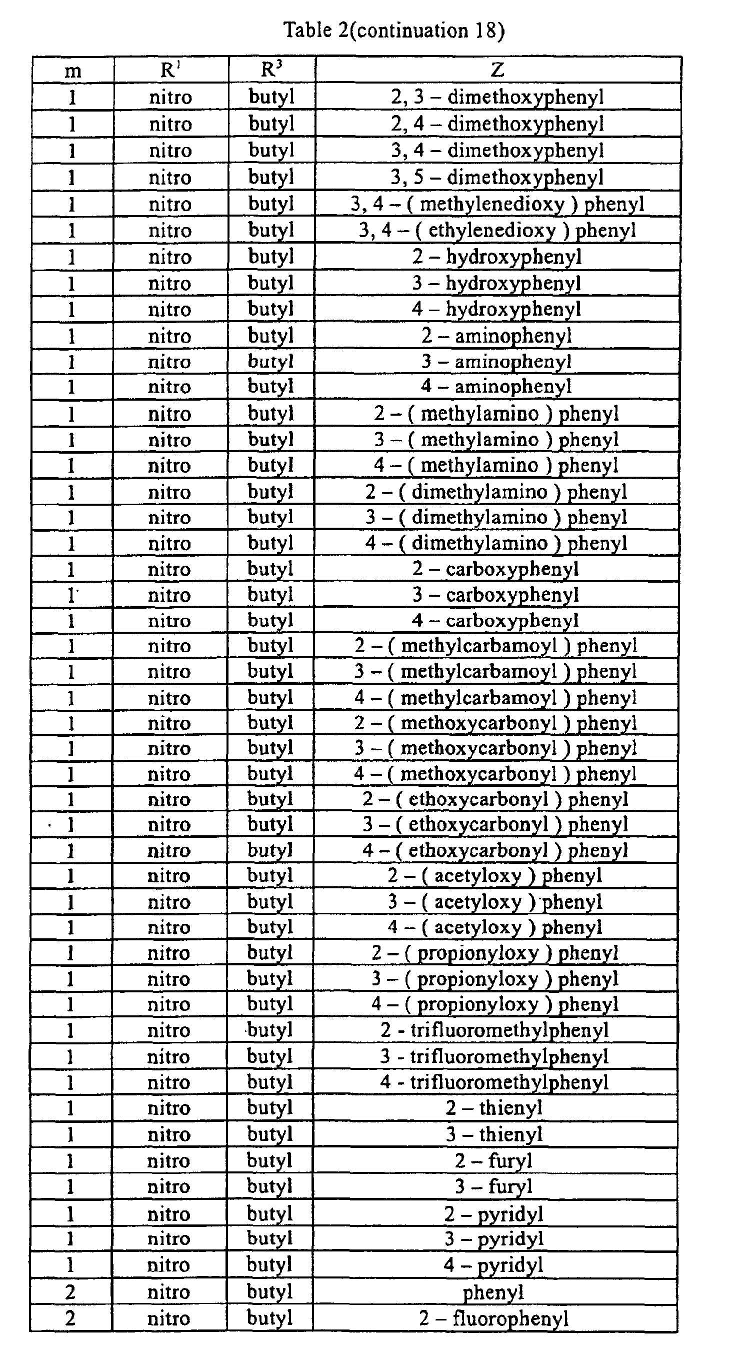

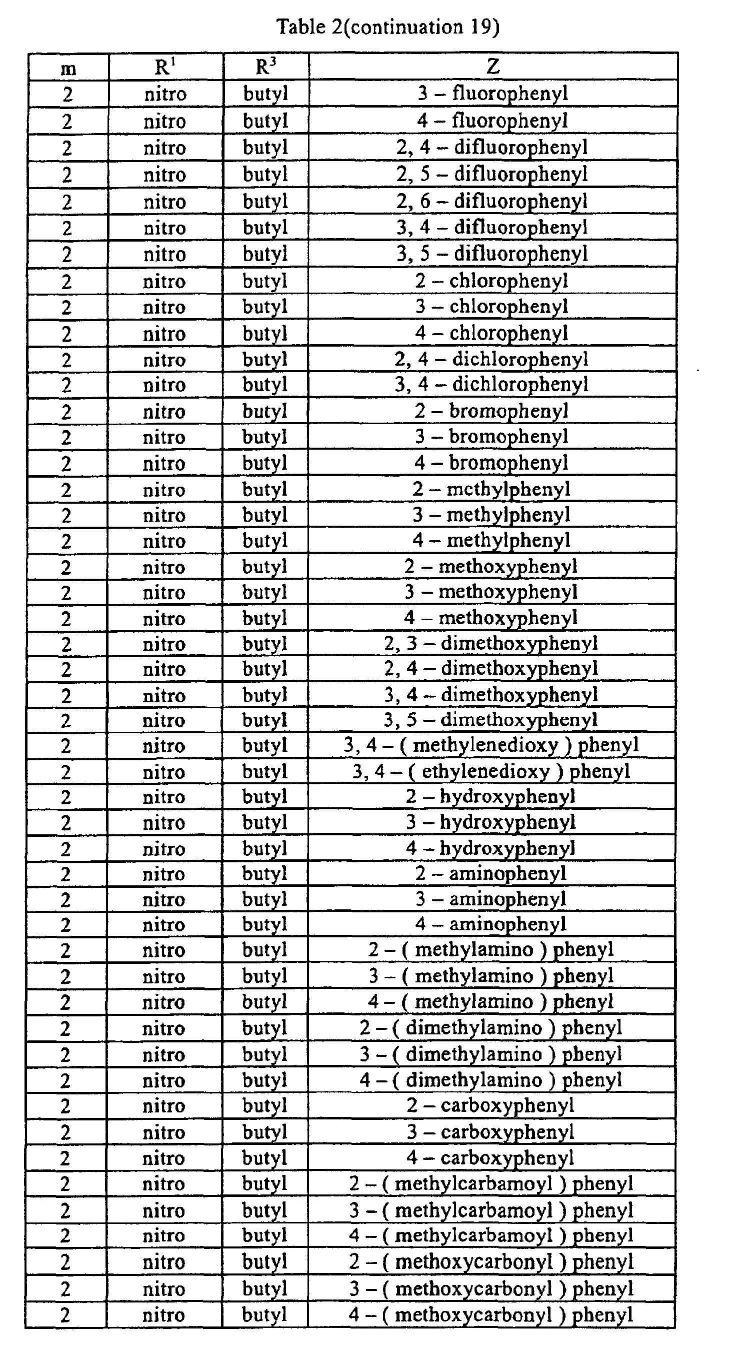

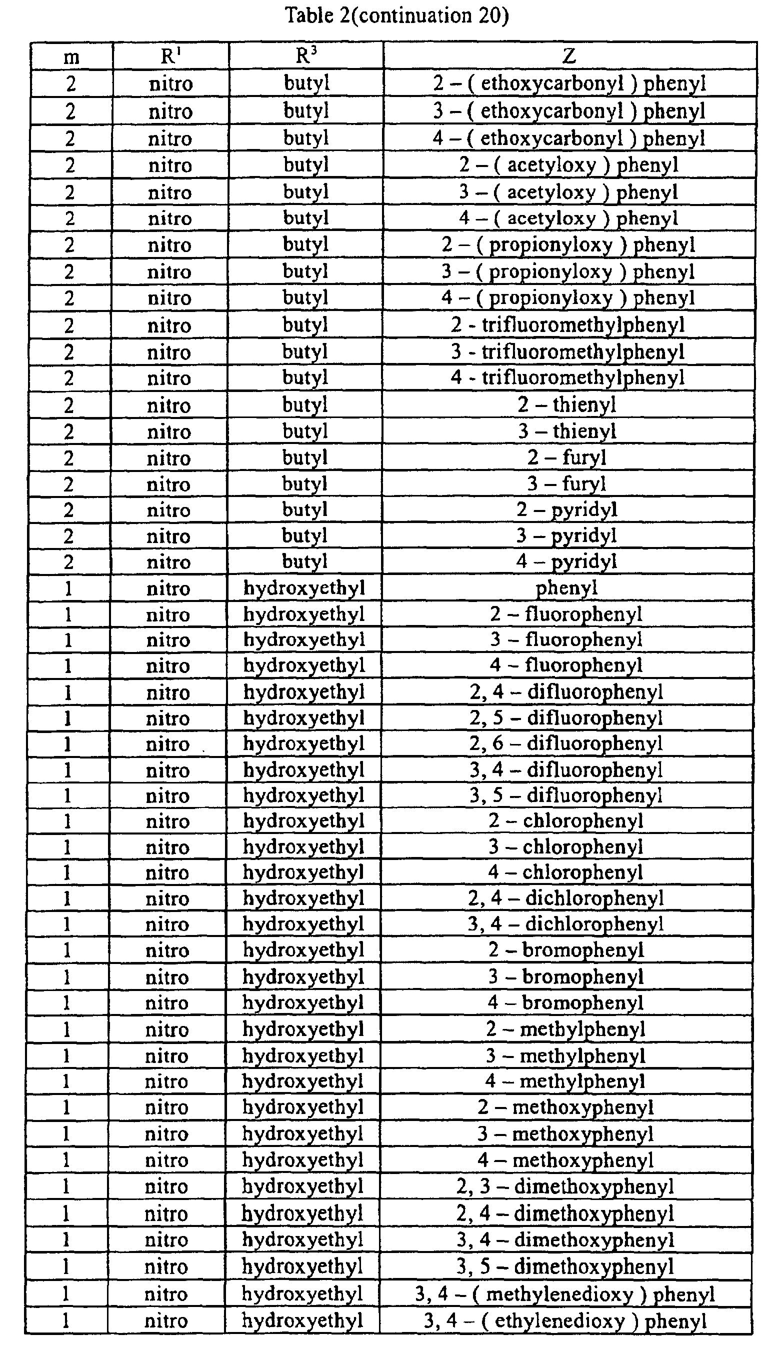

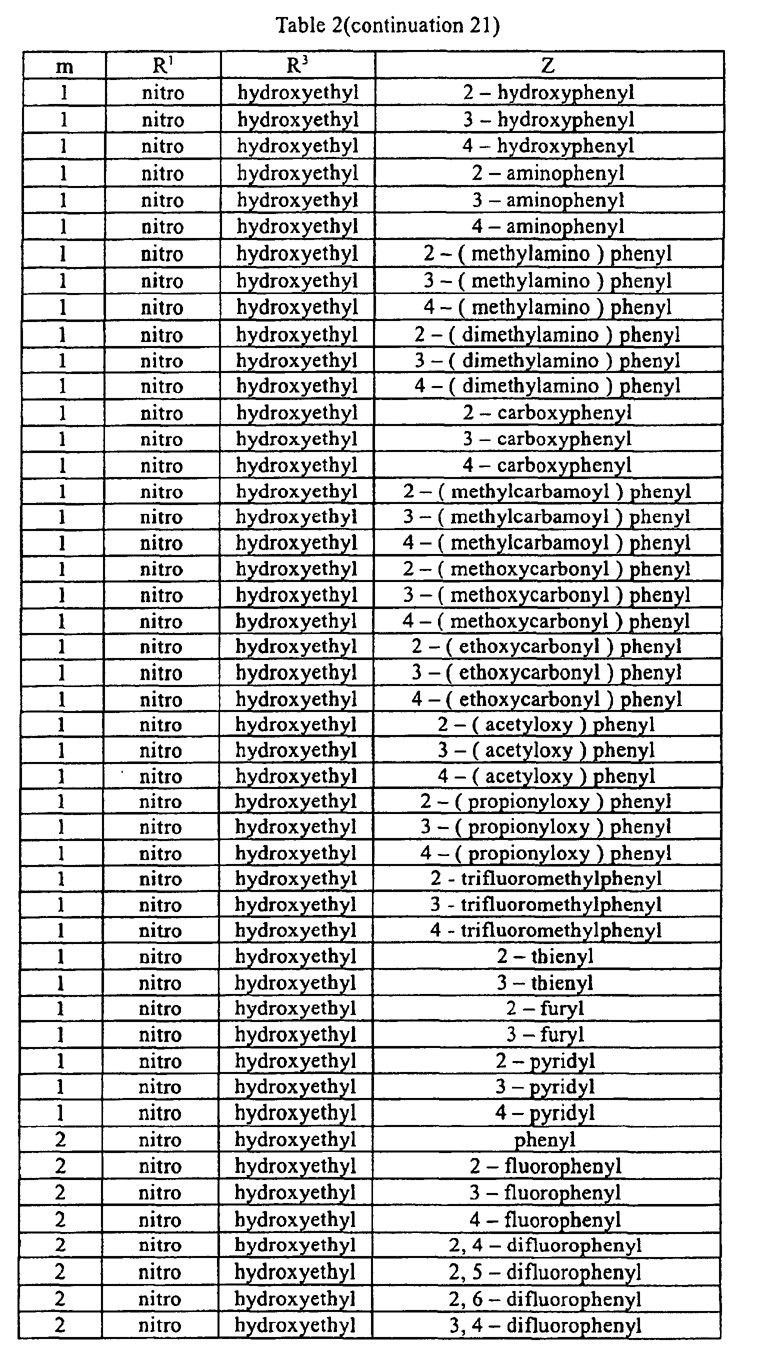

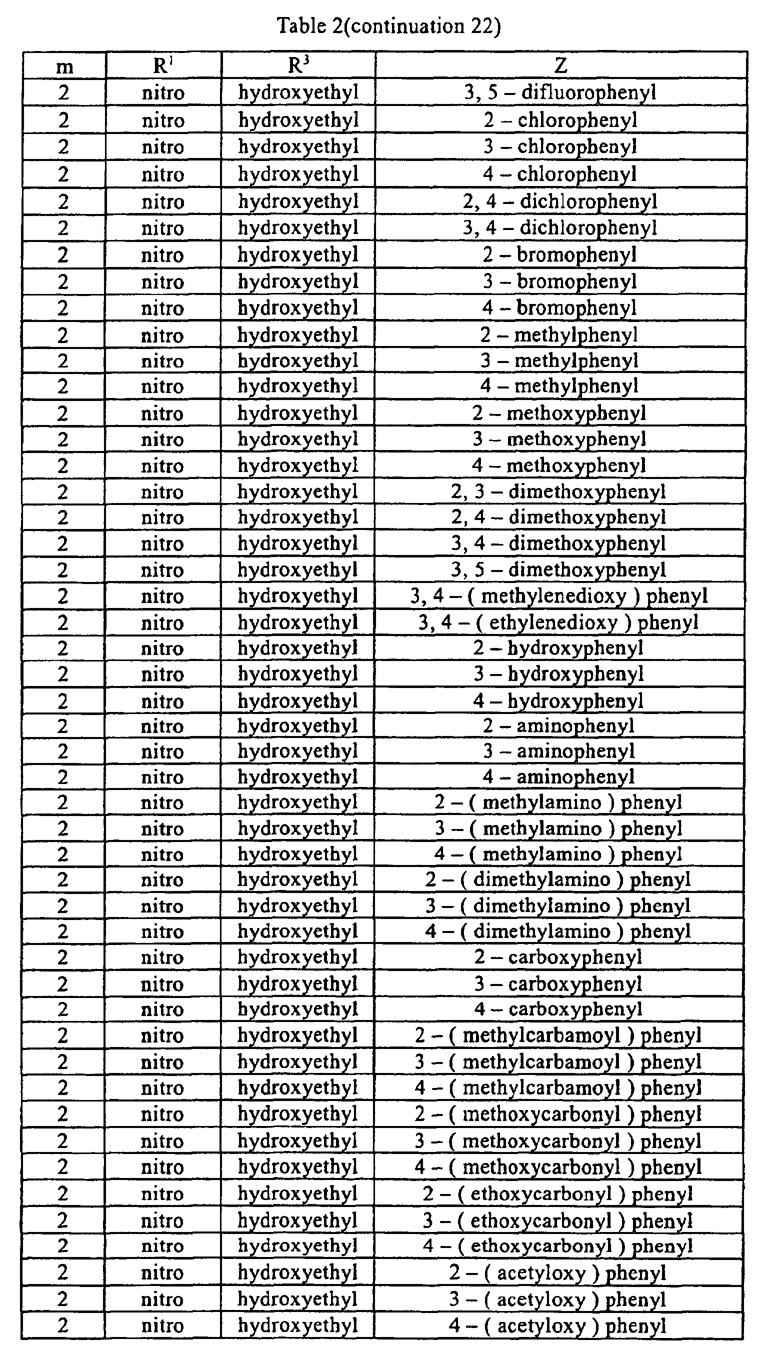

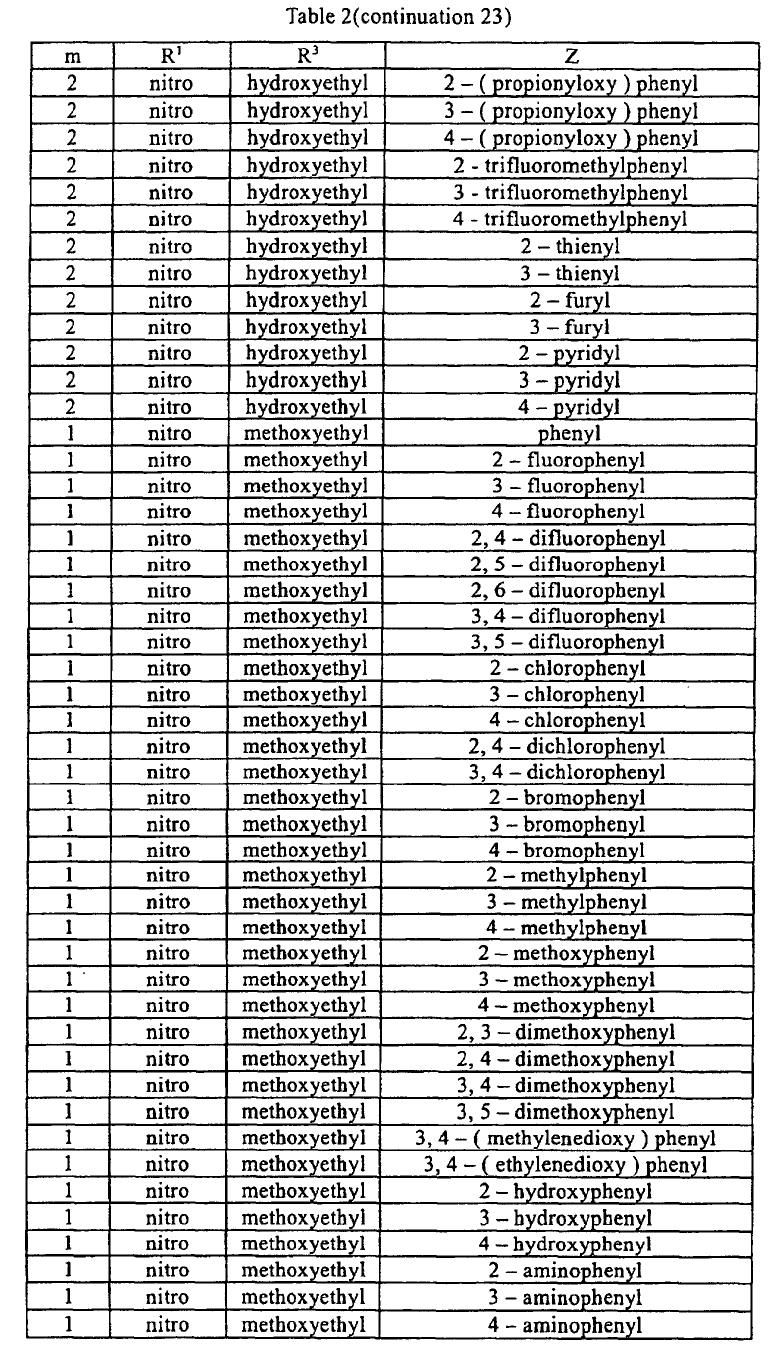

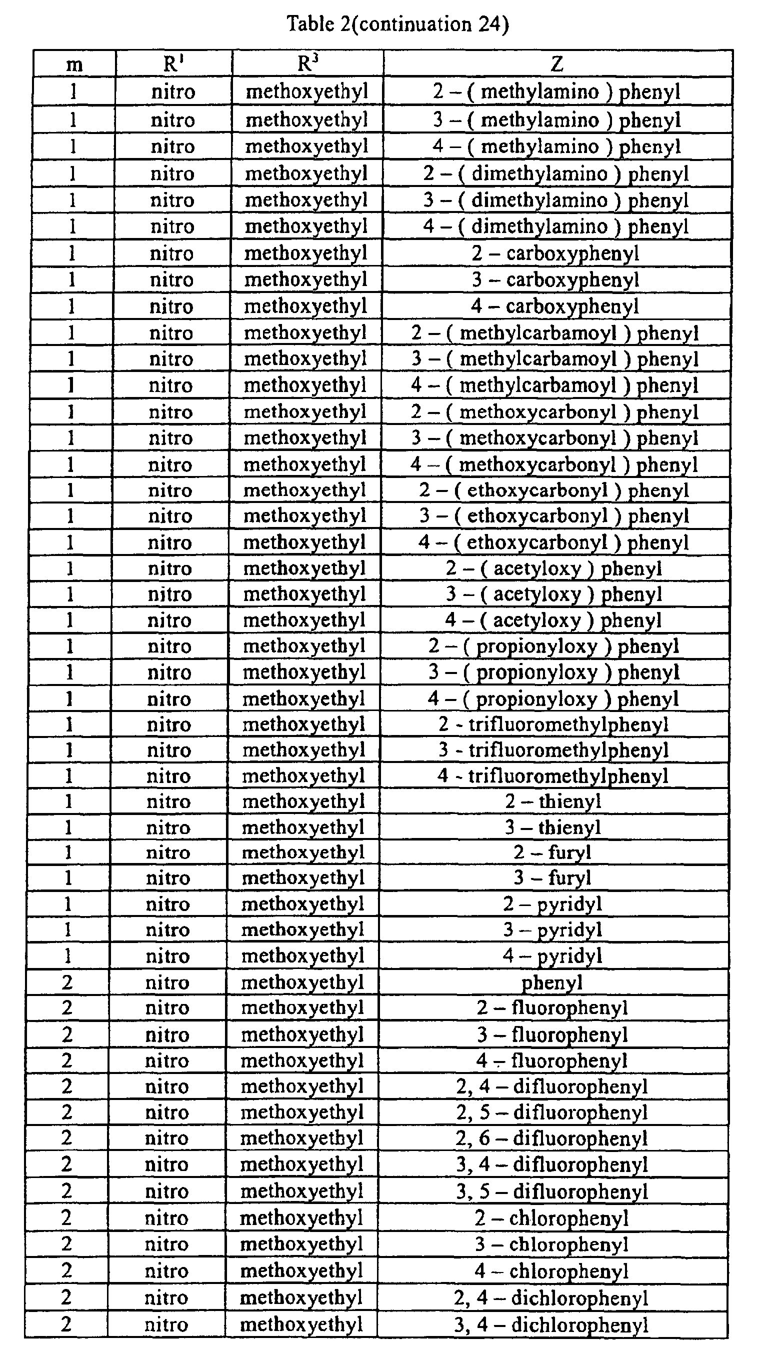

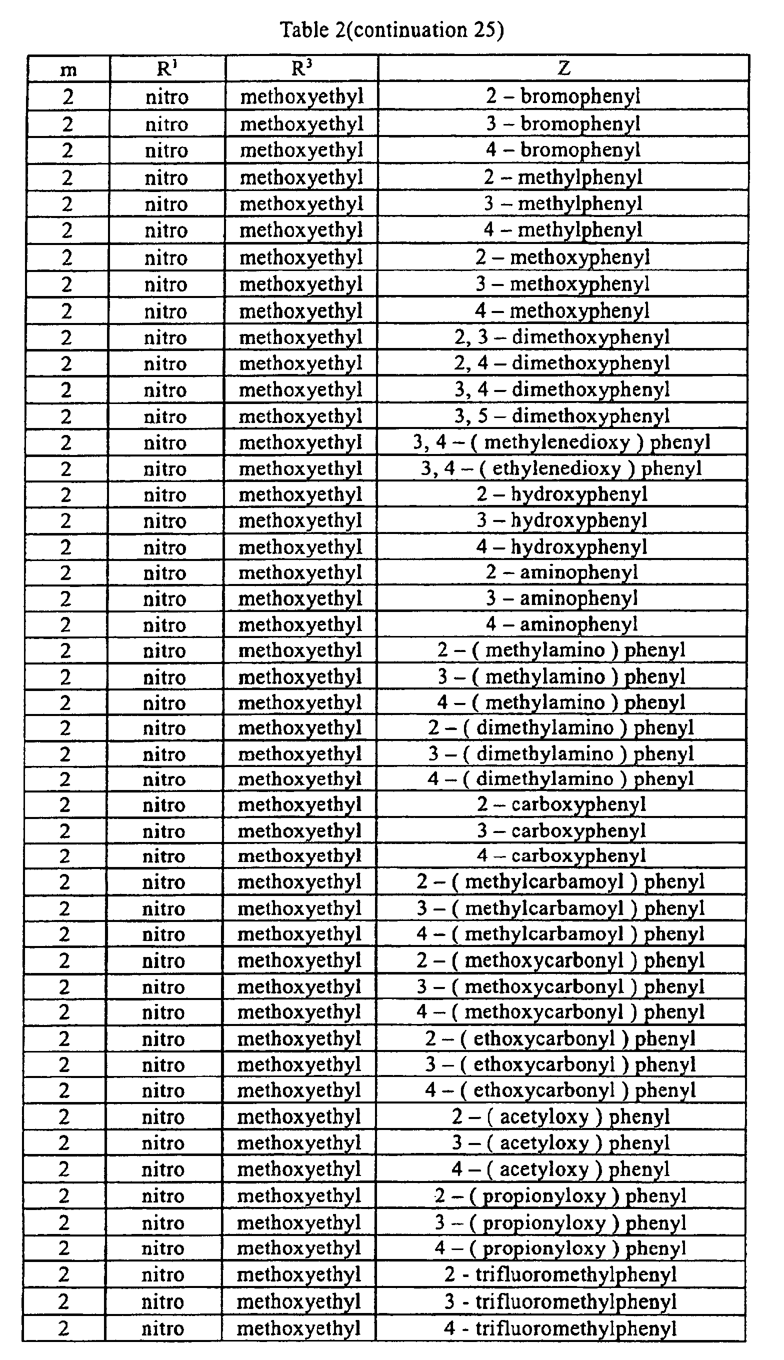

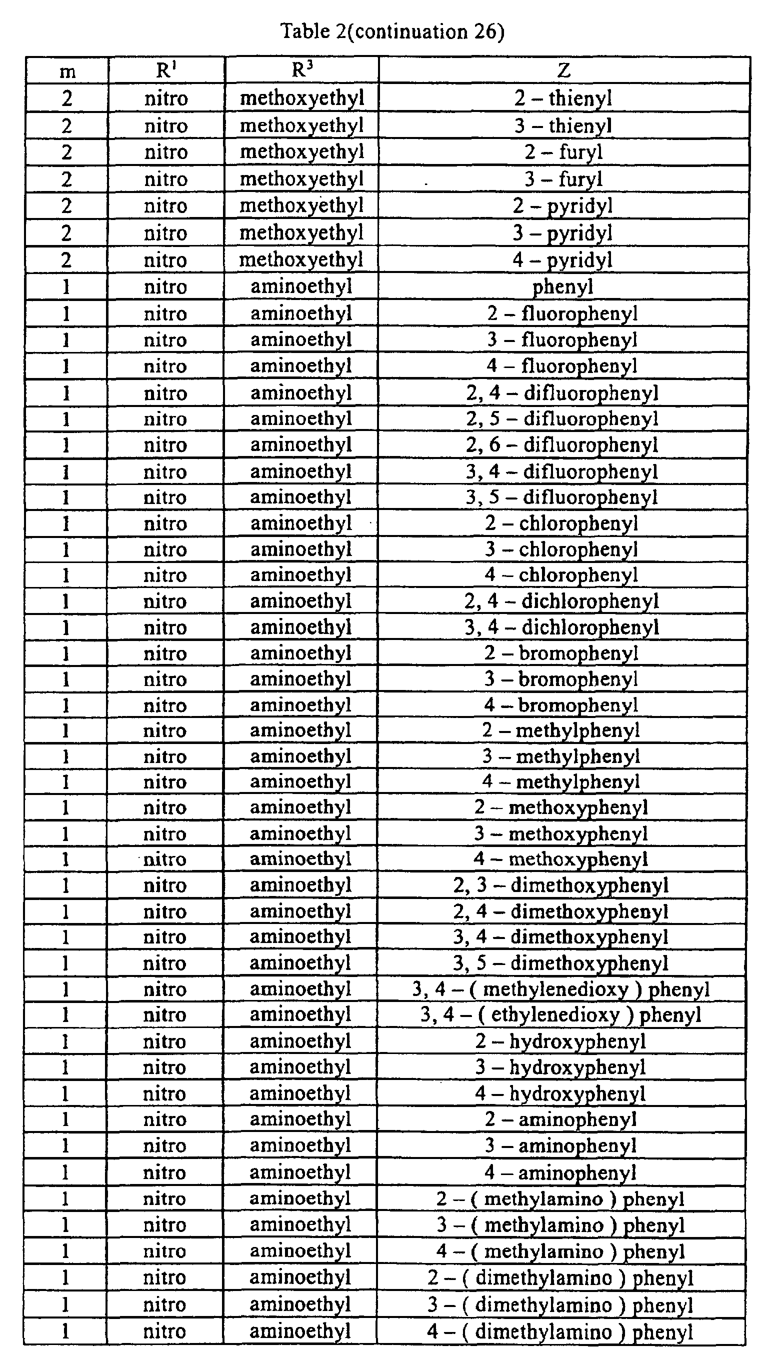

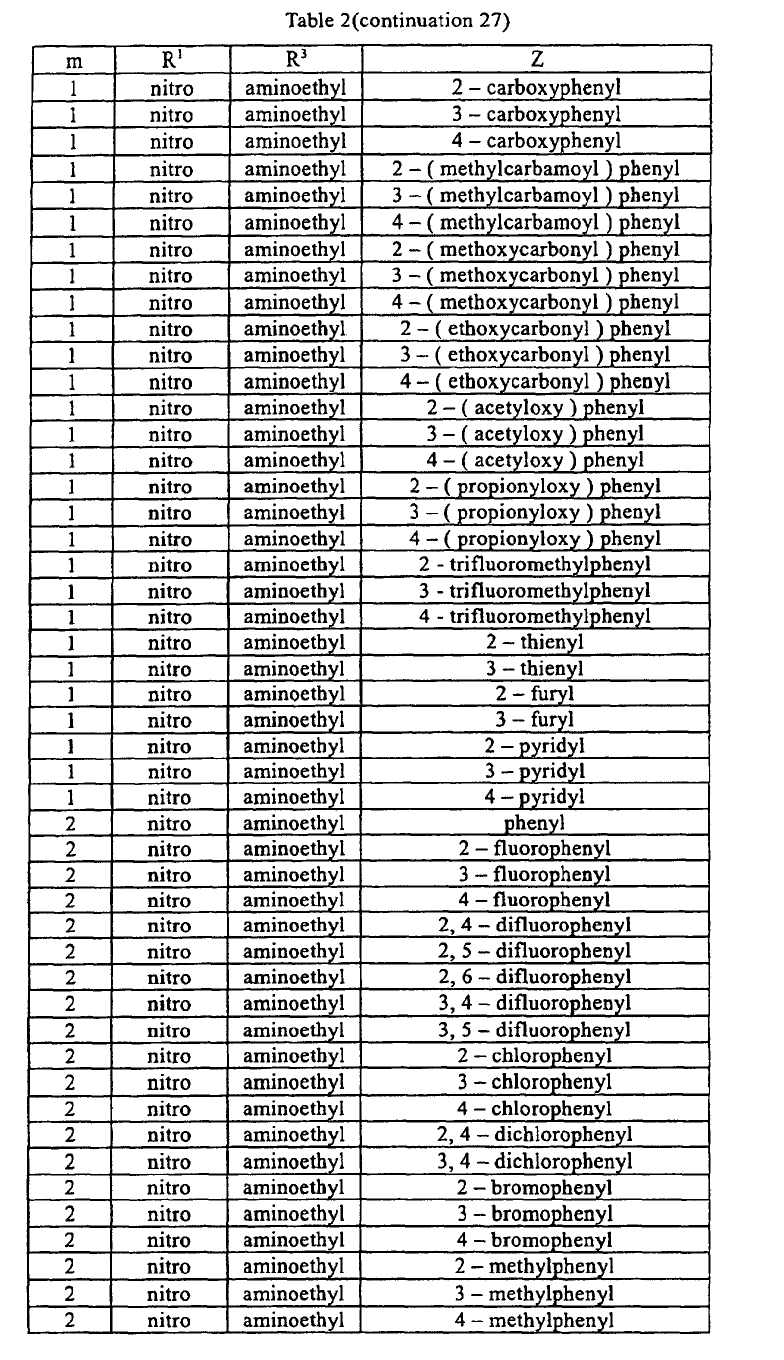

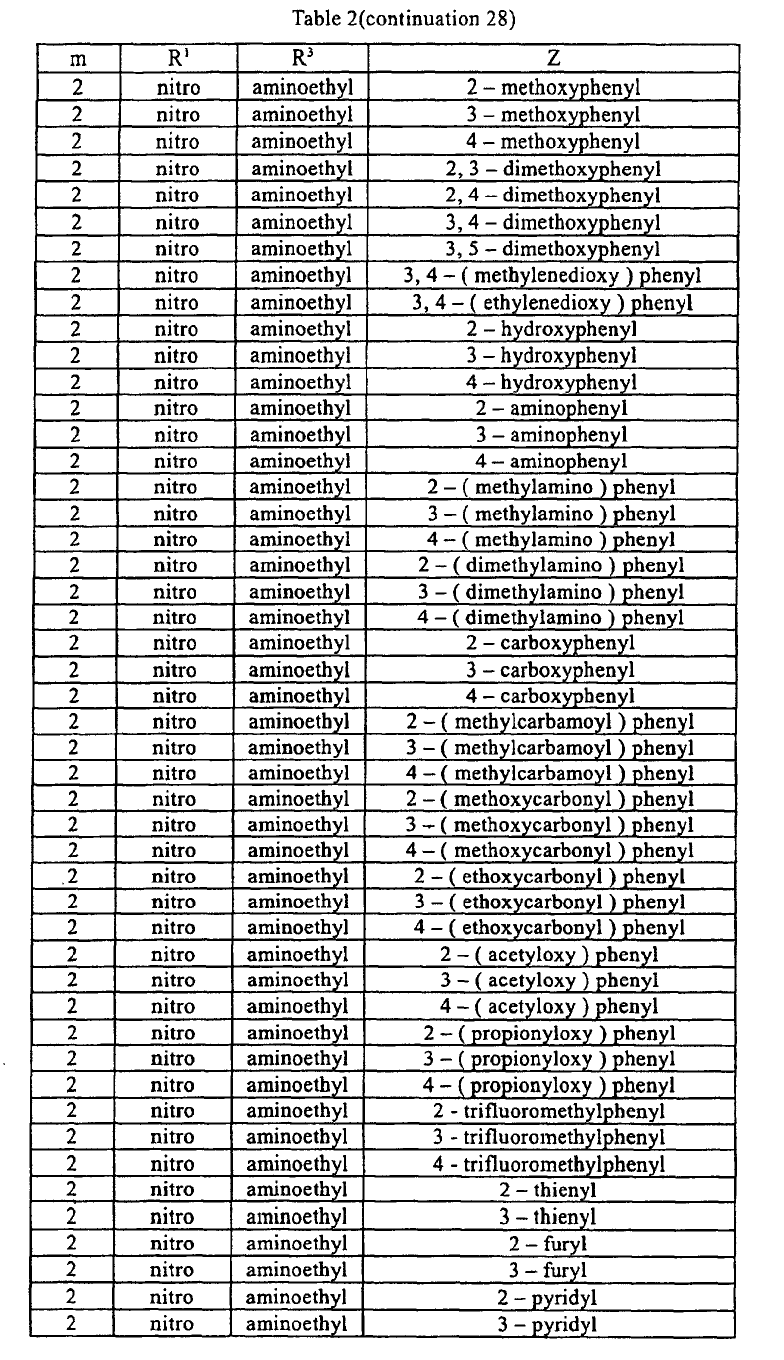

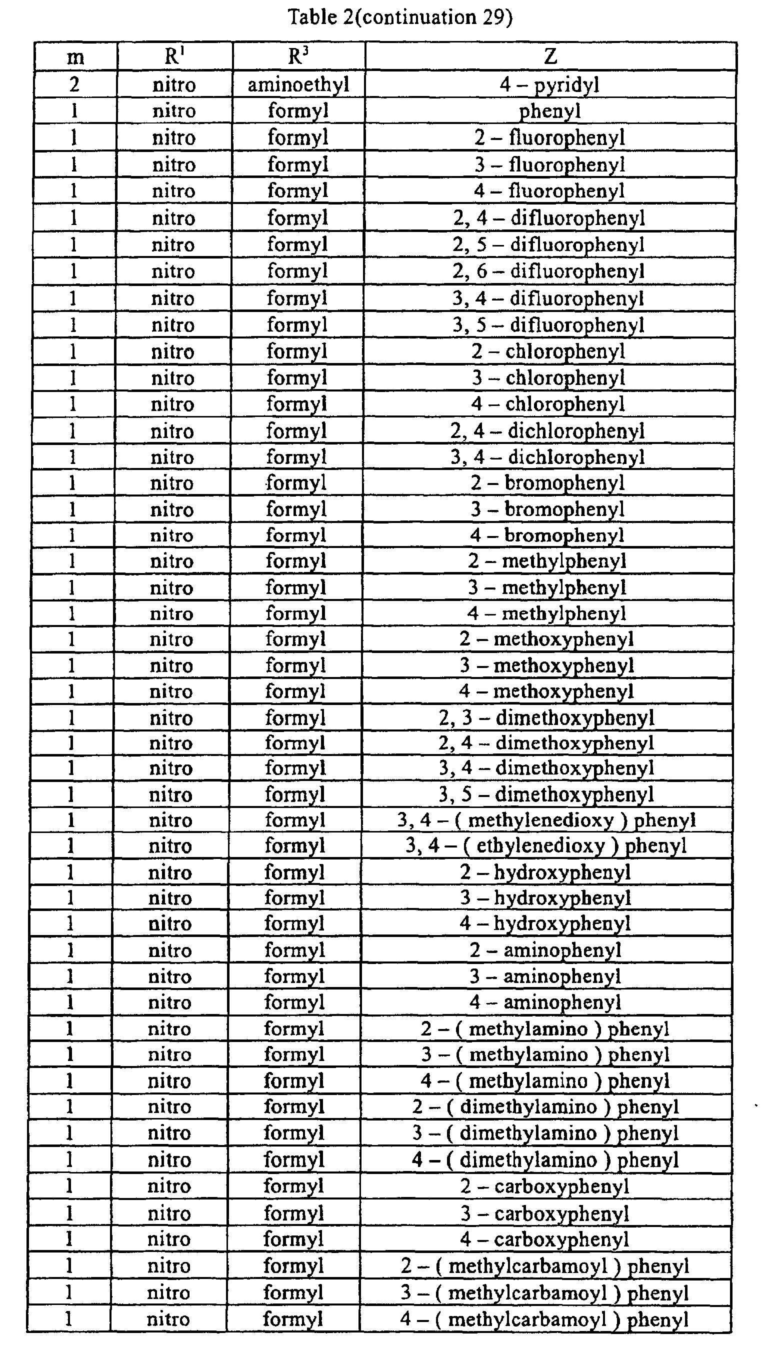

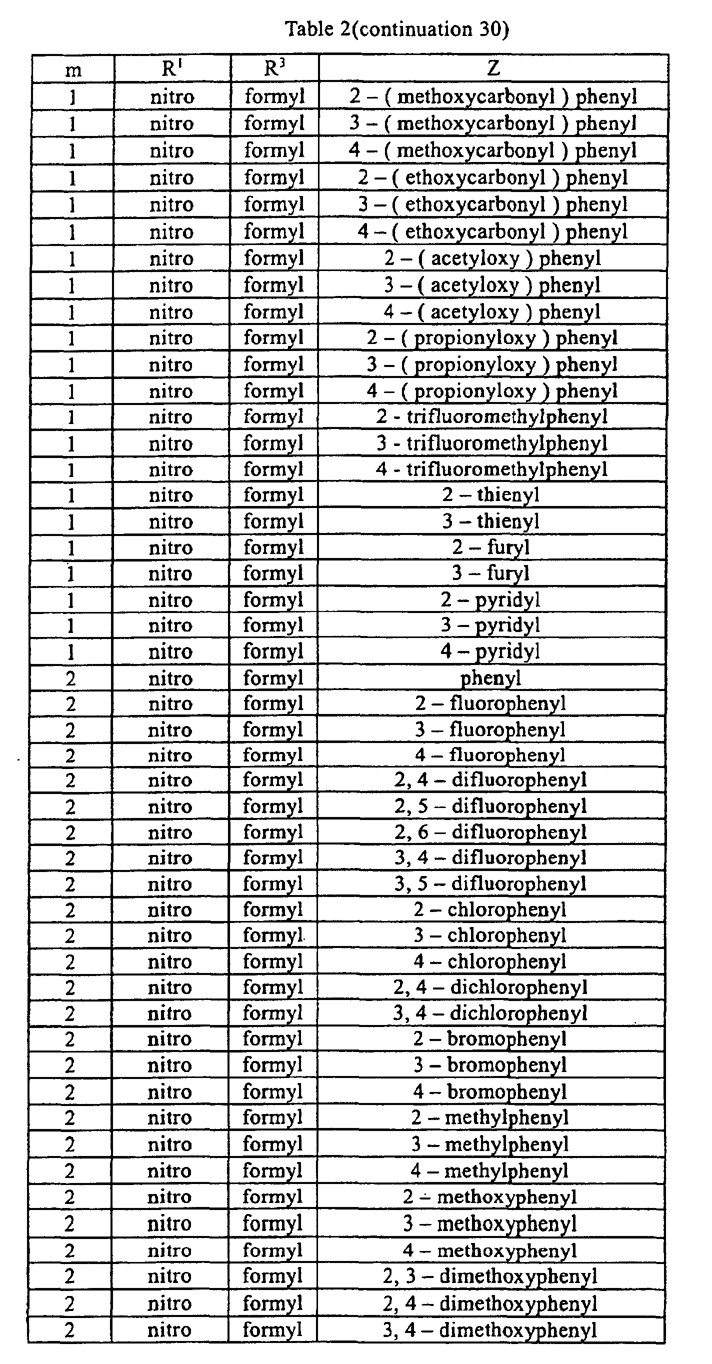

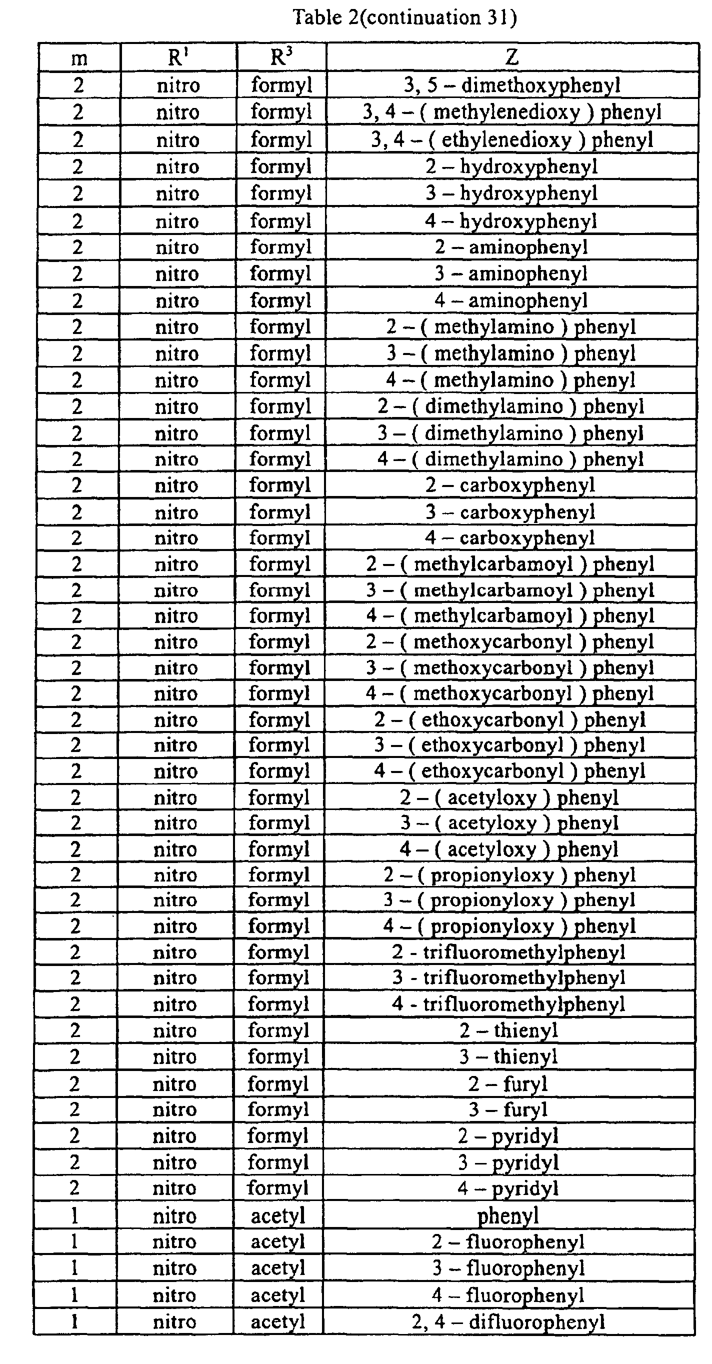

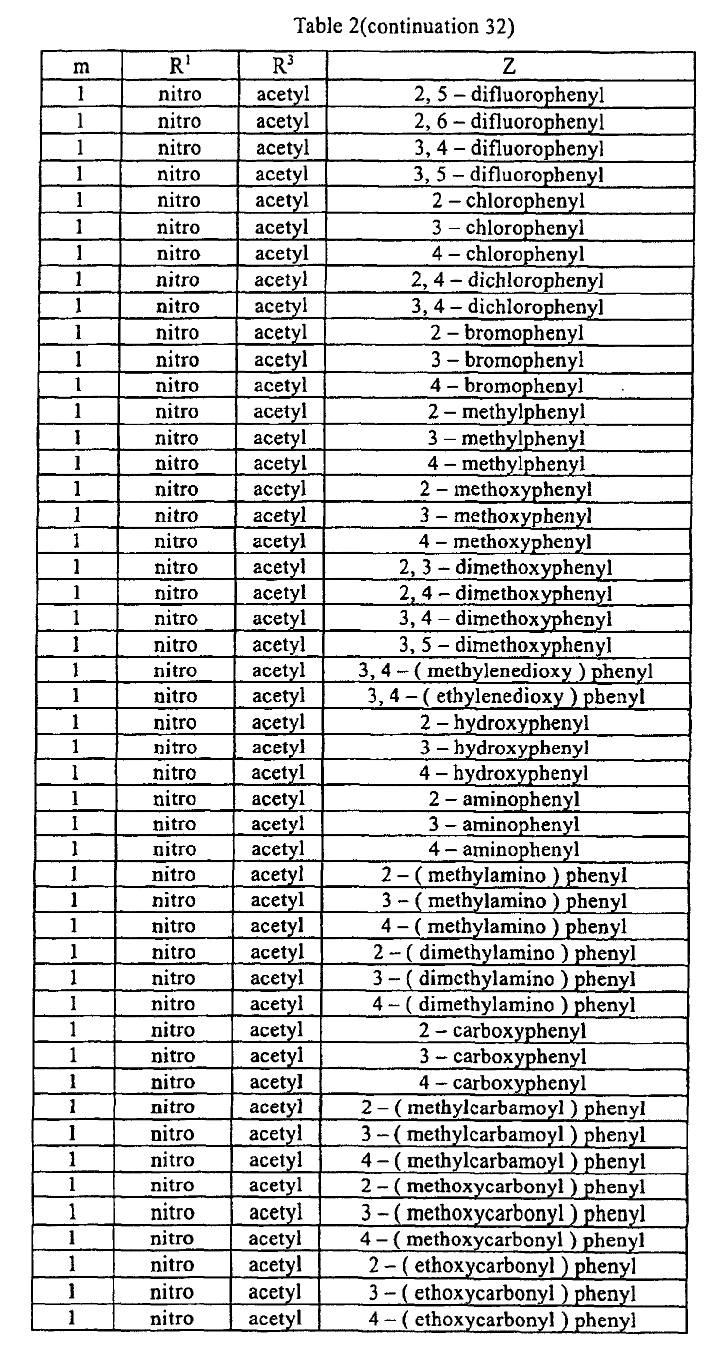

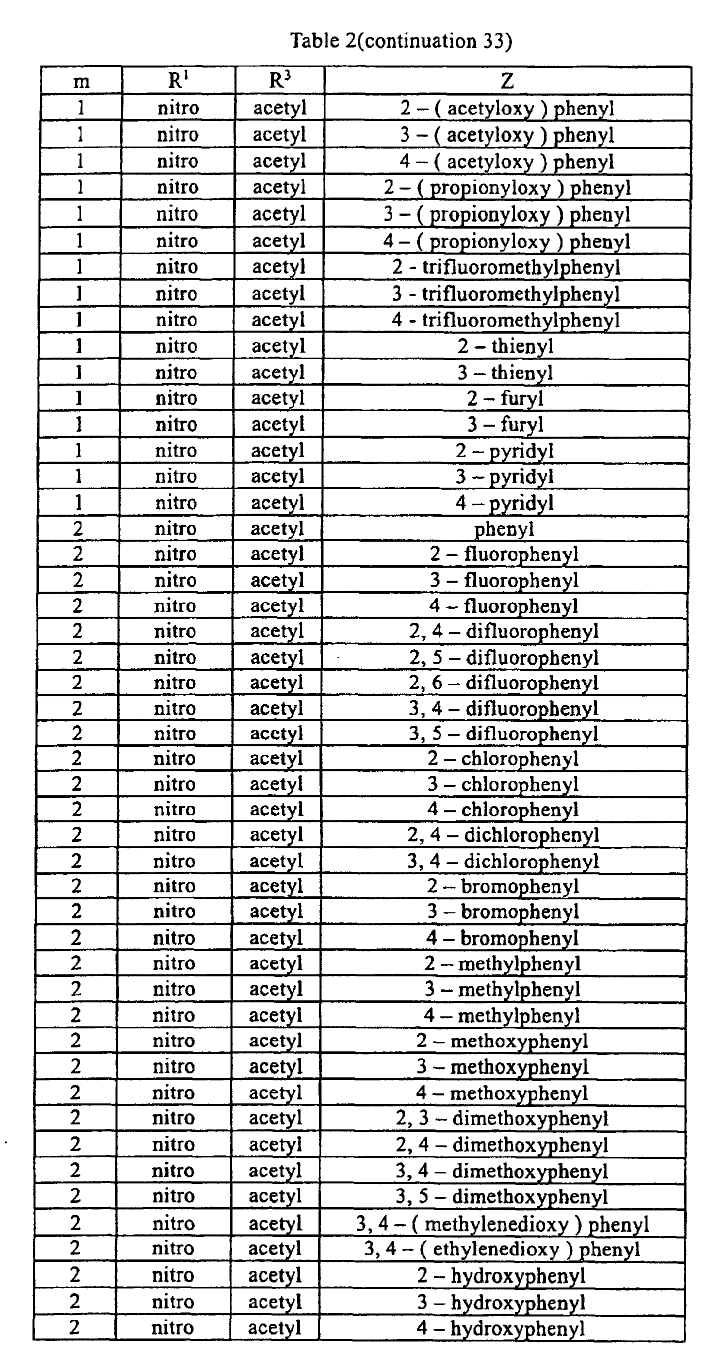

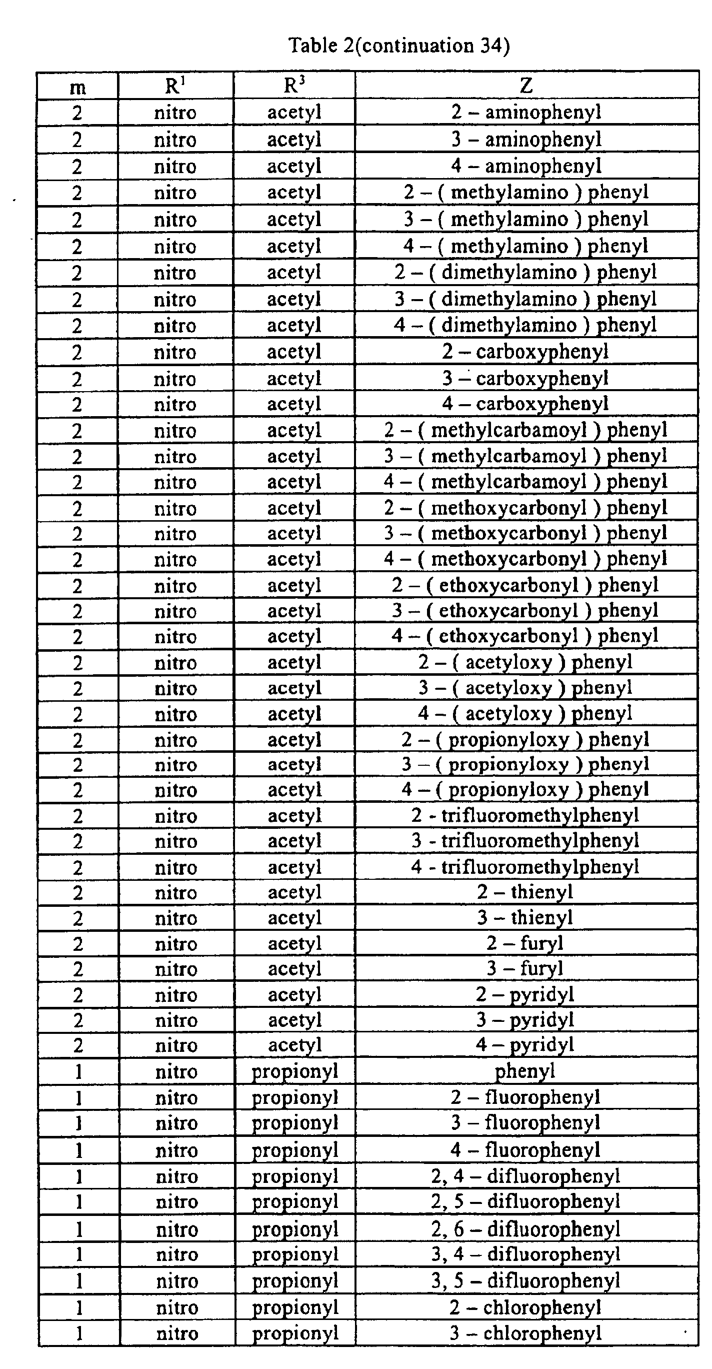

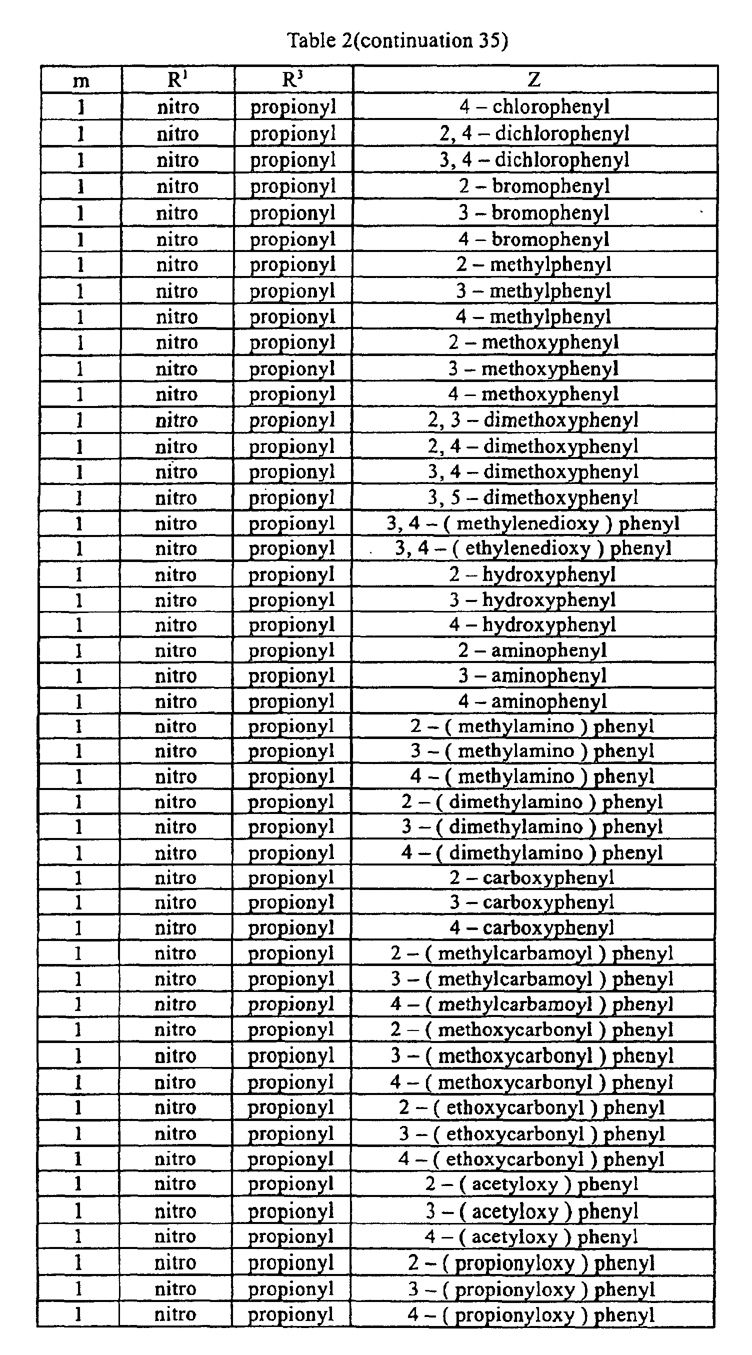

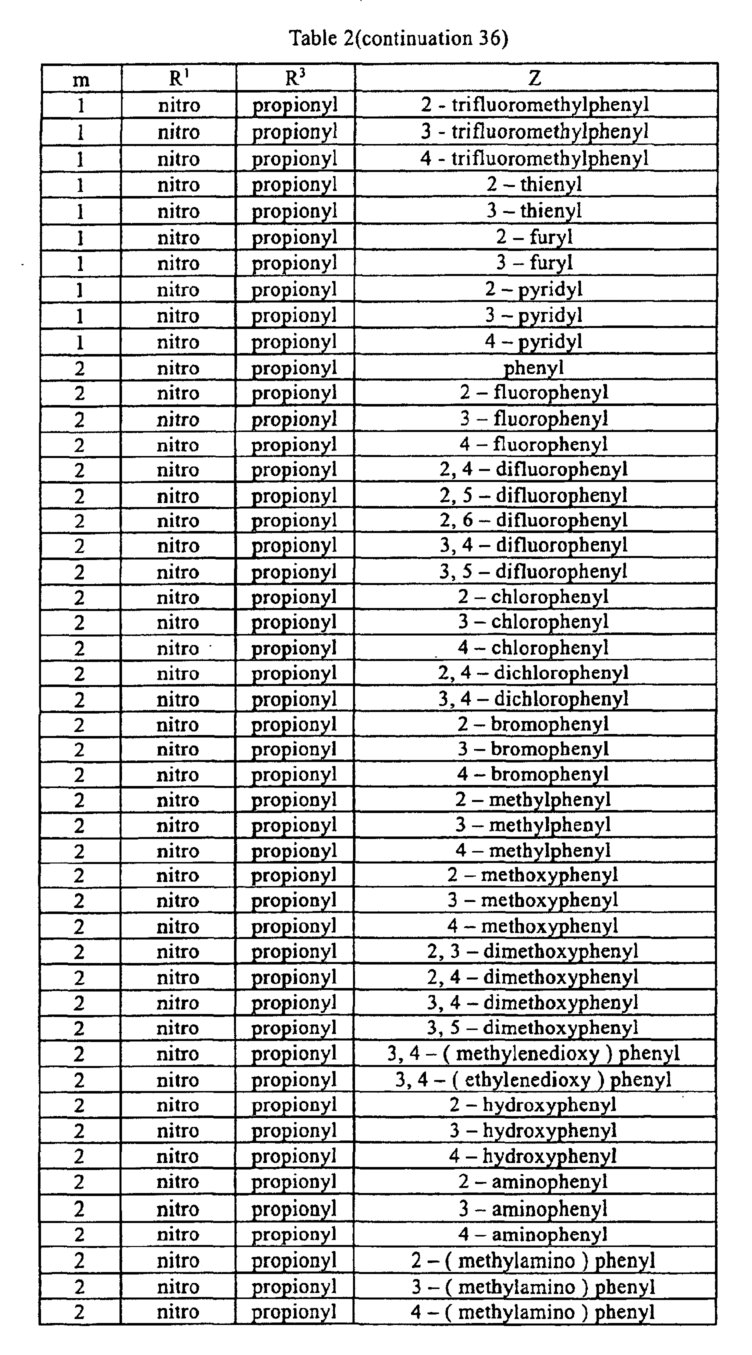

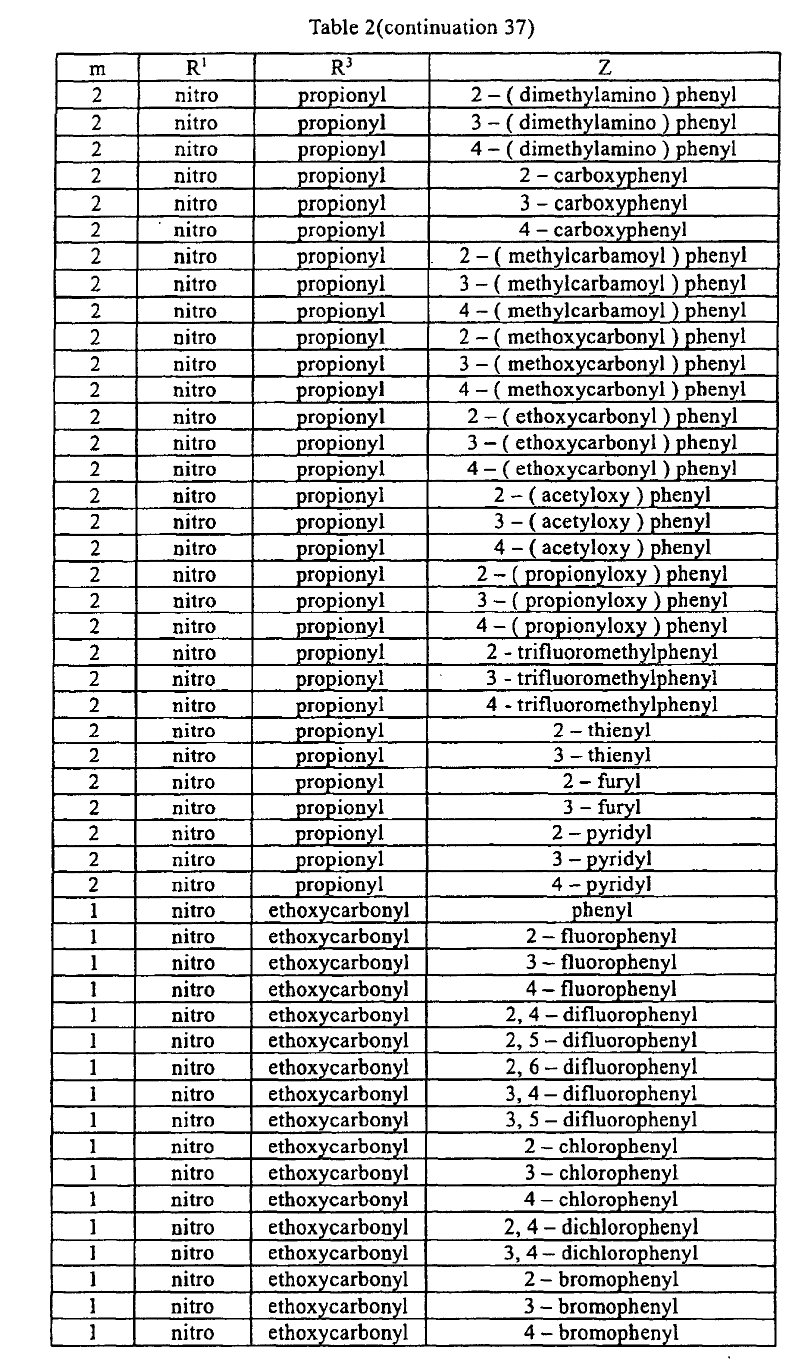

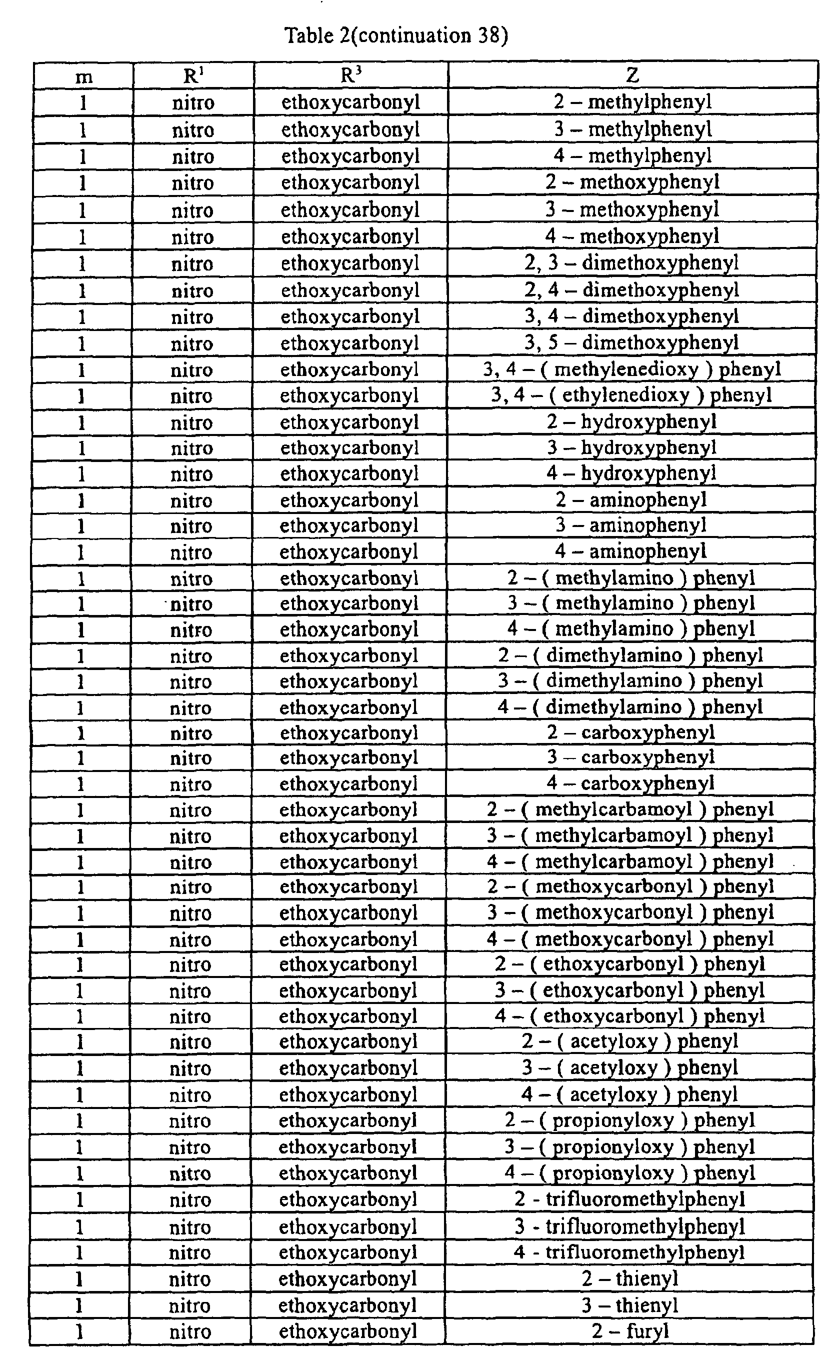

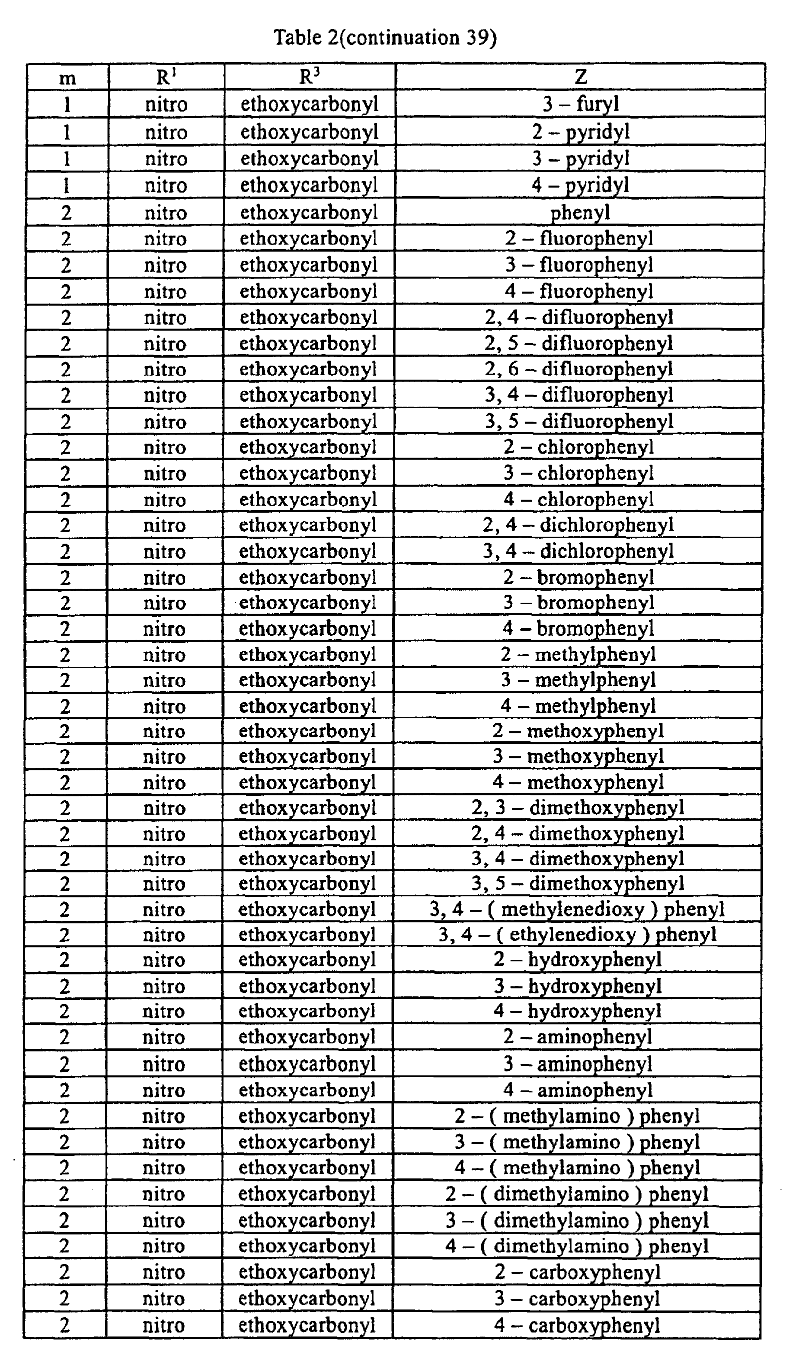

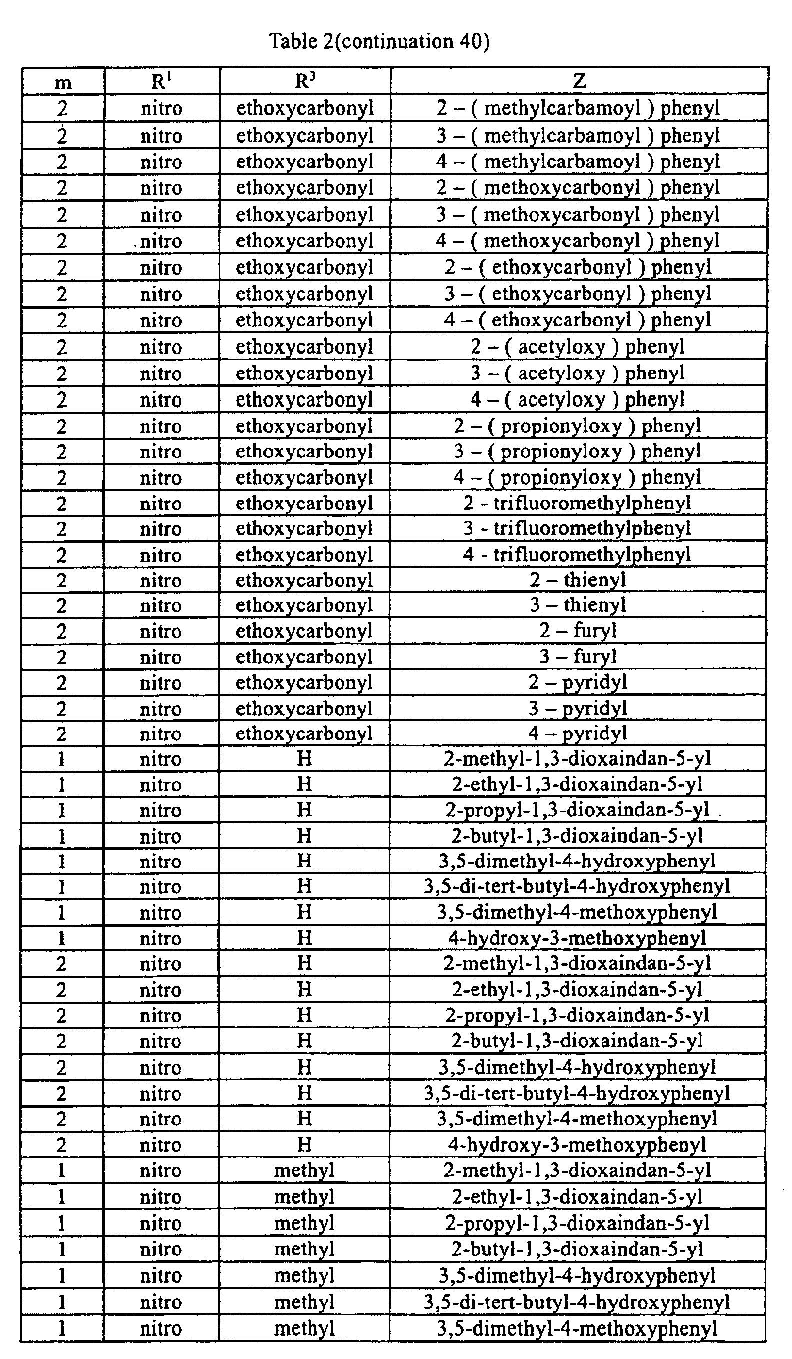

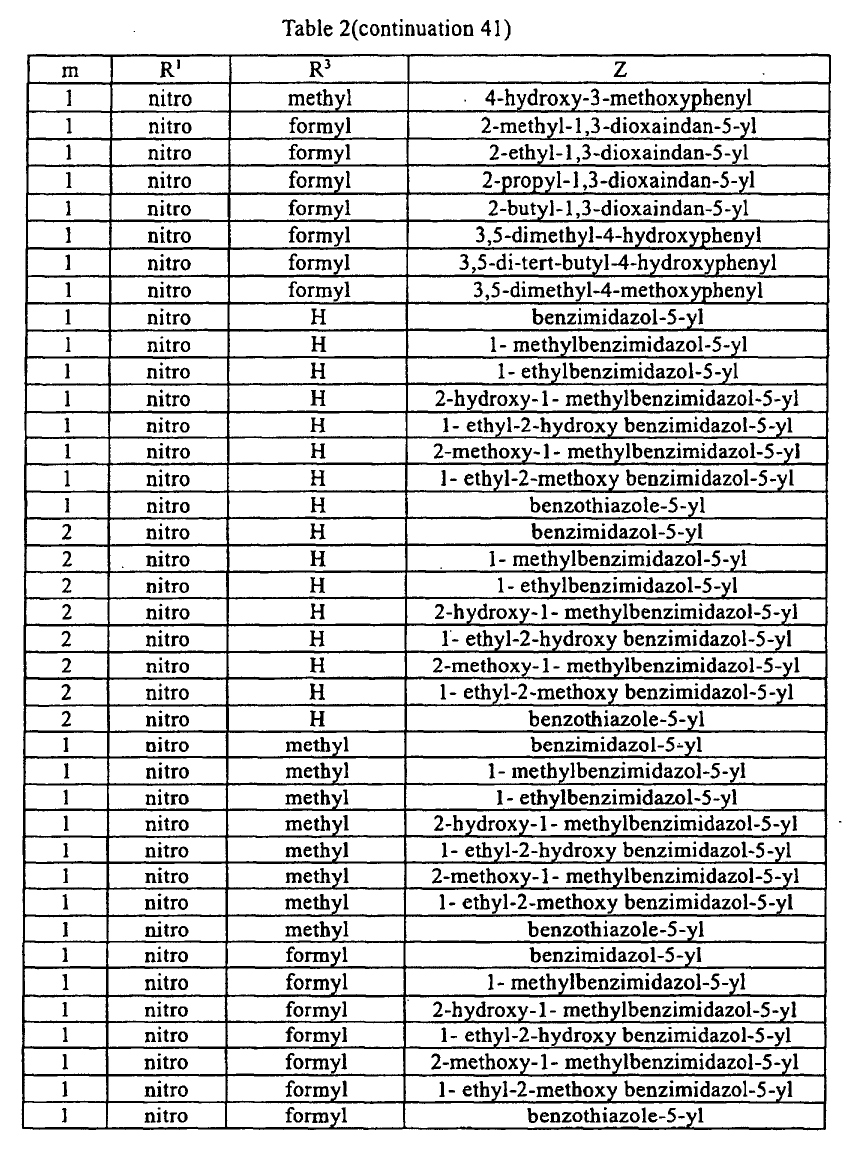

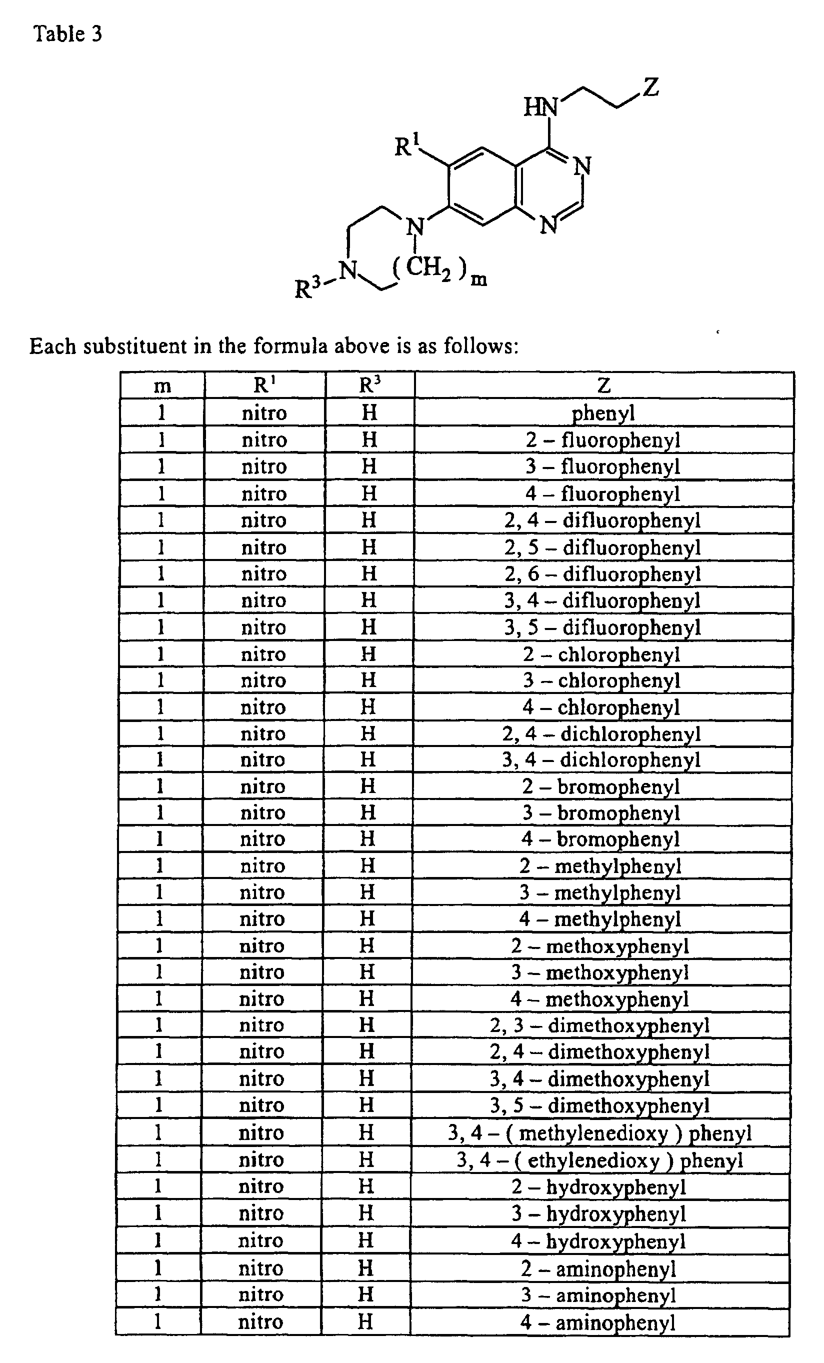

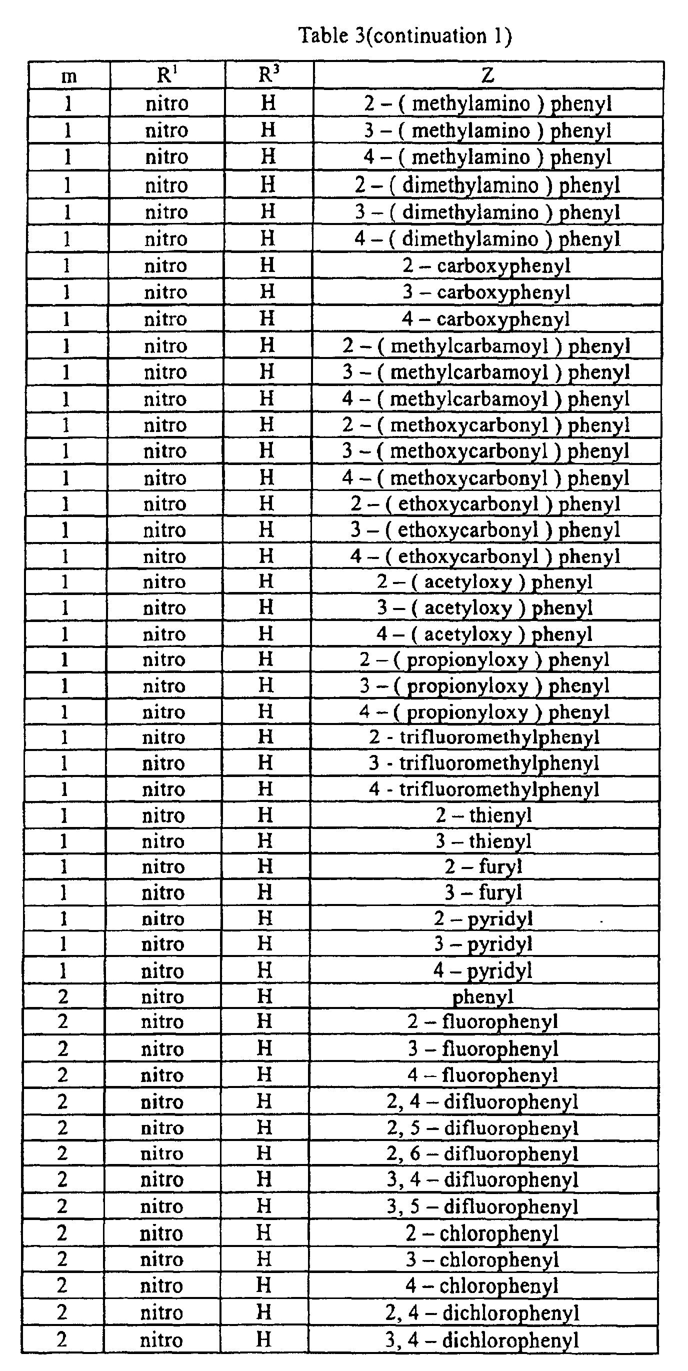

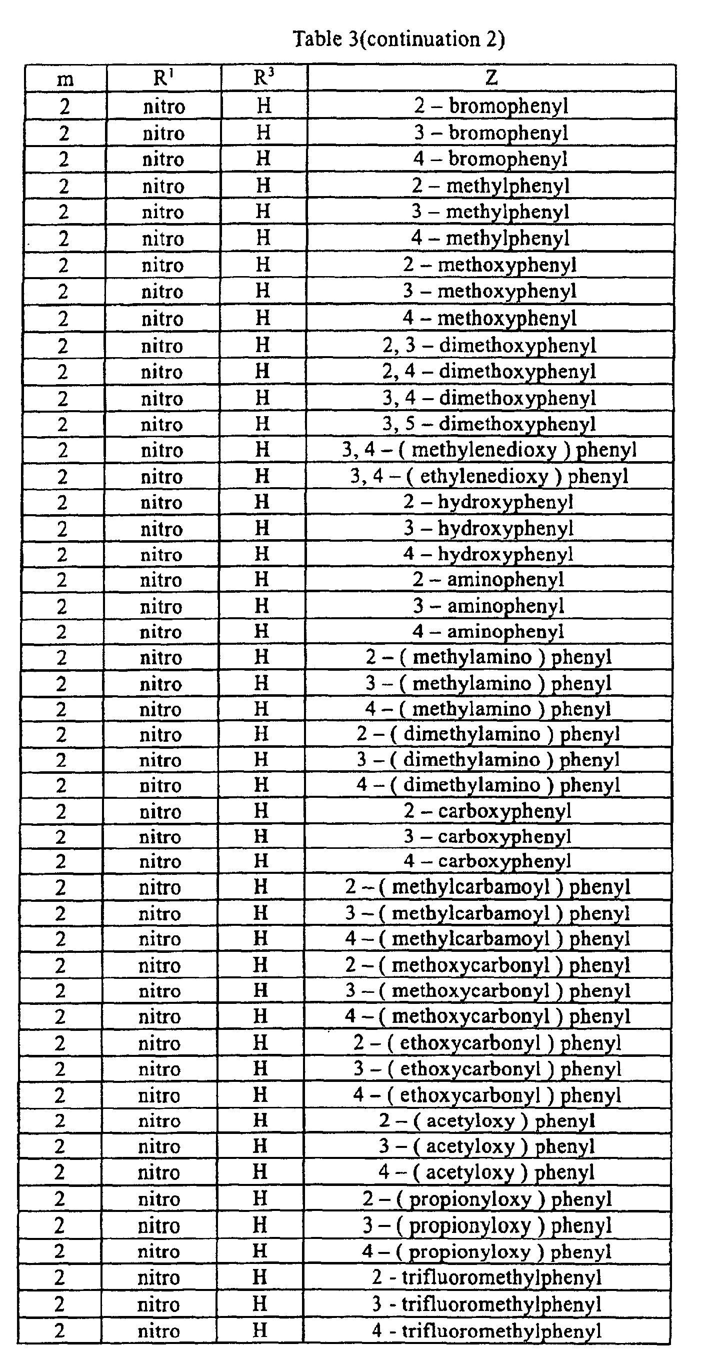

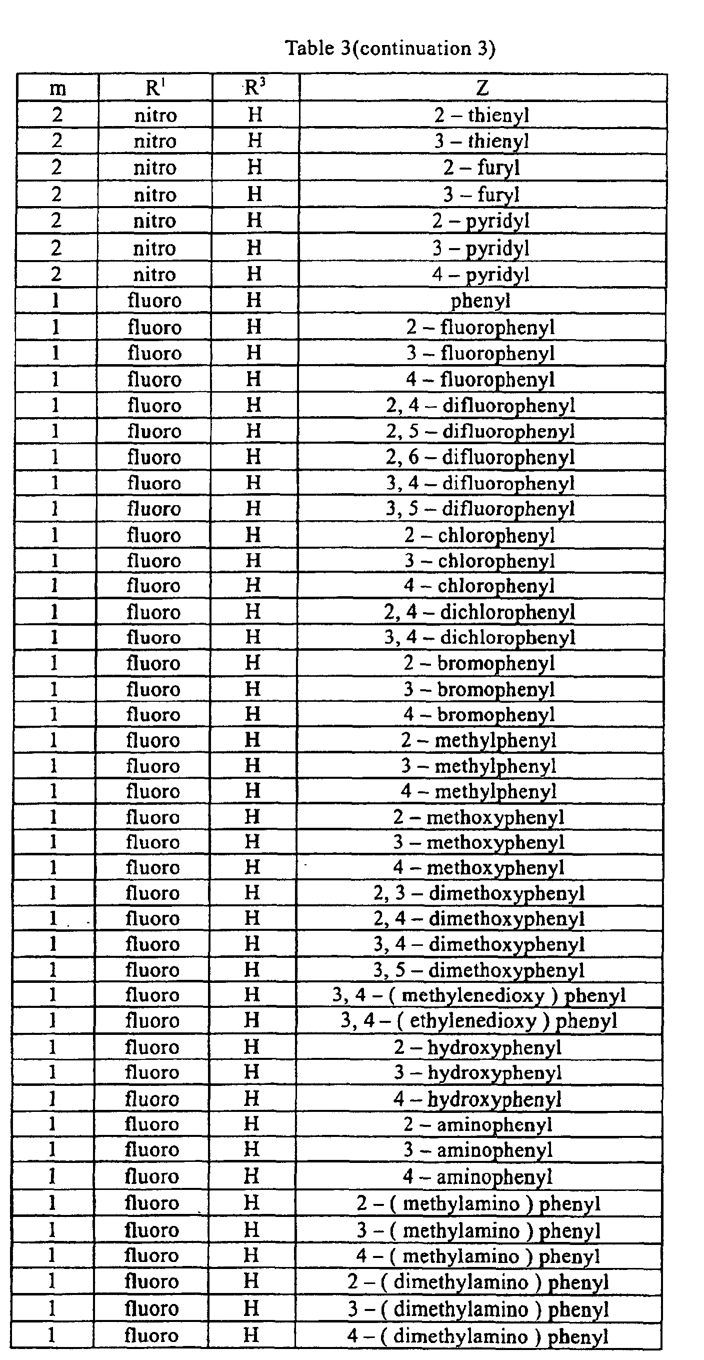

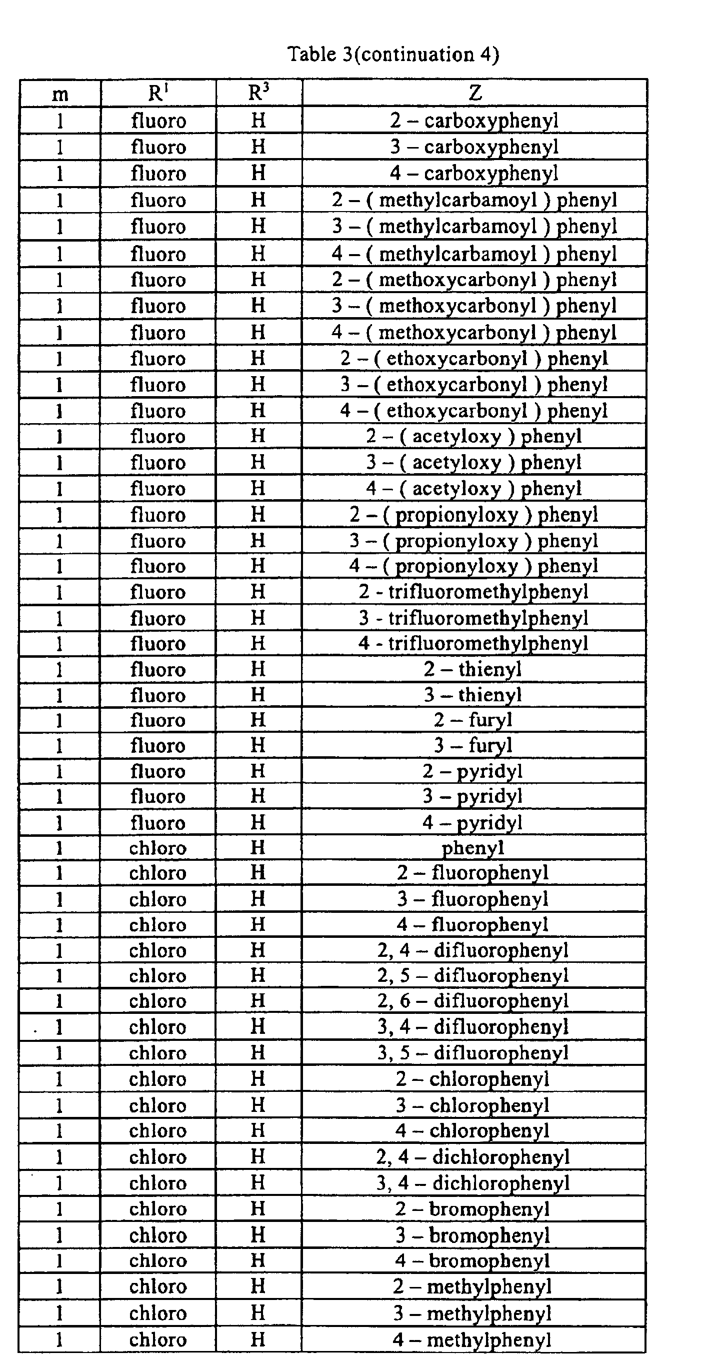

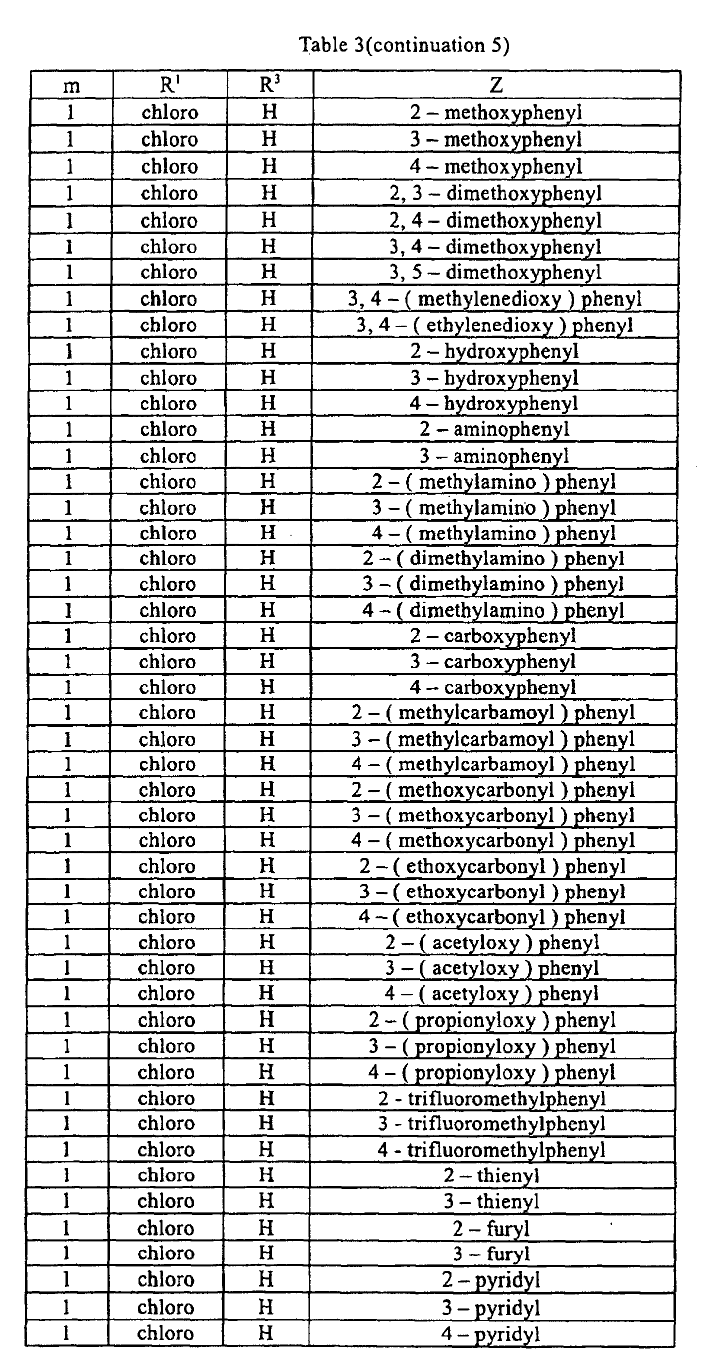

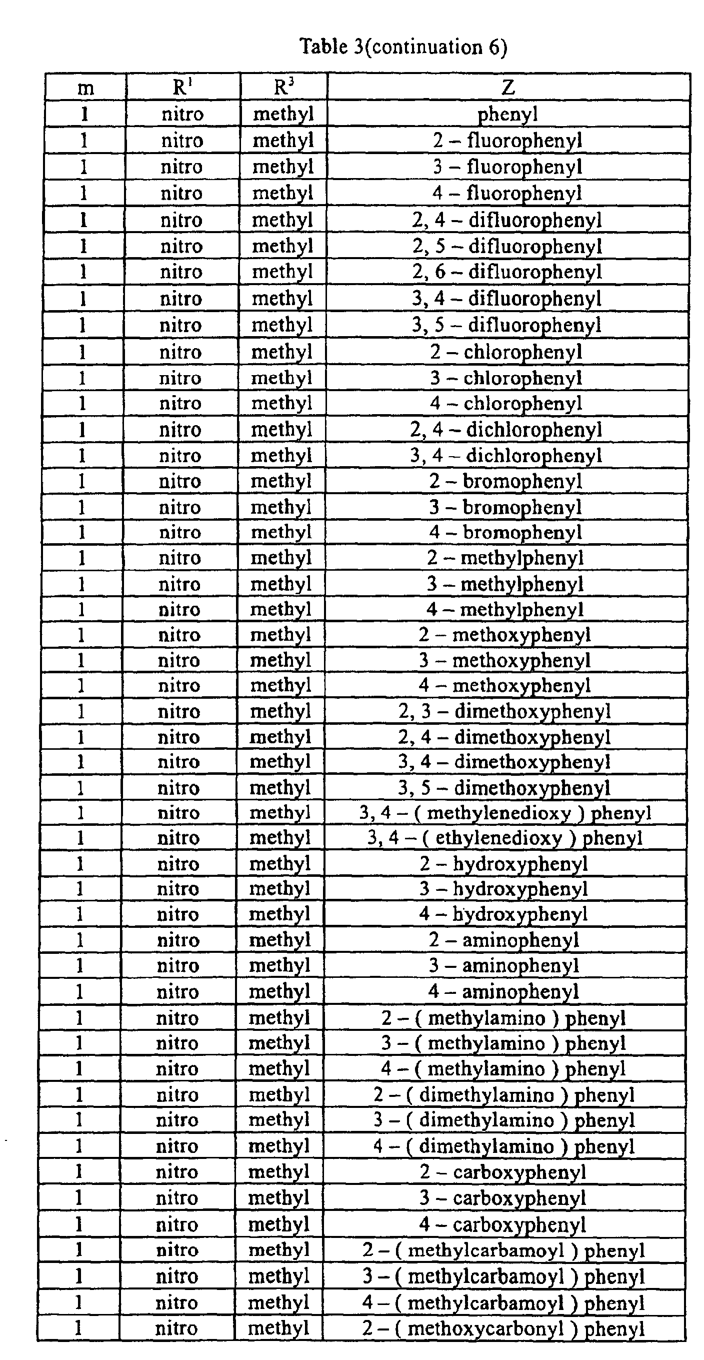

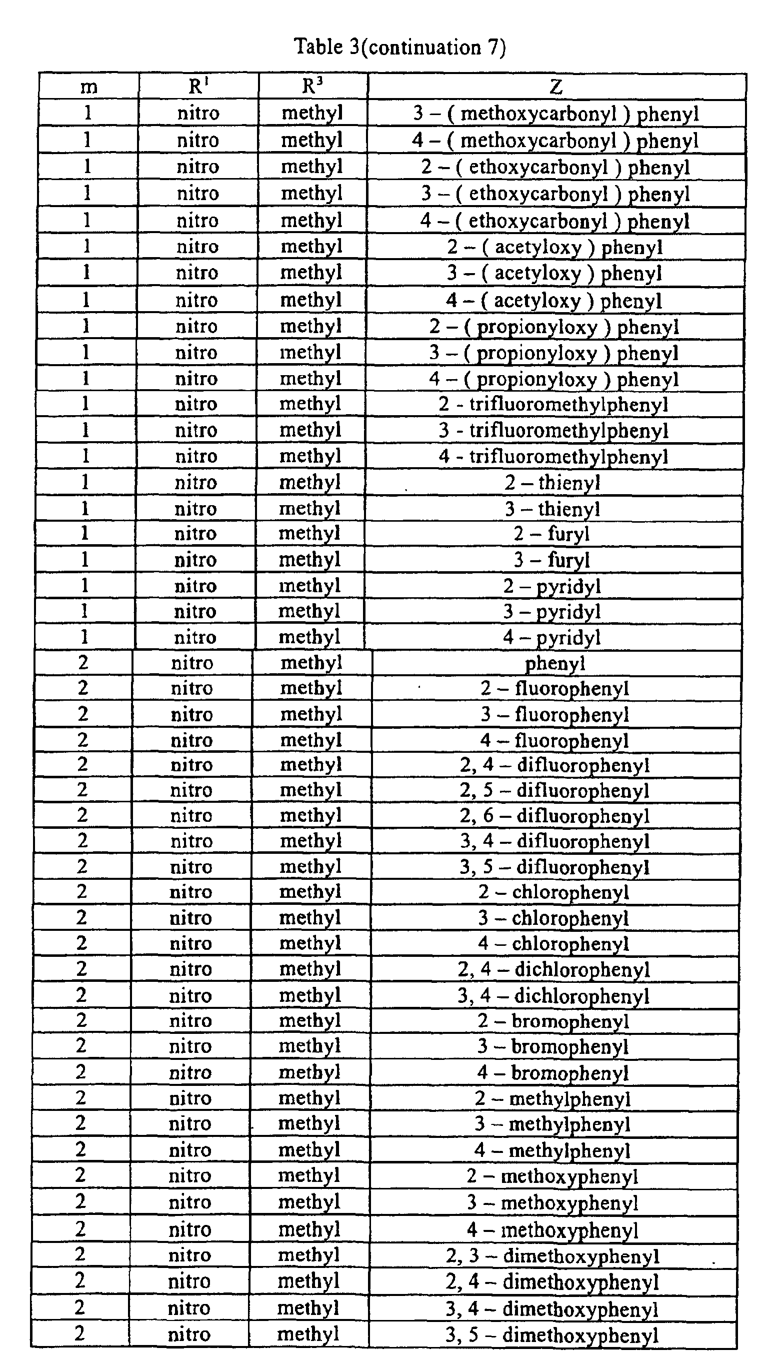

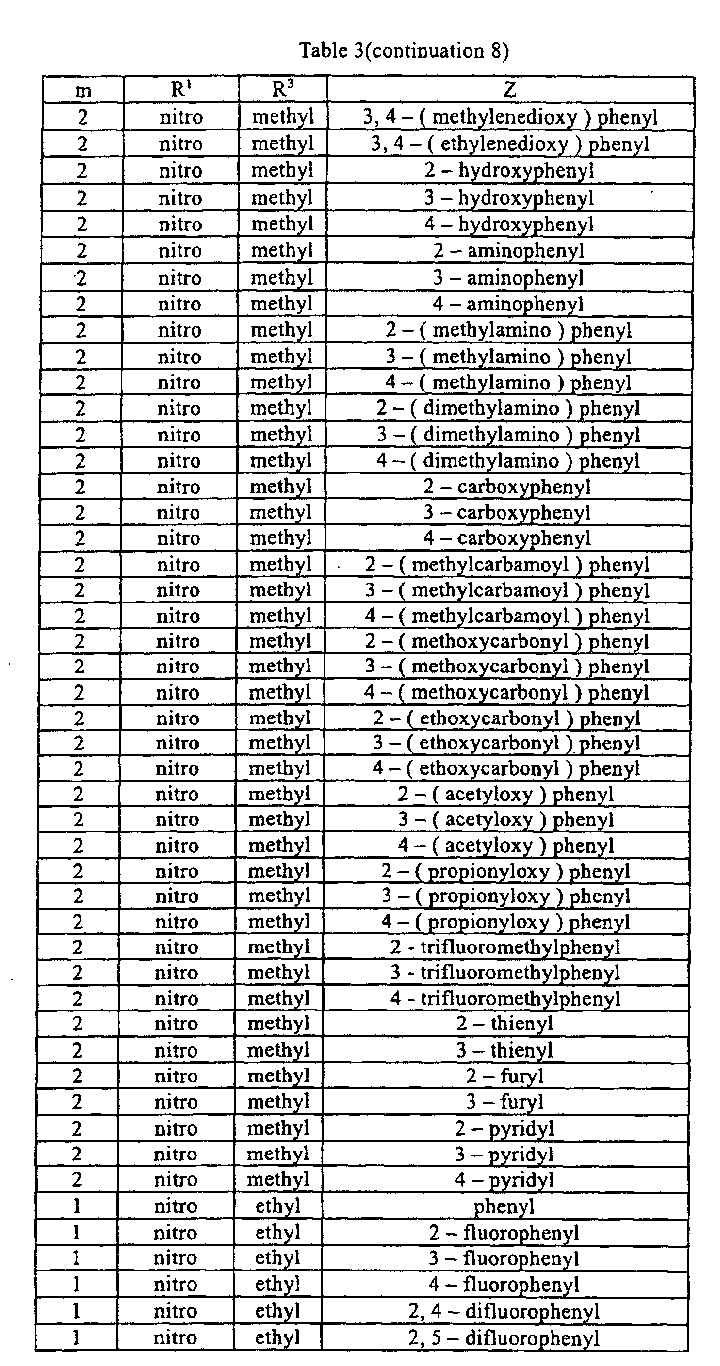

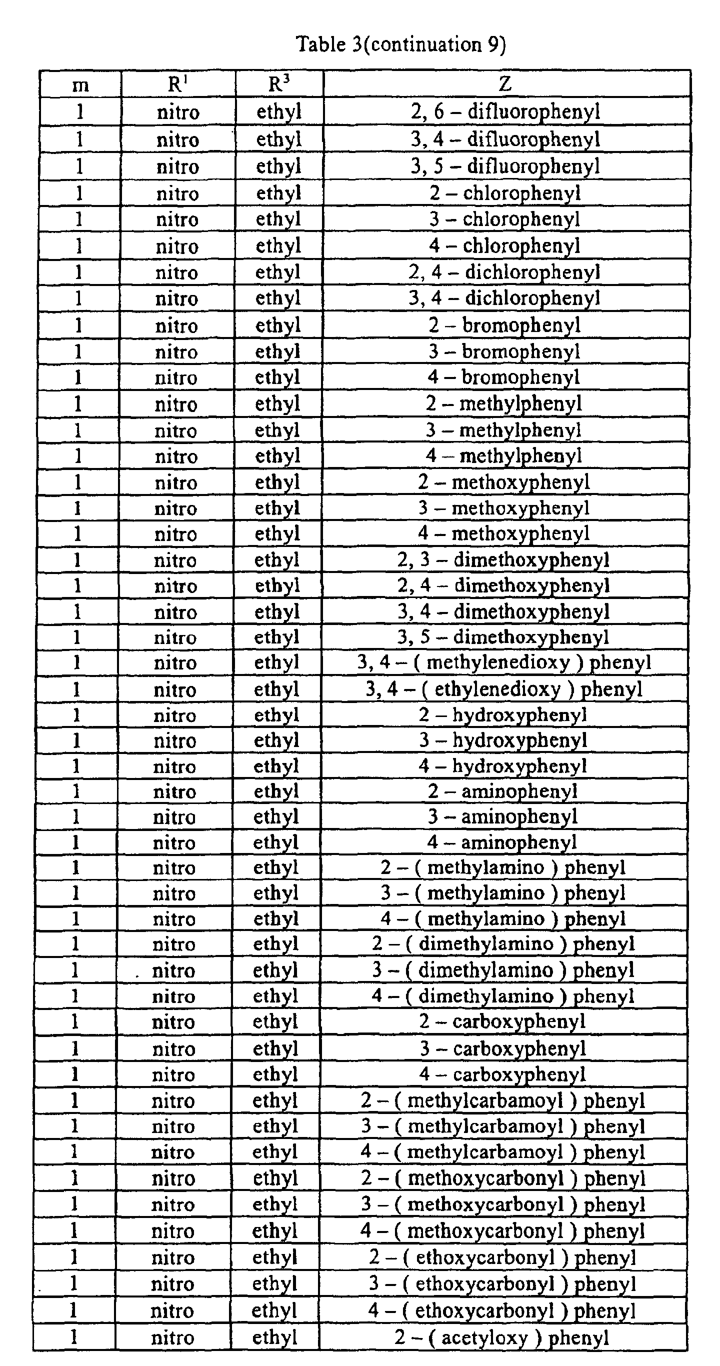

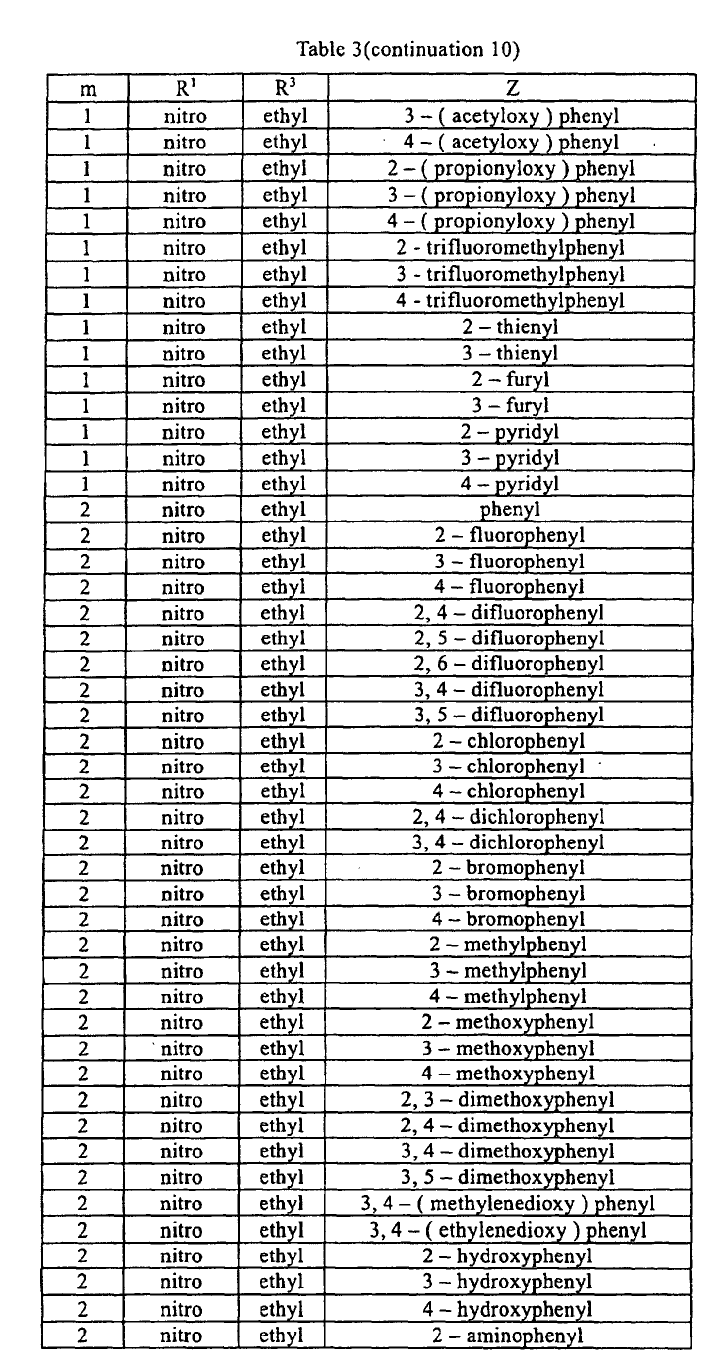

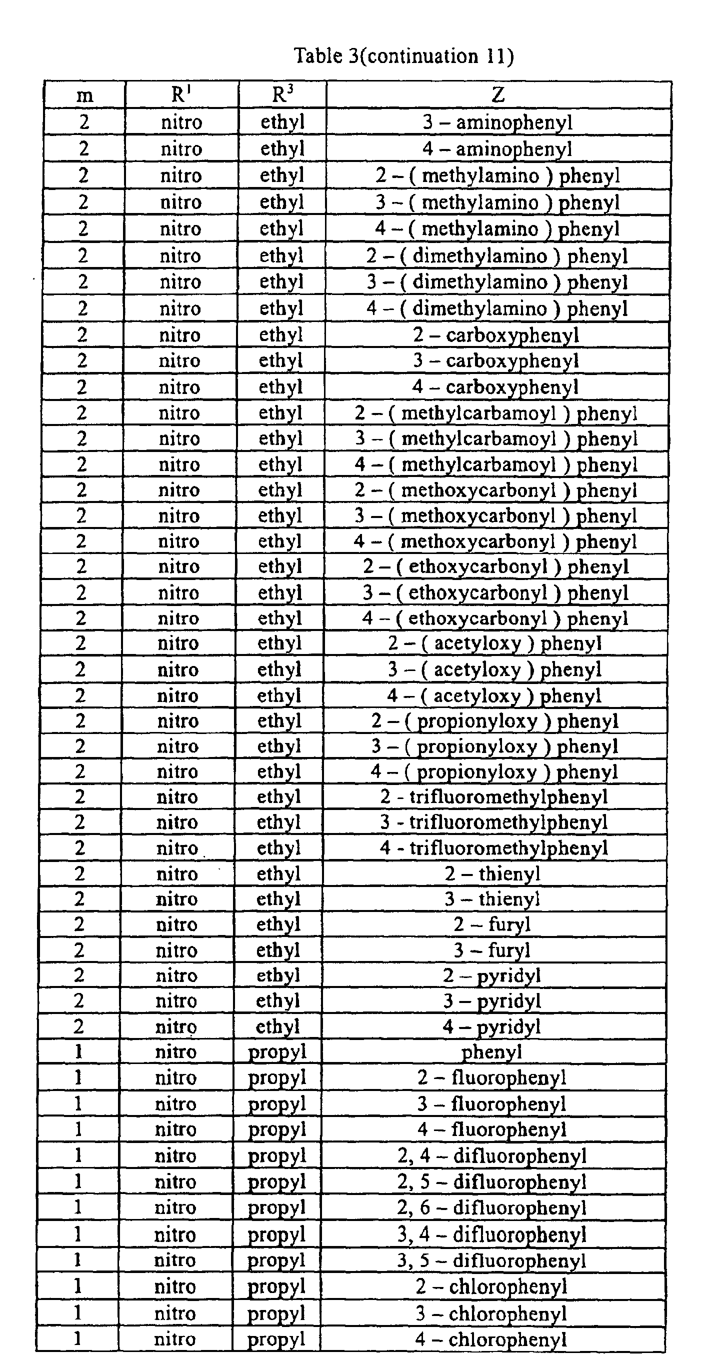

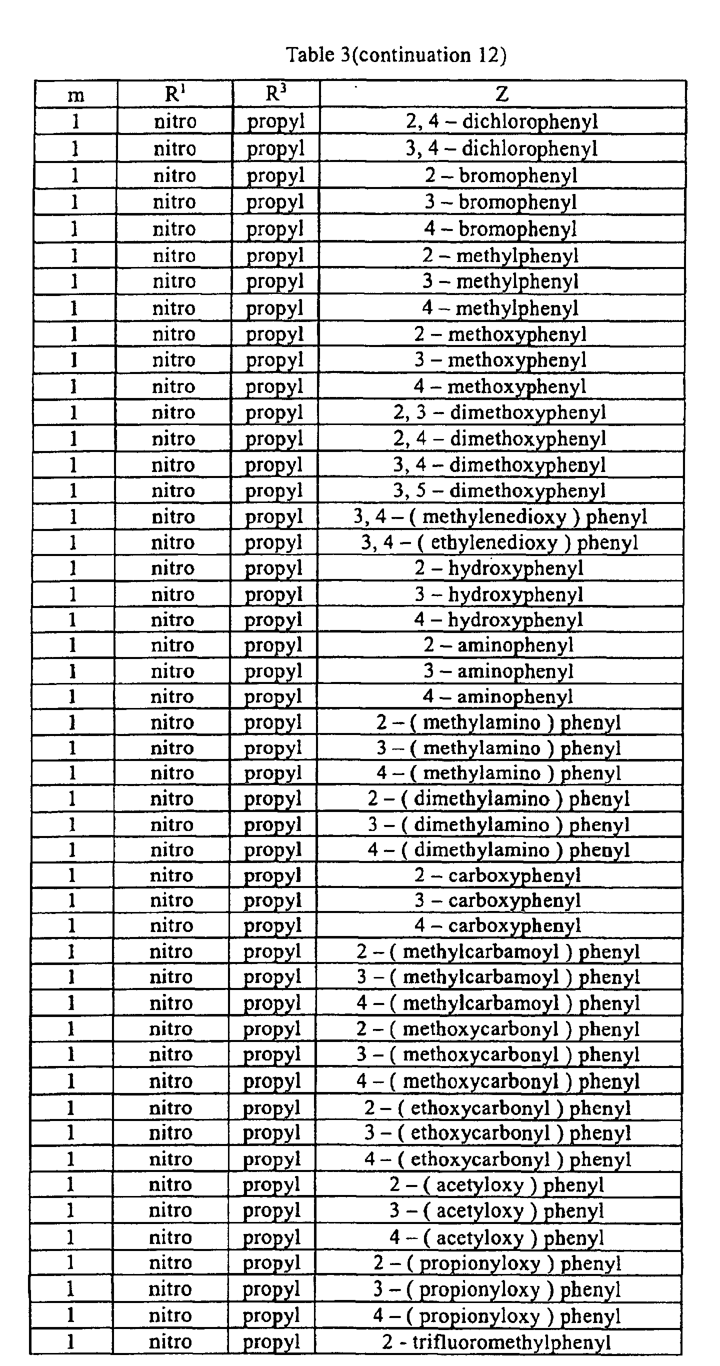

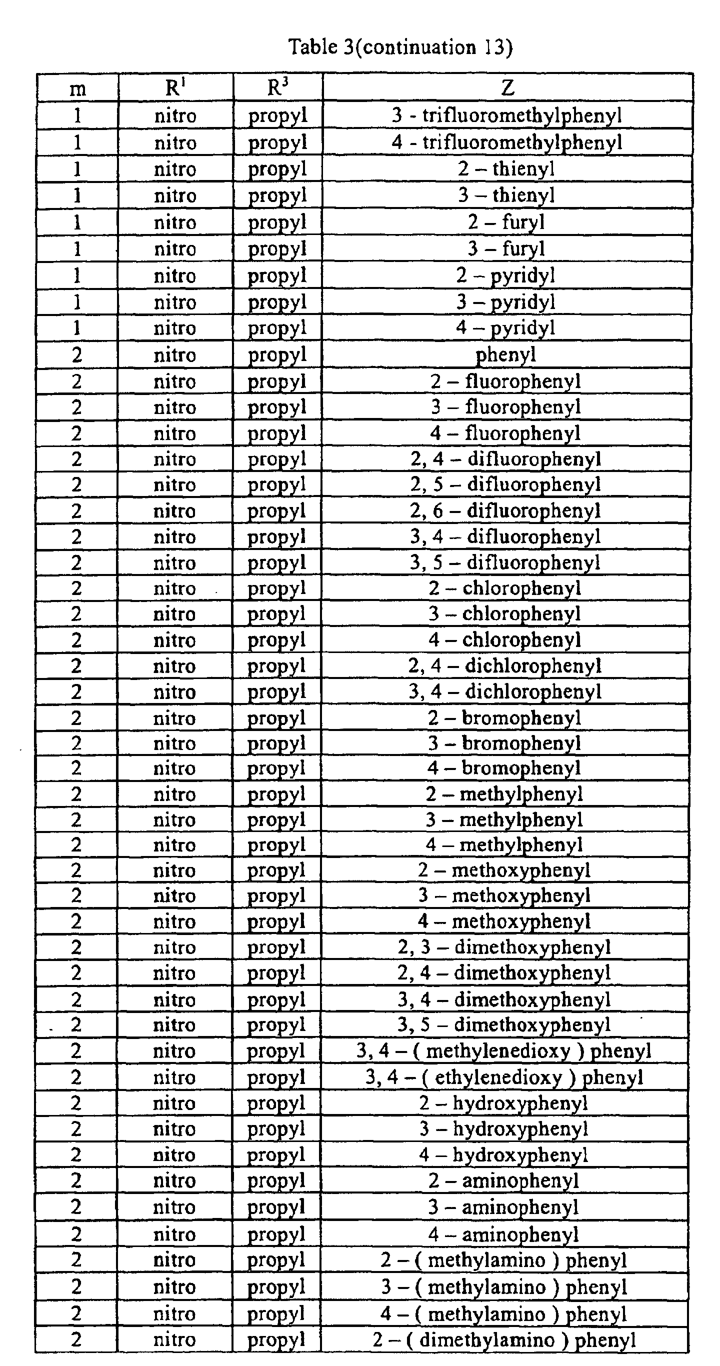

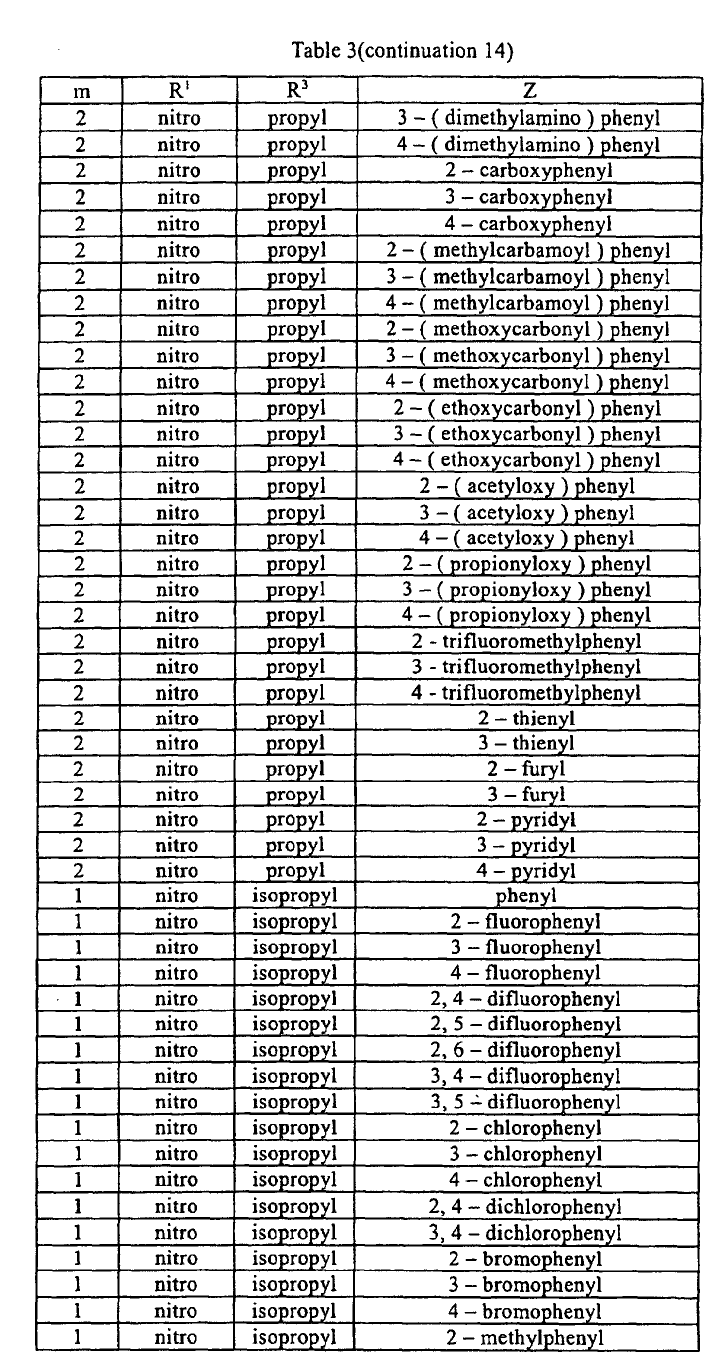

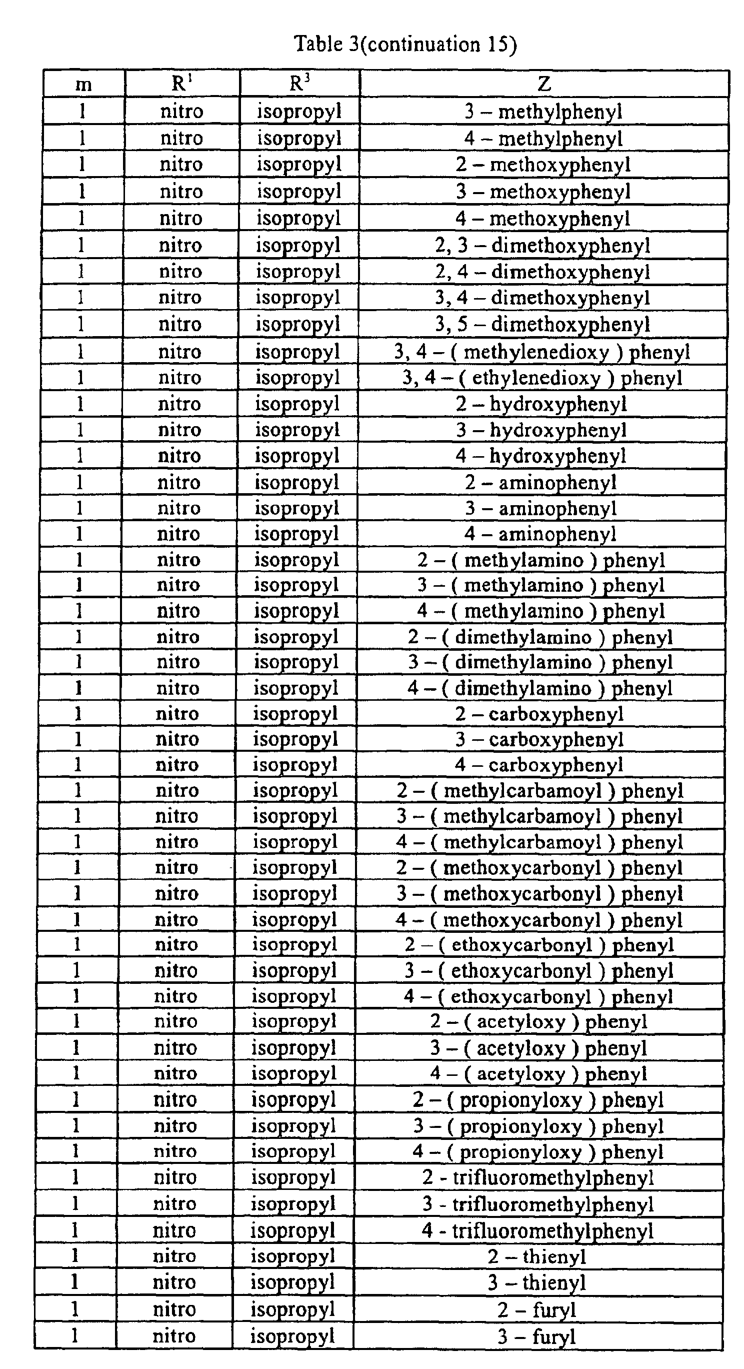

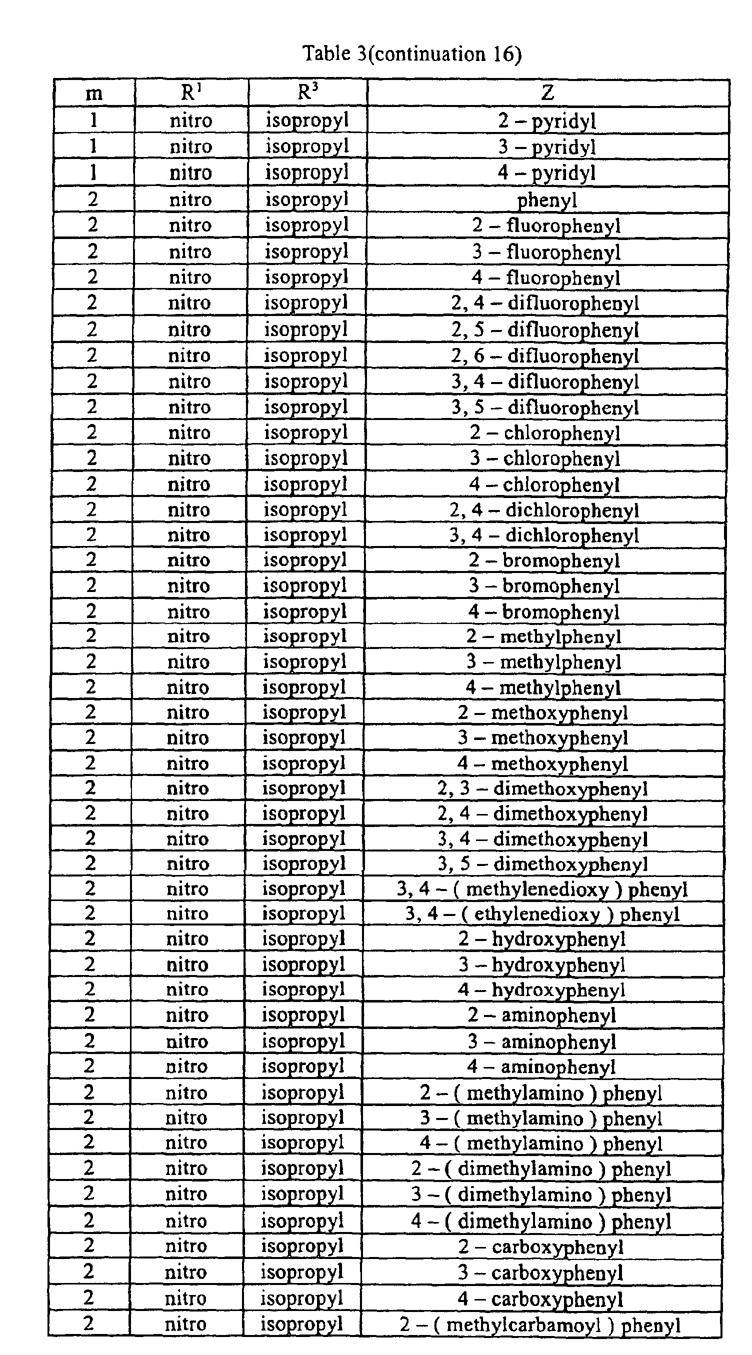

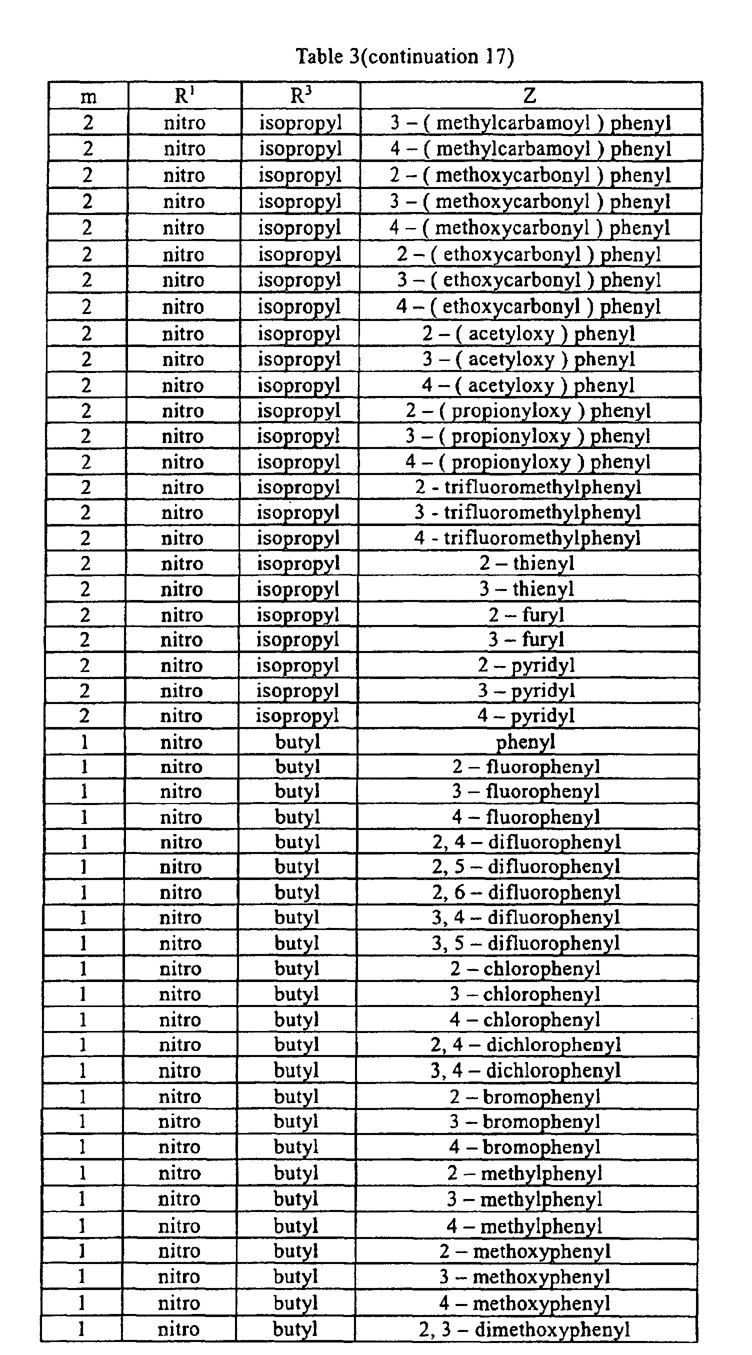

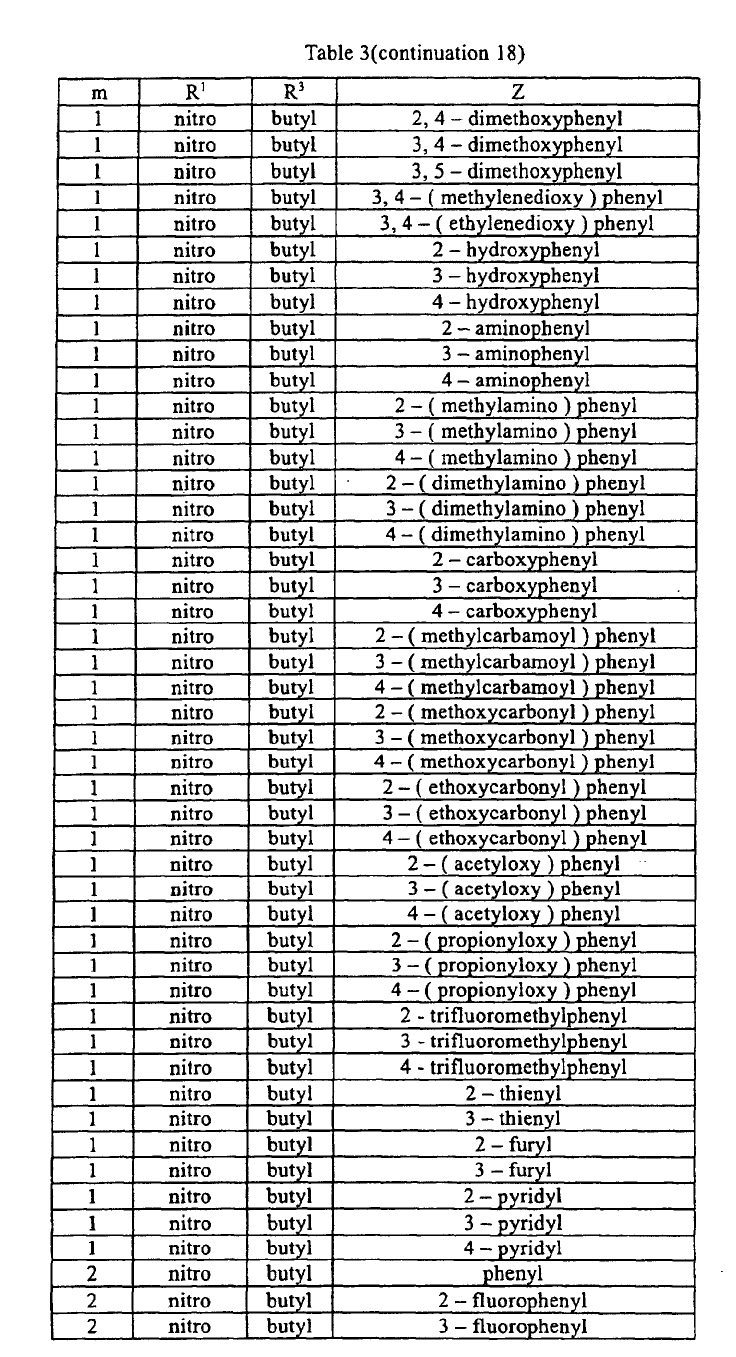

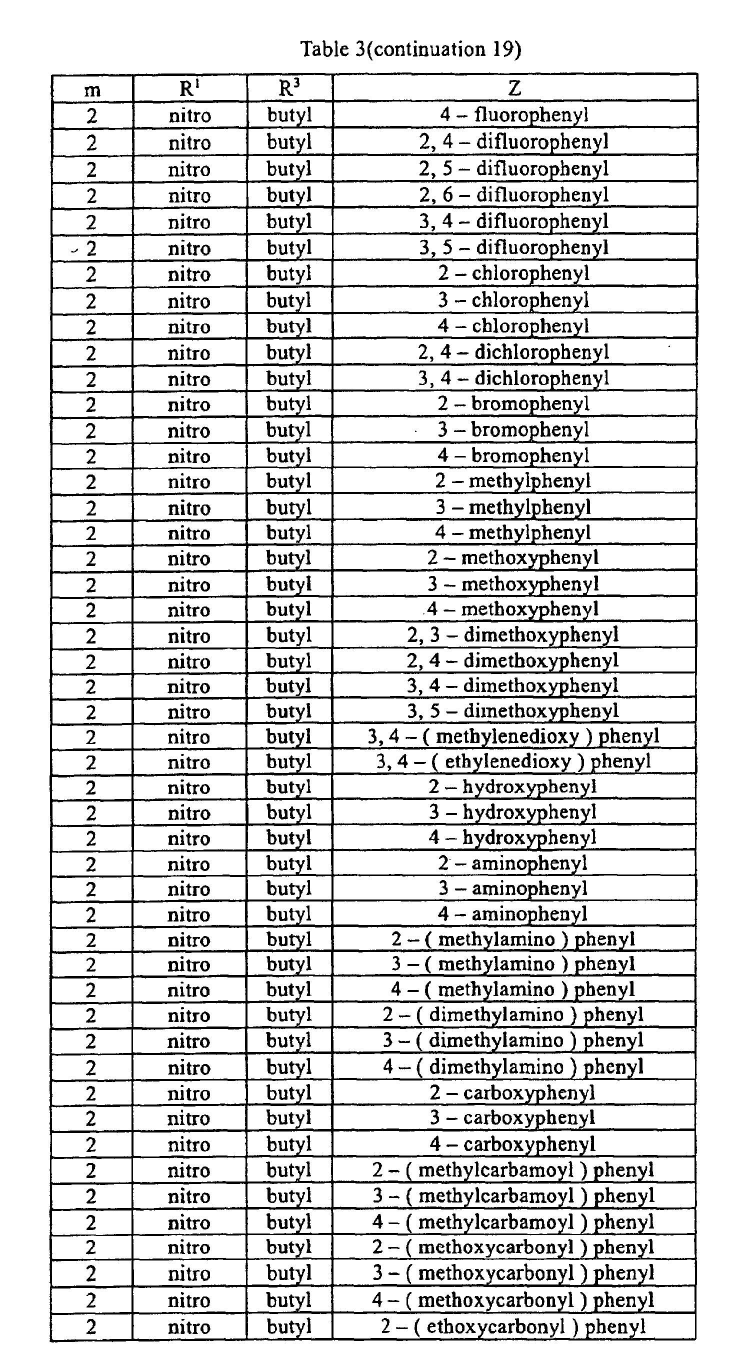

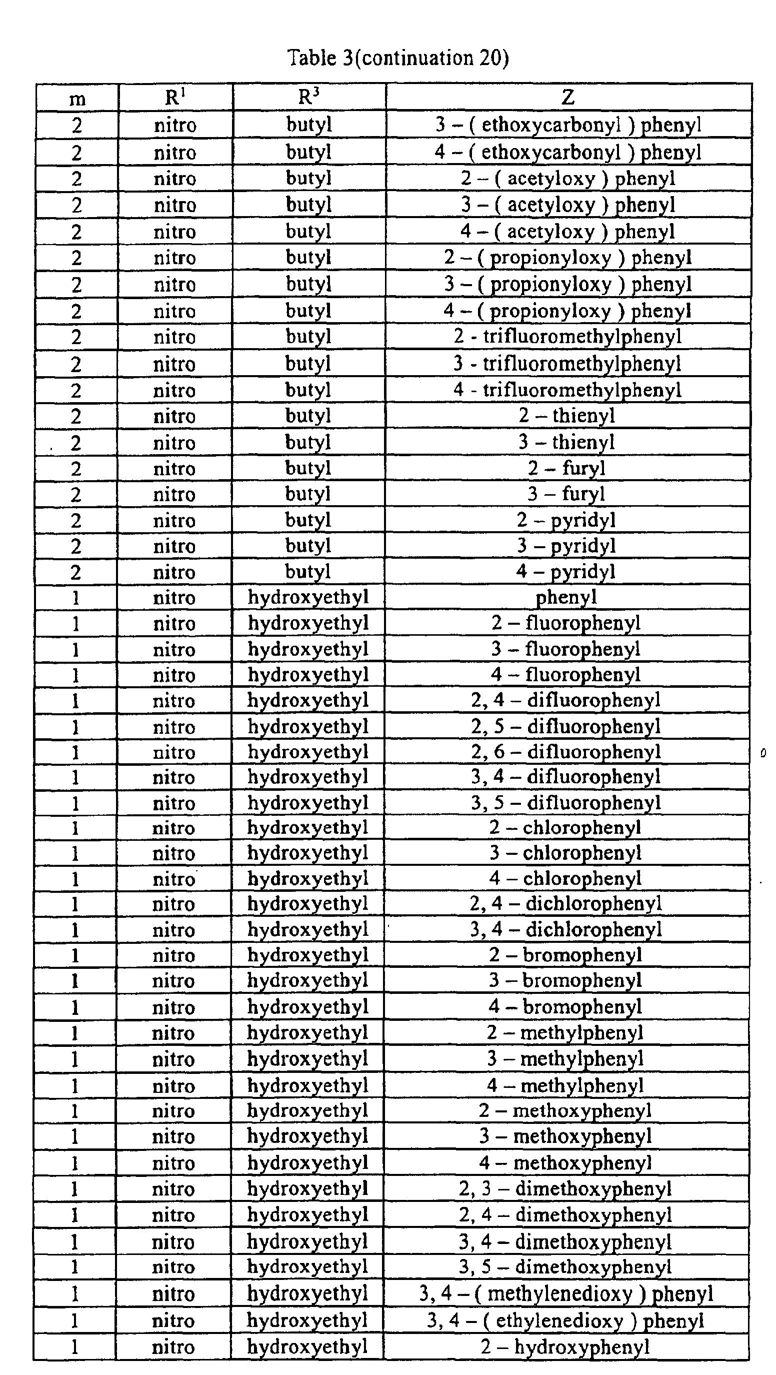

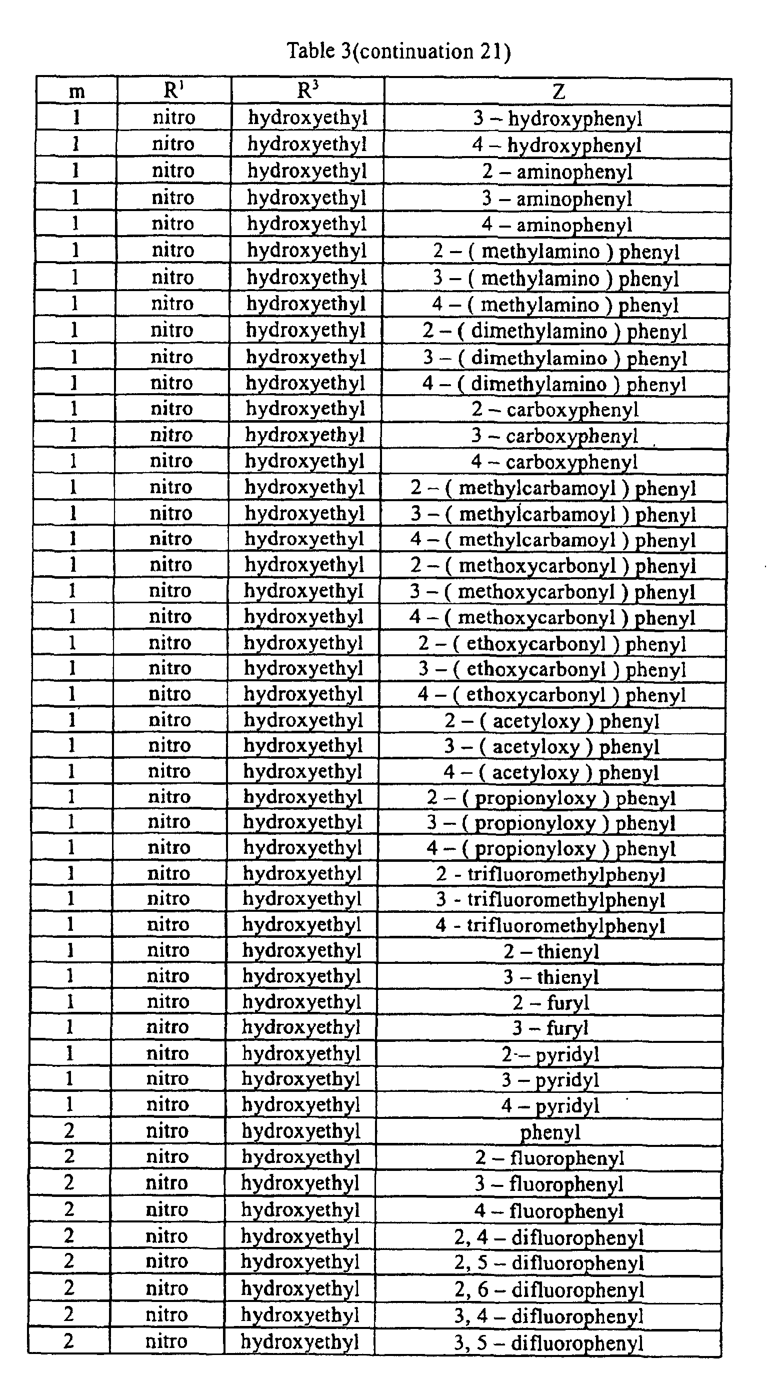

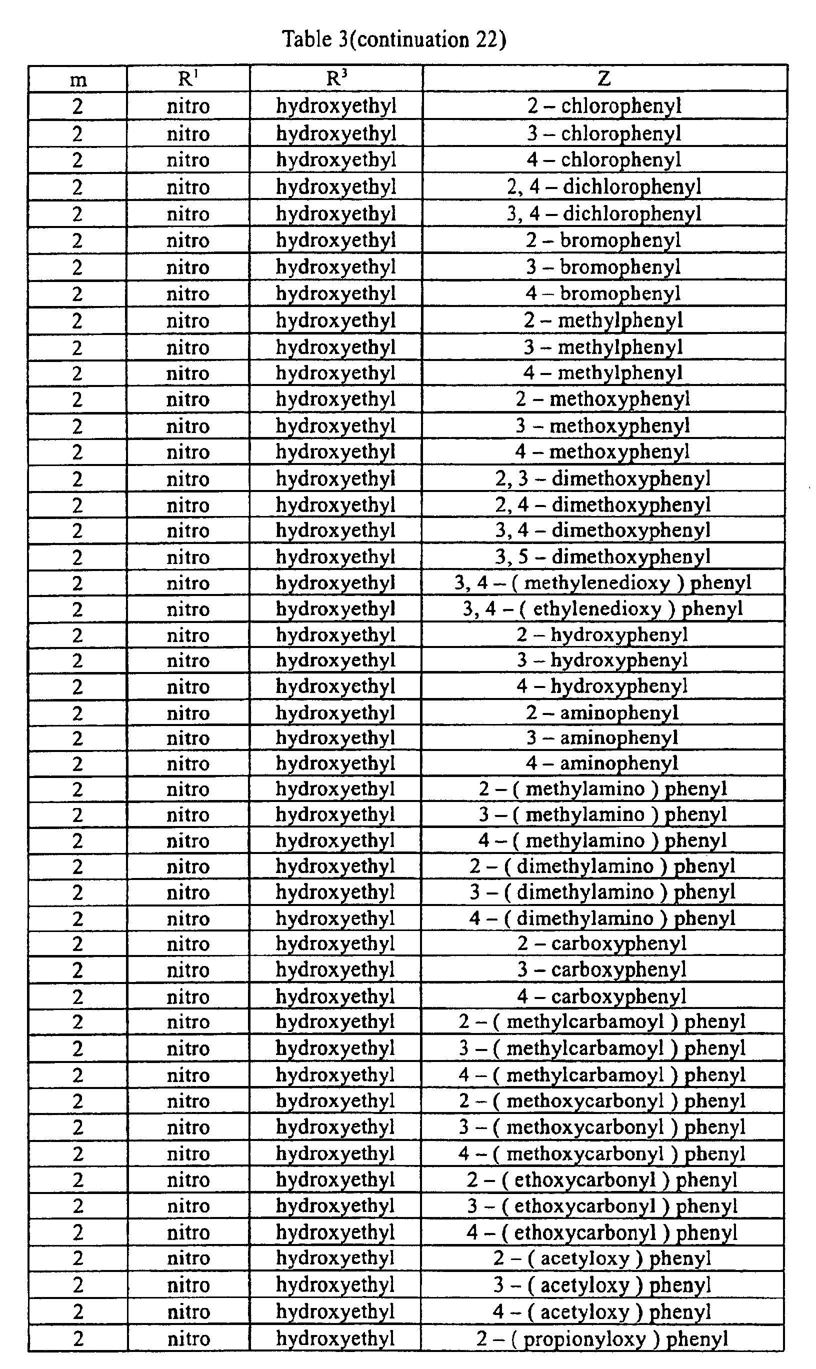

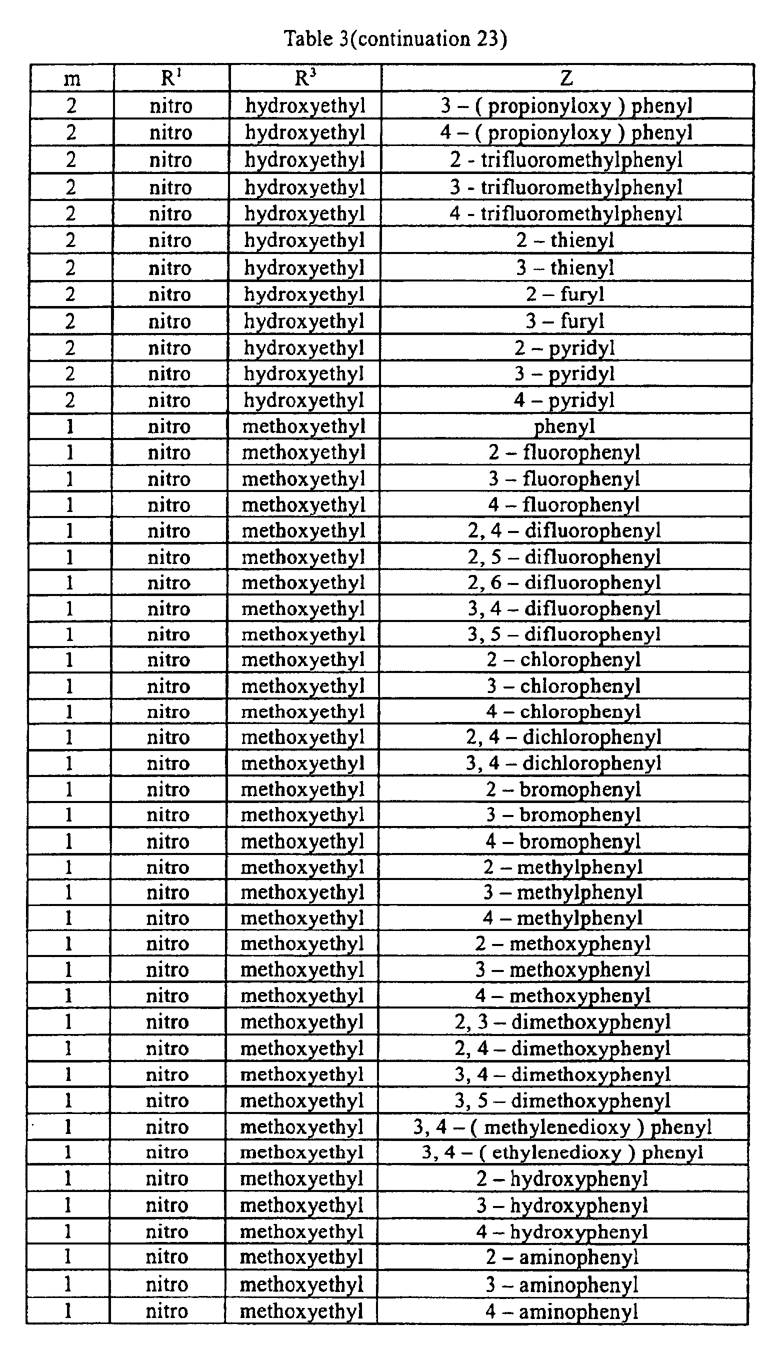

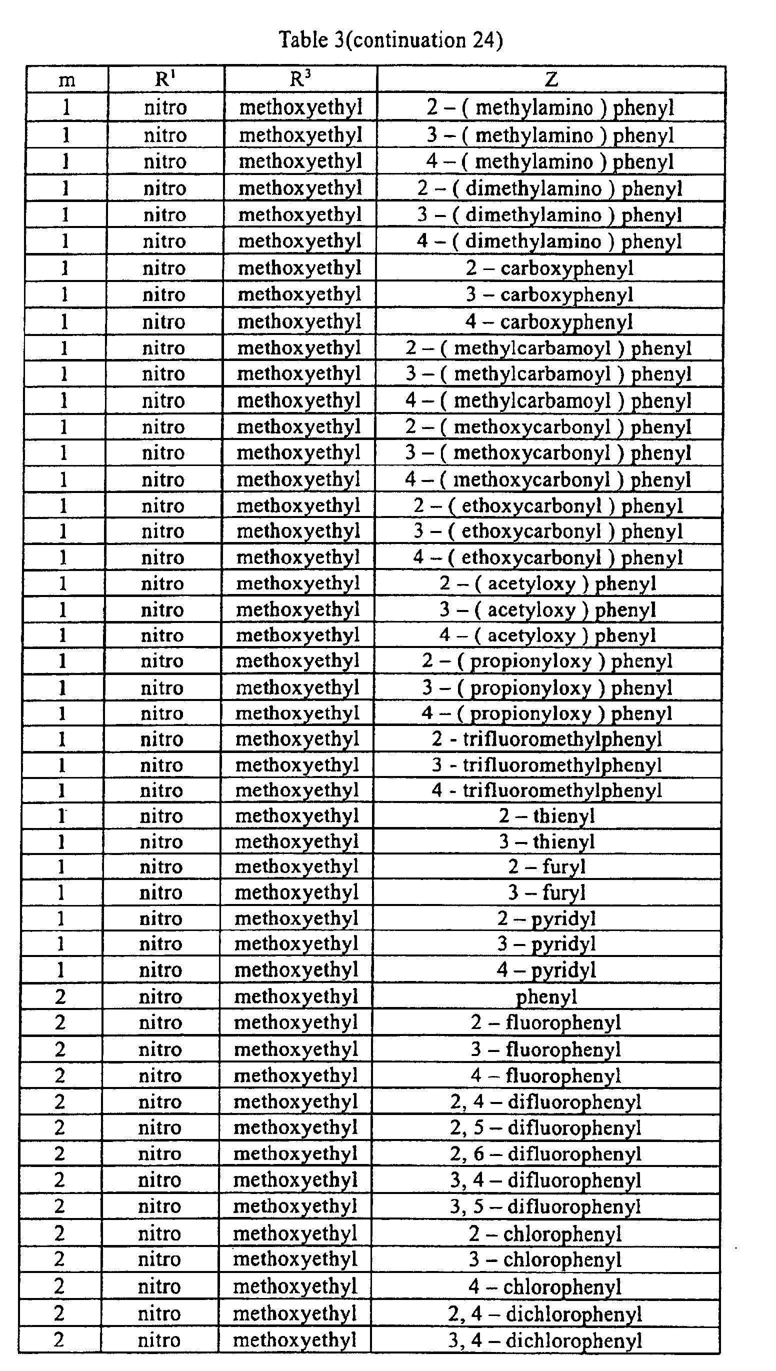

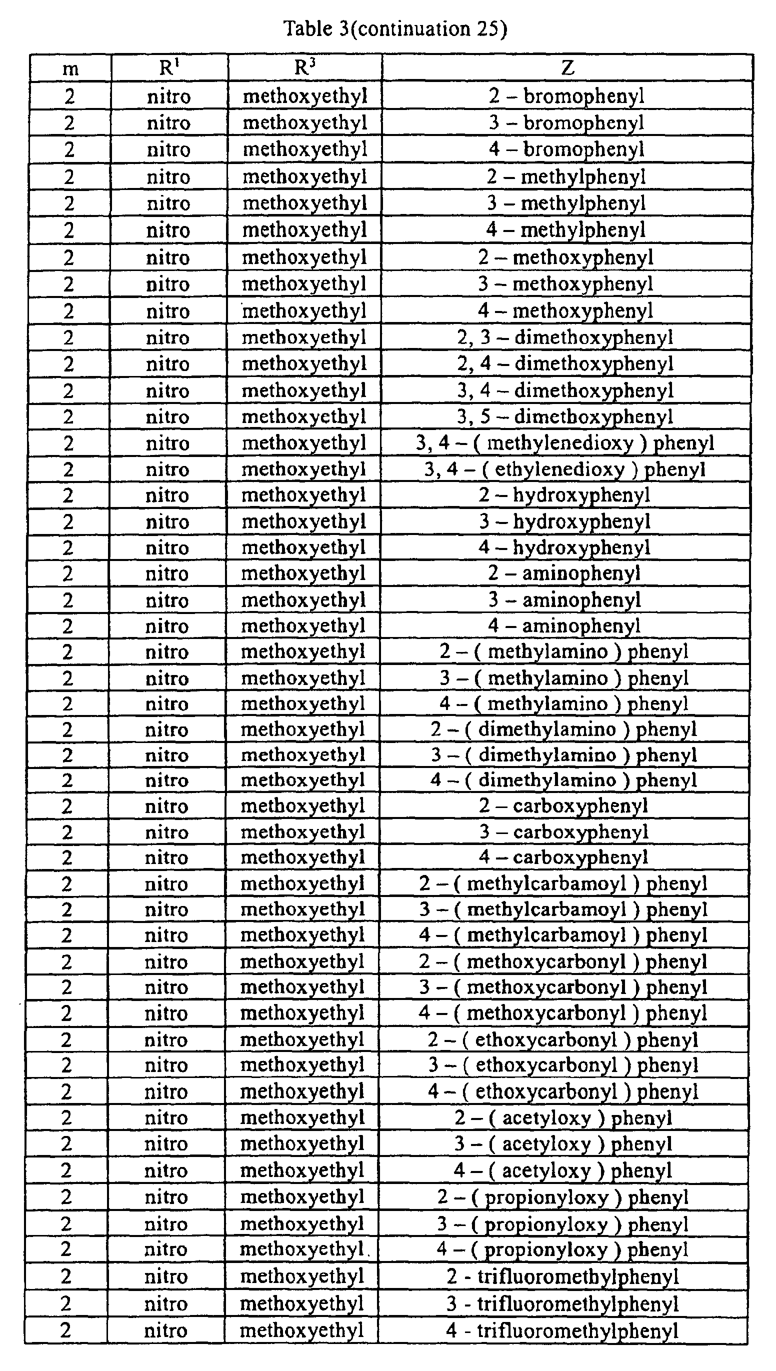

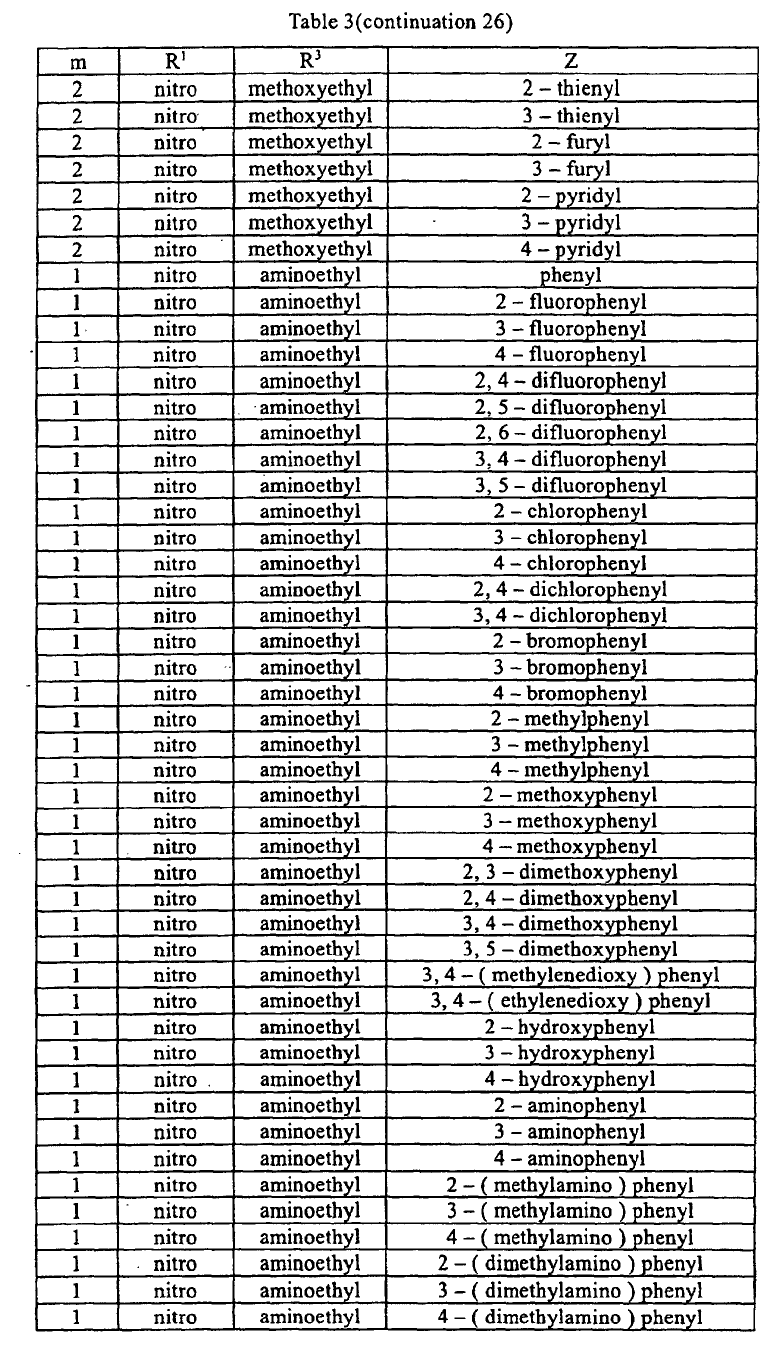

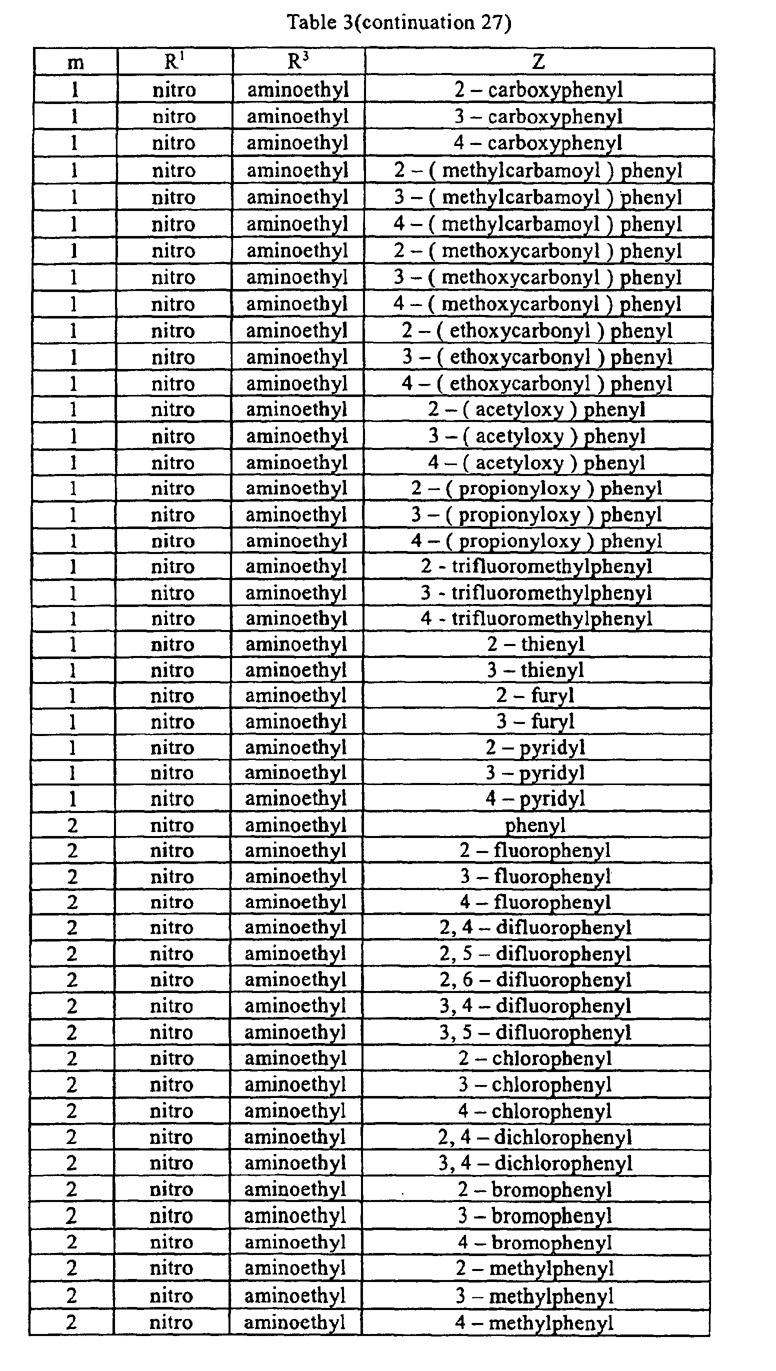

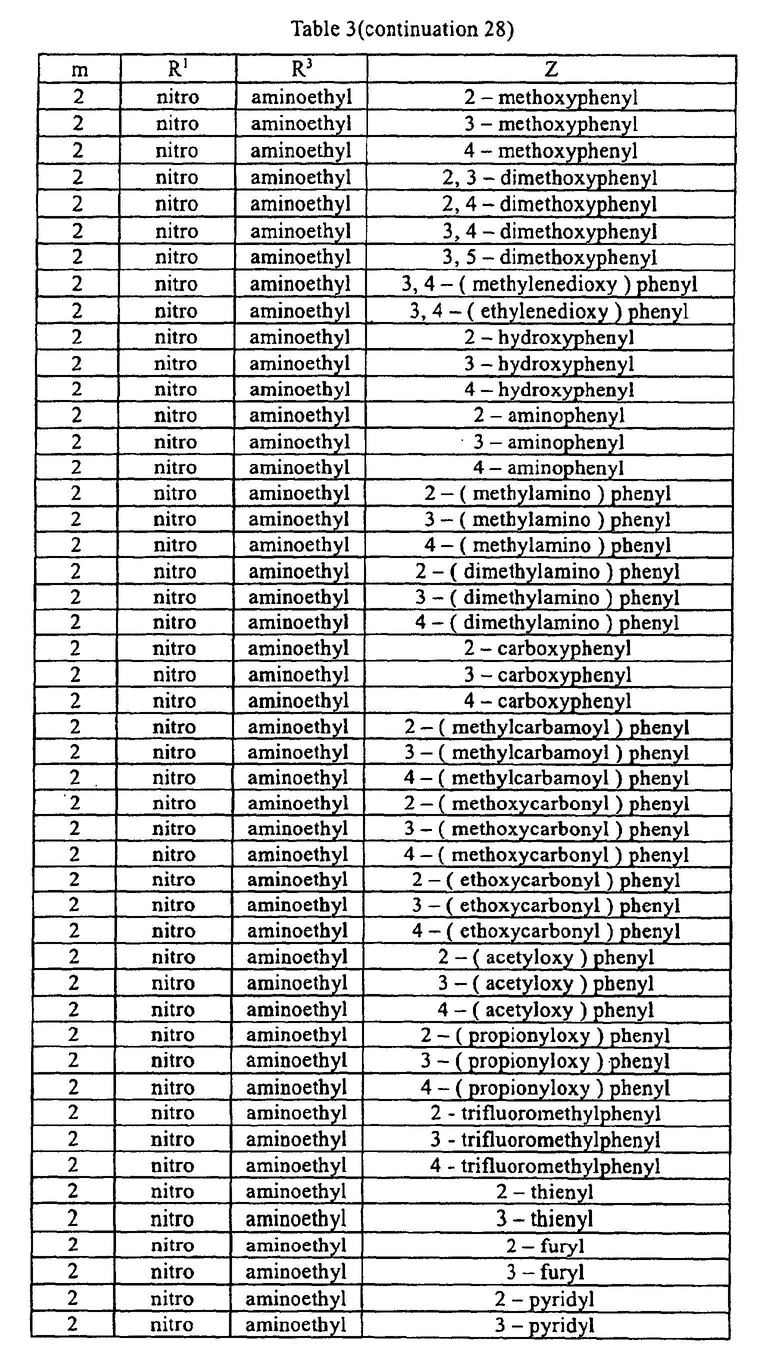

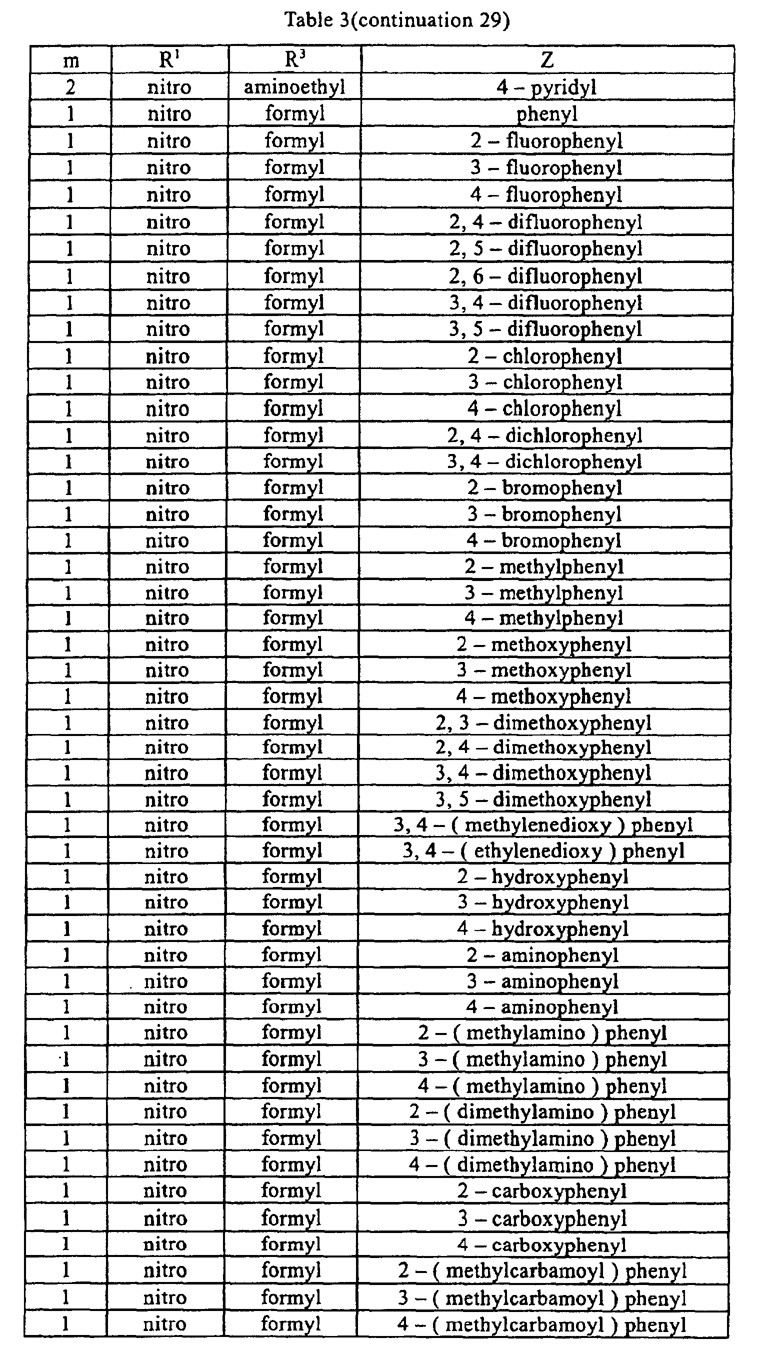

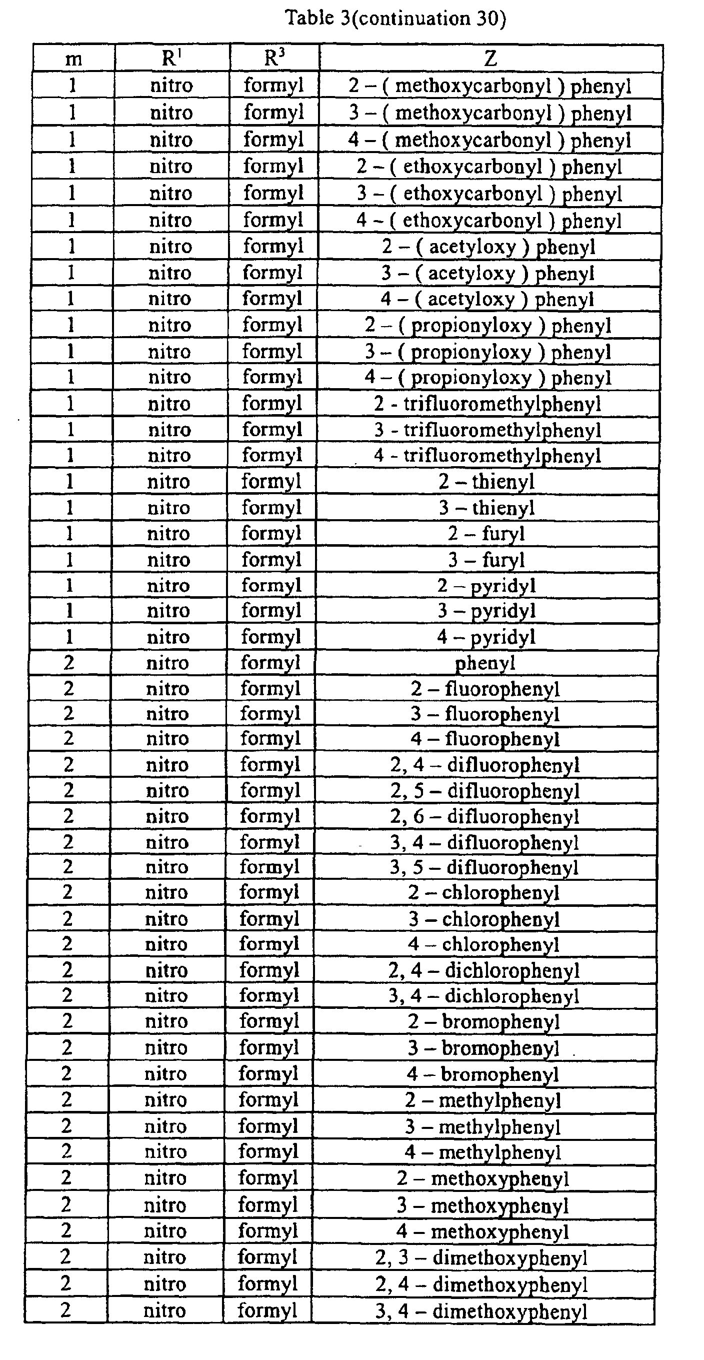

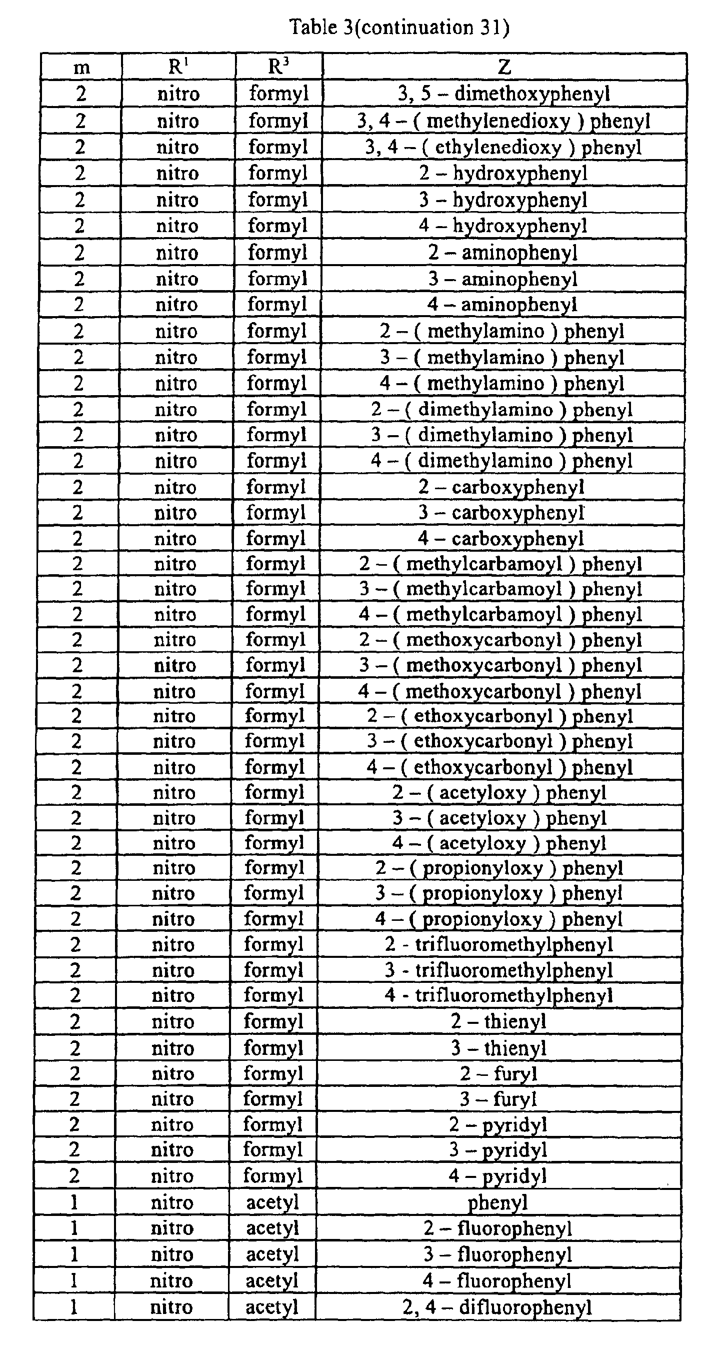

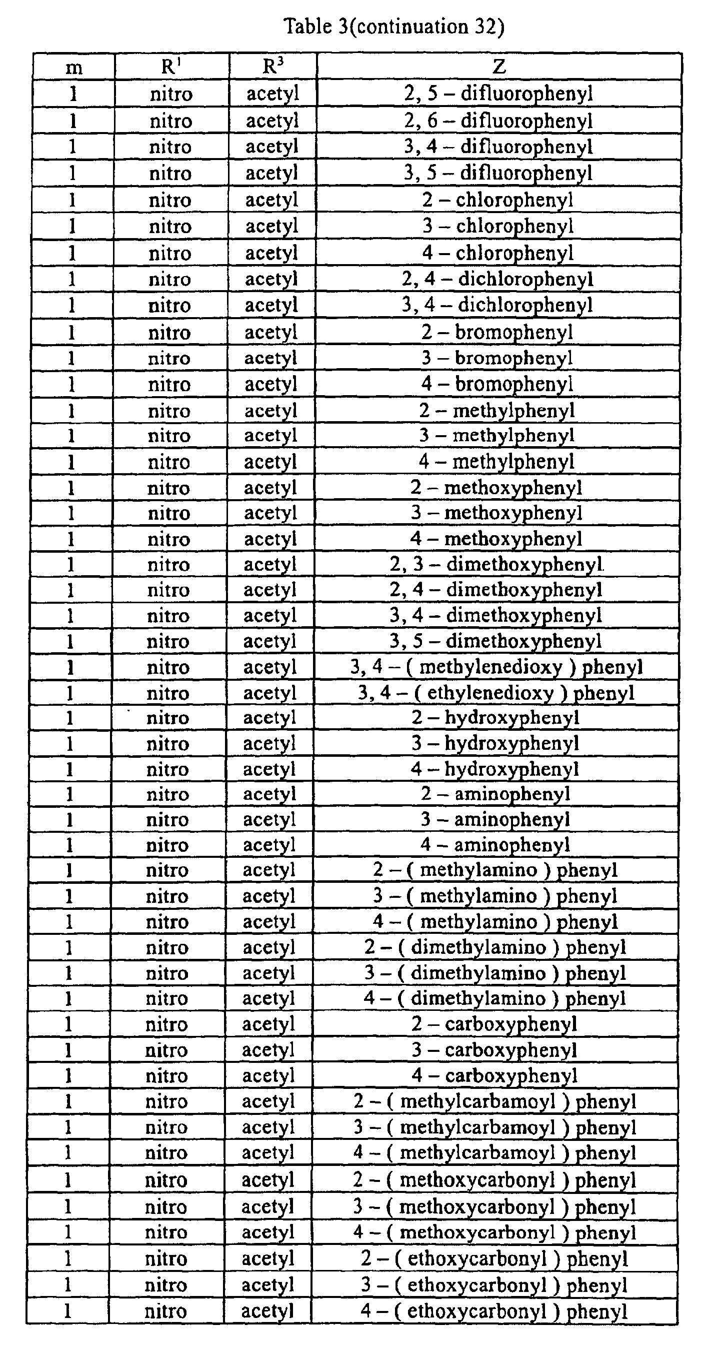

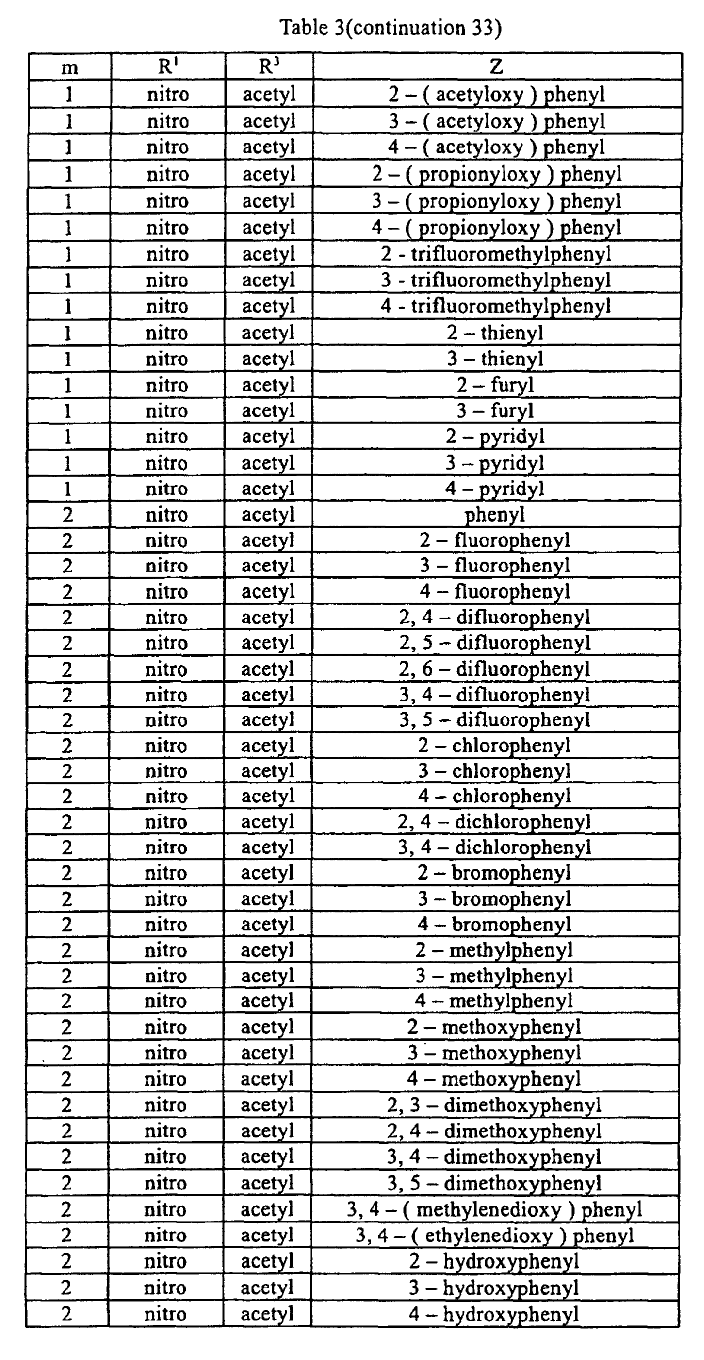

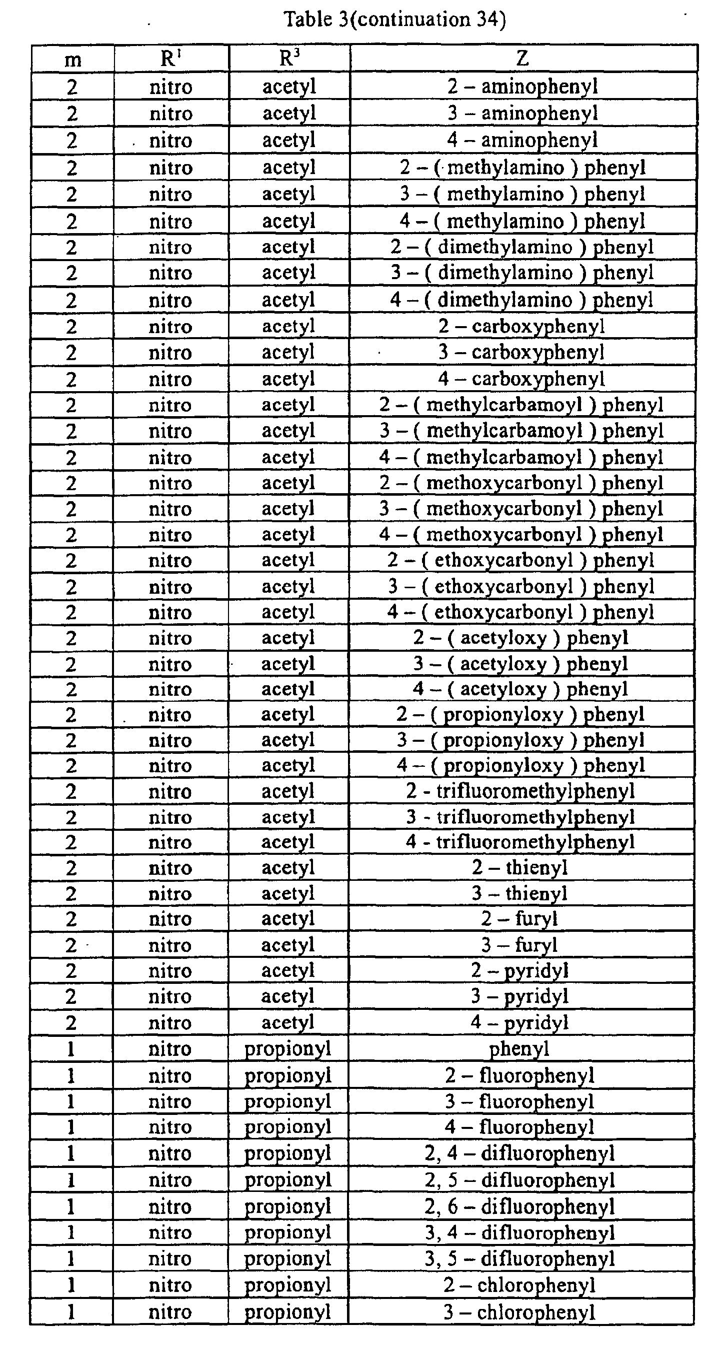

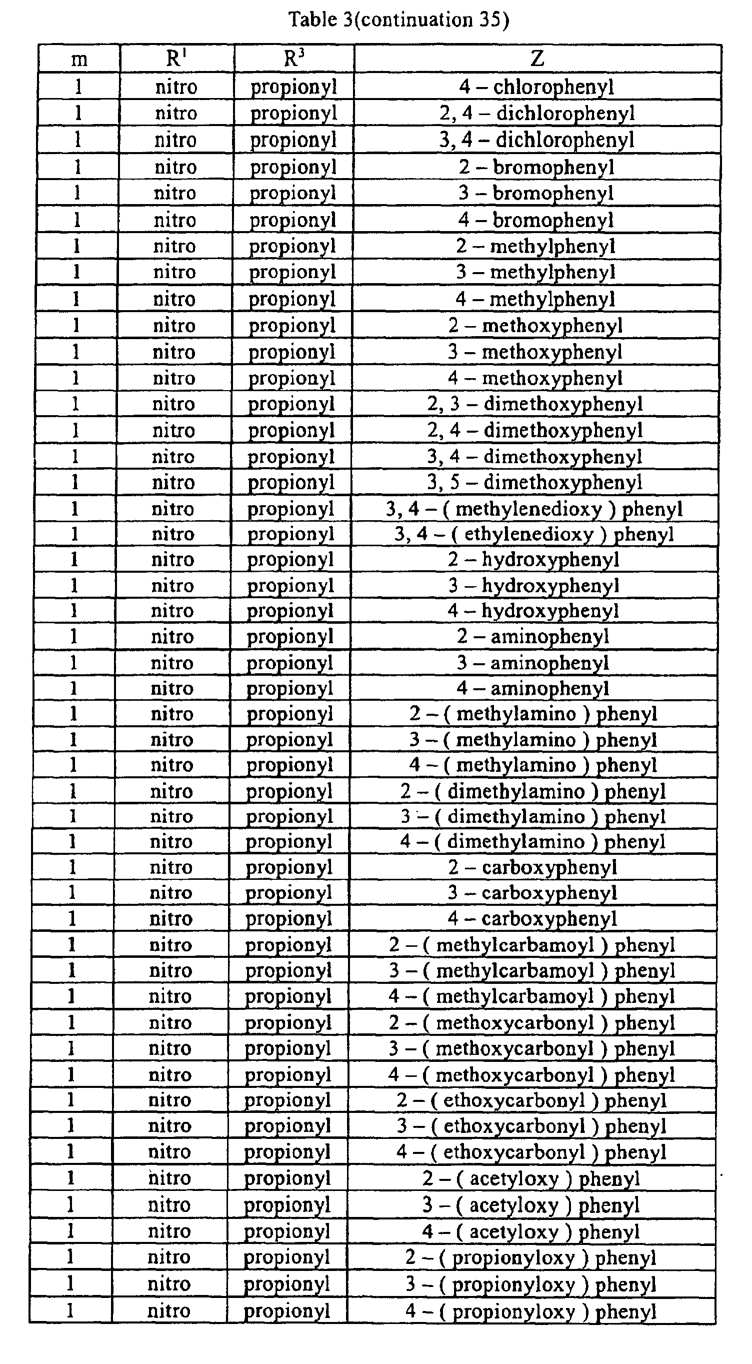

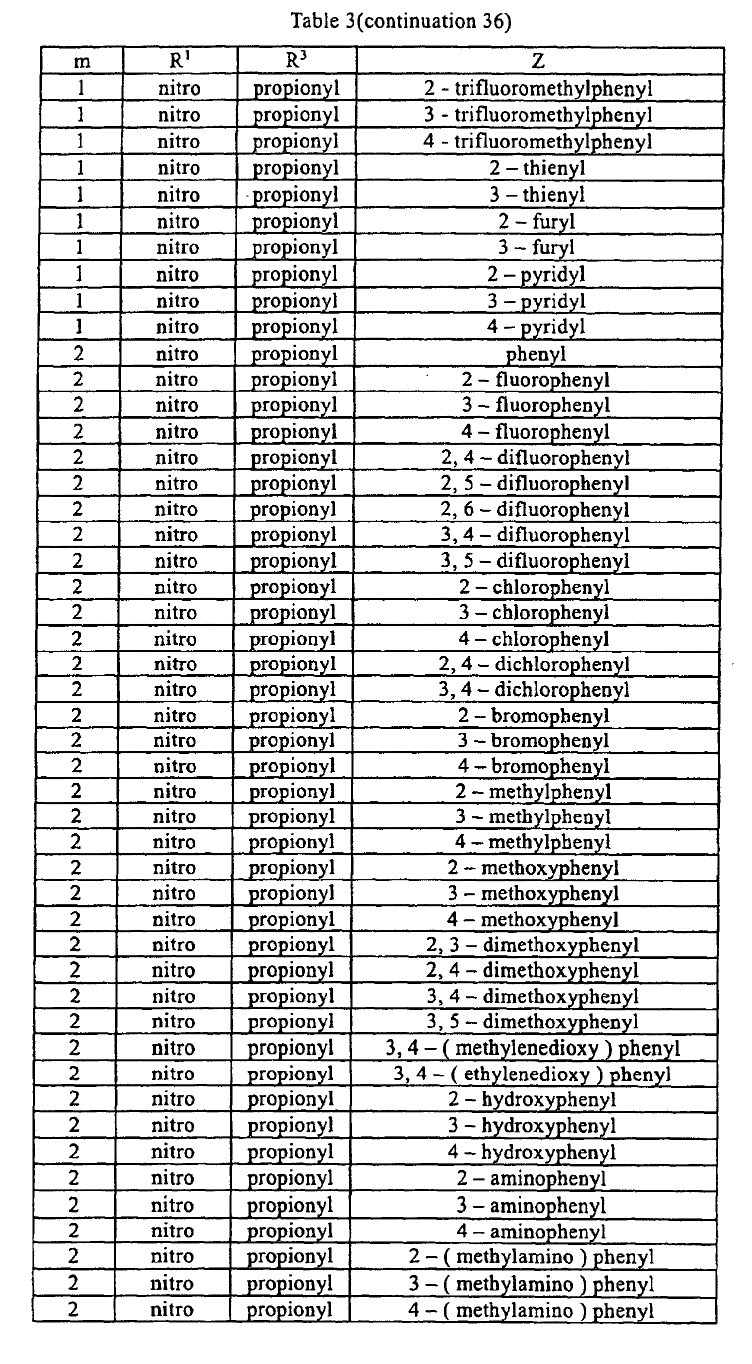

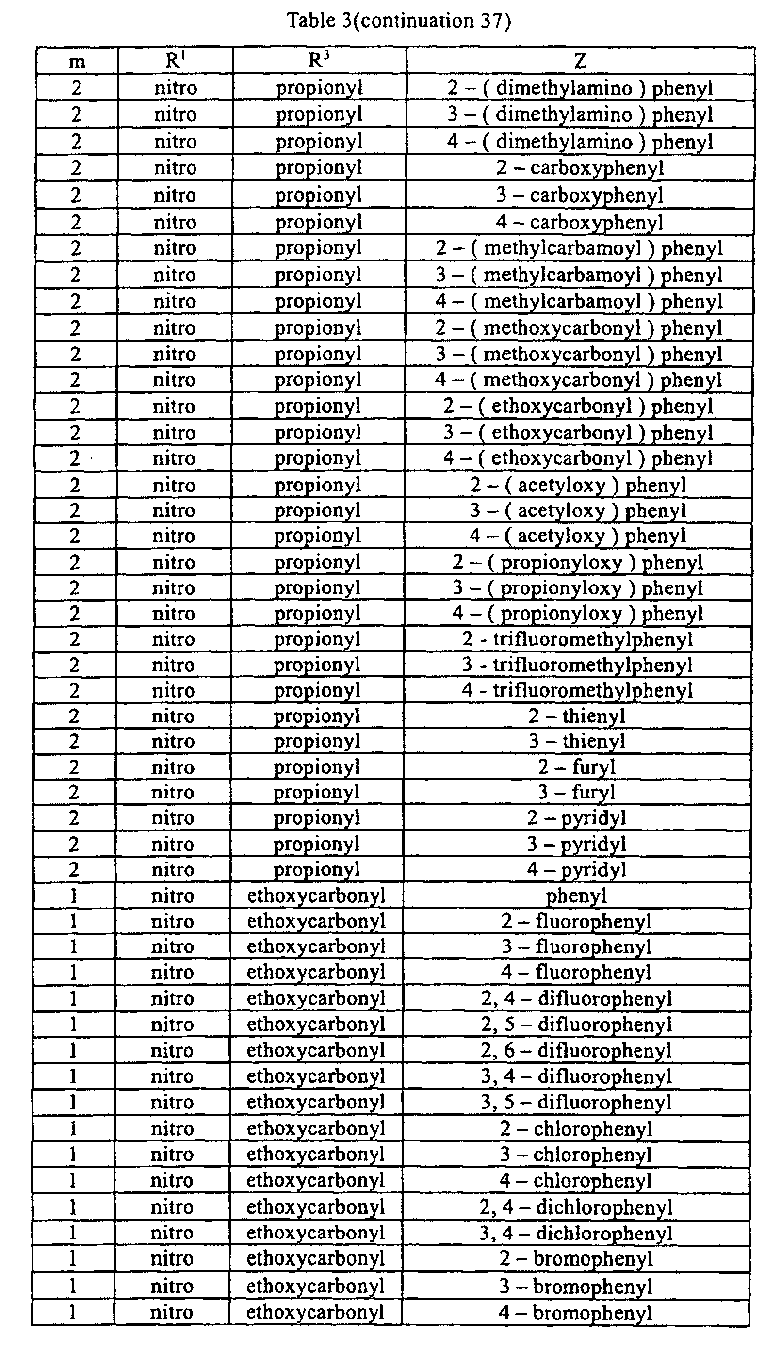

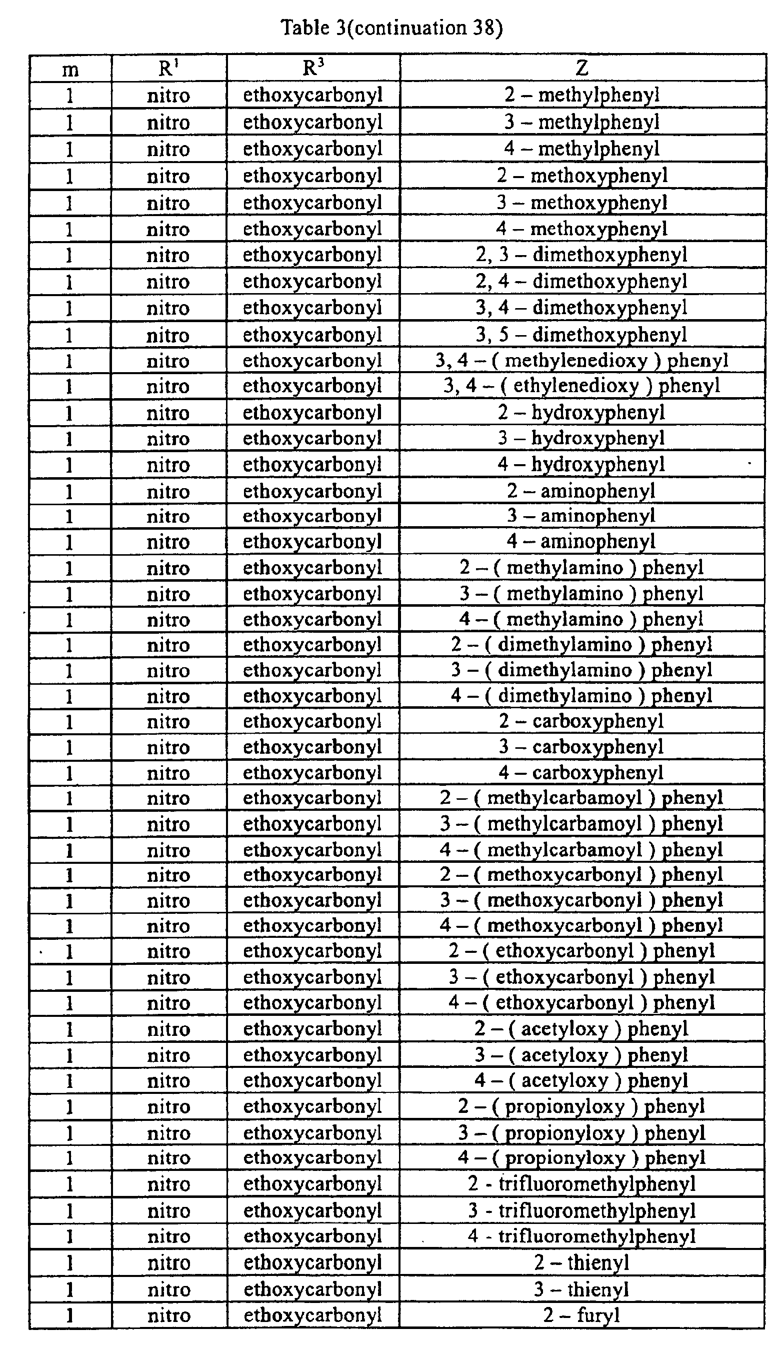

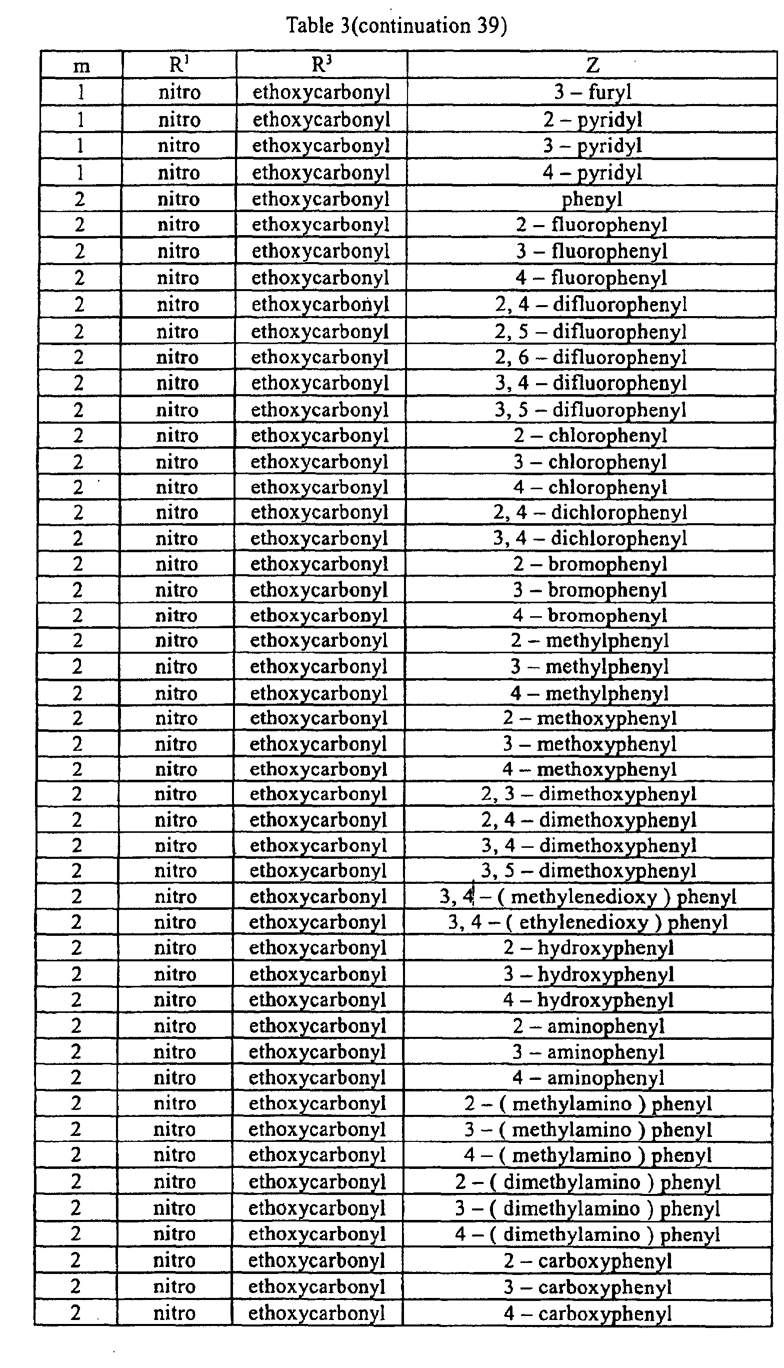

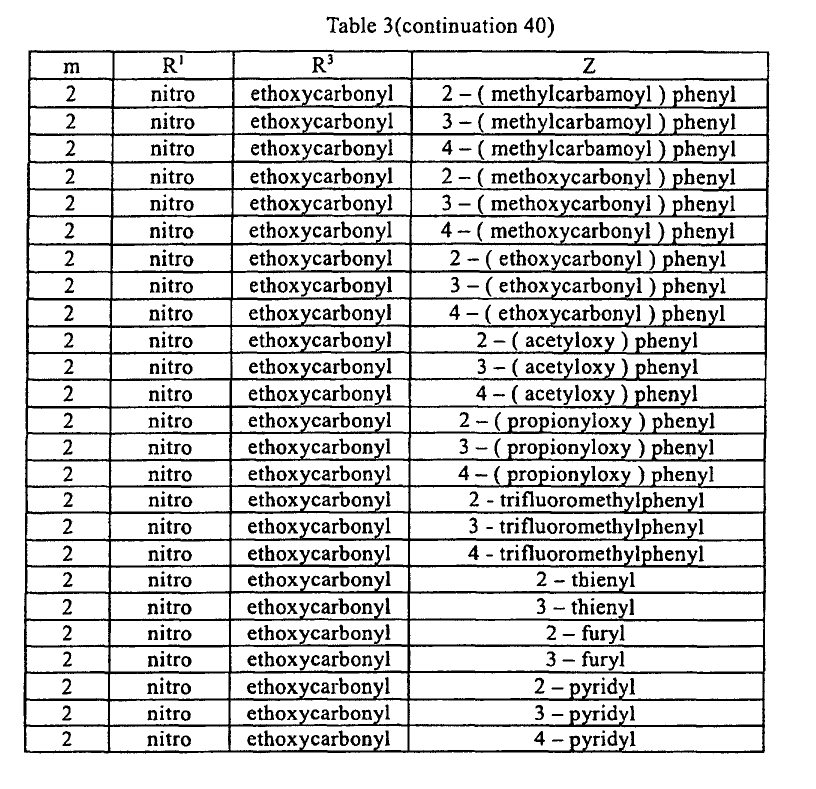

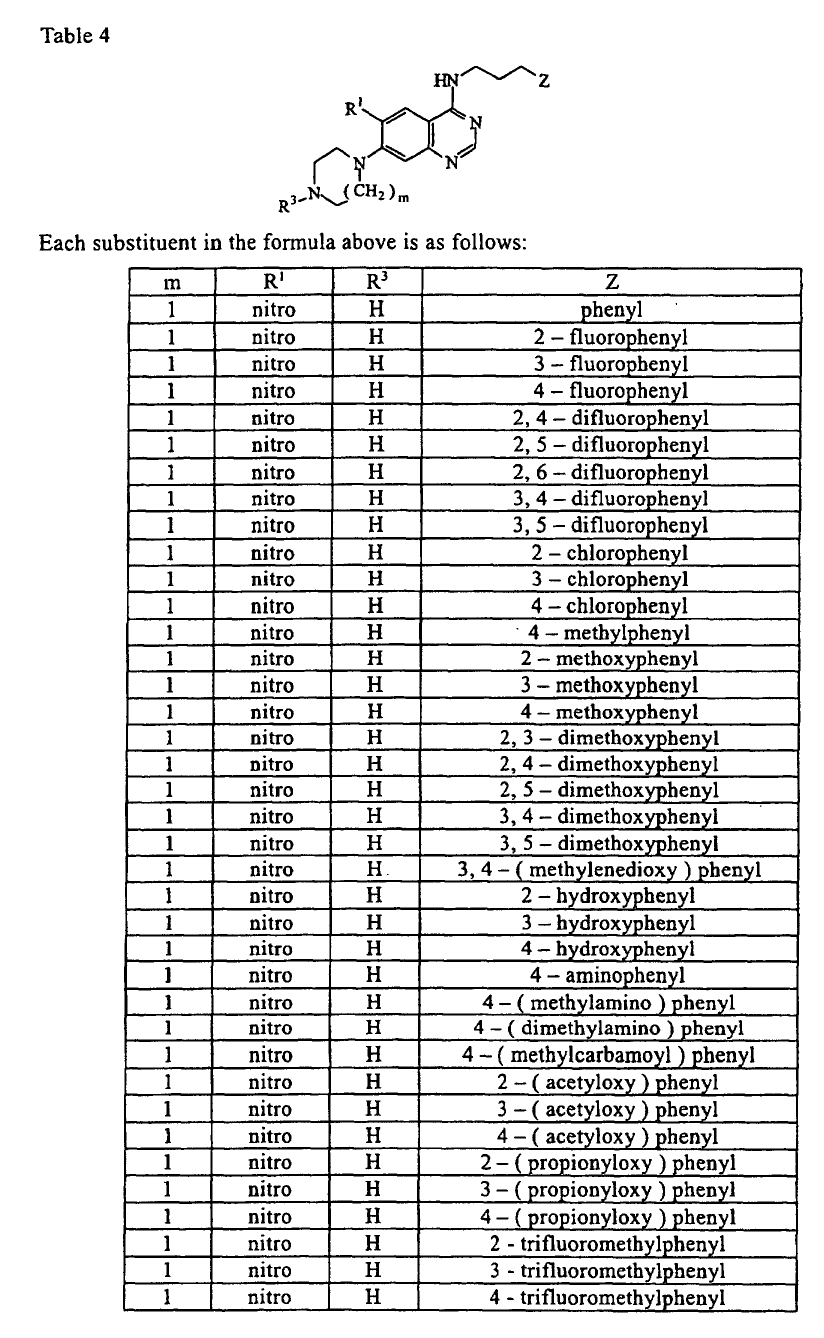

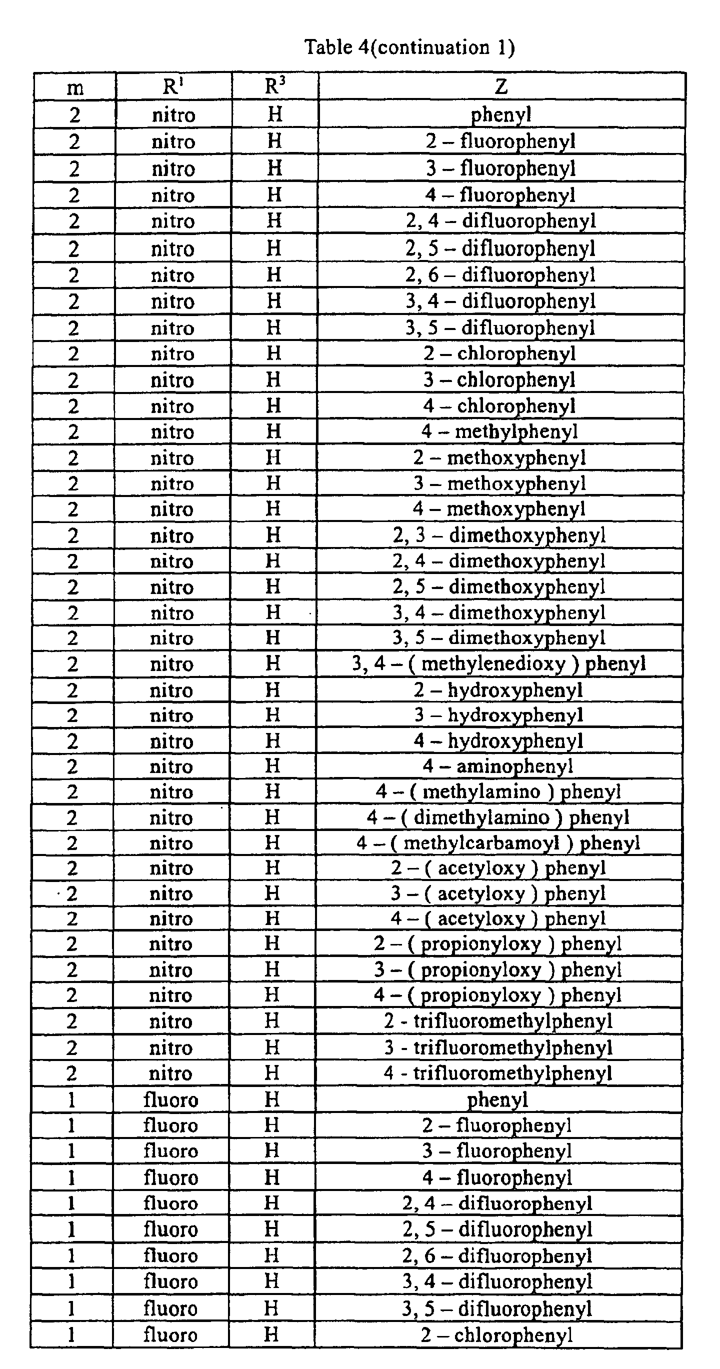

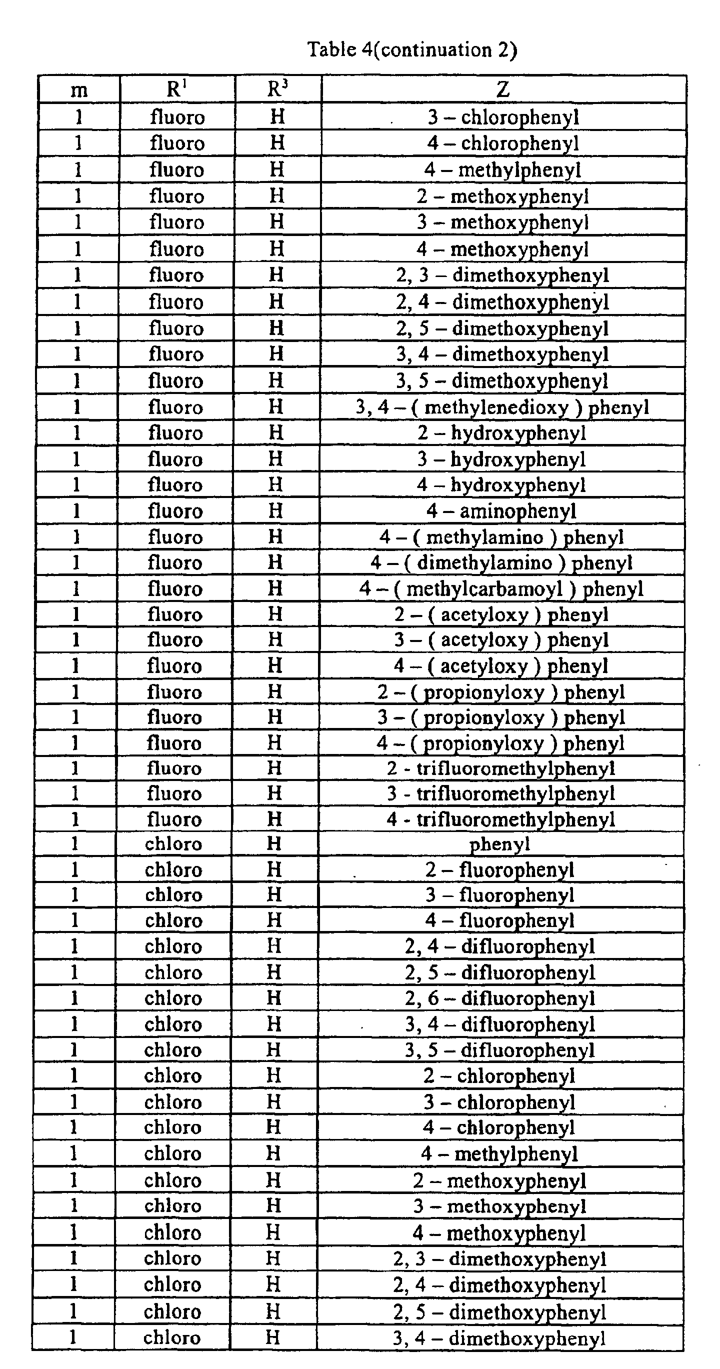

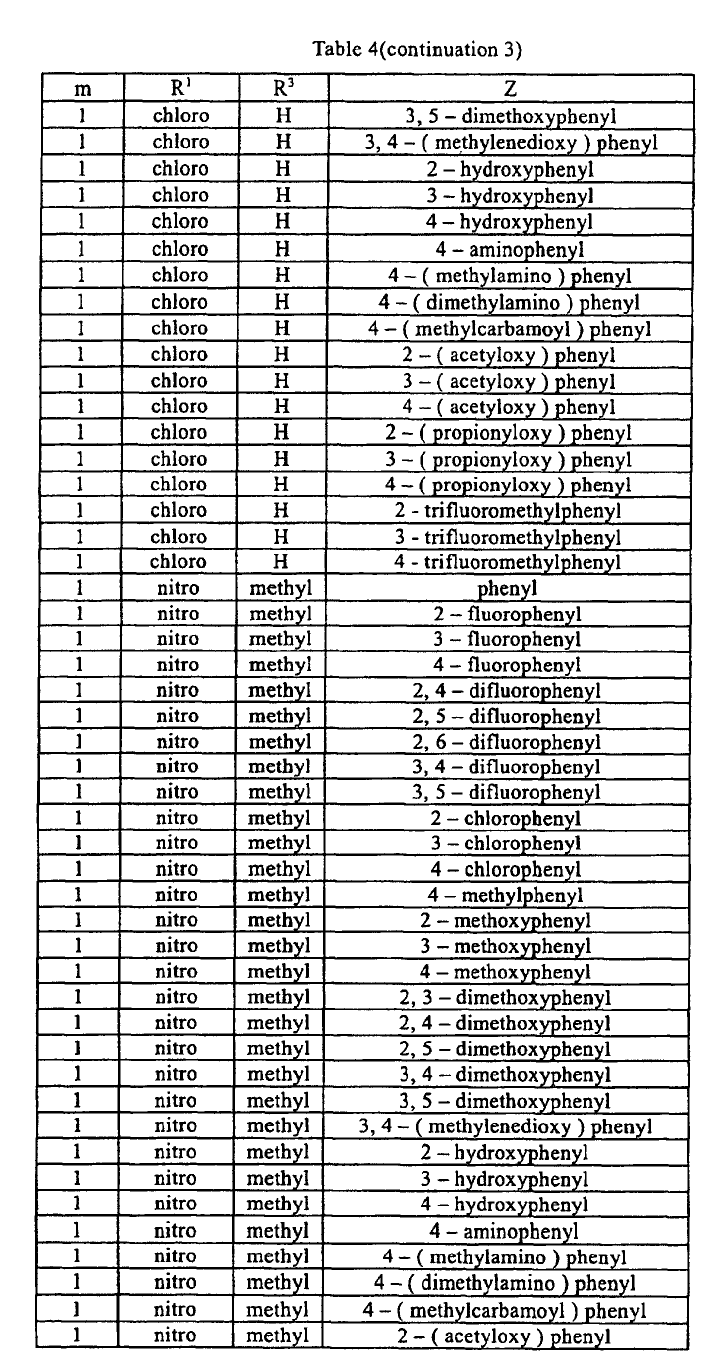

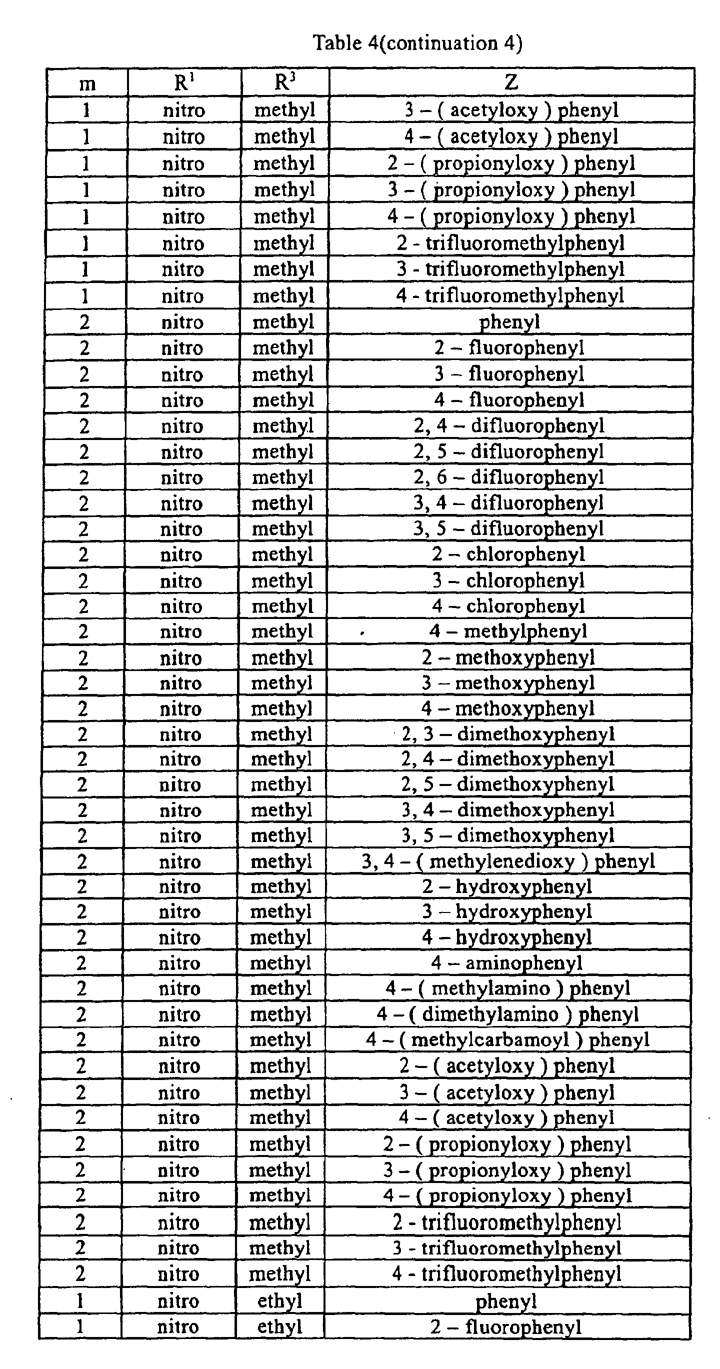

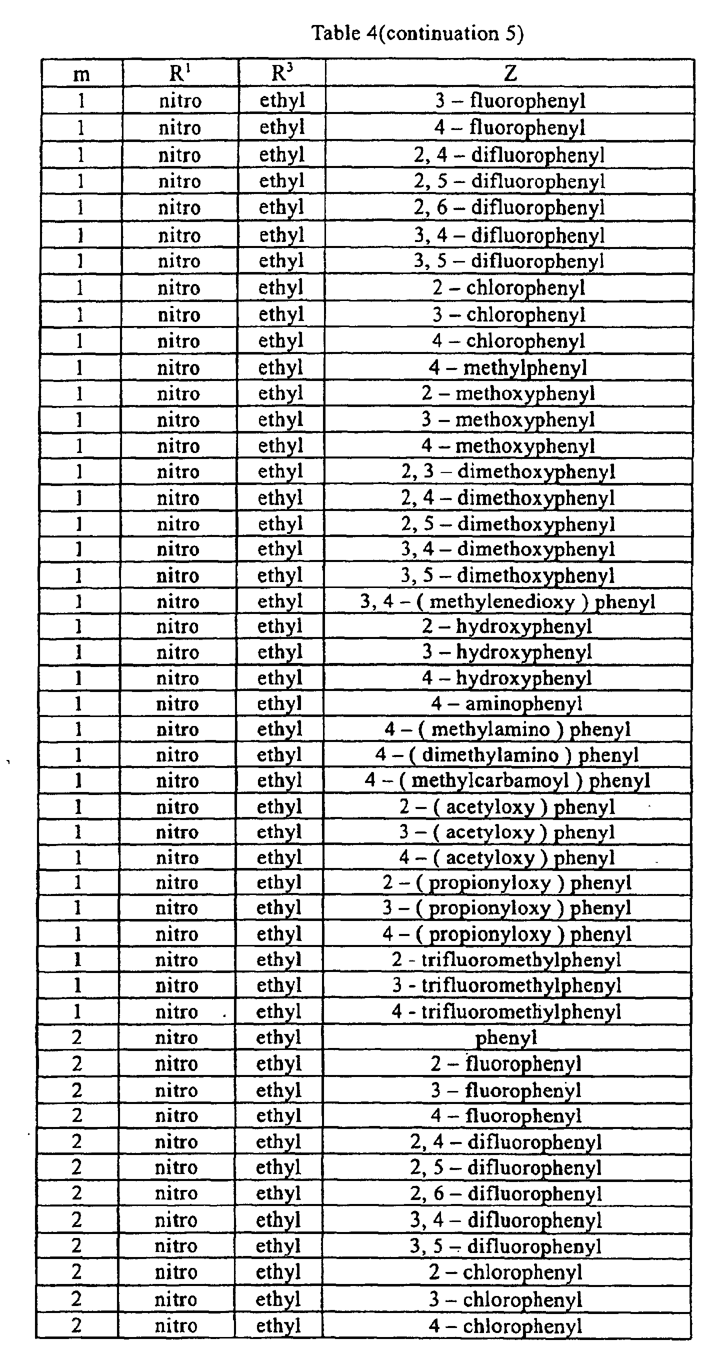

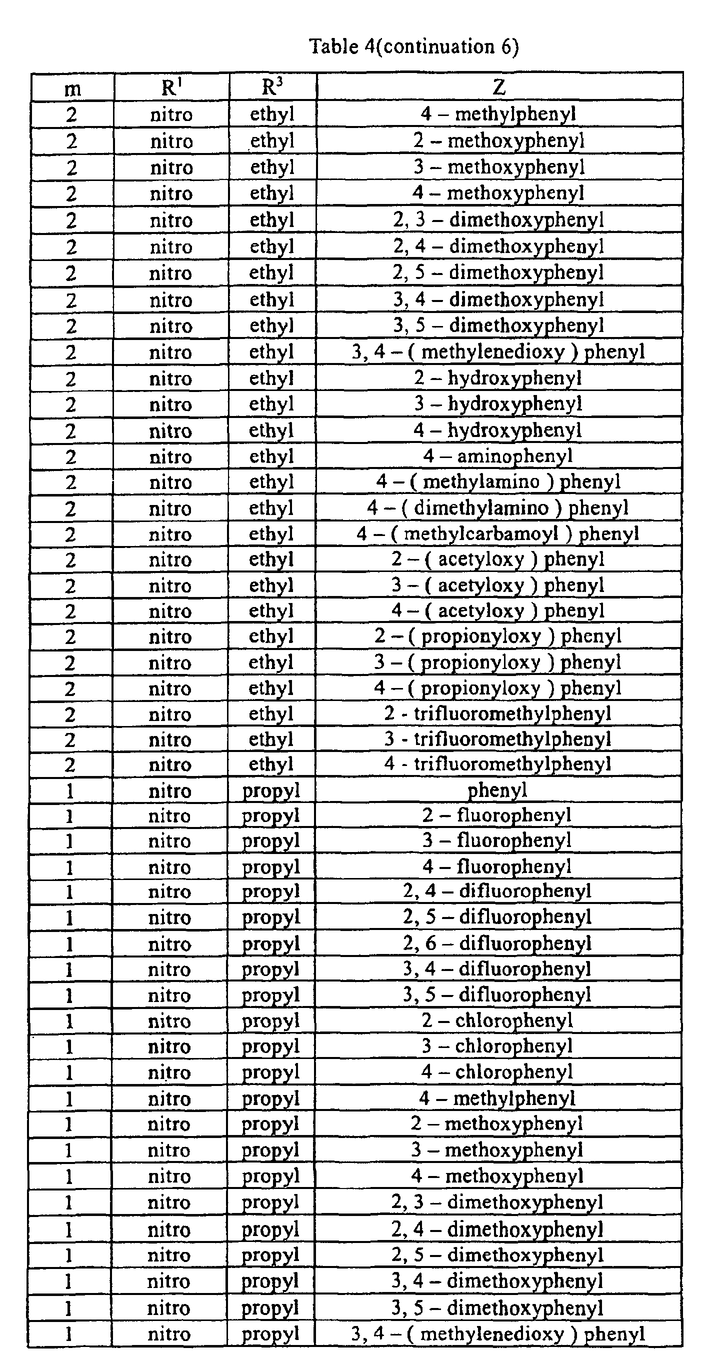

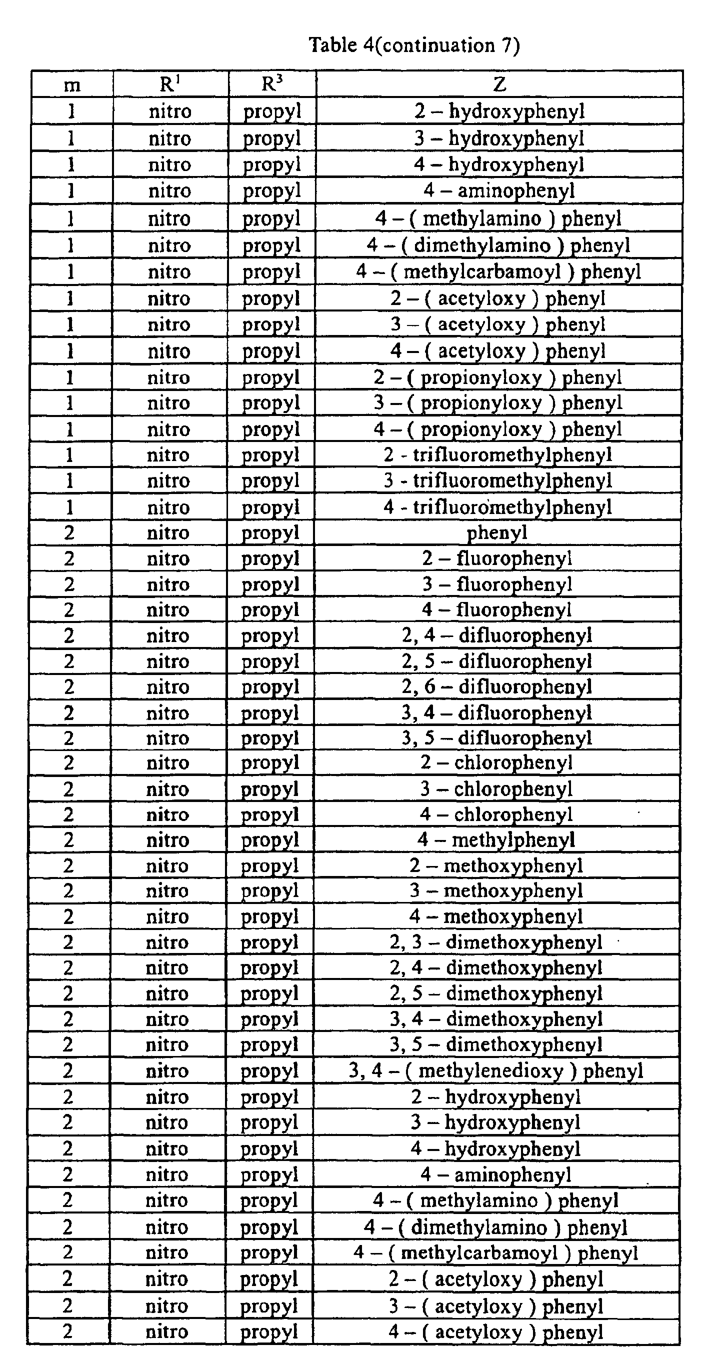

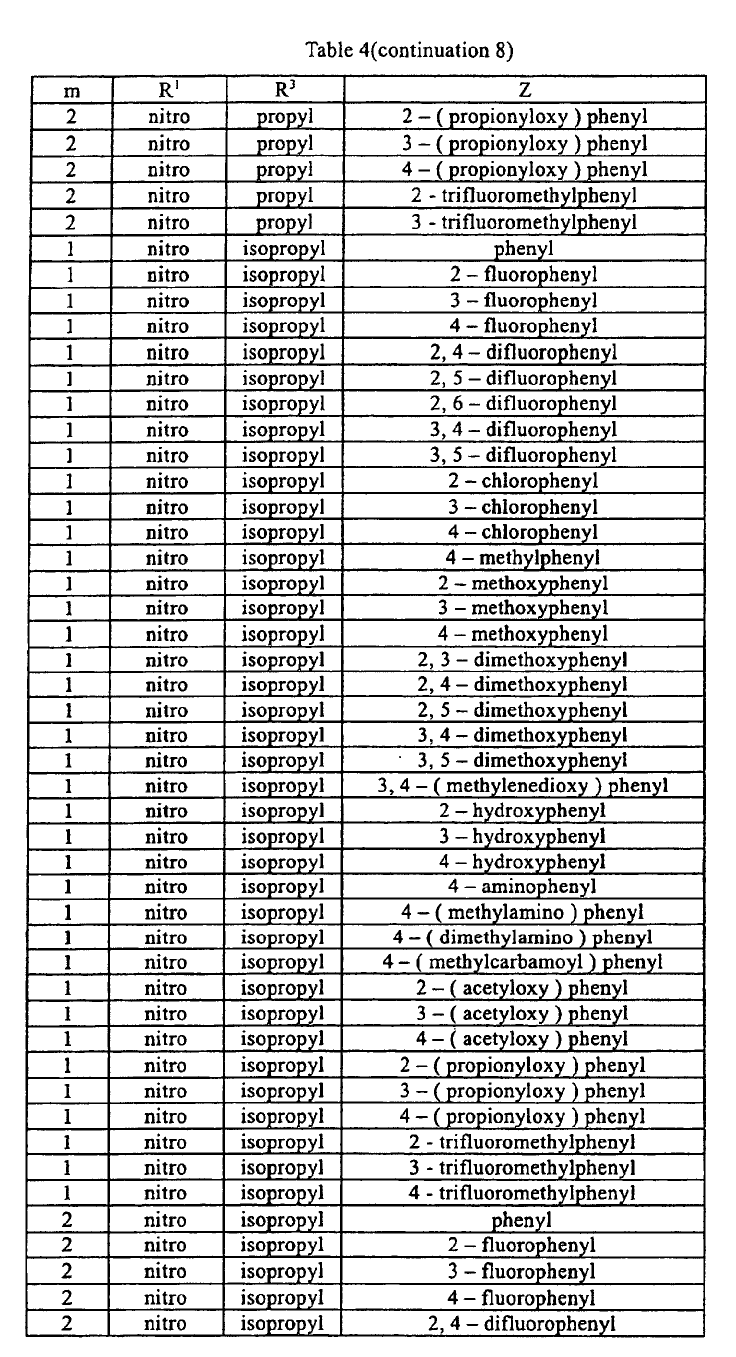

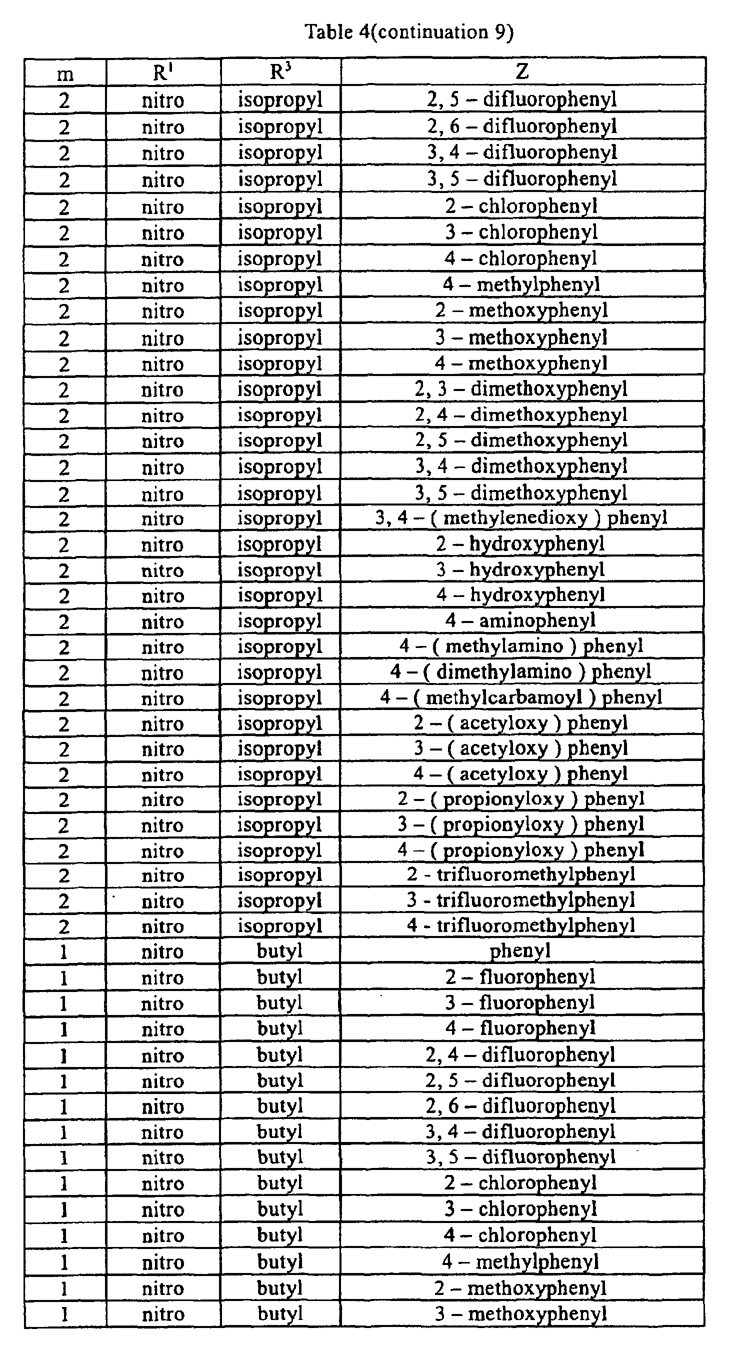

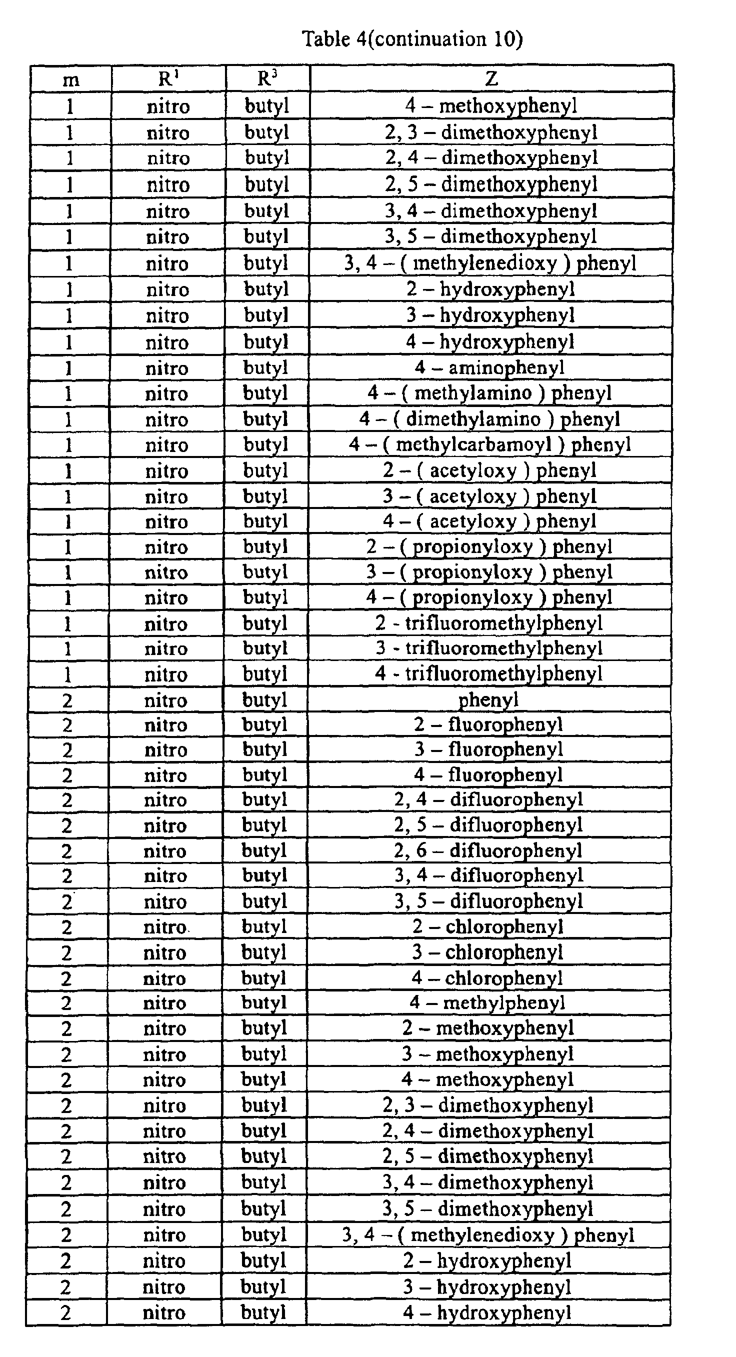

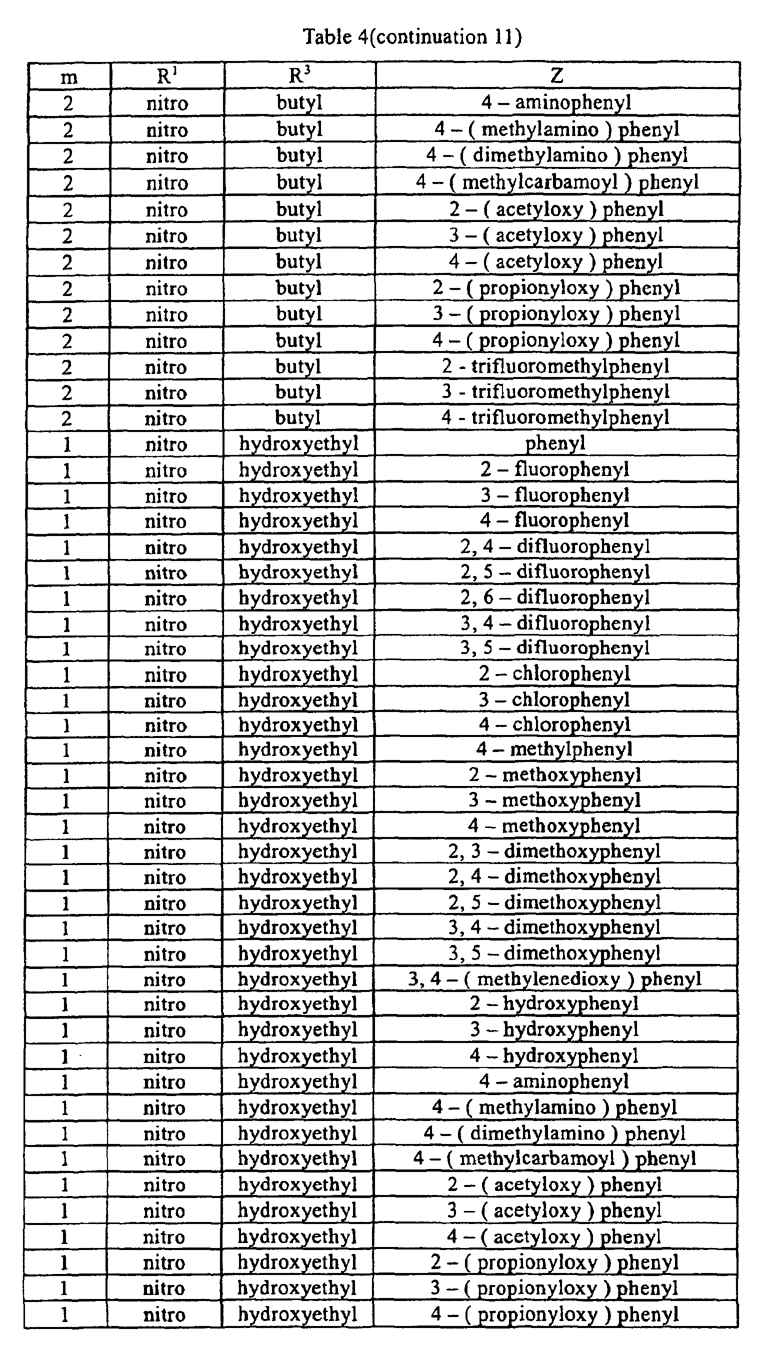

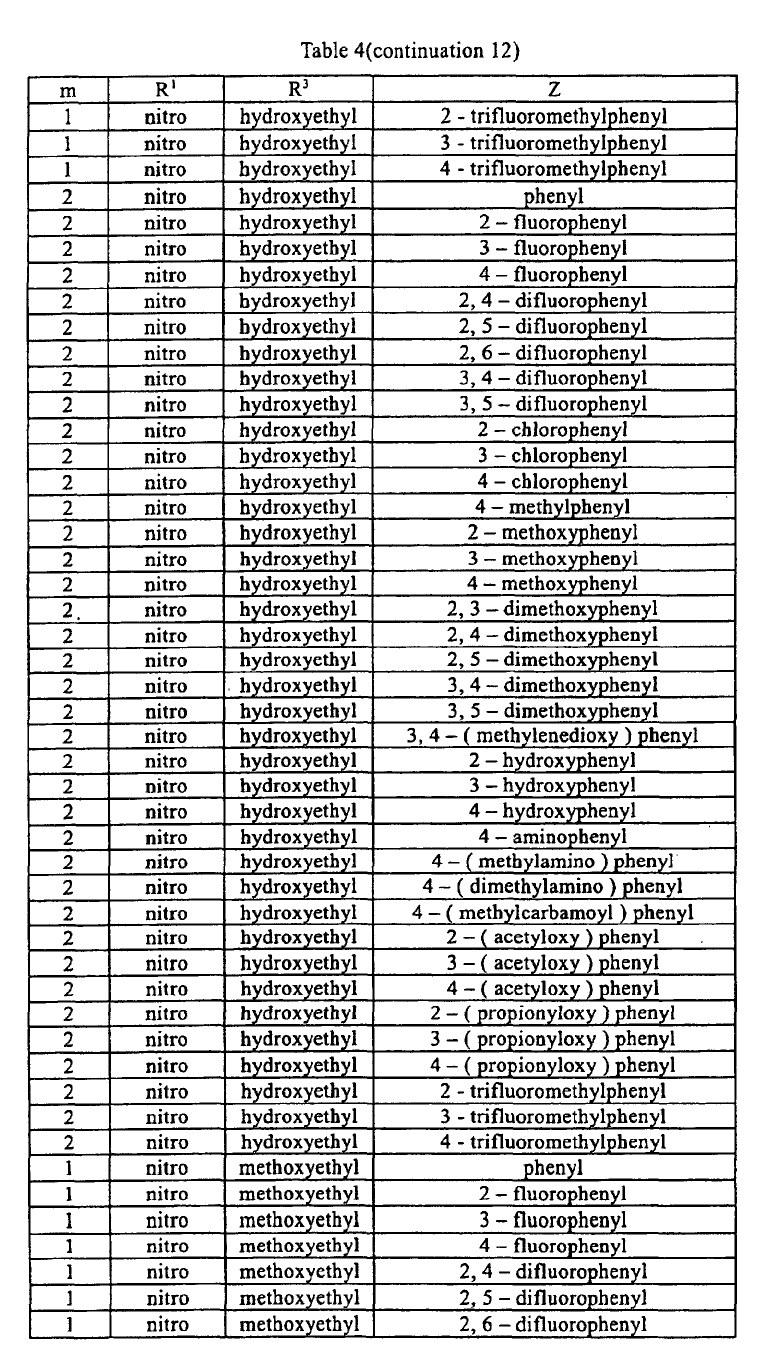

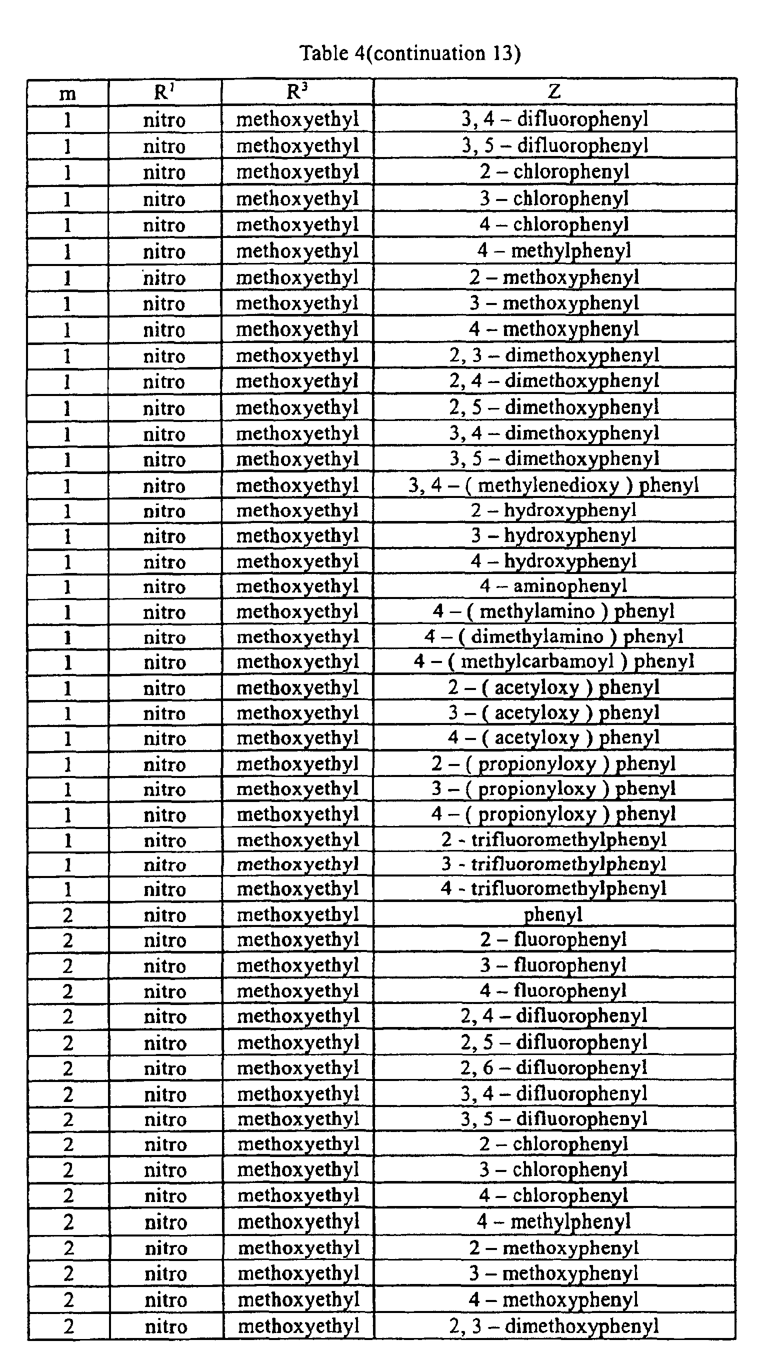

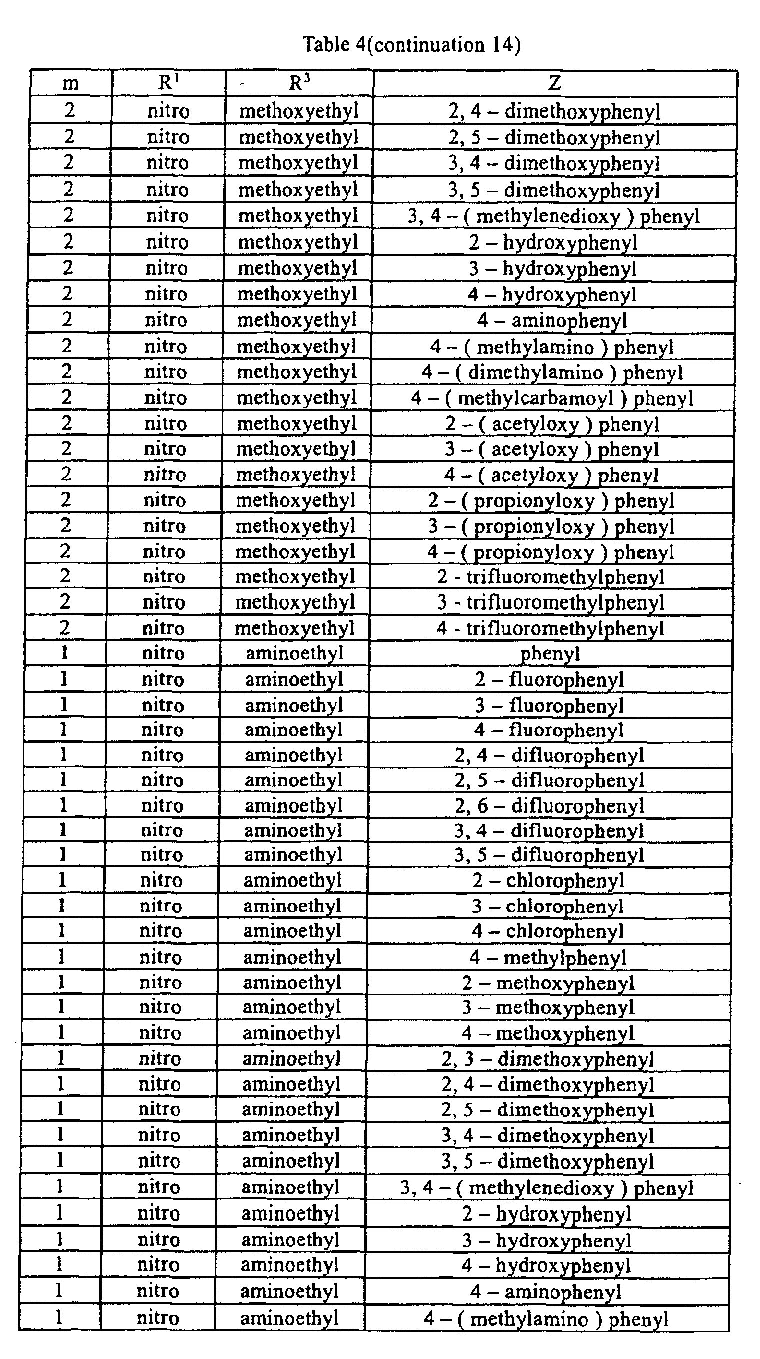

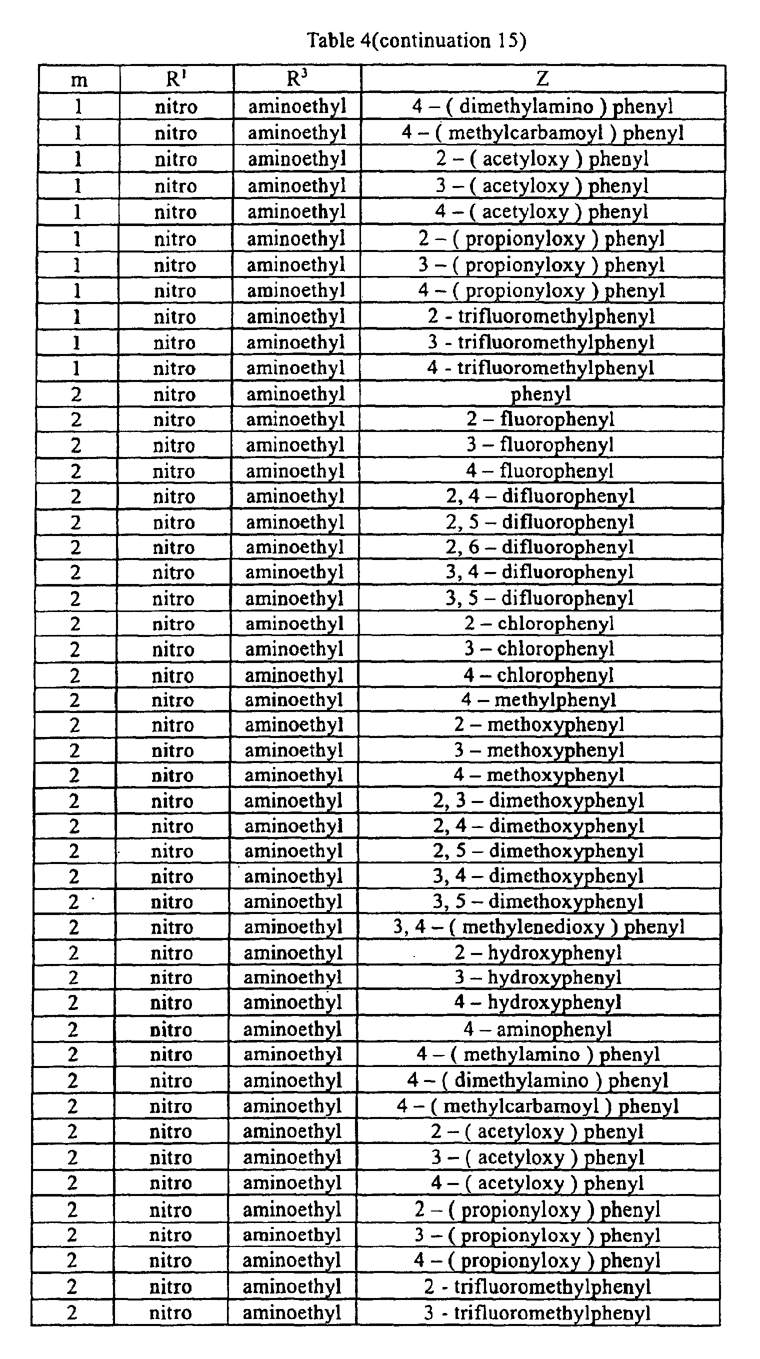

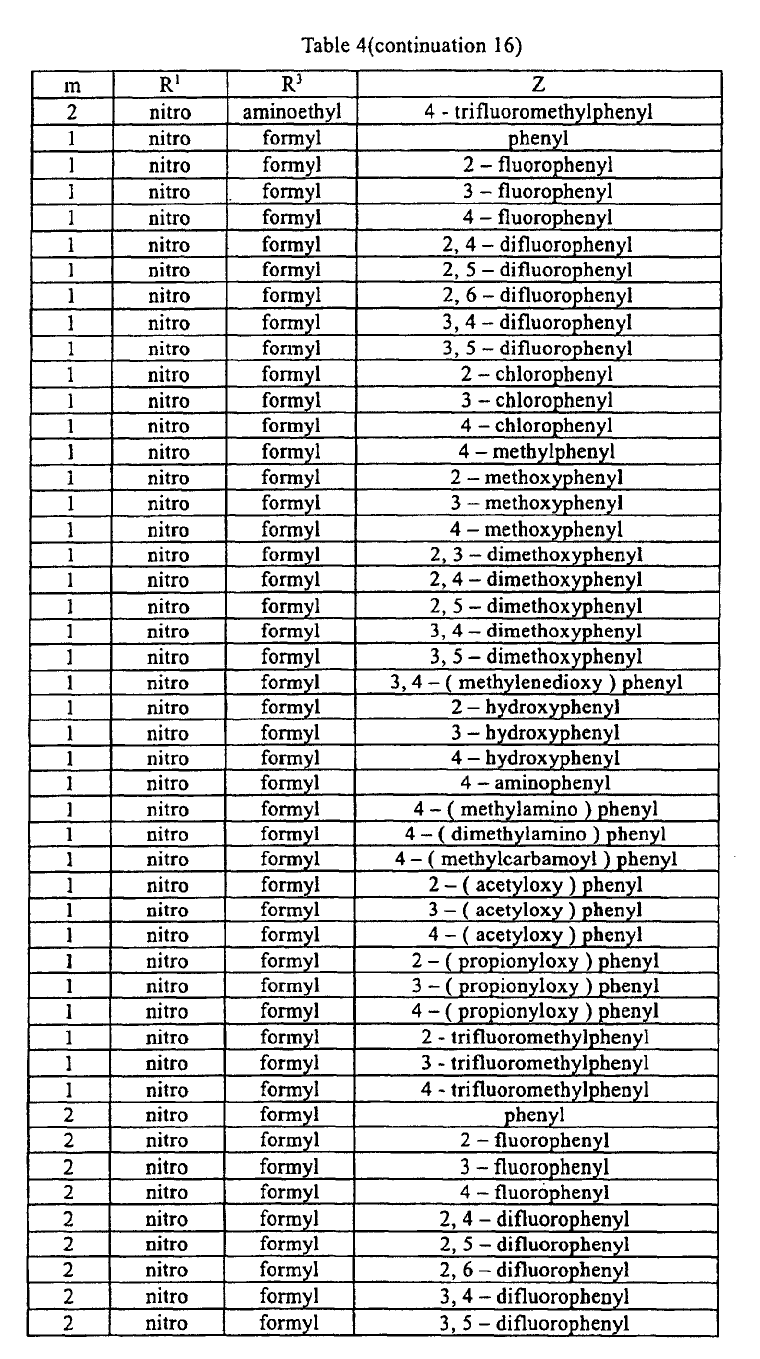

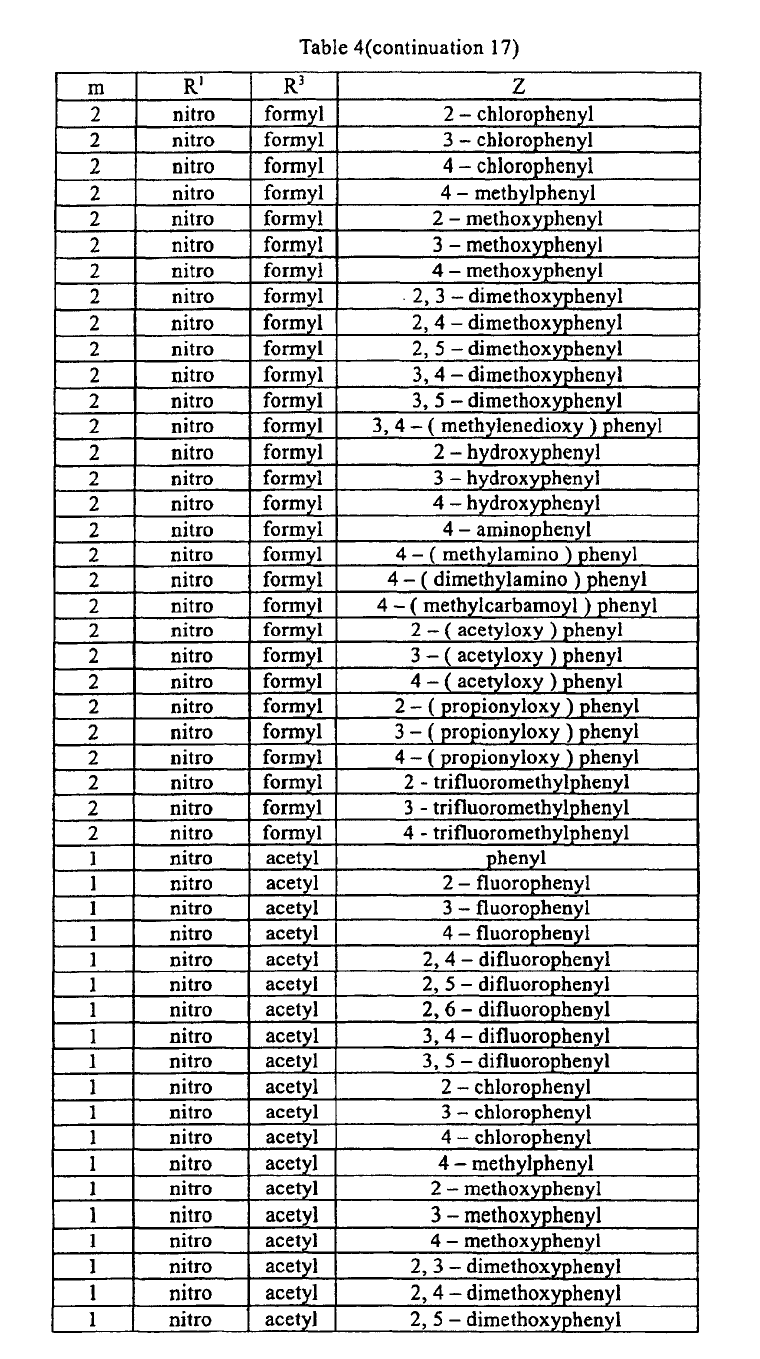

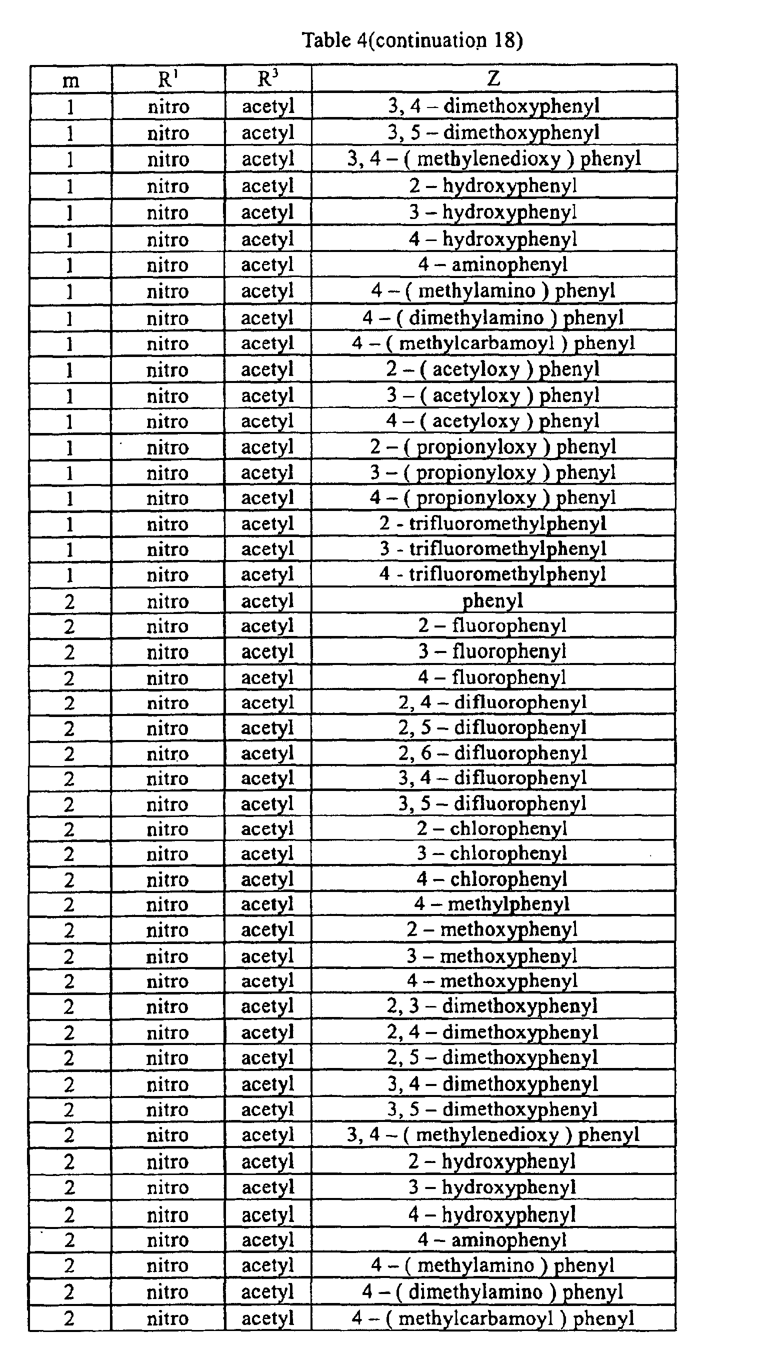

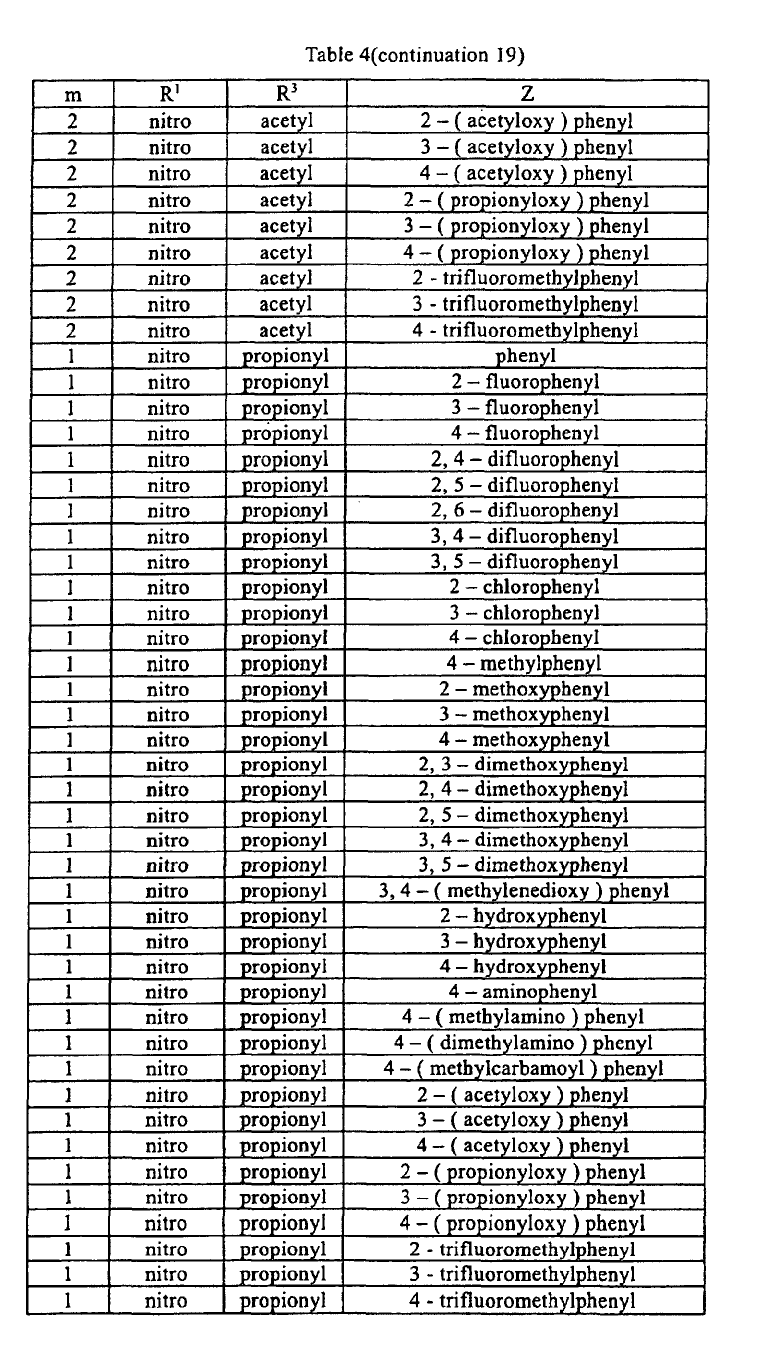

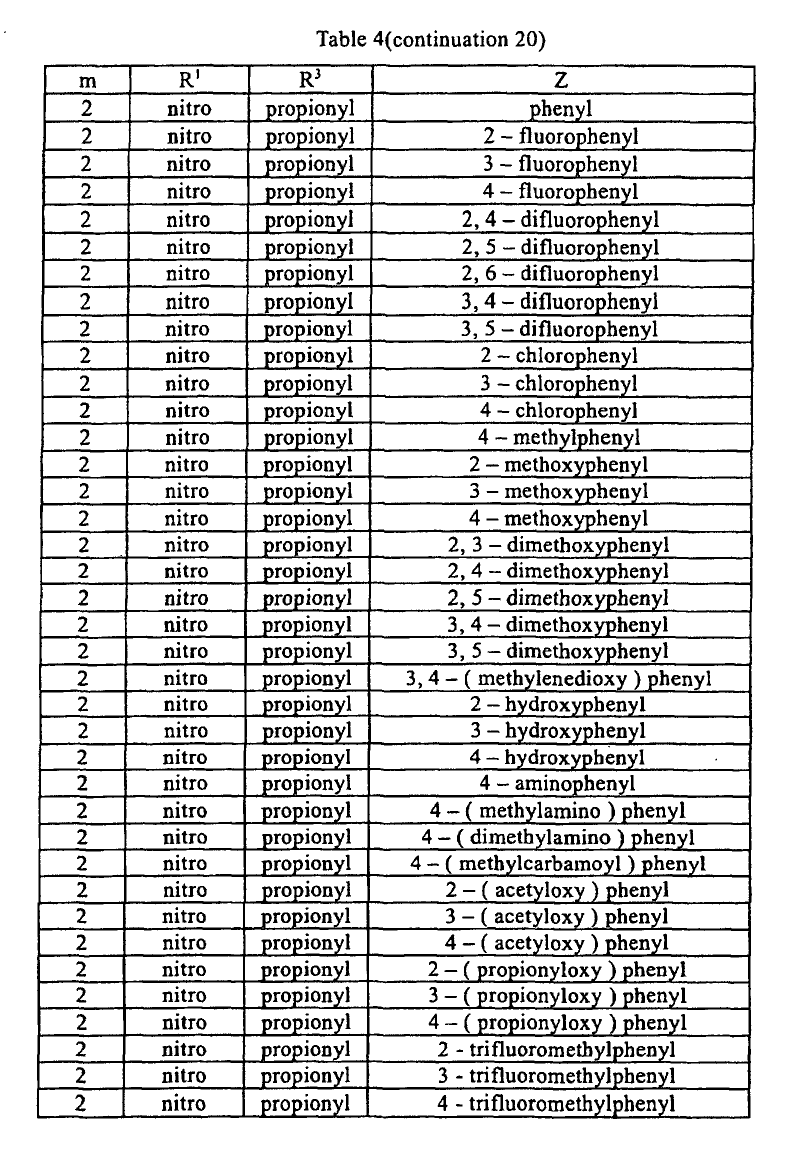

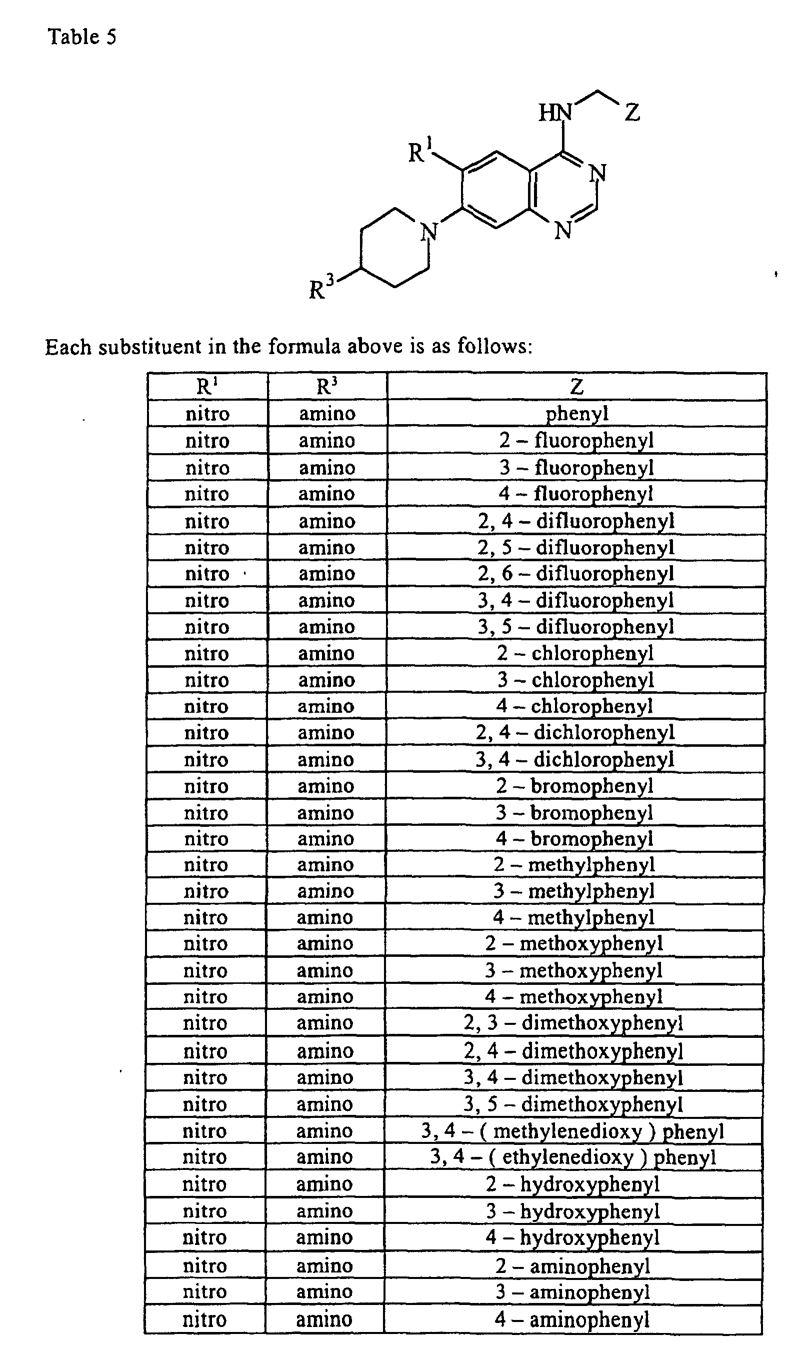

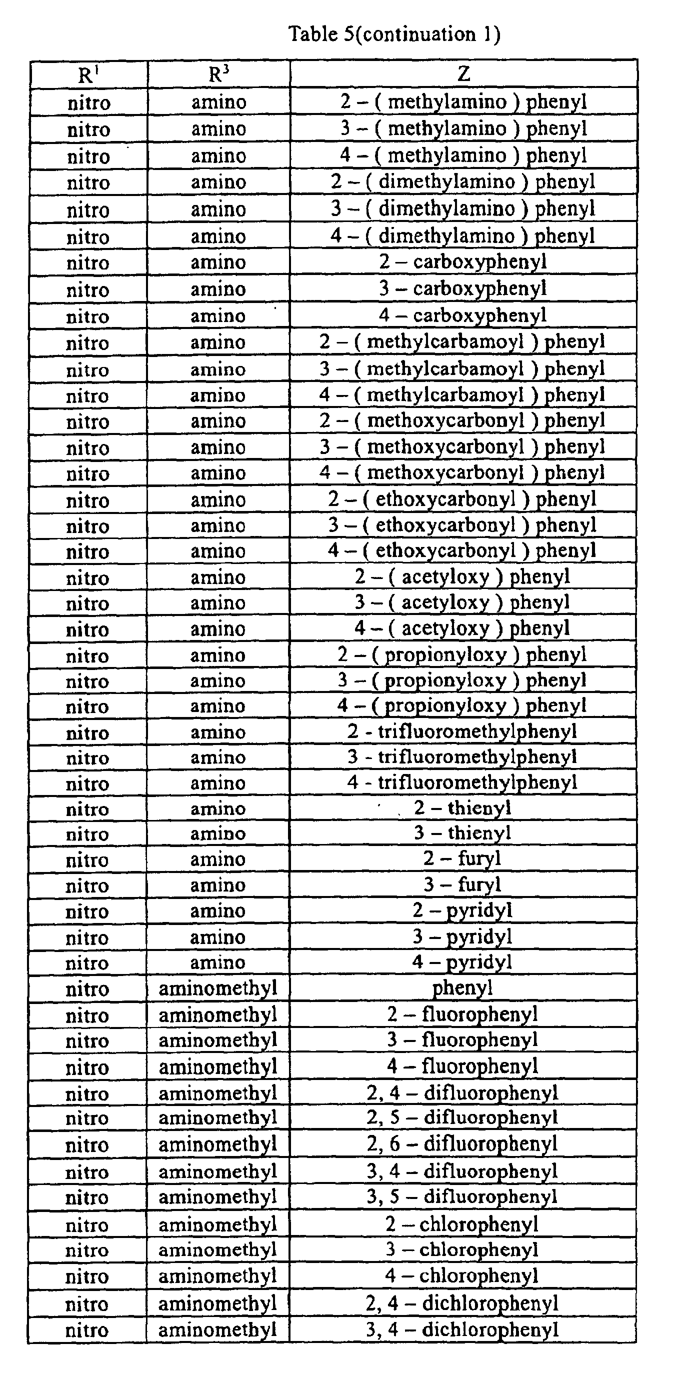

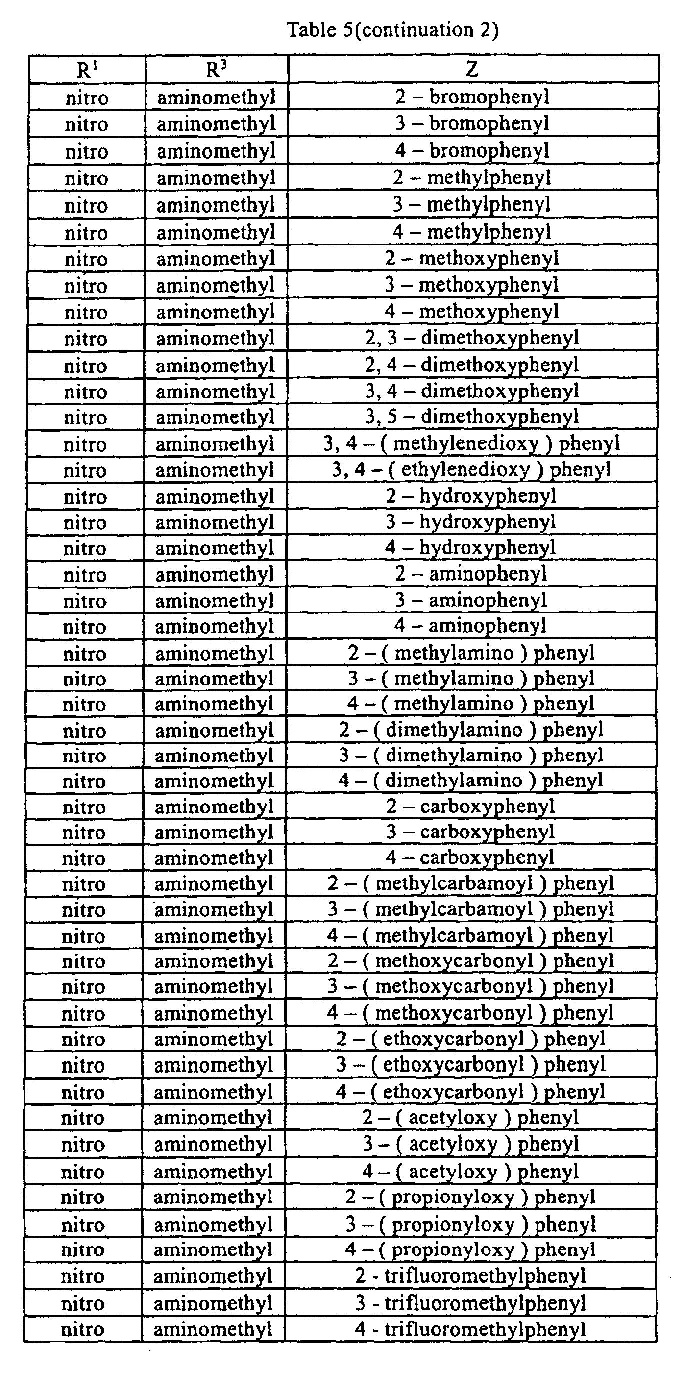

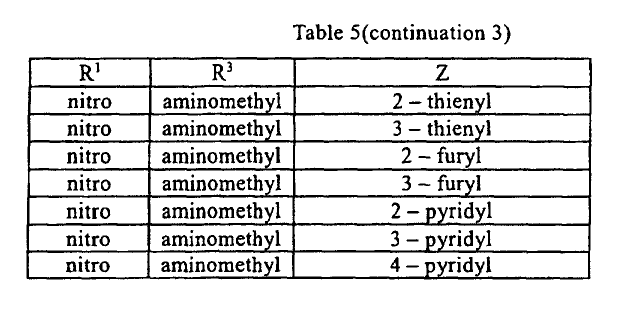

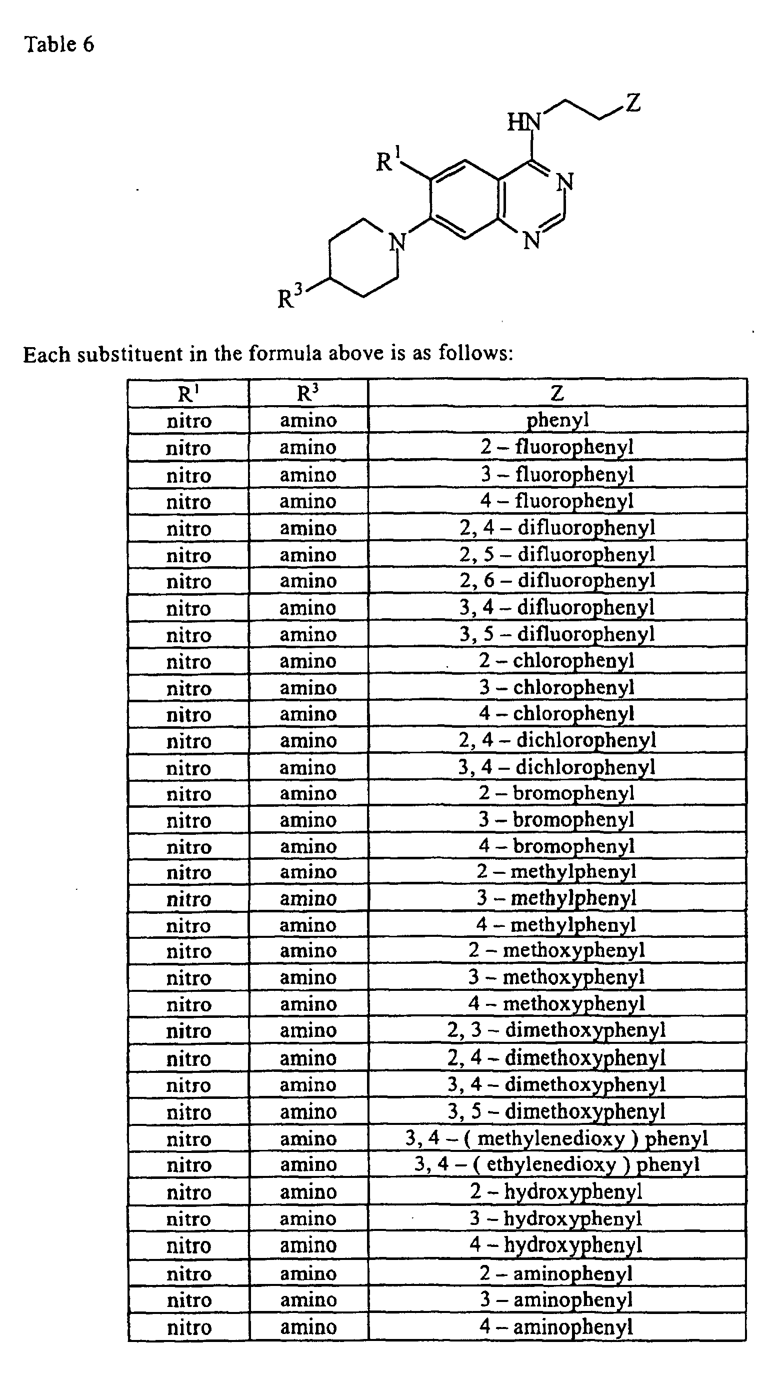

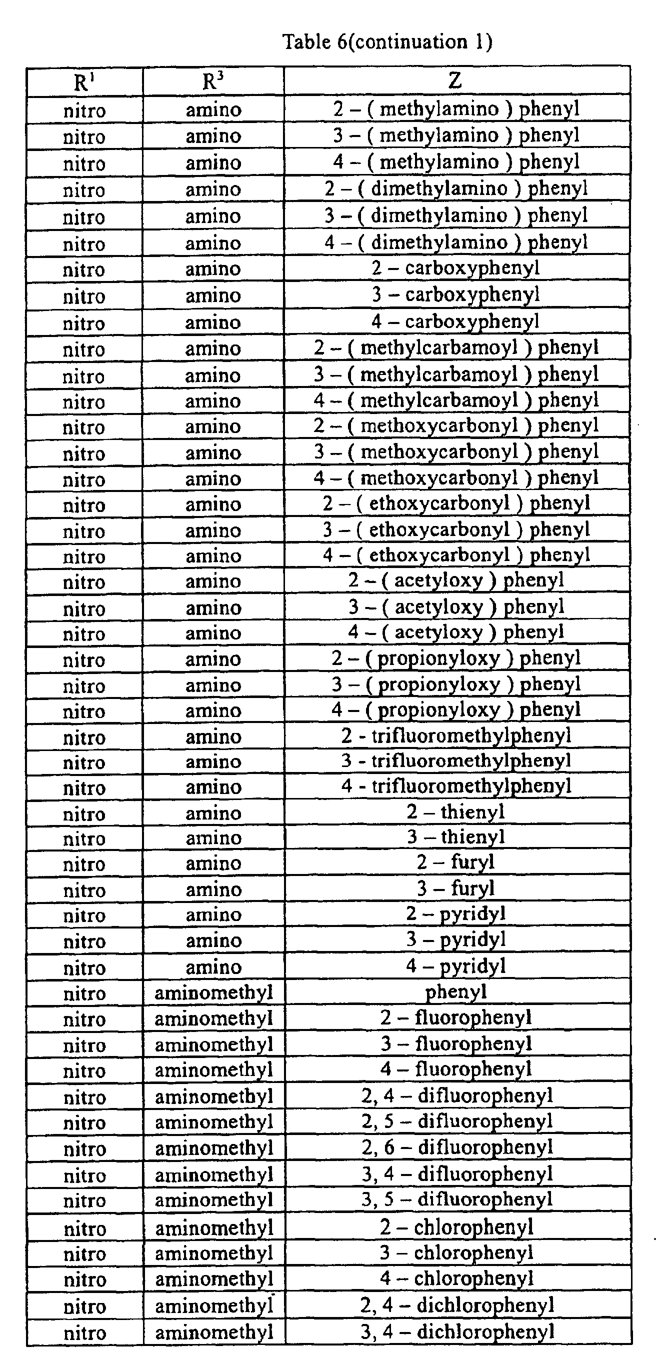

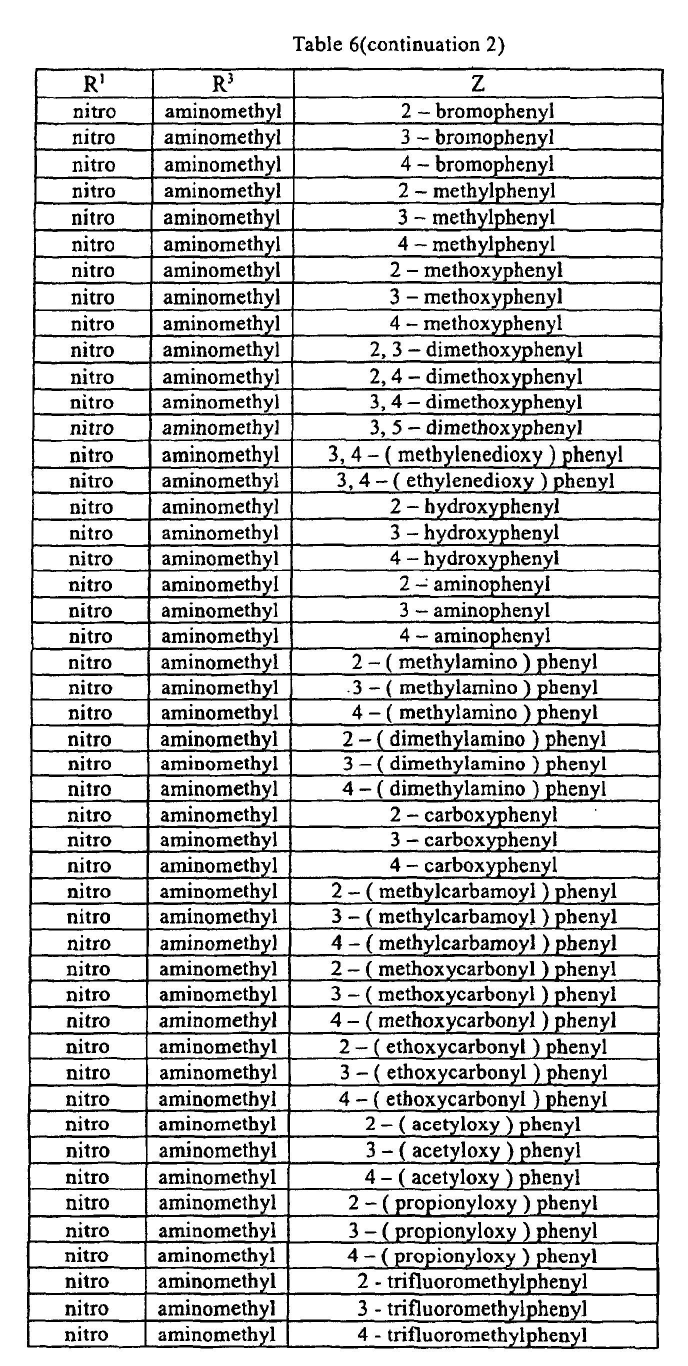

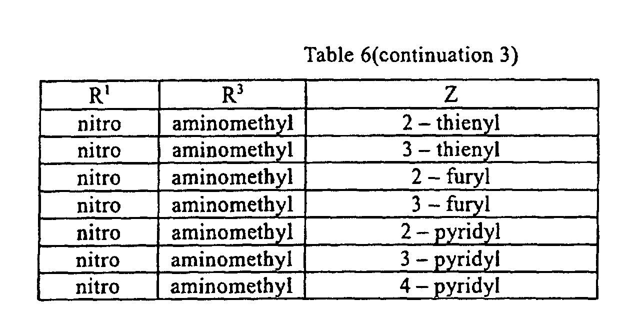

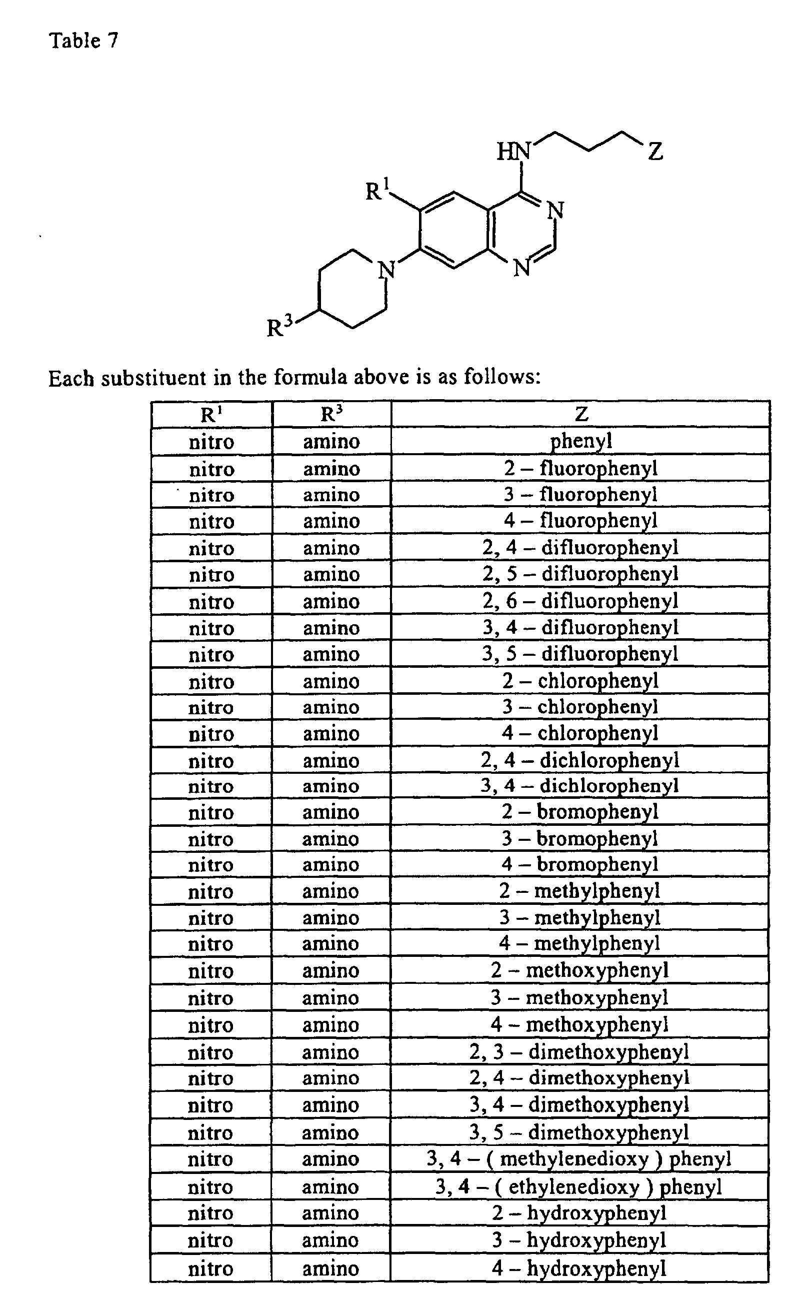

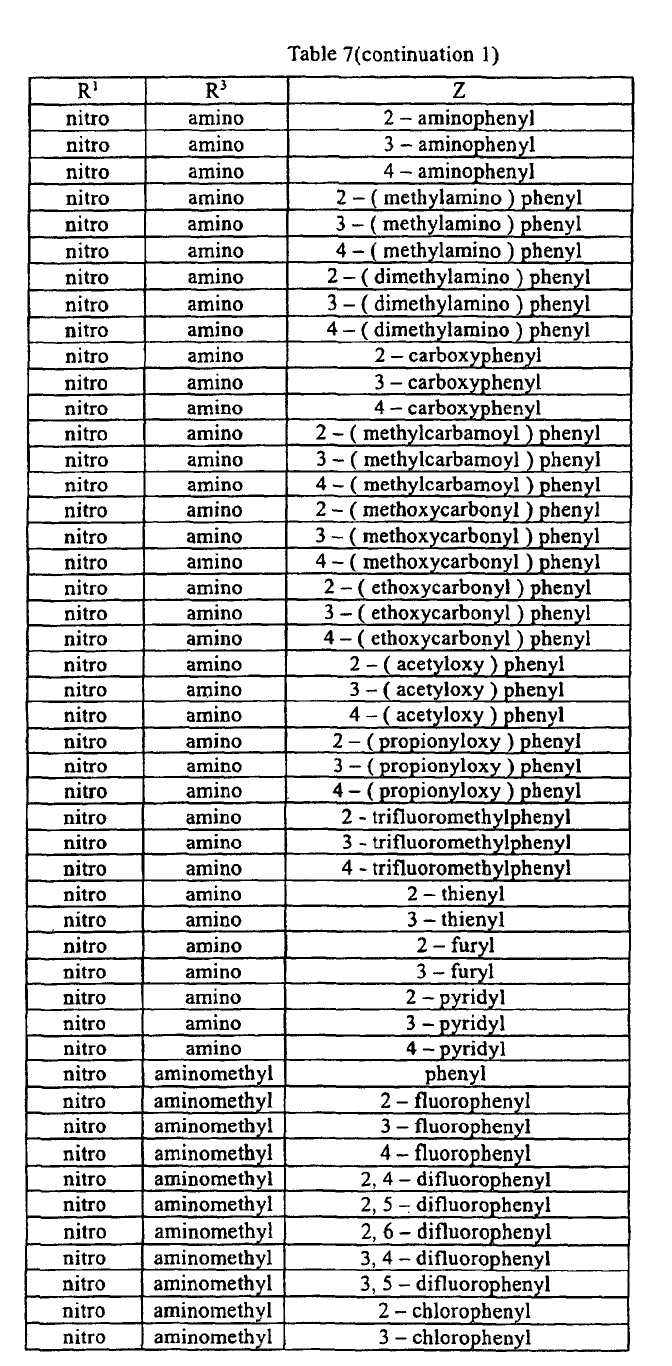

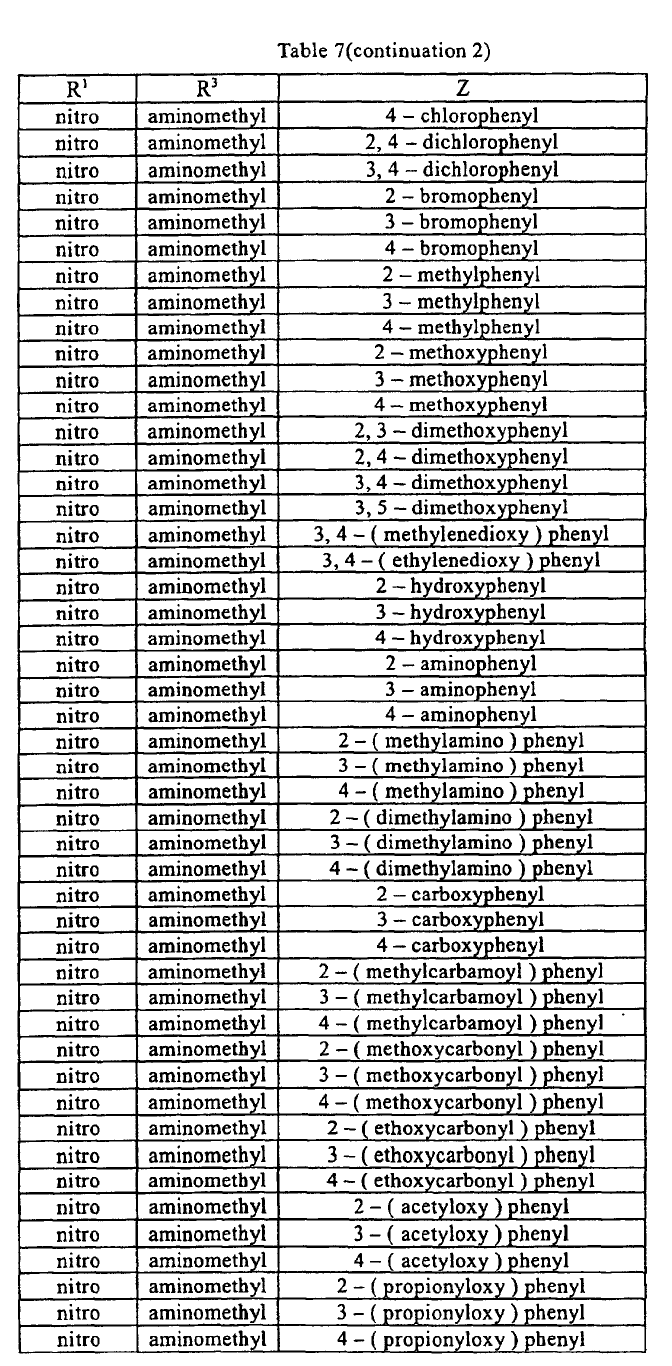

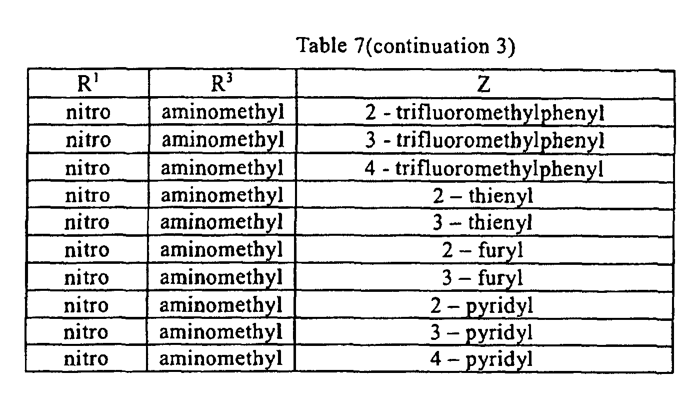

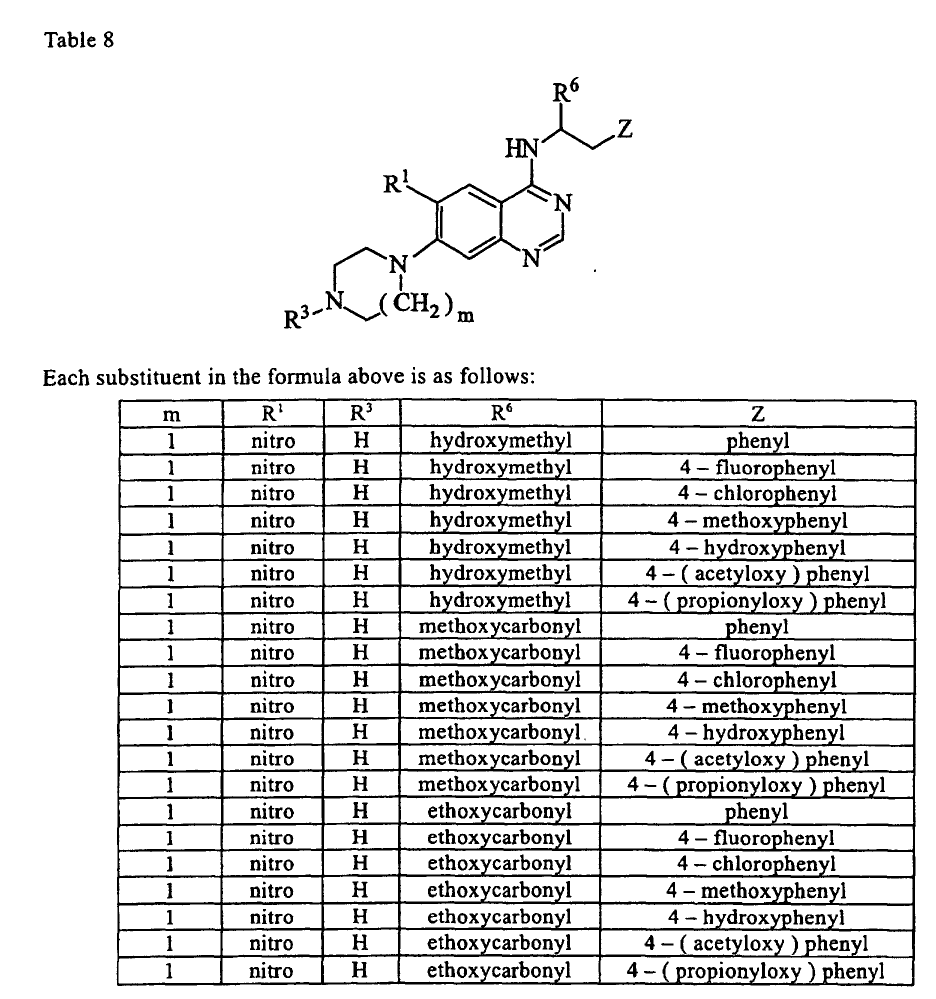

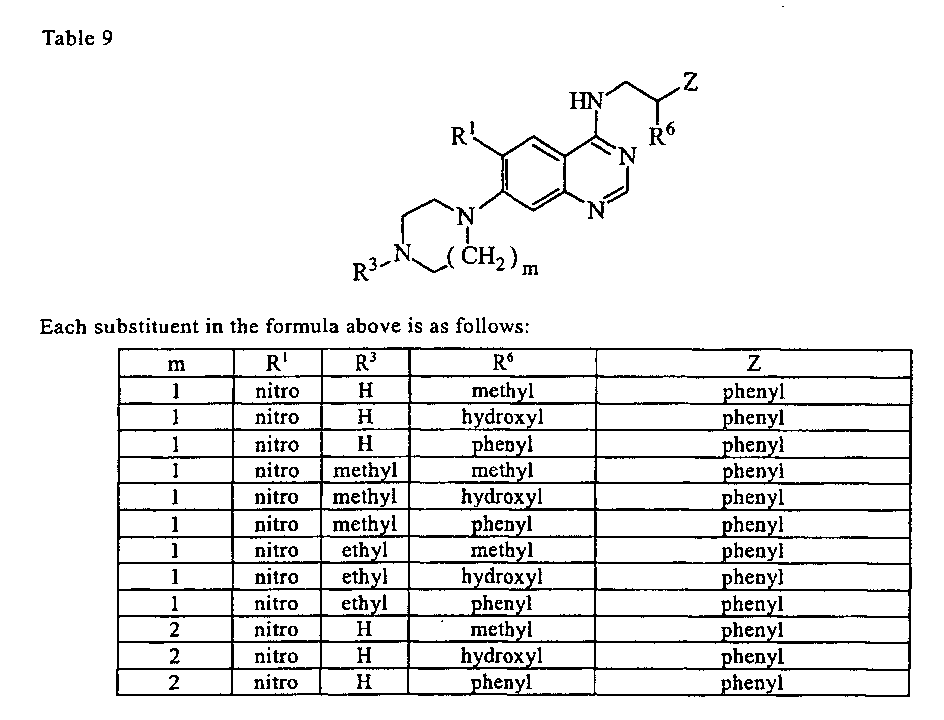

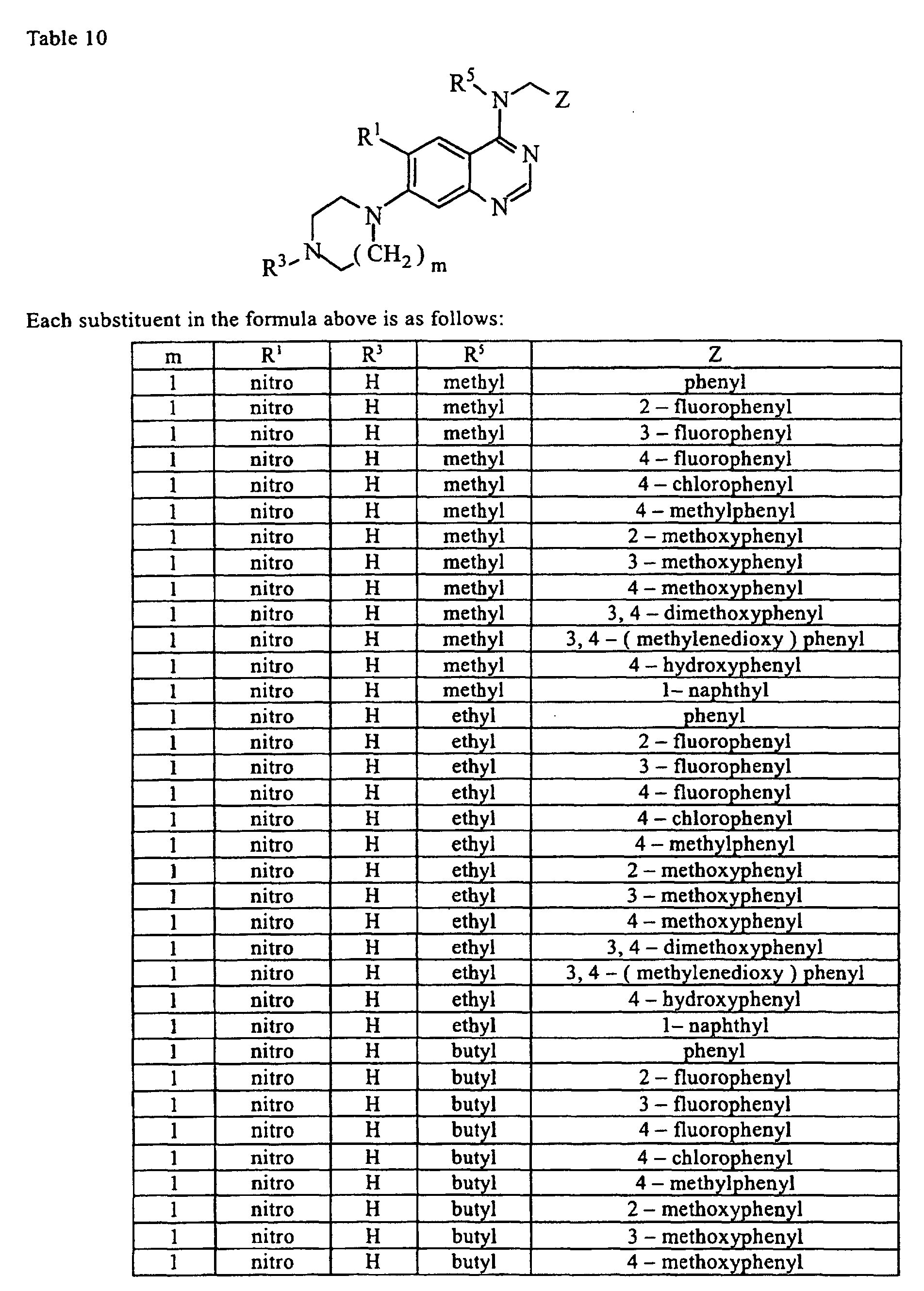

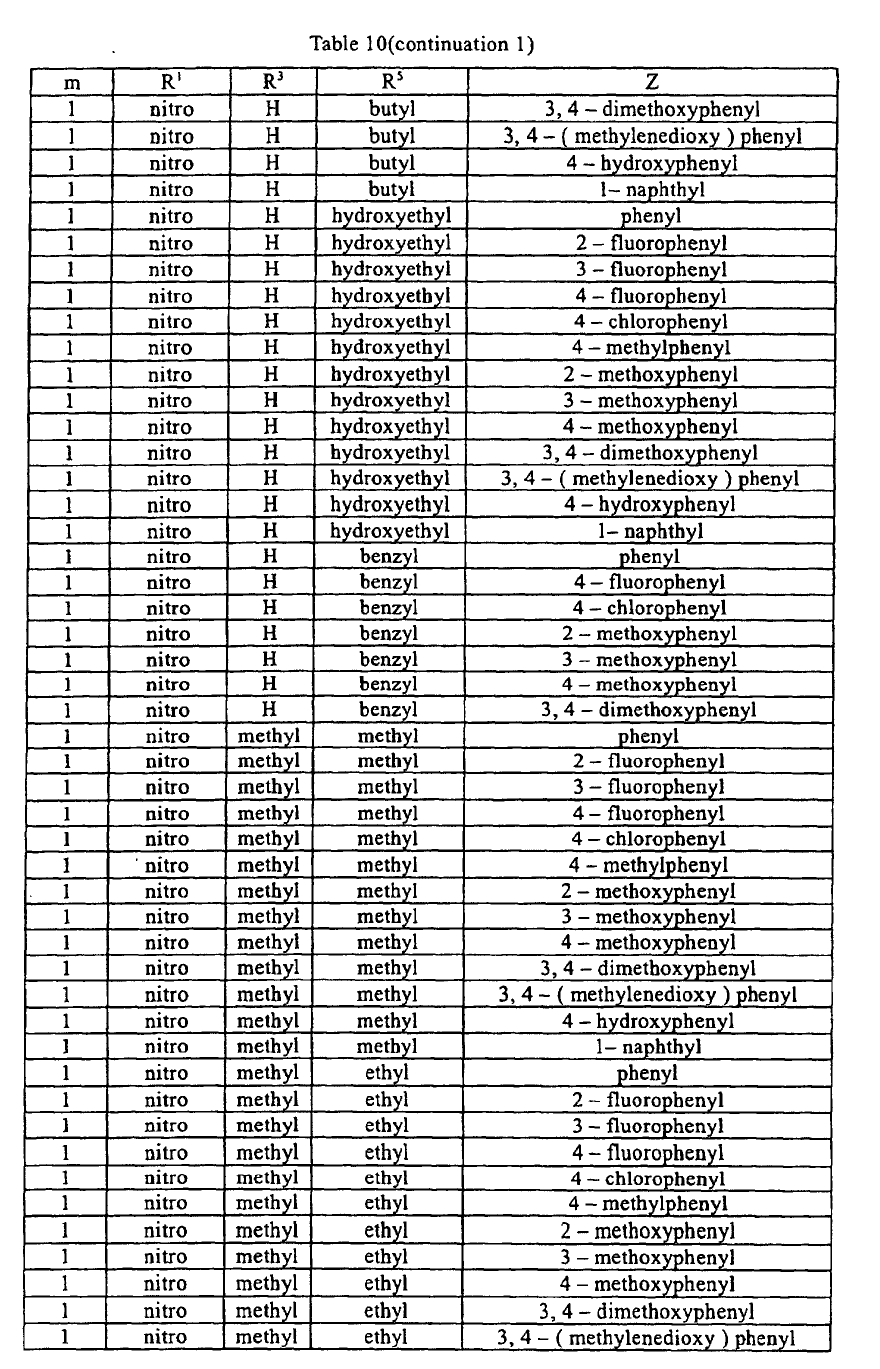

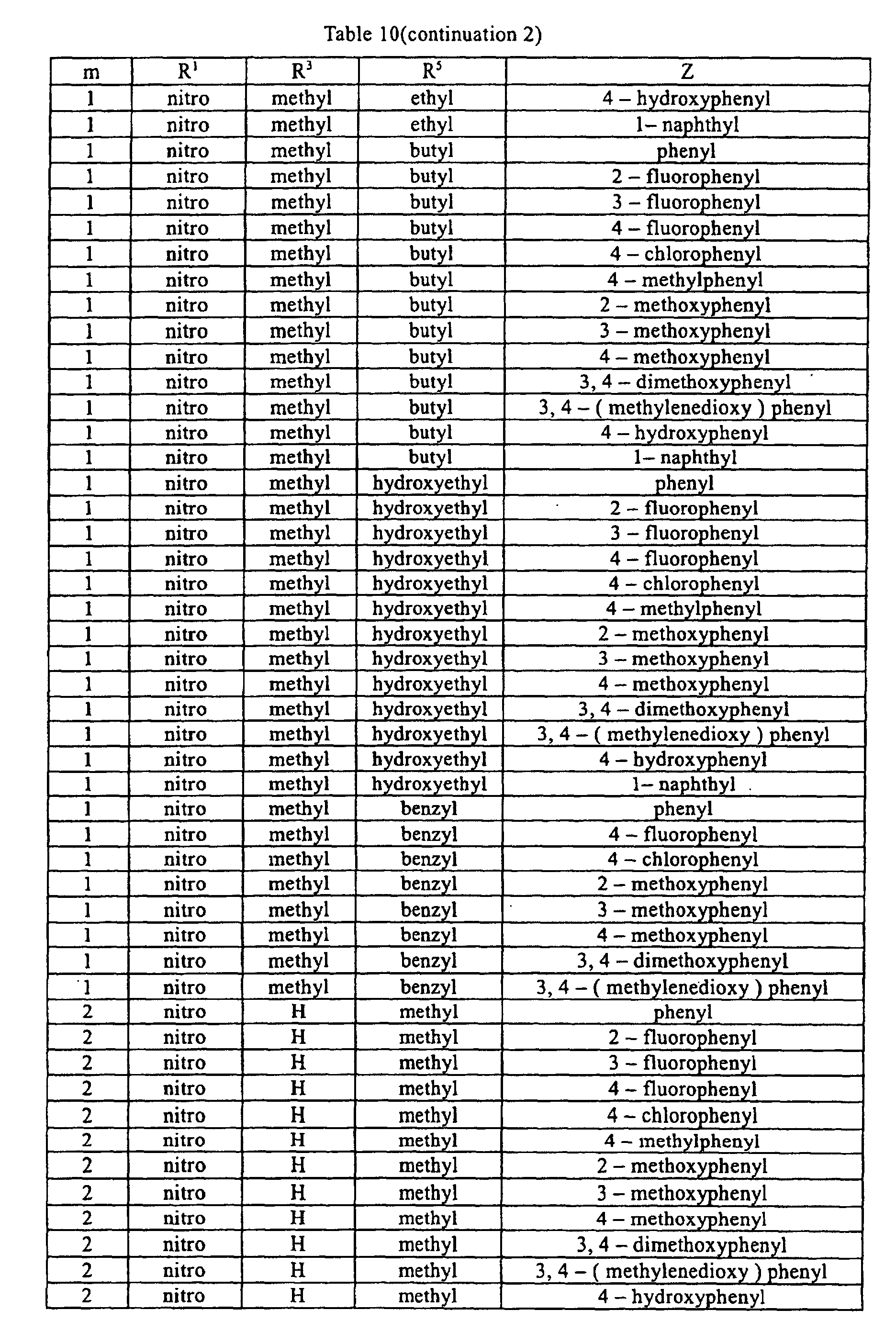

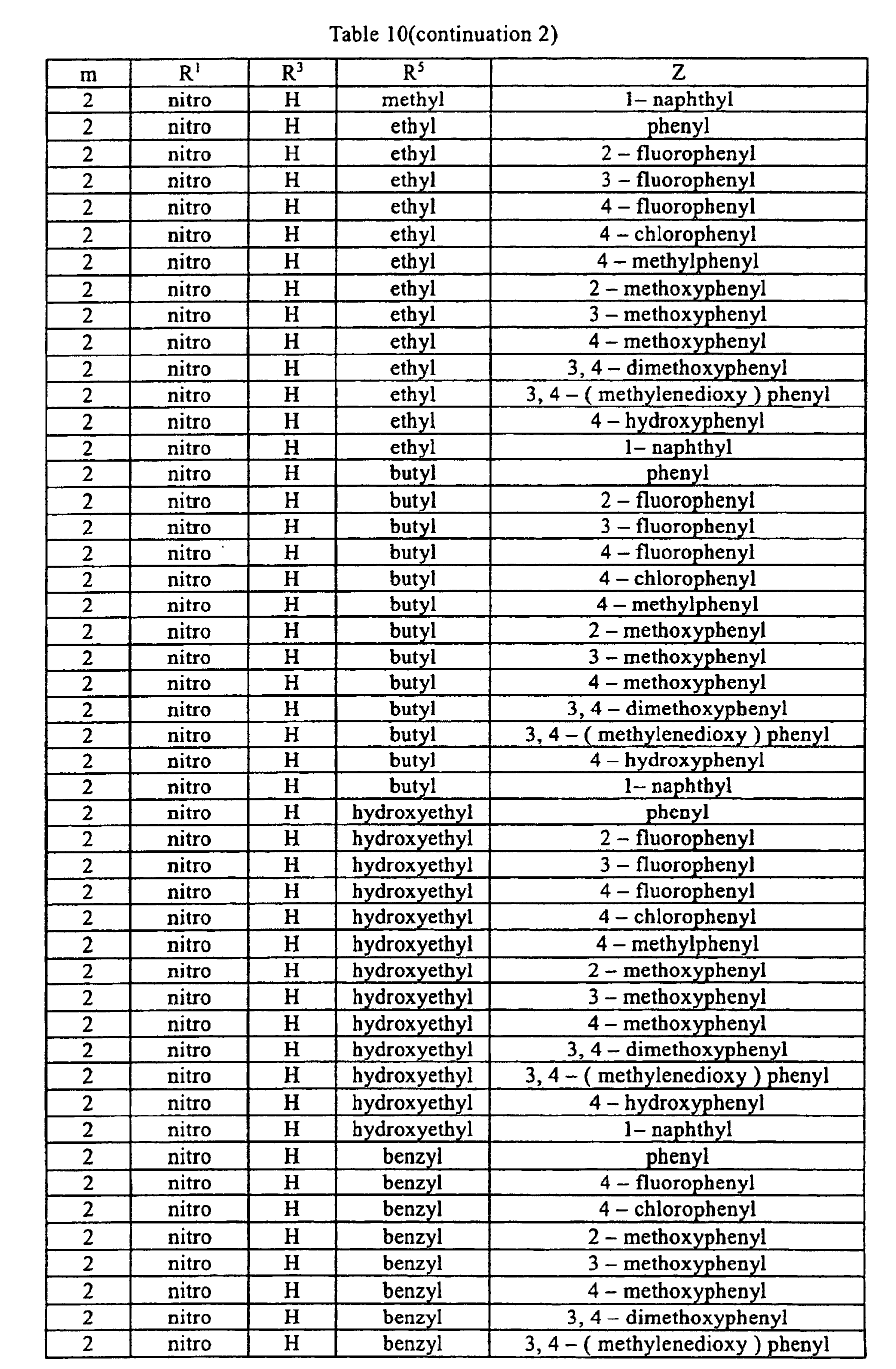

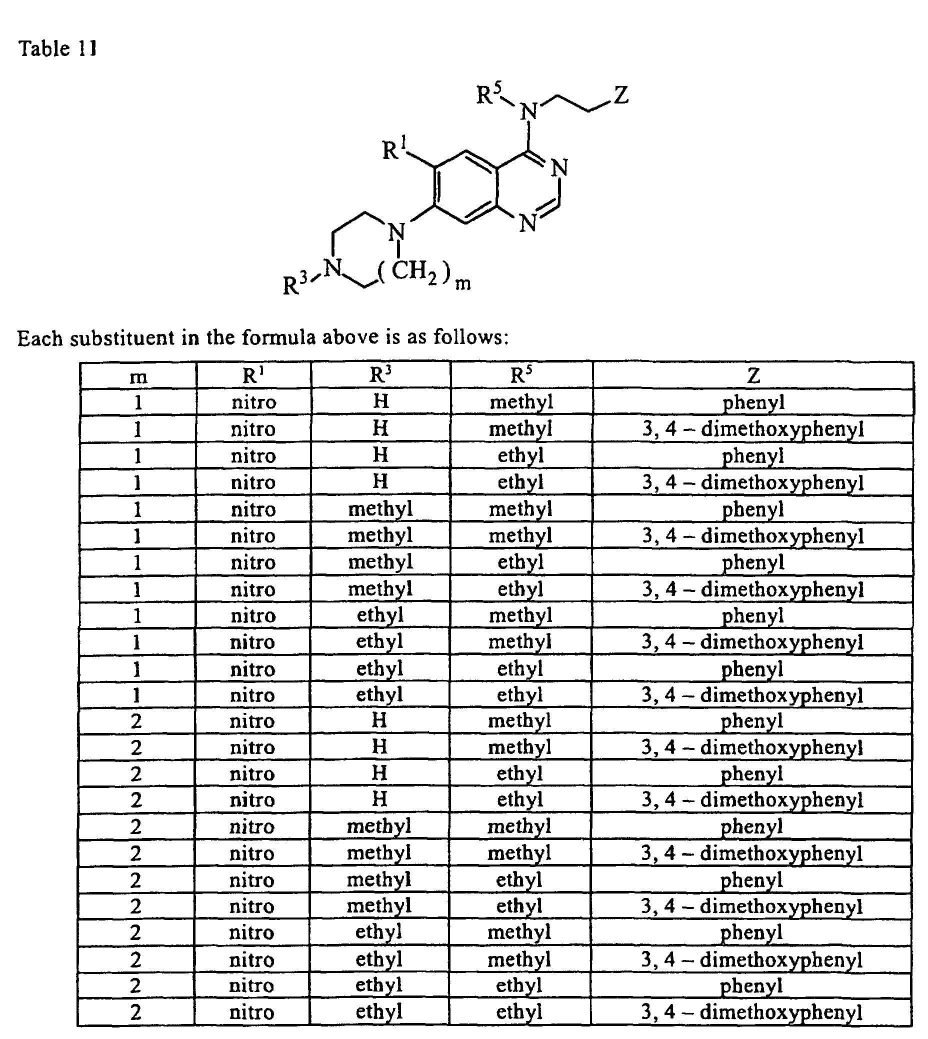

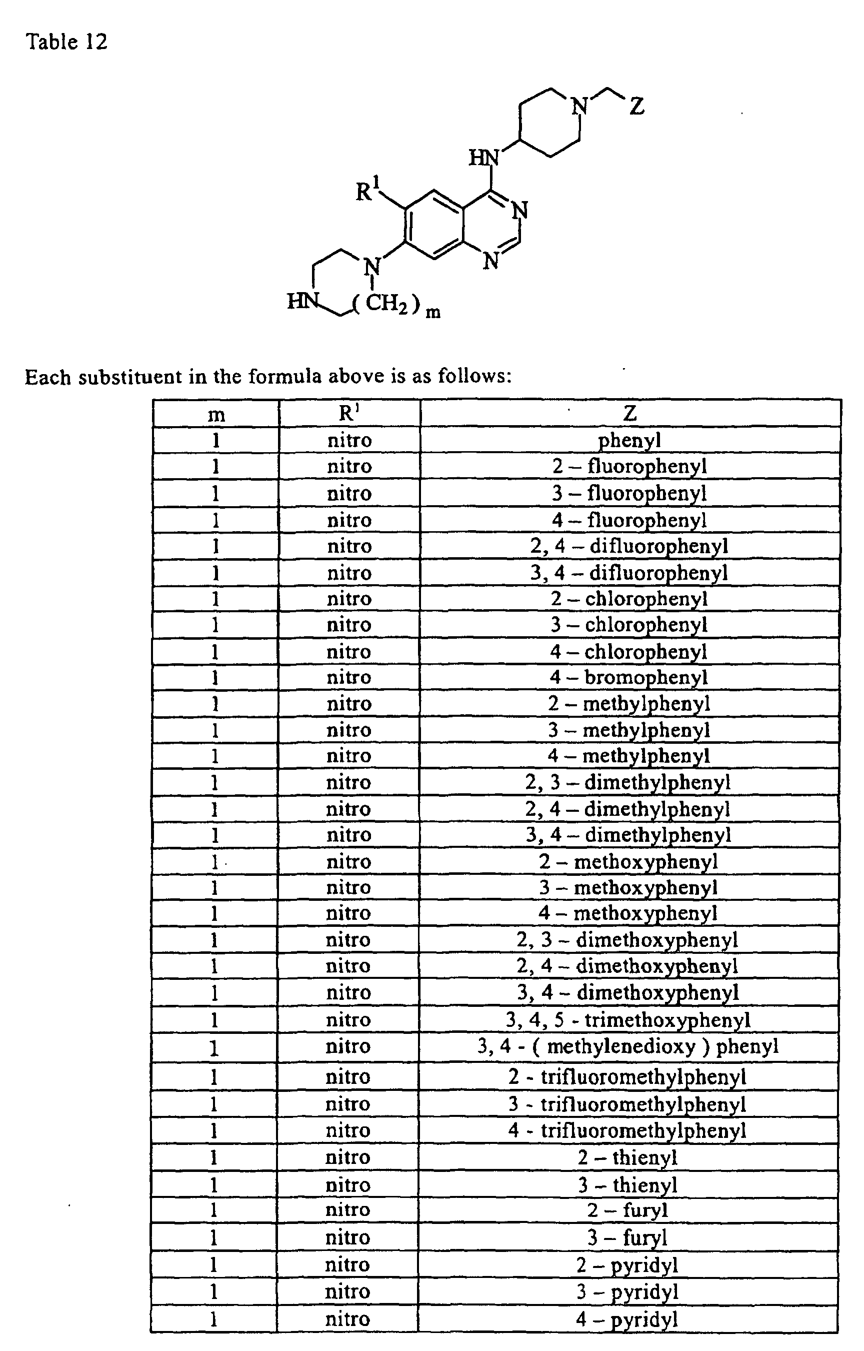

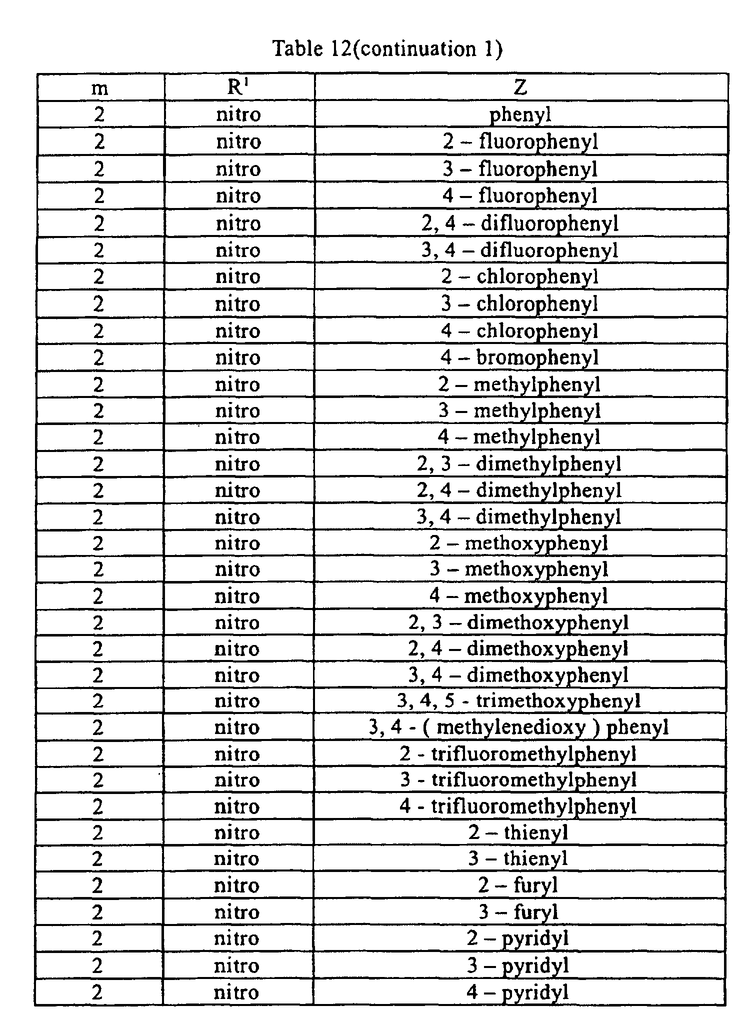

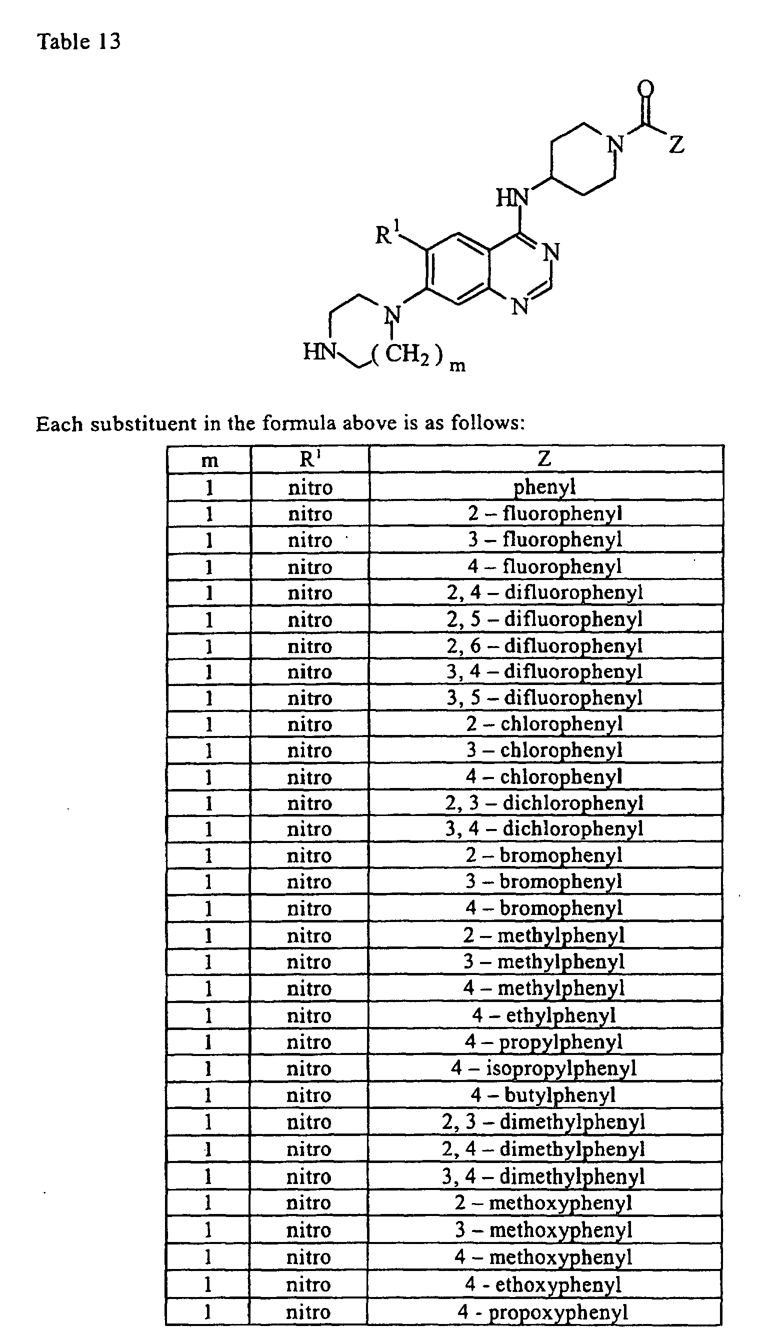

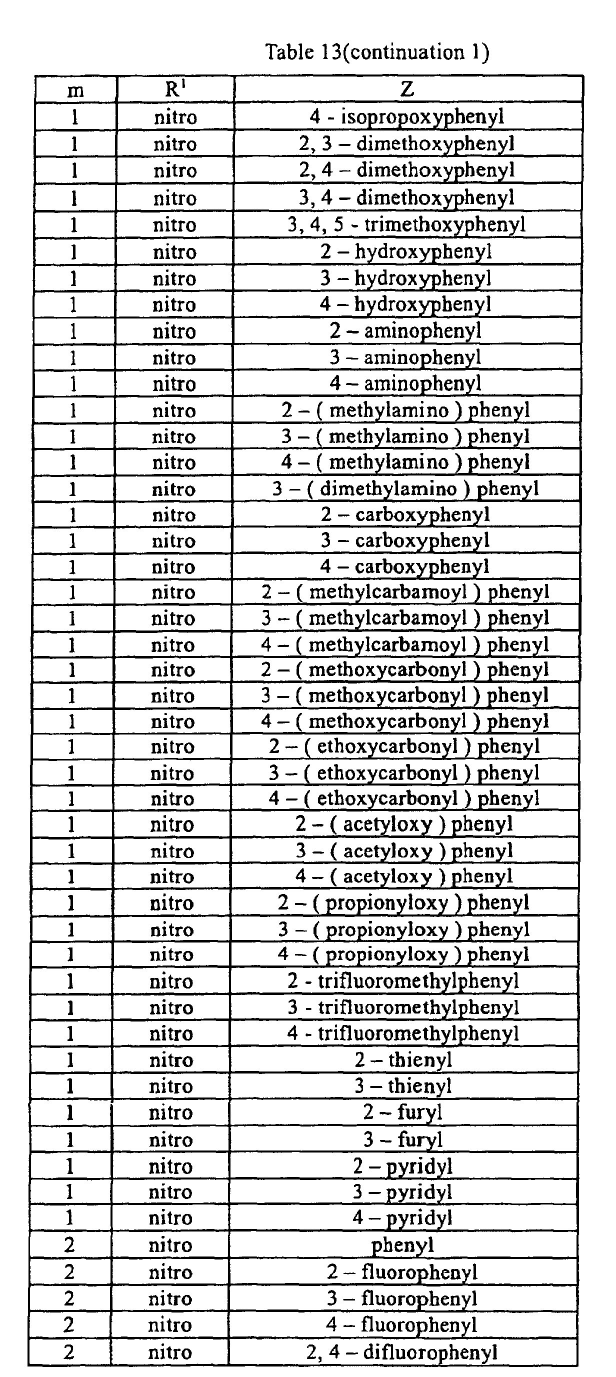

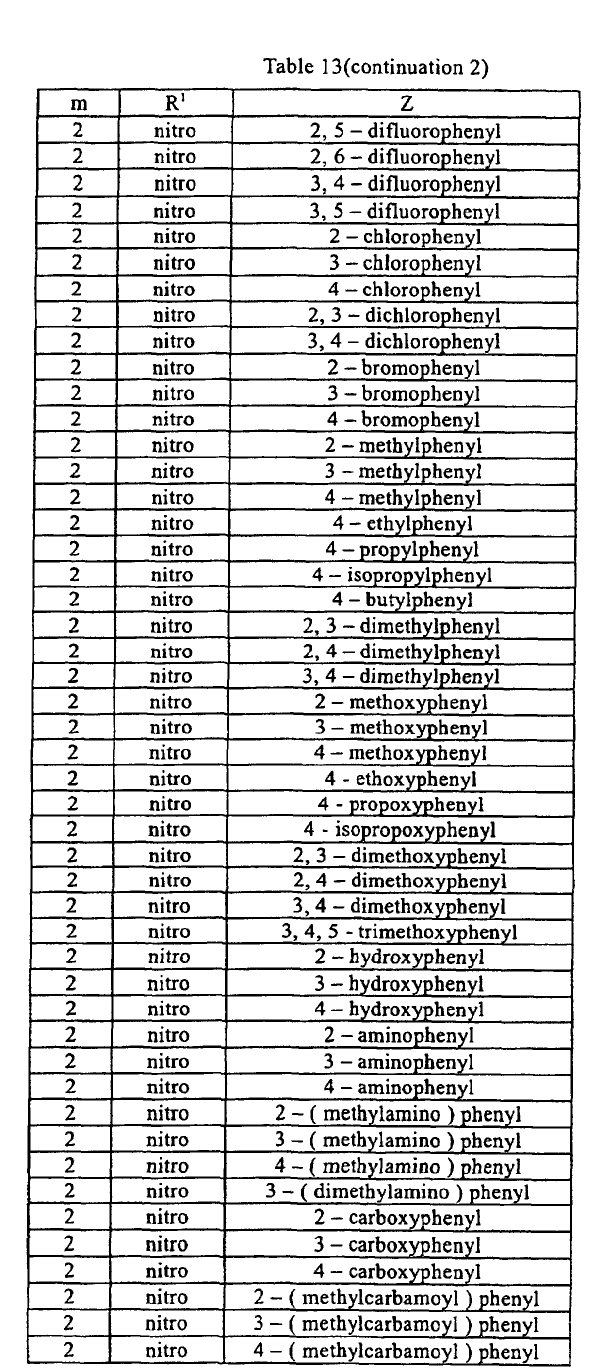

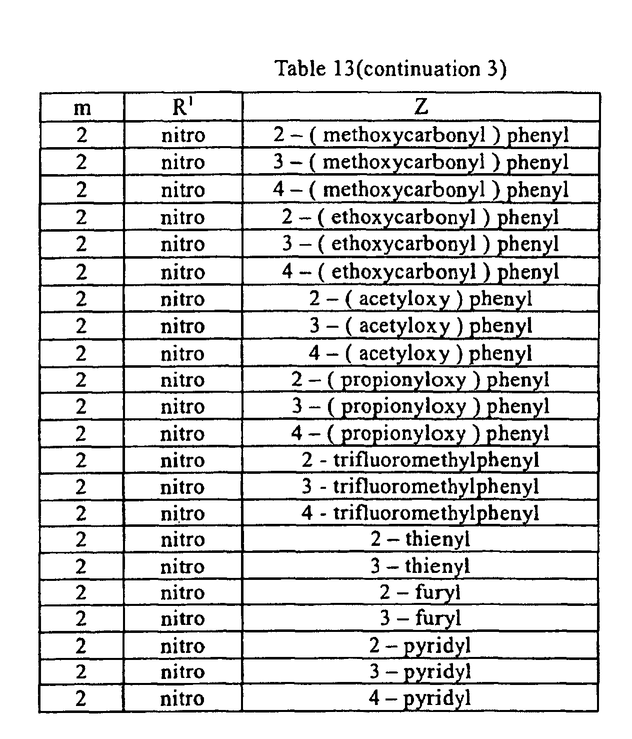

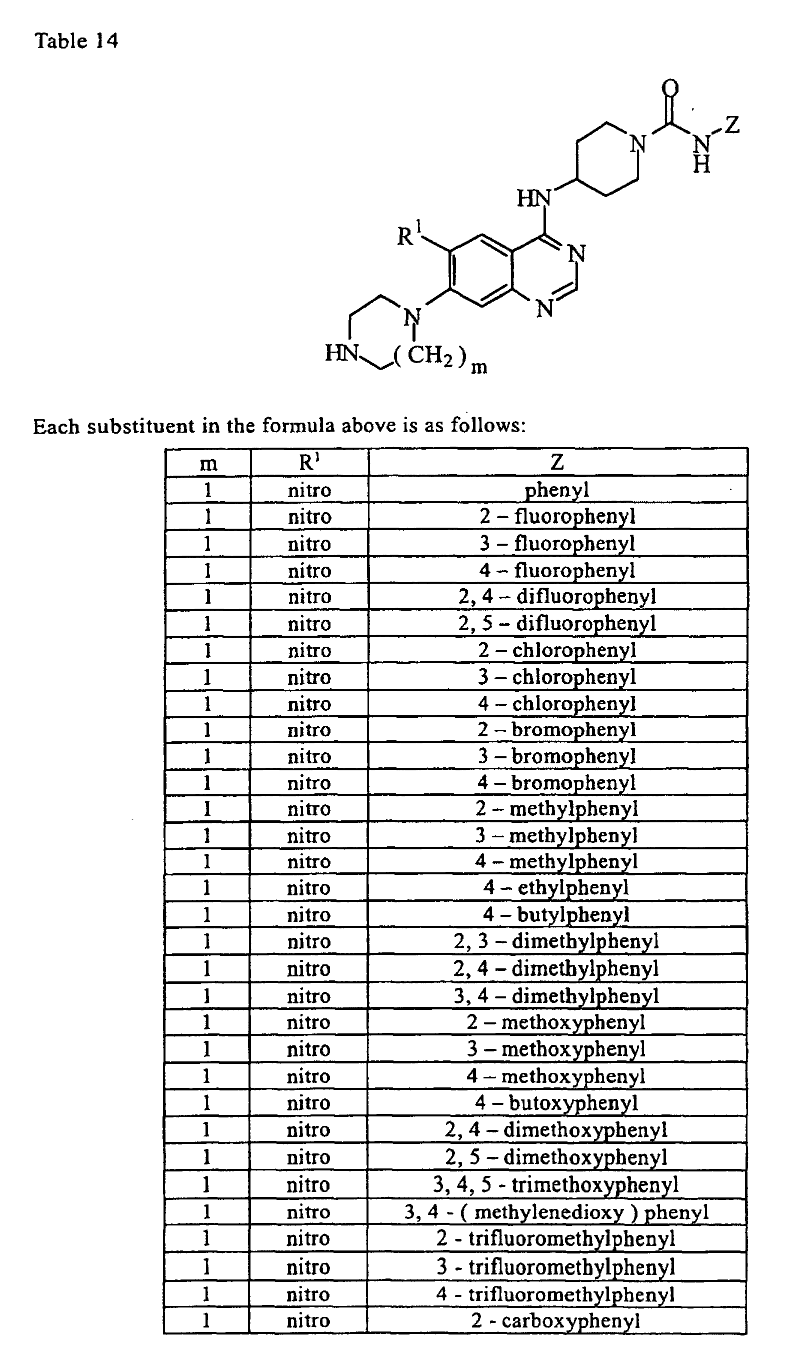

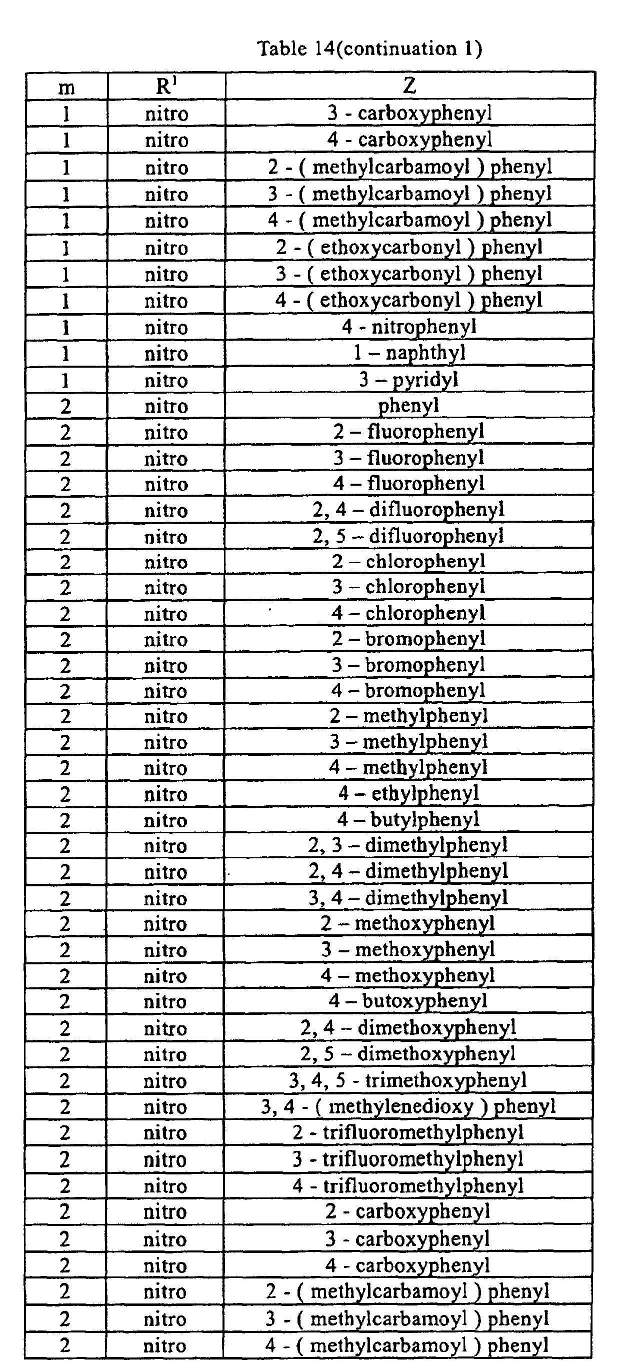

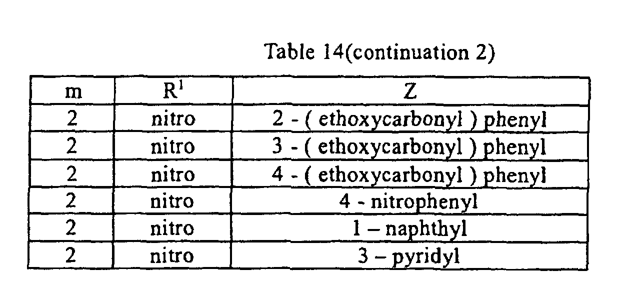

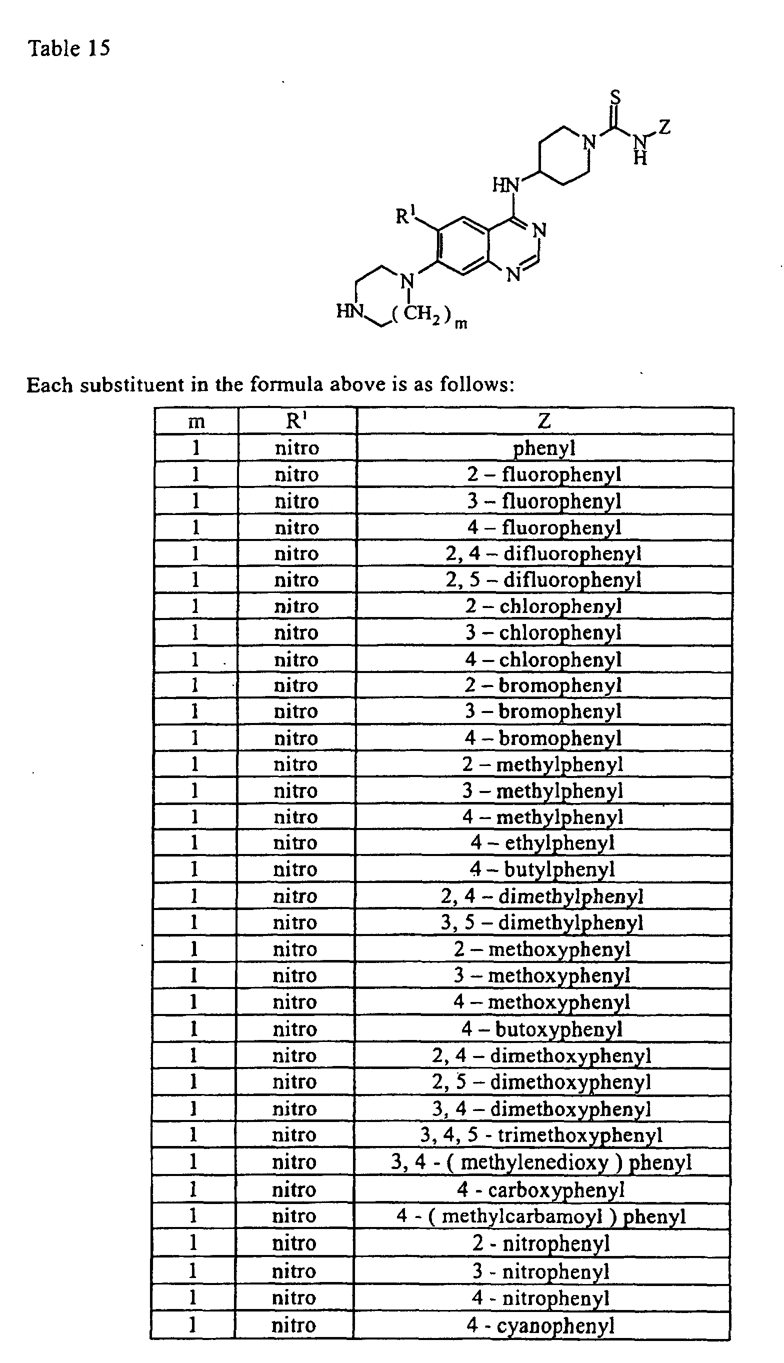

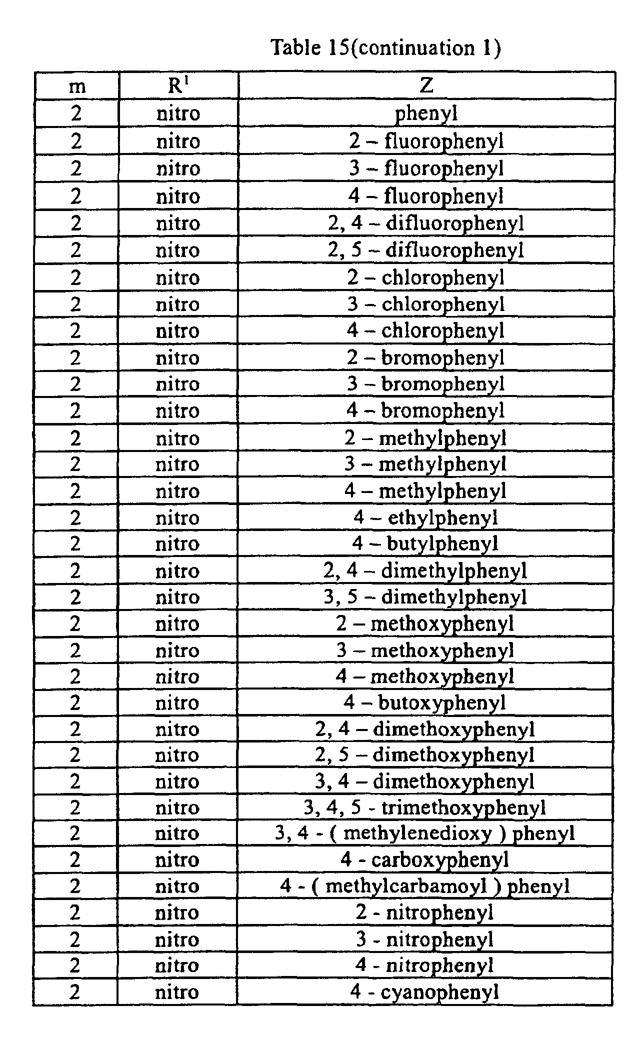

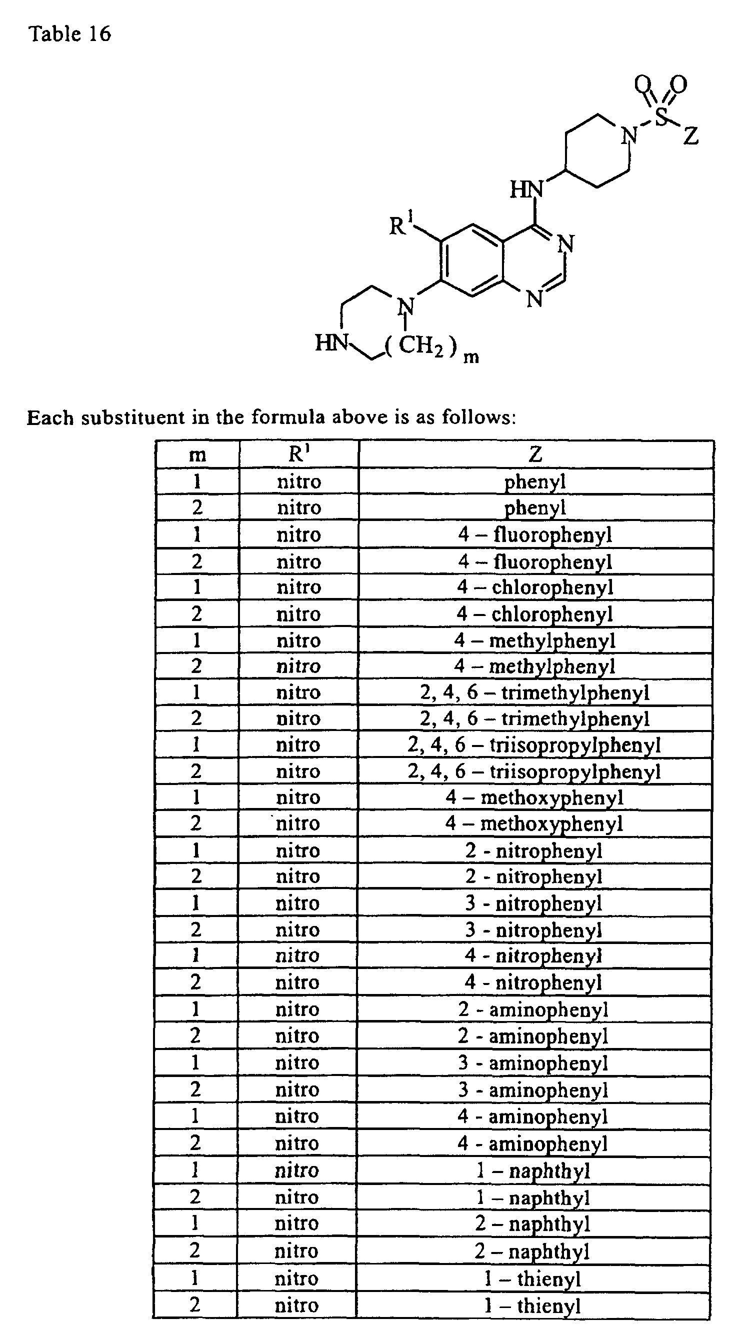

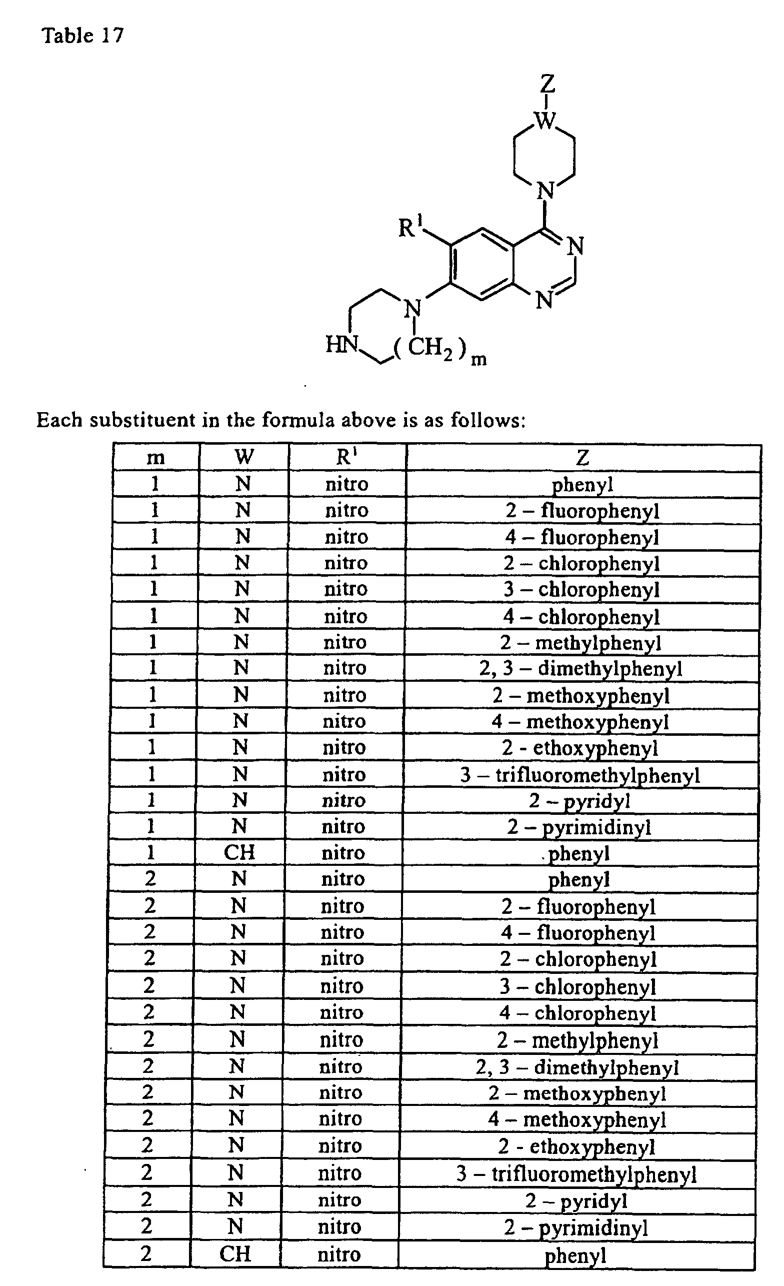

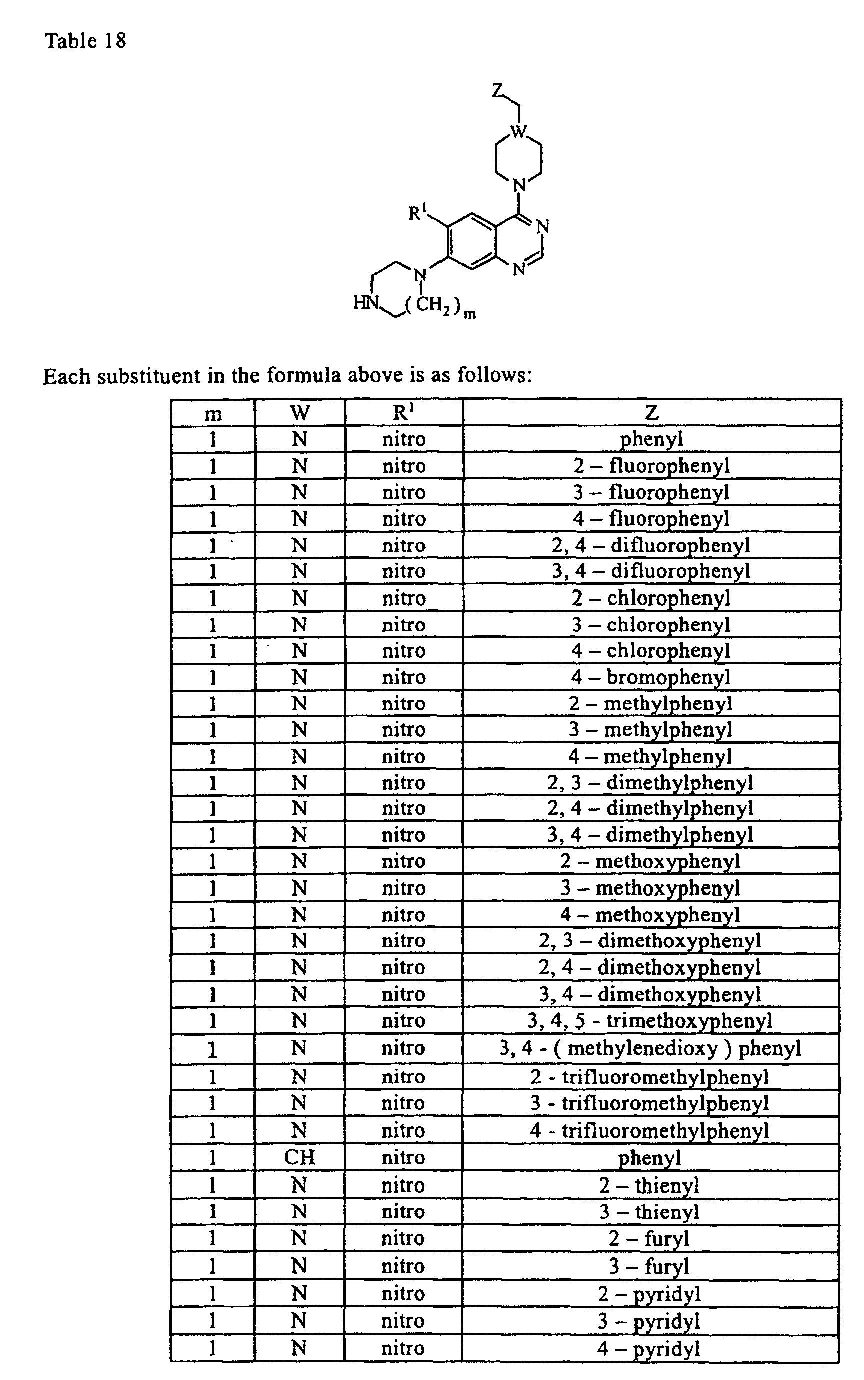

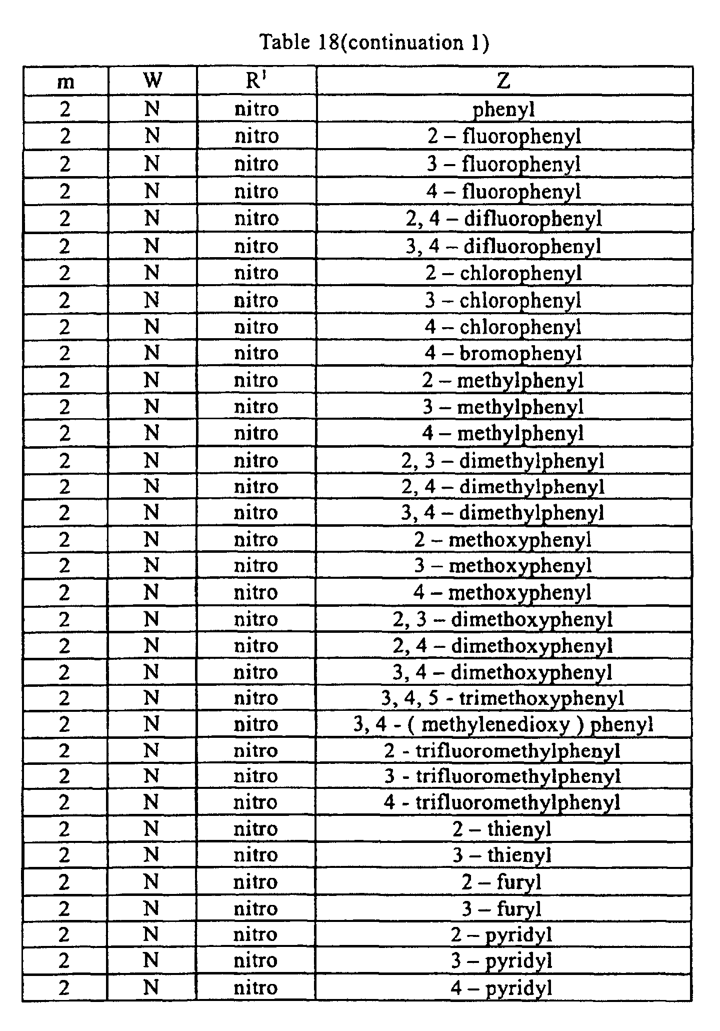

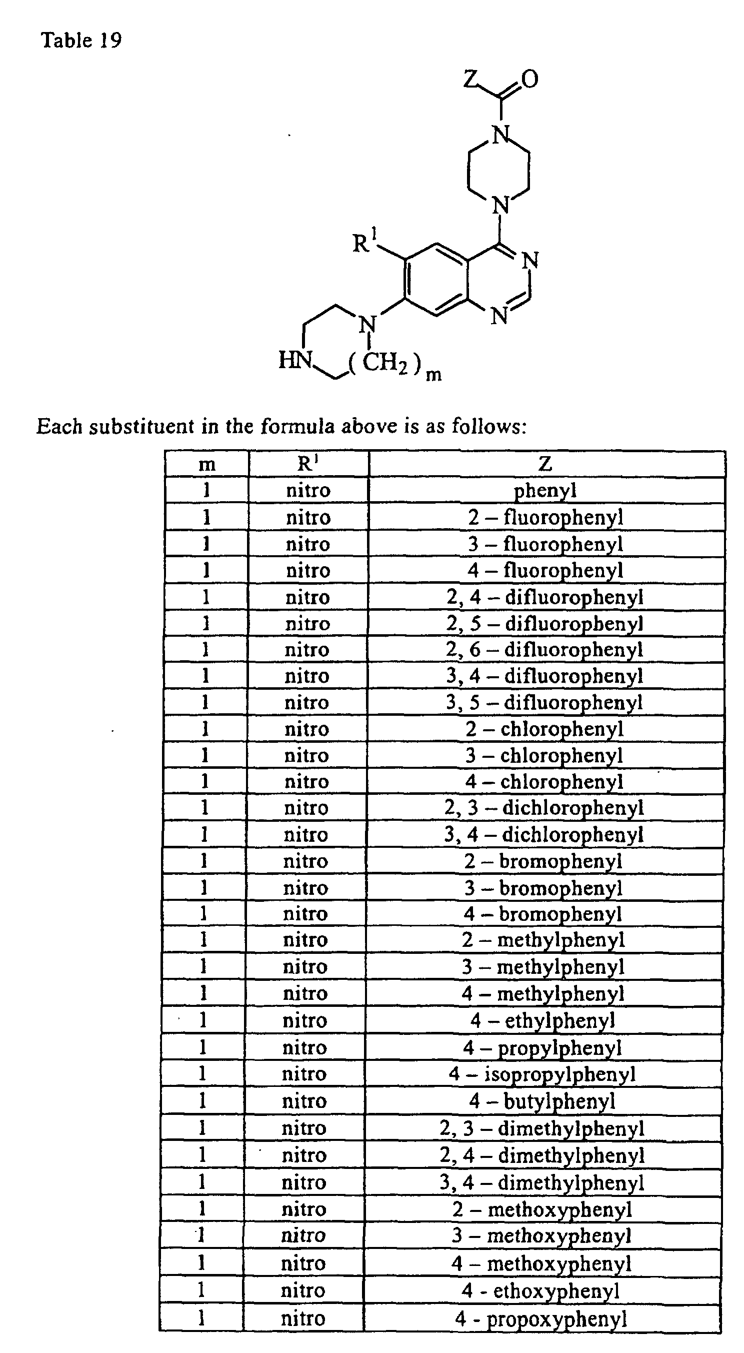

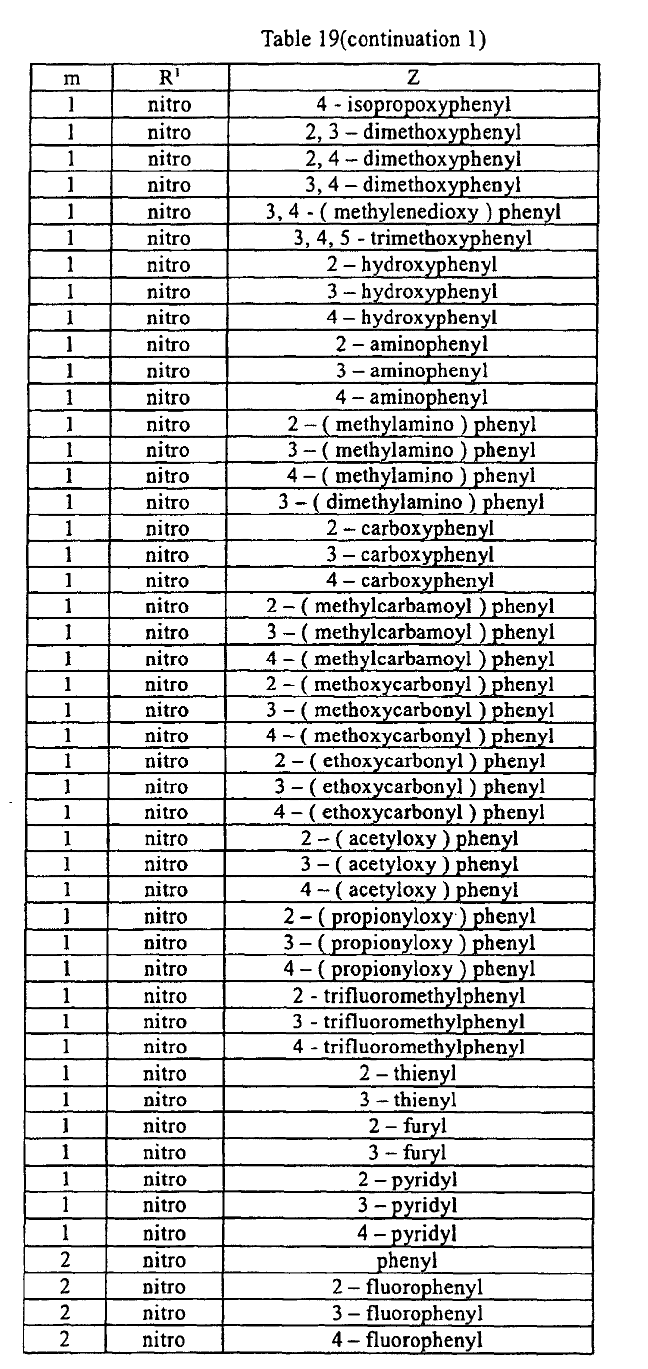

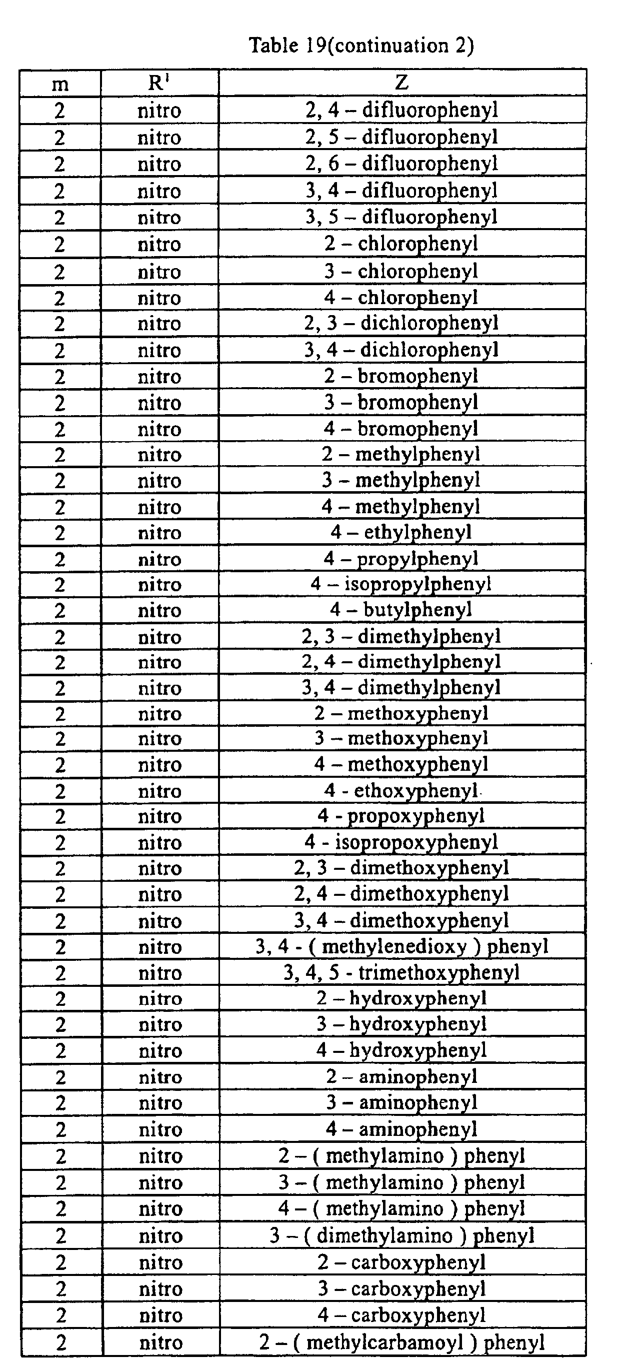

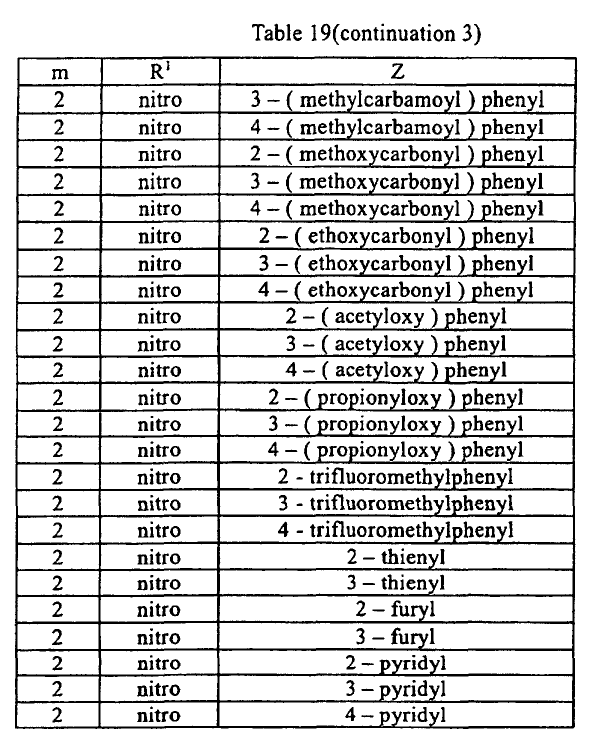

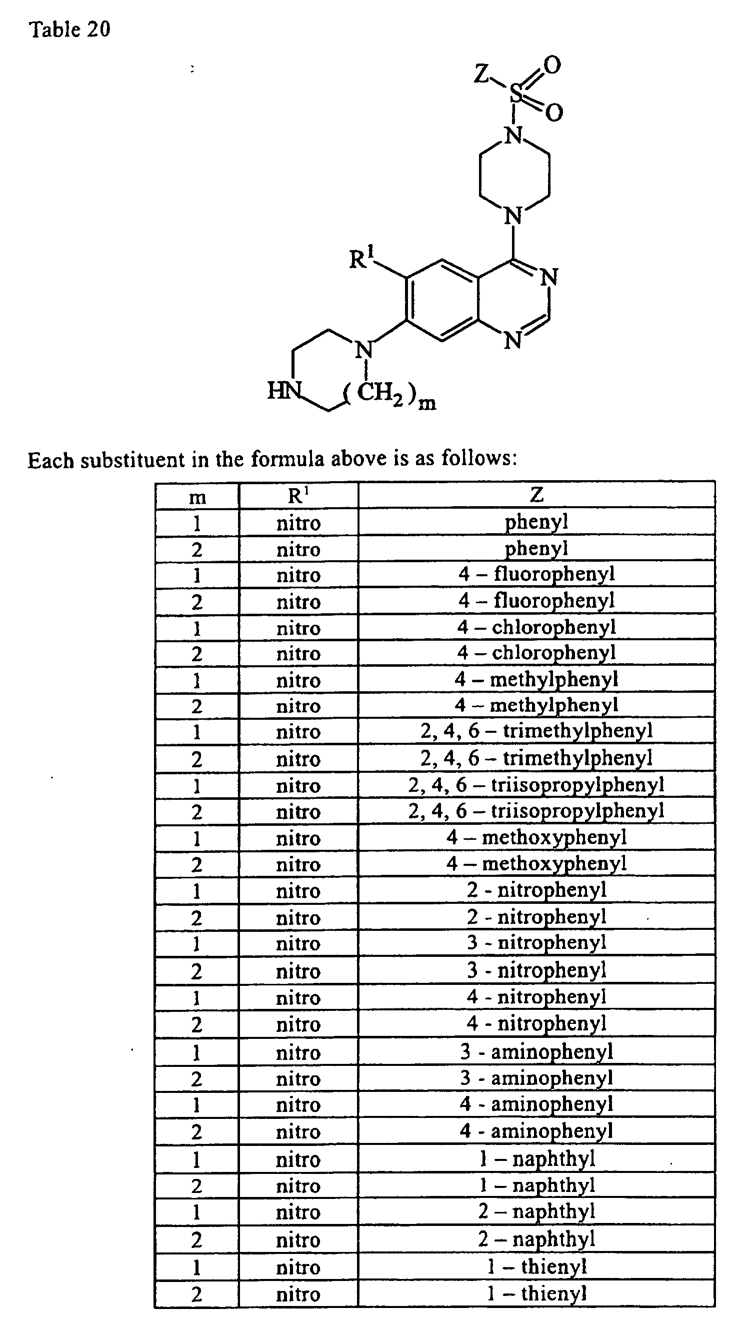

Next, the manufacturing methods of the compounds of the present invention will be explained in detail. The starting compounds which are not described in the following sections can be produced according to the following methods, or known methods or methods based thereon.

Manufacturing method 1: The compound (1a) wherein R₁ is a nitro group can be prepared according to the following reaction steps. 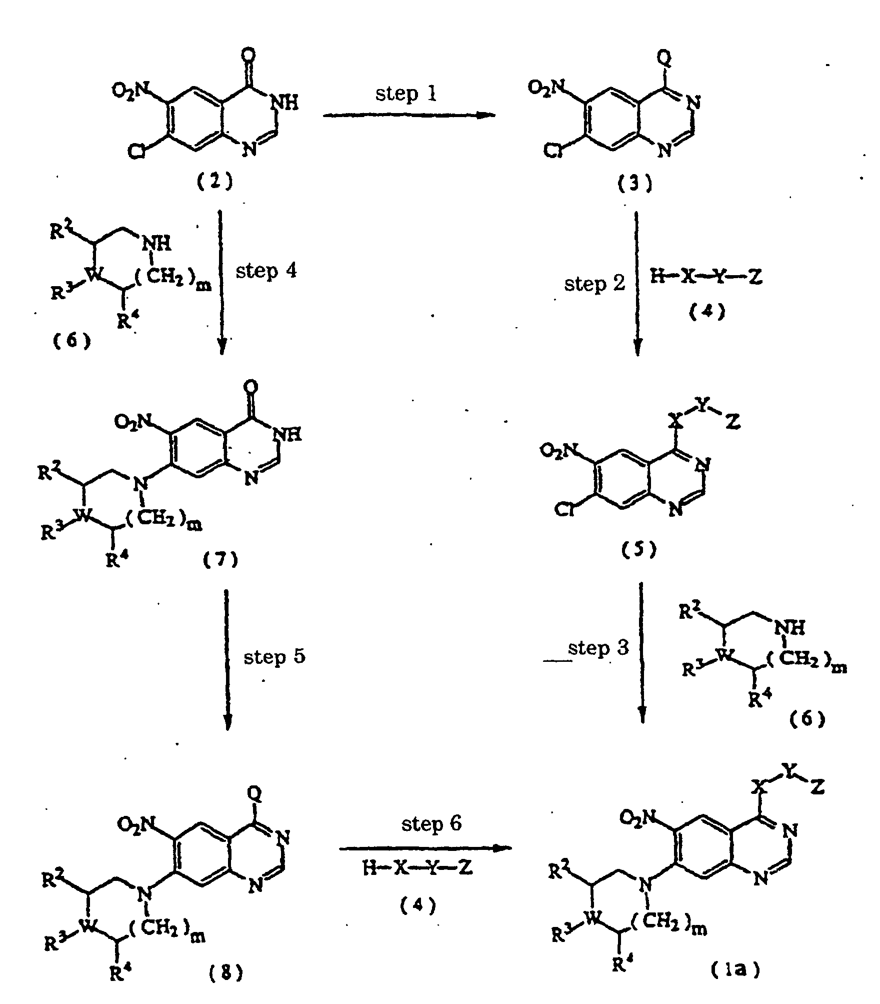 (wherein R², R³, R⁴, X, Y, Z, W and m are as defined above, Q is halogen atom, alkylsulfonyloxy group such as methanesulfonyloxy and ethanesulfonyloxy, or substituted or unsubstituted arylsulfonyloxy group such as benzenesulfonyloxy or methylbenzenesulfonyloxy.)

The starting material (2) can be obtained according to the known methods [J. Org. Chem., 40, 356(1975), etc.]

### (Step 1-1)

The compound (3a), wherein Q of the compound (3) is a halogen atom, can be obtained by reacting the compound (2) with a halogenating agent, without solvent or with a solvent of 1 to 100 times in volume, at between room temperature and the reflux temperature of the solvent used (in the case of non-solvent condition, reaction temperature is between room temperature and the boiling point of the halogenating agent) for 0.5 to 10 hours. The halogenating agent includes chlorination agent such as phosphorus oxychloride and phosphorus pentachloride and bromination agent such as phosphorus pentabromide and thionyl bromide. The solvent includes halogen-containing solvents such as chloroform, dichloromethane and dichloroethane, aromatic solvent such as benzene, toluene and xylene and ester solvent such as ethyl acetate, propyl acetate and butyl acetate.

### (Step 1-2)

The compound (3b), wherein Q of the compound(3) is alkylsulfonyloxy, or substituted or unsubstituted arysulfonyloxy group, can be obtained by reacting the compound (2) with an alkylsulfonyl chloride, or substituted or unsubstituted arylsulfonyl chloride, in the presence of a 2 to 20 equivalent base, without solvent or with a solvent of 1 to 100 times in volume, at between 0 °C and the reflux temperature of the solvent used, for 0.5 to 12 hours. The base includes an inorganic base such as sodium hydride, potassium carbonate and sodium carbonate, and an organic base such as triethyl amine, diisopropylethyl amine, pyridine and 2,6-lutidine. The solvent includes halogen-containing solvent such as chloroform, dichloromethane and dichloroethane, aromatic solvent such as benzene, toluene and xylene, ester solvent such as ethyl acetate, propyl acetate and butyl acetate, and N,N-dimethylformamide.

### (Step 2)

The compound (5) can be obtained by reacting the compound (3) with a primary or secondary amine (4), in the presence of a base, in a solvent of 1 to 100 times in volume, at between 0 °C and the reflux temperature of the solvent used, for 0.5 to 12 hours. The base includes an inorganic base such as sodium hydride, potassium carbonate and potassium tert-butoxide, and an organic base such as triethyl amine, diisopropylethyl amine, pyridine and 2,6-lutidine. The solvent includes ether solvent such as diethyl ether, tetrahydrofuran and dioxane, halogen-containing solvent such as chloroform, dichloromethane and dichloroethane, aromatic solvent such as benzene, toluene and xylene, ester solvent such as ethyl acetate, propyl acetate and butyl acetate, water, and N,N-dimethylformamide.

### (Step 3)

The compound (1) can be obtained by reacting the compound (5) with a cyclic amine (6), in the presence of a base, in a solvent of 1 to 100 times in volume, at between 0 °C and the reflux temperature of the solvent used, for 0.5 to 12 hours. The base includes an inorganic base such as sodium hydride, potassium carbonate and potassium tert-butoxide, and an organic base such as triethyl amine, diisopropylethyl amine, pyridine and 2,6-lutidine. The solvent includes ether solvent such as diethyl ether, tetrahydrofuran and dioxane, halogen-containing solvent such as chloroform, dichloromethane and dichloroethane, aromatic solvent such as benzene, toluene and xylene, ester solvent such as ethyl acetate, propyl acetate and butyl acetate, water, and N,N-dimethylformamide.

### (Step 4)

The compound (7) can be obtained by reacting the compound (2) with a cyclic amine (6), in the presence of a base, in a solvent of 1 to 100 times in volume, at between 0 °C and the reflux temperature of the solvent used, for 0.5 to 12 hours. The base includes an inorganic base such as sodium hydride, potassium carbonate and potassium tert-butoxide, and an organic base such as triethyl amine, diisopropylethyl amine, pyridine and 2,6-lutidine. The solvent includes ether solvent such as diethyl ether, tetrahydrofuran and dioxane, halogen-containing solvent such as chloroform, dichloromethane and dichloroethane, aromatic solvent such as benzene, toluene and xylene, ester solvent such as ethyl acetate, propyl acetate and butyl acetate, water, and N,N-dimethylformamide.

### (Step 5-1)

The compound (8a), wherein Q of the compound (8) is a halogen atom, can be obtained by reacting the compound (7) with a halogenating agent, without solvent or with a solvent of 1 to 100 times in volume, at between room temperature and the reflux temperature of the solvent used (in the case of non-solvent condition, reaction temperature is between room temperature and the boiling point of the halogenating agent) for 0.5 to 10 hours. The halogenating agent and solvent are the same as those used in the preparation of the compound (3a).

### (Step 5-2)

The compound (8b), wherein Q of the compound(8) is alkylsulfonyloxy, or substituted or unsubstituted arysulfonyloxy group, can be obtained by reacting the compound (7) with an alkylsulfonyl chloride, or substituted or unsubstituted arylsulfonyl chloride, in the presence of a 2 to 20 equivalent base, without solvent or with a solvent of 1 to 100 times in volume, at between 0 °C and the reflux temperature of the solvent used, for 0.5 to 12 hours. The base and solvent are the same as those used in the preparation of the compound (3b).

### (Step 6)

The compound (1) can be obtained by reacting the compound (8) with a primary or secondary amine (4), in the presence of a base, in a solvent of 1 to 100 times in volume, at between 0 °C and the reflux temperature of the solvent used, for 0.5 to 12 hours. The base and solvent are the same as those used in the preparation of the compound (5).

Manufacturing method 2: The compound (1b) wherein R, is a halogen atom can be prepared according to the following reaction steps. 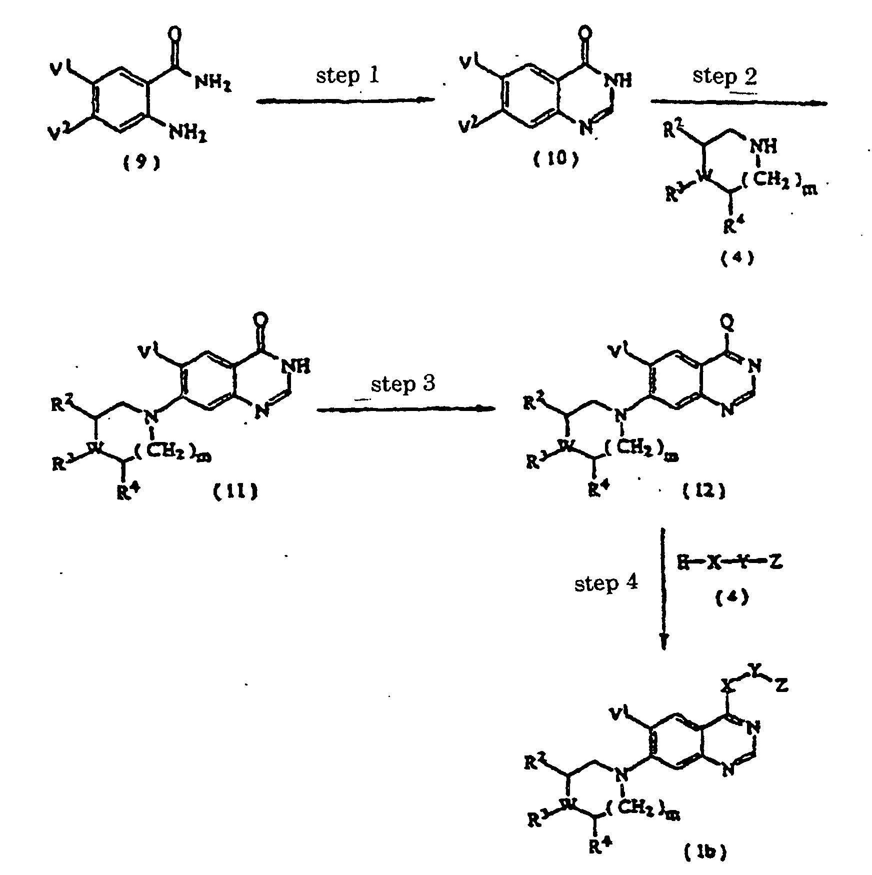 (wherein R², R³, R⁴, X, Y, Z, W and m are as defined above, V¹, V² and Q is halogen atom, alkylsulfonyloxy group such as methanesulfonyloxy and ethanesulfonyloxy, or substituted or unsubstituted arylsulfonyloxy group such as benzenesulfonyloxy or methylbenzenesulfonyloxy.)

### (Step 1)

The compound (10) can be obtained by reacting the compound (9) obtained according to the known methods with formic acid or trimethyl orthoformate, in the presence of an acid, at a temperature of between room temperature and 100 °C for 0.5 to 10 hours. The acid includes an inorganic acid such as hydrochloric acid and an organic acid such as p-toluenesulfonic acid and camphorsulfonic acid.

### (Step 2)

The compound (11) can be obtained by reacting the compound (10) with a cyclic amine (6), in the presence of a base, in a solvent of 1 to 100 times in volume, at between 0 °C and the reflux temperature of the solvent used, for 0.5 to 12 hours. The base includes an inorganic base such as sodium hydride, potassium carbonate and potassium tert-butoxide, and an organic base such as triethyl amine, diisopropylethyl amine, pyridine and 2,6-lutidine. The solvent includes ether solvent such as diethyl ether, tetrahydrofuran and dioxane, halogen-containing solvent such as chloroform, dichloromethane and dichloroethane, aromatic solvent such as benzene, toluene and xylene, ester solvent such as ethyl acetate, propyl acetate and butyl acetate, water, and N,N-dimethylformamide.

### (Step 3-1)

The compound (12a), wherein Q of the compound(12) is a halogen atom, can be obtained by reacting the compound (11) with a halogenating agent, without solvent or with a solvent of 1 to 100 times in volume, at between room temperature and the reflux temperature of the solvent used, for 0.5 to 10 hours. The halogenating agent includes chlorination agent such as phosphorus oxychloride and phosphorus pentachloride and bromination agent such as phosphorus pentabromide and thionyl bromide. The solvent includes halogen-containing solvent such as chloroform, dichloromethane and dichloroethane, aromatic solvent such as benzene, toluene and xylene and ester solvent such as ethyl acetate, propyl acetate and butyl acetate.

### (Step 3-2)

The compound (12b), wherein Q of the compound(12) is alkylsulfonyloxy, or substituted or unsubstituted arysulfonyloxy group, can be obtained by reacting the compound (11) with an alkylsulfonyl chloride, or substituted or unsubstituted arylsulfonyl chloride, in the presence of a 2 to 20 equivalent base, without solvent or with a solvent of 1 to 100 times in volume, at between 0 °C and the reflux temperature of the solvent used, for 0.5 to 12 hours. The base includes an inorganic base such as sodium hydride, potassium carbonate and sodium carbonate, and an organic base such as triethyl amine, diisopropylethyl amine, pyridine and 2,6-lutidine. The solvent includes halogen-containing solvent such as chloroform, dichloromethane and dichloroethane, aromatic solvent such as benzene, toluene and xylene, ester solvent such as ethyl acetate, propyl acetate and butyl acetate, and N,N-dimethylformamide.

### (Step 4)

The compound (1b) can be obtained by reacting the compound (12) with a primary or secondary amine (4), in the presence of a base, in a solvent of I to 100 times in volume, at between 0 °C and the reflux temperature of the solvent used, for 0.5 to 12 hours. The base includes an inorganic base such as sodium hydride, potassium carbonate and potassium tert-butoxide, and an organic base such as triethyl amine, diisopropylethyl amine, pyridine and 2,6-lutidine. The solvent includes ether solvent such as diethyl ether, tetrahydrofuran and dioxane, halogen-containing solvent such as chloroform, dichloromethane and dichloroethane, aromatic solvent such as benzene, toluene and xylene, ester solvent such as ethyl acetate, propyl acetate and butyl acetate, water, and N,N-dimethylformamide.

The intermediates and end products in the above-mentioned manufacturing methods can be isolated or purified by conventional methods. For example, they can be isolated or purified using neutralization, filtration, extraction, drying, concentration, recrystallization and various kinds of chromatography. The solvent for recrystallization includes, for example, alcohol solvent such as methanol, ethanol and 2-propanol, ether solvent such as diethyl ether, ester solvent such as ethyl acetate, aromatic hydrocarbon solvent such as toluene, ketone solvent such as acetone, hydrocarbon solvent such as hexane and mixed solvents thereof. It is also possible for the intermediates to be used in the next steps without any purification.

In case that a salt of the compound (1) is desired to be obtained, if the compound (1) is obtained in the form of a salt it is purified as it is, and if obtained in a free form it is dissolved or suspended in a suitable solvent and convert it into a salt by adding an acid. And, it is possible to transform the compound (1) obtained in the form of a salt into a free compound and then to convert it into the form of a desired salt.

Upon carrying out the above reactions, it is possible to use the techniques of protection and deprotection for functional group, which are described in detail in T. W. Green and P. G. M. Wuts, "Protecting Groups in Organic Synthesis", 1990.

As the medicaments of the present invention there can be used substances selected from the group consisting of compounds represented by the general formula (1) and pharmaceutically acceptable salts thereof, or there can be used hydrates or solvates thereof. It is also possible to use these substances in a proper combination of two or more. A substance per se selected from the group may be administered as a medicament of the present invention, but usually it is preferable to administer in the form of a medicament composition containing the above substance as an effective ingredient and pharmaceutically acceptable preparation additives.

The medicament composition applied for the living body can easily be produced according to the preparation methods commonly used in the field of pharmacology, that is, by mixing the above substance as effective ingredient and one or two or more kinds of pharmaceutically acceptable preparation additives. The administration route of the medicament of the present invention is not limited in particular, but it is preferable to properly choice the most effective route in the treatment and/or prevention. The medicinal composition suitable for oral administration includes, for example, capsules, powders, tablets, granules, fine grains, syrups, solutions and suspensions, and the medicament composition suitable for parenteral administration includes, for example, absorbents, nebulas, intrarectal administration medicaments, injections, drops, ointments, creams, transdermal absorbents, transmucosa absorbents, eyedrops, collunarium, eardrops, tapes and patches. The forms of the medicaments of the present invention, however, are not limited to these.

Of the medicinal compositions suitable for oral administration, liquid preparations such as emulsions and syrups can be manufactured using preparation additives such as water; saccharides such as saccharose, sorbit and fructose; glycols such as polyethylene glycol and propylene glycol; oils such as sesame oil, olive oil and soybean oil; antiseptics such as p-hydroxybenzoic acid ester; and flavors such as strawberry flavor and peppermint flavor. The solid preparations such as capsules, tablets, powders and granules can be manufactured using excipients such as lactose, glucose, saccharose and mannitol; disintegrating agents such as starch and sodium alginate; lubricants such as magnesium stearate and talc; binders such as polyvinyl alcohol, hydroxypropyl cellulose and gelatin; surfactants such as fatty acid ester; and plasticizers such as glycerol.

Of the medicament compositions suitable for parenteral administration, liquid preparations in the form of injections, drops and eyedrops can preferably be manufactured as sterilized isotonic liquid preparations. For example, the injections can be prepared using a salt solution, a glucose solution or an aqueous medium consisting of a mixture of salt water and glucose solution. The intrarectal administration agents can be prepared usually in the form of suppository using carriers such as cacao fat, hydrogenated fat and hydrogenated carboxylic acid. Further, for the preparation of the nebulas there can be used non-irritating carriers which disperse the above substance of the effective ingredient as fine particles to facilitate absorption. As such carriers, it is possible to enumerate lactose and glycerol and to select the forms of aerosol and drypowder as the form of the preparations. Further, in the production of the medicament compositions for parenteral administration, there can optionally be used one kind, or two or more kinds of preparation additives selected from diluents, flavors, antiseptics, excipients, disintegrating agents, lubricants, binders, surfactants and plasticizers which have been illustrated in the oral administration agents. The preparation additives used for the manufacture of the medicaments of the present invention are not limited to those mentioned above, and any can be used as long as it is utilizable for a person skilled in the art.

The dose and frequency of administration of the medicaments of the present invention are not limited in particular, and generally, in the case of oral administration, it is possible to administer a dose in the range of about 1 to about 1000 mg, preferably about 10 to about 500 mg per day for an adult in one portion or in several portions. In the case of administration as an injection medicament, the dose ranging about 0.1 to about 500 mg, preferably about 3 to about 100 mg can be administered in one portion or in several portions.

This specification includes part or all of the contents as disclosed in the specification of Japanese Patent Application No. 11-282078, which is the base of priority of the present invention.

### Best Mode for Embodying the Invention

The present invention will hereinafter be explained more in detail by way of Examples and Reference examples, but the scope of the present invention is limited in no way by the following Examples.

### Reference example 1: 2-amino-4-chlorobenzamide

4-Chloroanthranilic acid (75 g, 0.437 mol) was suspended in dichloromethane (1.09 1). To this suspension, triethylamine (73 ml, 0.524 mol) and diphenylphosphoryl chloride (141 g, 0.524 mol) were added successively, and the mixture was stirred at room temperature for 1 hour. Then, 28% aqueous ammonia (44 ml) was added, and the mixture was further stirred at room temperature for 2 hours. After completion of the reaction, the reaction solution was filtrated, the filtrated crude crystal was washed with methanol to give 51.0 g (yield 68%) of the title compound as yellow crystal.

### Reference example 2: 7-chloro-4(3H)-quinazolone

2-Amino-4-chlorobenzamide (25.6 g, 0.150 mol) obtained in Reference example 1 was dissolved in trimethyl orthoformate (560 ml), and to this added was concentrated hydrochloric acid (15 ml), and the mixture was stirred at room temperature for 1 hour. After completion of the reaction, the reaction solution was filtered, and the crude crystal filtered was suspended in water (250 ml) and neutralized with 3N NaOH aqueous solution. The neutralized solution was filtered, the solid being washed with water on the funnel to give 20.9 g (yield 77%) of the title compound as white crystal.

### Reference example 3: 7-chloro-6-nitro-4(3H)-quinazolone

7-Chloro-4(3H)-quinazolone (20.9 g, 0.116 mmol) obtained in Reference example 2 was dissolved in a mixed solution of concentrated sulfuric acid (45 ml) and fuming nitric acid (45 ml) and stirred at 80 °C for 2 hours. After completion of the reaction, the reaction solution was cooled to room temperature and poured into ice water (900 ml). The precipitated solid was filtered off, and the filtered crude crystal was suspended in acetic acid (440 ml) and stirred at 80 °C for 1 hour. After cooling to 40 °C, the precipitated crystal was subjected to filtration and washed with water on the funnel to give 15.7 g (yied 60%) of the title compound as pale yellow crystal.

### Reference example 4: 4,7-dichloro-6-nitro-4(3H)-quinazolone

7-Chloro-6-nitro-4(3H)-quinazolone (1.00 g, 4.43 mmol) obtained in Reference example 3 was added in phosphorus oxychloride (22 ml) and heated under reflux for 1 hour. After completion of the reaction, the reaction solution was cooled to room temperature, the solvent was removed under reduced pressure, the residue was added with a saturated sodium bicarbonate aqueous solution, and the solution was extracted with chloroform. The organic layer was washed with a 10% saline and dried, the solvent being removed. The precipitated crystal was washed with ethanol to give 888 mg (yield 82%) of the title compound as pale yellow crystal.

### Reference example 5: 7-chloro-4-[2-(4-chlorophenyl)ethylamino]-6-nitroquinazoline

4,7-Dichloro-6-nitro-4(3H)-quinazolone (5.95 g, 24.4 mmol) obtained in Reference example 4 was suspended in ethanol (150 ml). To this suspension, 2-(4-chlorophenyl)ethylamine (4.07 ml, 29.3 mmol) and triethylamine (4.08 ml, 29.3 mmol) were added successively, and the mixture was heated under reflux for 5 hours. The reaction solution was cooled to room temperature, the solvent was removed under reduced pressure, and the residue was washed with dichloromethane to give 7.32 g (yield 83%) of the title compound as yellow crystal.

### Reference example 6: 7-chloro-4-(phenethylamino)-6-nitroquinazoline

Using the compound obtained in Reference example 4 as starting material, the title compound was obtained according to the same method as Reference example 5.

### Reference example 7: 7-chloro-4-(benzylamino)-6-nitroquinazoline

Using the compound obtained in Reference example 4 as starting material, the title compound was obtained according to the same method as Reference example 5.

### Reference example 8: 7-chloro-4-(4-fluorobenzylamino)-6-nitroquinazoline

Using the compound obtained in Reference example 4 as starting material, the title compound was obtained according to the same method as Reference example 5.

### Reference example 9: 7-chloro-4-[3,4-(methylenedioxy)benzylamino]-6-nitroquinazoline

Using the compound obtained in Reference example 4 as starting material, the title compound was obtained according to the same method as Reference example 5.

### Reference example 10: 7-chloro-6-nitro-4-[3-(phenyl)propylamino]quinazoline

Using the compound obtained in Reference example 4 as starting material, the title compound was obtained according to the same method as Reference example 5.

### Reference example 11: 7-chloro-6-nitro-4-[(2-pyridyl)methylamino]quinazoline

Using the compound obtained in Reference example 4 as starting material, the title compound was obtained according to the same method as Reference example 5.

### Reference example 12: 7-chloro-6-nitro-4-[2-(2-pyridyl)ethylamino]quinazoline

Using the compound obtained in Reference example 4 as starting material, the title compound was obtained according to the same method as Reference example 5.

### Reference example 13: 7-chloro-6-nitro-4-[2-(3-pyridyl)ethylamino]quinazoline

Using the compound obtained in Reference example 4 as starting material, the title compound was obtained according to the same method as Reference example 5.

### Reference example 14: 7-chloro-6-vitro-4-[2-(4-pyridyl)ethylamino]quinazoline

Using the compound obtained in Reference example 4 as starting material, the title compound was obtained according to the same method as Reference example 5.

### Reference example 15: 7-chloro-6-nitro-4-[(2-thienyl)methylamino]quinazoline

Using the compound obtained in Reference example 4 as starting material, the title compound was obtained according to the same method as Reference example 5.

### Reference example 16: 7-chloro-6-nitro-4-[2-(2-thienyl)ethylamino]quinazoline

Using the compound obtained in Reference example 4 as starting material, the title compound was obtained according to the same method as Reference example 5.

### Reference example 17: 7-chloro-4-[(2-furyl)methylamino]-6-nitroquinazoline

Using the compound obtained in Reference example 4 as starting material, the title compound was obtained according to the same method as Reference example 5.

### Reference example 18: 7-chloro-4-[(1-naphthyl)methylamino]-6-nitroquinazoline

Using the compound obtained in Reference example 4 as starting material, the title compound was obtained according to the same method as Reference example 5.

### Reference example 19: 7-chloro-4-(4-chlorobenzylamino)-6-nitroquinazoline

Using the compound obtained in Reference example 4 as starting material, the title compound was obtained according to the same method as Reference example 5.

### Reference example 20: 7-chloro-4-(2-fluorobenzylamino)-6-nitroquinazoline

Using the compound obtained in Reference example 4 as starting material, the title compound was obtained according to the same method as Reference example 5.

### Reference example 21: 7-chloro-4-(3-fluorobenzylamino)-6-nitroquinazoline

Using the compound obtained in Reference example 4 as starting material, the title compound was obtained according to the same method as Reference example 5.

### Reference example 22: 7-chloro-6-nitro-4-[4-(1,1,1-trifluoromethyl)benzylamino]quinazoline

Using the compound obtained in Reference example 4 as starting material, the title compound was obtained according to the same method as Reference example 5.

### Reference example 23: 7-chloro-4-(4-methylbenzylamino)-6-nitroquinazoline

Using the compound obtained in Reference example 4 as starting material, the title compound was obtained according to the same method as Reference example 5.

### Reference example 24: 7-chloro-4-(2-methoxybenzylamino)-6-nitroquinazoline

Using the compound obtained in Reference example 4 as starting material, the title compound was obtained according to the same method as Reference example 5.

### Reference example 25: 7-chloro-4-(3-methoxybenzylamino)-6-nitroquinazoline

Using the compound obtained in Reference example 4 as starting material, the title compound was obtained according to the same method as Reference example 5.

### Reference example 26: 7-chloro-4-(4-methoxybenzylamino)-6-nitroquinazoline

Using the compound obtained in Reference example 4 as starting material, the title compound was obtained according to the same method as Reference example 5.

### Reference example 27: 7-chloro-4-(3,4-dimethoxybenzylamino)-6-nitroquinazoline

Using the compound obtained in Reference example 4 as starting material, the title compound was obtained according to the same method as Reference example 5.

### Reference example 28: 7-chloro-6-nitro-4-(3,4,5-trimethoxybenzylamino)quinazoline

Using the compound obtained in Reference example 4 as starting material, the title compound was obtained according to the same method as Reference example 5.

### Reference example 29: 7-chloro-4-[2-(2-chlorophenyl)ethylamino]-6-nitroquinazoline

Using the compound obtained in Reference example 4 as starting material, the title compound was obtained according to the same method as Reference example 5.

### Reference example 30: 7-chloro-4-[2-(3-chlorophenyl)ethylamino]-6-nitroquinazoline

Using the compound obtained in Reference example 4 as starting material, the title compound was obtained according to the same method as Reference example 5.

### Reference example 31: 4-[2-(4-bromophenyl)ethylamino]-7-chloro-6-nitroquinazoline

Using the compound obtained in Reference example 4 as starting material, the title compound was obtained according to the same method as Reference example 5.

### Reference example 32: 7-chloro-4-[2-(2-fluorophenyl)ethylamino]-6-nitroquinazoline

Using the compound obtained in Reference example 4 as starting material, the title compound was obtained according to the same method as Reference example 5.

### Reference example 33: 7-chloro-4-[2-(3-fluorophenyl)ethylamino]-6-nitroquinazoline

Using the compound obtained in Reference example 4 as starting material, the title compound was obtained according to the same method as Reference example 5.

### Reference example 34: 7-chloro-4-[2-(4-fluorophenyl)ethylamino]-6-nitroquinazoline

Using the compound obtained in Reference example 4 as starting material, the title compound was obtained according to the same method as Reference example 5.

### Reference example 35: 7-chloro-4-[2-(4-methylphenyl)ethylamino]-6-nitroquinazoline

Using the compound obtained in Reference example 4 as starting material, the title compound was obtained according to the same method as Reference example 5.

### Reference example 36: 7-chloro-4-[2-(2-methoxyphenyl)ethylamino]-6-nitroquinazoline

Using the compound obtained in Reference example 4 as starting material, the title compound was obtained according to the same method as Reference example 5.

### Reference example 37: 7-chloro-4-[2-(3-methoxyphenyl)ethylamino]-6-nitroquinazoline

Using the compound obtained in Reference example 4 as starting material, the title compound was obtained according to the same method as Reference example 5.

### Reference example 38: 7-chloro-4-[2-(4-methoxyphenyl)ethylamino]-6-nitroquinazoline

Using the compound obtained in Reference example 4 as starting material, the title compound was obtained according to the same method as Reference example 5.

### Reference example 39: 7-chloro-4-[2-(3,4-dimethoxyphenyl)ethylamino]-6-nitroquinazoline

Using the compound obtained in Reference example 4 as starting material, the title compound was obtained according to the same method as Reference example 5.

### Reference example 40: 7-chloro-4-[2-(4-hydroxyphenyl)ethylamino]-6-nitroquinazoline

Using the compound obtained in Reference example 4 as starting material, the title compound was obtained according to the same method as Reference example 5.

### Reference example 41: 4-[2-(4-anilino)ethylamino]-7-chloro-6-nitroquinazoline

Using the compound obtained in Reference example 4 as starting material, the title compound was obtained according to the same method as Reference example 5.

### Reference example 42: 7-chloro-4-(2-methyl-2-phenylethylamino)-6-nitroquinazoline

Using the compound obtained in Reference example 4 as starting material, the title compound was obtained according to the same method as Reference example 5.

### Reference example 43: 7-chloro-4-(2,2-diphenylethylamino)-6-nitroquinazoline

Using the compound obtained in Reference example 4 as starting material, the title compound was obtained according to the same method as Reference example 5.

### Reference example 44: 7-chloro-4-(N-methylbenzylamino)-6-nitroquinazoline

Using the compound obtained in Reference example 4 as starting material, the title compound was obtained according to the same method as Reference example 5.

### Reference example 45: 7-chloro-4-(dibenzylamino)-6-nitroquinazoline

Using the compound obtained in Reference example 4 as starting material, the title compound was obtained according to the same method as Reference example 5.

### Reference example 46: 4-[(1-benzyl-4-piperidyl)amino-7-chloro-6-nitroquinazoline

Using the compound obtained in Reference example 4 as starting material, the title compound was obtained according to the same method as Reference example 5.

### Reference example 47: N-(1-benzyl-4-piperidyl)-tert-butoxyformamide

The title compound was obtained by reacting 4-amino-1-benzylpiperidine with di-tert-butyl dicarbonate in THF at room temperature for 3 hours in the presence of triethylamine.

### Reference example 48: tert-butoxy-N-(4-piperidyl)formamide

Using the compound obtained in Reference example 47 as starting material, the title compound was obtained by catalytic hydrogenation reduction in ethanol using 5% palladium on carbon as the catalyst.

### Reference example 49: N-[1-(4-methoxybenzyl-4-piperidyl)]-tert-butoxyformamide

Using the compound obtained in Reference example 48 as starting material, the title compound was obtained by reacting it with 4-methoxybenzyl chloride in dimethylformamide in the presence of potassium carbonate.

### Reference example 50: 1-(4-methoxybenzyl)-4-piperidylamine

Using the compound obtained in Reference example 49 as starting material, the title compound was obtained by performing the reaction in a 4N hydrochloric acid-dioxane solution.

### Reference example 51: N-(1-benzoyl-piperidyl)-tert-butoxyformamide

Using the compound obtained in Reference example 48 as starting material, the title compound was obtained by reacting it with benzoyl chloride in dichloromethane in the presence of triethylamine.

### Reference example 52: 4-aminopiperidyl-phenylketone

Using the compound obtained in Reference example 51 as starting material, the title compound was obtained by performing the reaction in a 4N hydrochloric acid-dioxane solution.

### Reference example 53: N-(1-(3,4-methylenedioxy)benzoyl-piperidyl)-tert-butoxyformamide

Using the compound obtained in Reference example 48 as starting material, the title compound was obtained by reacting it with piperonyloyl chloride in dichloromethane in the presence of triethylamine.

### Reference example 54: 4-aminopiperidyl-[(3,4-methylenedioxy)phenyl]ketone

Using the compound obtained in Reference example 53 as starting material, the title compound was obtained by performing the reaction in a 4N hydrochloric acid-dioxane solution.

### Reference example 55: N-(1-nicotinoyl-piperidyl)-tert-butoxyformamide

Using the compound obtained in Reference example 48 as starting material, the title compound was obtained by reacting it with nicotinoyl chloride in dichloromethane in the presence of triethylamine.

### Reference example 56: 4-aminopiperidyl-(3-pyridyl)ketone

Using the compound obtained in Reference example 55 as starting material, the title compound was obtained by performing the reaction in a 4N hydrochloric acid-dioxane solution.

### Reference example 57: N-[1-(N-phenylcarbamoyl)-4-piperidyl)-tert-butoxyformamide

Using the compound obtained in Reference example 48 as starting material, the title compound was obtained by reacting it with phenylisocyanate in acetonitrile.

### Reference example 58: N-phenyl-4-aminopiperidylformamide

Using the compound obtained in Reference example 49 as starting material, the title compound was obtained by performing the reaction in a 4N hydrochloric acid-dioxane solution.

### Reference example 59: N-[1-[N-(4-fluorophenyl)carbamoyl]-4-piperidyl)-tert-butoxyformamide

Using the compound obtained in Reference example 48 as starting material, the title compound was obtained by reacting it with 4-fluorophenylisocyanate in acetonitrile.

### Reference example 60: N-(4-fluorophenyl)-4-aminopiperidylformamide

Using the compound obtained in Reference example 59 as starting material, the title compound was obtained by performing the reaction in a 4N hydrochloric acid-dioxane solution.

### Reference example 61: N-[1-[N-(4-methoxyphenyl)carbamoyl]-4-piperidyl]-tert-butoxyformamide

Using the compound obtained in Reference example 48 as starting material, the title compound was obtained by reacting it with 4-methoxyphenyl isocyanate in acetonitrile.

### Reference example 62: N-(4-methoxyphenyl)-4-aminopiperidylformamide

Using the compound obtained in Reference example 61 as starting material, the title compound was obtained by performing the reaction in a 4N hydrochloric acid-dioxane solution.

### Reference example 63: N-[1-((phenylamino)thioxomethyl-4-piperidyl]-tert-butoxyformamide

Using the compound obtained in Reference example 48 as starting material, the title compound was obtained by reacting it with phenyl isothiocyanate in acetonitrile.

### Reference example 64: (4-aminopiperidyl)(phenylamino) methane-1-thione

Using the compound obtained in Reference example 63 as starting material, the title compound was obtained by performing the reaction in a 4N hydrochloric acid-dioxane solution.

### Reference example 65: N-[1-(phenylsulfonyl)-4-piperidyl]-tert-butoxyformamide

Using the compound obtained in Reference example 48 as starting material, the title compound was obtained by reacting it with benzenesulfonyl chloride in dichloromethane in the presence of triethylamine.

### Reference example 66: 1-(phenylsulfonyl)-4-piperidylamine

Using the compound obtained in Reference example 65 as starting material, the title compound was obtained by performing the reaction in a 4N hydrochloric acid-dioxane solution.

### Reference example 67: N-[1-(4-methoxyphenylsulfonyl)-4-piperidyl]-tert-butoxyformamide

Using the compound obtained in Reference example 48 as starting material, the title compound was obtained by reacting it with 4-methoxybenzenesulfonyl chloride in dichloromethane in the presence of triethylamine.

### Reference example 68: 1-(4-methoxyphenylsulfonyl)-4-piperidylamine

Using the compound obtained in Reference example 67 as starting material, the title compound was obtained by performing the reaction in a 4N hydrochloric acid-dioxane solution.

### Reference example 69: N-[1-(4-fluorophenylsulfonyl)-4-piperidyl]-tert-butoxyformamide

Using the compound obtained in Reference example 48 as starting material, the title compound was obtained by reacting it with 4-fluorobenzenesulfonyl chloride in dichloromethane in the presence of triethylamine.

### Reference example 70: 1-(4-fluorophenylsulfonyl)-4-piperidylamine

Using the compound obtained in Reference example 69 as starting material, the title compound was obtained by performing the reaction in a 4N hydrochloric acid-dioxane solution.

### Reference example 71: 7-chloro-6-nitro-4-(4-phenylpiperazinyl)quinazoline

4,7-Dichloro-6-nitro-4(3H)-quinazolone (138 mg, 0.576 mmol) obtained in Reference example 4 was suspended in ethanol (3 ml). To this suspension, 1-(4-chlorophenyl)piperazine dihydrochloride (186 mg, 0.689 mmol) and N,N-diisopropylethylamine (360 µl, 2.07 mmol) were added successively, and the mixture was stirred at room temperature over night. After completion of the reaction, the reaction solution was filtered, and the crude crystal was washed with ethanol and water to give 72 mg (yield 31%) of the title compound as yellow crystal.

### Reference example 72: 4-benzylpiperazinylphenylketone

Using 1-benzylpiperazine as starting material, the title compound was obtained by reacting it with benzoyl chloride in dichloromethane in the presence of triethylamine.

### Reference example 73: piperazinylphenylketone

Using the compound obtained in Reference example 72 as starting material, the title compound was obtained by catalytic hydrogenation reduction in ethanol using 5% palladium on carbon as the catalyst.

### Reference example 74: 4-(7-chloro-6-nitroquinazolin-4-yl)piperazinyl-4-phenylketone

Using the compound obtained in Reference example 73, the title compound was obtained according to the same method as Reference example 71.

### Reference example 75: 4-benzylpiperazinyl-4-fluorophenylketone

Using 1-benzylpiperazine as starting material, the title compound was obtained by reacting it with 4-fluorobenzoyl chloride in dichloromethane in the presence of triethylamine.

### Reference example 76: 4-fluorophenyl-piperazinylketone

Using the compound obtained in Reference example 75 as starting material, the title compound was obtained by catalytic hydrogenation reduction in ethanol using 5% palladium on carbon as the catalyst.

### Reference example 77: 4-(7-chloro-6-nitroquinazolin-4-yl) piperazinyl-4-fluorophenylketone

Using the compound obtained in Reference example 76 as starting material, the title compound was obtained according to the same method as Reference example 71.

### Reference example 78: 4-benzylpiperazinyl-4-methoxyphenylketone

Using 1-benzylpiperazine as starting material, the title compound was obtained by reacting it with 4-methoxybenzoyl chloride in dichloromethane in the presence of triethylamine.

### Reference example 79: 4-methoxyphenyl-piperazinylketone

Using the compound obtained in Reference example 78 as starting material, the title compound was obtained by catalytic hydrogenation reduction in ethanol using 5% palladium on carbon as the catalyst.

### Reference example 80: 4-(7-chloro-6-nitroquinazolin-4-yl) piperazinyl-4-methoxyphenylketone

Using the compound obtained in Reference example 79 as starting material, the title compound was obtained according to the same method as Reference example 71.

### Reference example 81: 4-benzylpiperazinyl-4-(3,4-methylenedioxy)phenylketone

Using 1-benzylpiperazine as starting material, the title compound was obtained by reacting it with piperonyloyl chloride in dichloromethane in the presence of triethylamine.

### Reference example 82: (3,4-methylenedioxy)phenyl-piperazinylketone

Using the compound obtained in Reference example 81 as starting material, the title compound was obtained by catalytic hydrogenation reduction in ethanol using 5% palladium on carbon as the catalyst.

### Reference example 83: 4-(7-chloro-6-nitroquinazolin-4-yl) piperazinyl-(3,4-methylenedioxy)phenylketone

Using the compound obtained in Reference example 82 as starting material, the title compound was obtained according to the same method as Reference example 71. Reference example 84: 4-(phenylsulfonyl)-1-benzylpiperazine Using 1-benzylpiperazine as starting material, the title compound was obtained by reacting it with benzenesulfonyl chloride in dichloromethane in the presence of triethylamine.

### Reference example 85: phenylsulfonyl piperazine

Using the compound obtained in Reference example 84 as starting material, the title compound was obtained by catalytic hydrogenation reduction in ethanol using 5% palladium on carbon as the catalyst.

### Reference example 86: 1-(7-chloro-6-nitroquinazolin-4-yl)-4-(phenylsulfonyl)piperazine

Using the compound obtained in Reference example 85 as starting material, the title compound was obtained according to the same method as Reference example 71.

### Reference example 87: 4-(4-fluorophenylsulfonyl)-1-benzylpiperazine

Using 1-benzylpiperazine as starting material, the title compound was obtained by reacting it with 4-fluorobenzenesulfonyl chloride in dichloromethane in the presence of triethylamine.

### Reference example 88: 4-fluorophenylsulfonyl piperazine

Using the compound obtained in Reference example 87 as starting material, the title compound was obtained by catalytic hydrogenation reduction in ethanol using 5% palladium on carbon as the catalyst.

### Reference example 89: 1-(7-chloro-6-nitroquinazolin-4-yl)-4-[(4-fluorophenyl)sulfonyl]piperazine

Using the compound obtained in Reference example 88 as starting material, the title compound was obtained according to the same method as Reference example 71.

### Reference example 90: 2-amino-4,5-difluorobenzamide

Using 4,5-difluoroanthranilic acid as starting material, the title compound was obtained according to the same method as Reference example 1.

### Reference example 91: 6,7-difluoro-4(3H)-quinazolone

Using the compound obtained in Reference example 90 as starting material, the title compound was obtained according to the same method as Reference example 2.

### Reference example 92: 4-chloro-6,7-difluoro-4(3H)-quinazolone

Using the compound obtained in Reference example 91 as starting material, the title compound was obtained according to the same method as Reference example 4.

### Reference example 93: 6,7-difluoro-4-[2-(4-chlorophenyl)ethylamino]quinazoline

Using the compound obtained in Reference example 92 as starting material, the title compound was obtained according to the same method as Reference example 5.

### Reference example 94: 6,7-difluoro-4-[2-(4-fluorophenyl)ethylamino]quinazoline

Using the compound obtained in Reference example 92 as starting material, the title compound was obtained according to the same method as Reference example 5.

### Reference example 95: 6,7-difluoro-4-(4-fluorobenzylamino)quinazoline

Using the compound obtained in Reference example 92 as starting material, the title compound was obtained according to the same method as Reference example 5.

### Reference example 96: 6,7-difluoro-4-[3,4-(methylenedioxy)benzylamino)quinazoline

Using the compound obtained in Reference example 92 as starting material, the title compound was obtained according to the same method as Reference example 5.

### Reference example 97: 6,7-difluoro-4-[(4-fluoro)anilino]quinazoline

Using the compound obtained in Reference example 92 as starting material, the title compound was obtained according to the same method as Reference example 5.

### Reference example 98: 6,7-difluoro-4-[(3,4-methylenedioxy)anilino]quinazoline

Using the compound obtained in Reference example 92 as starting material, the title compound was obtained according to the same method as Reference example 5.

### Reference example 99: 6,7-difluoro-4-[2-[(4-fluoro)phenyl]ethylamino]quinazoline

Using the compound obtained in Reference example 92 as starting material, the title compound was obtained according to the same method as Reference example 5.

### Reference example 100: 6,7-difluoro-4-[2-[(3,4-methylenedioxy)phenyl]ethylamino]quinazoline

Using the compound obtained in Reference example 92 as starting material, the title compound was obtained according to the same method as Reference example 5.

### Reference example 101: 6,7-difluoro-4-[3-[(4-fluoro)phenyl] propylamino]quinazoline

Using the compound obtained in Reference example 92 as starting material, the title compound was obtained according to the same method as Reference example 5.

### Reference example 102: 6,7-difluoro-4-[3-[(3,4-methylenedioxy)phenyl]propylamino]quinazoline

Using the compound obtained in Reference example 92 as starting material, the title compound was obtained according to the same method as Reference example 5.

### Reference example 103: 6,7-difluoro-4-[(3,4-ethylenedioxy)benzylamino]quinazoline

Using the compound obtained in Reference example 92 as starting material, the title compound was obtained according to the same method as Reference example 5.

### Reference example 104: 6,7-difluoro-4-[[N-methyl-N'-(3,4-methylenedioxybenzyl)]amino]quinazoline

Using the compound obtained in Reference example 92 as starting material, the title compound was obtained according to the same method as Reference example 5.

### Reference example 105: 7-chloro-4-[(4-fluoro)anilino]-6-nitroquinazoline

Using the compound obtained in Reference example 4 as starting material, the title compound was obtained according to the same method as Reference example 5.

### Reference example 106: 7-chloro-4-[(3,4-methylenedioxy)anilino]-6-nitroquinazoline

Using the compound obtained in Reference example 4 as starting material, the title compound was obtained according to the same method as Reference example 5.

### Reference example 107: 7-chloro-4-[2-(3,4-methylenedioxy)phenyl]ethylamino]-6-nitroquinazoline

Using the compound obtained in Reference example 4 as starting material, the title compound was obtained according to the same method as Reference example 5.

### Reference example 108: 7-chloro-4-[3-[(4-fluoro)phenyl]propylamino]-6-nitroquinazoline

Using the compound obtained in Reference example 4 as starting material, the title compound was obtained according to the same method as Reference example 5.

### Reference example 109: 7-chloro-4-[3-[(3,4-methylenedioxy)phenyl]propylamino]-6-nitroquinazoline

Using the compound obtained in Reference example 4 as starting material, the title compound was obtained according to the same method as Reference example 5.

### Reference example 110: 7-chloro-4-[(3,4-difluoro)benzylamino]-6-nitroquinazoline

Using the compound obtained in Reference example 4 as starting material, the title compound was obtained according to the same method as Reference example 5.

### Reference example 111: 7-chloro-4-[(4-ethoxycarbonyl)benzylamino]-6-nitroquinazoline

Using the compound obtained in Reference example 4 as starting material, the title compound was obtained according to the same method as Reference example 5.

### Reference example 112: 7-chloro-4-[(3,4-ethylenedioxy)benzylamino]-6-nitroquinazoline

Using the compound obtained in Reference example 4 as starting material, the title compound was obtained according to the same method as Reference example 5.

### Reference example 113: 7-chloro-4-[(3,5-di-tert-butyl-4-hydroxy)benzylamino]-6-nitroquinazoline

Using the compound obtained in Reference example 4 as starting material, the title compound was obtained according to the same method as Reference example 5.

### Reference example 114: 7-chloro-4-[(3-methoxy-4-propoxy)benzylamino]-6-nitroquinazoline

Using the compound obtained in Reference example 4 as starting material, the title compound was obtained according to the same method as Reference example 5.

### Reference example 115: 7-chloro-4-[(4-cyclopentyloxy-3-methoxy)benzylamino]-6-nitroquinazoline

Using the compound obtained in Reference example 4 as starting material, the title compound was obtained according to the same method as Reference example 5.

### Reference example 116: 7-chloro-6-nitro-4-[(4-nitro) benzylamino]quinazoline

Using the compound obtained in Reference example 4 as starting material, the title compound was obtained according to the same method as Reference example 5.

### Reference example 117: 7-chloro-4-[[6-chloro-(3,4-methylenedioxy)]benzylamino]-6-nitroquinazoline

Using the compound obtained in Reference example 4 as starting material, the title compound was obtained according to the same method as Reference example 5.

### Reference example 118: 7-chloro-4-[[5-methoxy-(3,4-methylenedioxy)]benzylamino]-6-nitroquinazoline

Using the compound obtained in Reference example 4 as starting material, the title compound was obtained according to the same method as Reference example 5.

### Reference example 119: 4-[(4-benzyloxy)benzylamino]-7-chloro-6-nitroquinazoline

Using the compound obtained in Reference example 4 as starting material, the title compound was obtained according to the same method as Reference example 5.

### Reference example 120: 4-[(4-acetamide)benzylamino]-7-chloro-6-nitroquinazoline

Using the compound obtained in Reference example 4 as starting material, the title compound was obtained according to the same method as Reference example 5.

### Reference example 121: 7-chloro-4-[(4-cyclopropylcarbamoyl)benzylamino]-6-nitroquinazoline

Using the compound obtained in Reference example 4 as starting material, the title compound was obtained according to the same method as Reference example 5.

### Reference example 122: 7-chloro-4-[(3,5-dimethyl-4-hydroxy)benzylamino]-6-nitroquinazoline

Using the compound obtained in Reference example 4 as starting material, the title compound was obtained according to the same method as Reference example 5.

### Reference example 123: 7-chloro-4-[(3-cyclopentyloxy-4-methoxy)benzylamino]-6-nitroquinazoline

Using the compound obtained in Reference example 4 as starting material, the title compound was obtained according to the same method as Reference example 5.

### Reference example 124: 7-chloro-4-[(4-methoxy-3-pivaloyloxy)benzylamino]-6-nitroquinazoline

Using the compound obtained in Reference example 4 as starting material, the . title compound was obtained according to the same method as Reference example 5.

### Reference example 125: 7-chloro-4-[(4-hydroxy)benzylamino]-6-nitroquinazoline

Using the compound obtained in Reference example 4 as starting material, the title compound was obtained according to the same method as Reference example 5.

### Reference example 126: 7-chloro-4-[(4-cyano)benzylamino]-6-nitroquinazoline

Using the compound obtained in Reference example 4 as starting material, the title compound was obtained according to the same method as Reference example 5.

### Example 1: 4-[2-(4-chlorophenyl)ethylamino]-6-nitro-7-(piperazin-1-yl)quinazoline (compound 1)

7-Chloro-4-[2-(4-chlorophenyl)ethylamino]-6-nitroquinazoline (800 mg, 2.20 mmol) obtained in Reference example 5 was suspended in n-butanol (22 ml). To this suspension, anhydrous piperazine (569 mg, 6.60 mmol) and N,N-diisopropylethylamine (575 µl, 3.30 mmol) were added, and the mixture was stirred at 120 °C for 7 hours. After completion of the reaction, the solvent was distilled off under reduced pressure, the residue was added with water (40 ml), and the solution was stirred at room temperature for 1 hour. The suspension was filtered to give 735 mg (yield 81%) of the title compound as yellow orange crystal.
¹H-NMR(DMSO-d₆) δ(ppm): 8.87(s,1H), 8.68-8.64(br.t,1H), 8.48(s,1H), 7.37-7.26(m,4H), 7.21(s,1H), 3.74 (dt,2H, J=6.3,5.9Hz), 3.07-3.02(m,5H), 2.97-2.92(m,6H)

### Example 2: 4-[2-(4-chlorophenyl)ethylamino]-7-(homopiperazin-1-yl)-6-nitroquinazoline (compound 2)

7-Chloro-4-[2-(4-chlorophenyl)ethylamino]-6-nitroquinazoline (100 mg, 0.275 mmol) obtained in Reference example 5 was suspended in n-butanol (6 ml). To this suspension, homopiperazine (83 mg, 0.825 mmol) and N,N-diisopropylethylamine (72 µl, 0.413 mmol) were added, and the mixture was stirred at 120 °C for 16 hours. After completion of the reaction, the solvent was distilled off under reduced pressure, the residue was added with a mixed solution of dichloromethane and hexane, and the solution was stirred at room temperature for 30 minutes. The suspension was filtered to give 106 mg (yield 90%) of the title compound as yellow orange crystal.
¹H-NMR(DMSO-d₆) δ(ppm): 8.79(s,1H), 8.60-8.57(br.-t,1H), 8.41(s,1H), 7.37-7.26(m,4H), 7.14(s,1H), 3.76-3.69(m,2H),3.42-3.33(m,2H), 2.98-2.93(m,9H), 1.93-1.91(m,2H)

### Example 3: 4-[2-(4-chlorophenyl)ethylamino]-7-(4-methylpiperazin-1-yl)-6-nitroquinazoline (compound 3)

7-Chloro-4-[2-(4-chlorophenyl)ethylamino]-6-nitroquinazoline (100 mg, 0.275 mmol) obtained in Reference example 5 was suspended in n-butanol (6 ml). To this suspension, 1-methylpiperazine (92 µl, 0.825 mmol) and N,N-diisopropylethylamine (144 µl, 0.825 mmol) were added, and the mixture was stirred at 120 °C for 13 hours. After completion of the reaction, the solvent was distilled off under reduced pressure, the residue was added with a mixed solution of dichloromethane and hexane, and the solution was stirred at room temperature for 30 minutes. The suspension was filtered to give 73 mg (yield 62%) of the title compound as yellow orange crystal.
¹H-NMR(DMSO-d₆) δ(ppm): 8.84(s,1H), 8.64-8.62(br.-t,1H), 8.47(s,1H), 7.36-7.62(m,4H), 7.20(s,1H), 3.72(dt,2H, J=7.0,5.9Hz), 3.08-3.05(m,4H), 2.94(t,2H,7.0Hz), 2.50-2.45(m,4H), 2.23(s,3H)

### Example 4: 4-[2-(4-chlorophenyl)ethylamino]-7-(4-ethylpiperazin-1-yl)-6-nitroquinazoline (compound 4)

7-Chloro-4-[2-(4-chlorophenyl)ethylamino]-6-nitroquinazoline (70 mg, 0.193 mmol) obtained in Reference example 5 was suspended in n-butanol (5 ml). To this suspension, 1-ethylpiperazine (123 µl, 0.965 mmol) and N,N-diisopropylethylamine (202 µl, 1.16 mmol) were added, and the mixture was stirred at 120 °C for 8 hours. After completion of the reaction, the solvent was distilled off under reduced pressure, the residue was added with water (10 ml), and the solution was stirred at room temperature for 30 minutes. The suspension was filtered to give 61 mg (yield 72%) of the title compound as yellow orange crystal.
¹H-NMR(CDCl₃) δ(ppm): 8.65(s,1H), 8.10(s,1H), 7.40-7.28(m,5H), 5.74-5.72(bt.-t,1H), 3.85(dt,2H, J=6.8,6.2Hz), 3.20-3.18(m,4H), 3.00(t,2H,J=6.8Hz), 2.72-2.70(m,4H), 2.54(q,2H, J=7.1Hz), 1.17(t,3H.J=7.0Hz)

### Example 5: 4-[2-(4-chlorophenyl)ethylamino]-7-[4-(2-hydroxyethyl)piperazin-1-yl)-6-nitroquinazoline (compound 5)

7-Chloro-4-[2-(4-chlorophenyl)ethylamino]-6-nitroquinazoline (70 mg, 0.193 mmol) obtained in Reference example 5 was suspended in n-butanol (5 ml). To this suspension, 1-(2-hydroxyethyl)piperazine (330 µl, 2.69 mmol) and N,N-diisopropylethylamine (337 µl, 2.61 mmol) were added, and the mixture was stirred at 120 °C for 8 hours. After completion of the reaction, the solvent was distilled off under reduced pressure, the residue was added with water (10 ml), and the solution was stirred at room temperature for 30 minutes. The suspension was filtered to give 61 mg (yield 70%) of the title compound as yellow orange crystal.
¹H-NMR(DMSO-d₆) δ(ppm): 8.84(s,1H), 8.64-8.62(br.-t,1H), 8.47(s,1H), 7.36-7.26(m,4H), 7.19(s,1H), 4.44(t,1H,J=5.4Hz), 3.73(dt,2H, J=7.3,5.8Hz), 3.53(dt,2H,J=5.7,5.4Hz), 3.06-3.04(m,4H), 2.94(t,2H,7.3Hz), 2.47-2.43(m,6H)

### Example 6: 4-[2-(4-chlorophenyl)ethylamino]-7-(4-formylpiperazin-1-yl)-6-nitroquinazoline (compound 6)

To formic acid (20 ml), anhydrous acetic acid (2.2 ml, 2.42 mmol) was added and stirred at room temperature for 20 minutes. Then, the compound 1 (200 mg, 0.484 mmol) obtained in Example 1 and N,N-diisopropylethylamine (211 µl, 1.21 mmol) were added, and the mixture was stirred at 80 °C for 2 hours. After completion of the reaction, the solvent was distilled off under reduced pressure, and the residue was added with water (20 ml) and neutralized with 1N NaOH aqueous solution. The neutralized solution being distilled off under reduced pressure, the residue was added with methanol and water and stirred under ice-cooling for 30 minutes. The precipitated crystal was filtered off to give 196 mg (yield 92%) of the title compound as orange crystal. ¹H-NMR(DMSO-d₆) δ(ppm): 8.91(s,1H), 8.72-8.68(br.-t,1H), 8.49(s,1H), 8.08(s,1H), 7.36-7.27(m,5H), 3.74(dt,2H, J=6.3,5.9Hz), 3.52-3.51(m,4H), 3.13-2.92(m,6H)

### Example 7: 7-(4-acetylpiperazin-1-yl)-4-[2-(4-chlorophenyl)ethylamino]-6-nitroquinazoline (compound 7)

The compound 1 (80 mg, 0.194 mmol) obtained in Example 1 was suspended in dichloromethane (3 ml). To this suspension, anhydrous acetic acid (28 µl, 0.291 mmol) and pyridine(31 µl, 0.388 mmol) were added, and the mixture was stirred at room temperature for 4 hours. After completion of the reaction, the solvent was distilled off under reduced pressure, the residue was added with water (15 ml), and the solution was stirred at room temperature for 30 minutes. The precipitated solid was filtered off to give 69 mg (yield 78%) of the title compound as yellow crystal.
¹H-NMR(CDCl₃) δ(ppm): 8.66(s,1H), 8.13(s,1H), 7.41-7.29(m,5H), 5.84-5.82(bt.-t,1H), 3.95-3.90(m,2H), 3.87-3.83(m,2H), 3.72-3.68(m,2H), 3.20-3.07(m,6H), 2.20(s,3H)

### Example 8: 7-(4-aminopiperidin-1-yl)-4-[2-(4-chlorophenyl)ethylamino]-6-nitroquinazoline (compound 8)

7-Chloro-4-[2-(4-chlorophenyl)ethylamino]-6-nitroquinazoline (100 mg, 0.275 mmol) obtained in Reference example 5 was suspended in n-butanol (5 ml). To this suspension, tert-butoxy-N-(4-piperidyl)formamide (220 mg, 1.10 mmol) obtained in Reference example 48 and N,N-diisopropylethylamine (240 µl, 1.38 mmol) were added, and the mixture was stirred at 120 °C for 5 hours. After completion of the reaction, the solvent was distilled off under reduced pressure, the residue was added with water (15 ml), and the solution was stirred at room temperature for 30 minutes. The suspension was filtered, and then the obtained solid was dissolved in 4N hydrochloric acid-dioxane and stirred for 2 hours. The reaction solution was distilled off under reduced pressure, and the residue was neutralized with NaOH water. The precipitated solid was subjected to filtration to give 54 mg (yield 46%) of the title compound as yellow crystal.
¹H-NMR(DMSO-d₆) δ(ppm): 8.81(s,1H), 8.61-8.59(br.-t,1H), 8.45(s,1H), 7.36-7.29(m,4H), 7.18(s,1H), 3.73(dt,2H, J=7.2,6.0Hz), 3.28-3.26(m,3H), 2.97-2.91(m,5H), 1.80-1.76 (m,3H), 1.37-1.33(m,2H)

### Example 9: 7-(4-aminomethylpiperidin-1-yl)-4-[2-(4-chlorophenyl)ethylamino]-6-nitroquinazoline (compound 9)

7-Chloro-4-[2-(4-chlorophenyl)ethylamino]-6-nitroquinazoline (100 mg, 0.275 mmol) obtained in Reference example 5 was suspended in n-butanol (5 ml). To this suspension, 4-(aminomethyl)piperidine (157 mg, 1.38 mmol) and N,N-diisopropylethylamine (240 µl, 1.38 mmol) were added, and the mixture was stirred at 120 °C for 6 hours. After completion of the reaction, the solvent was distilled off under reduced pressure, the residue was added with water (20 ml), and the solution was stirred at room temperature for 30 minutes. The suspension was filtered to give 81 mg (yield 67%) of the title compound as yellow orange crystal.
¹H-NMR(DMSO-d₆) δ(ppm): 8.82(s,1H), 8.63-8.59(br.-t,1H), 8.45(s,1H), 7.36-7.29(m,4H), 7.18(s,1H), 3.73(dt,2H, J=7.2,5.7Hz), 3.28-3.26(m,2H), 2.94(t,2H,J=7.2Hz), 2.87-2.75(m,2H), 2.50-2.45(m,2H), 1.80-1.76 (m,2H), 1.59-1.15 (m,5H)

### Example 10: 6-nitro-4-[2-(phenyl)ethylamino]-7-(piperazin-1-yl)quinazoline (compound 10)

Using the compound obtained in Reference example 6 as starting material, the title compound was obtained according to the same method as Example 1.
¹H-NMR(DMSO-d₆) δ(ppm): 9.00(s,1H), 8.85-8.83(br.-t,1H), 8.51(s,1H), 7.33-7.18(m,6H), 3.76(dt,2H, J=7.2,6.0Hz), 3.19-3.15(m,5H), 2.99-2.92(m,6H)

### Example 11: 7-(homopiperazin-1-yl)-6-nitro-4-[2-(phenyl)ethylamino]quinazoline (compound 11)

Using the compound obtained in Reference example 6 as starting material, the title compound was obtained according to the same method as Example 2.
¹H-NMR(DMSO-d₆) δ(ppm): 8.77(s,1H), 8.59-8.57(br.-t,1H), 8.41(s,1H), 7.35-7.28(m,5H), 7.16(s,1H), 3.74-3.67(m,2H), 3.40-3.30(m,2H), 3.00-2.95(m,9H), 1.95-1.92(m,2H)

### Example 12: 7-(4-methylpiperazin-1-yl)-6-nitro-4-[2-(phenyl)ethylamino]quinazoline (compound 12)

Using the compound obtained in Reference example 6 as starting material, the title compound was obtained according to the same method as Example 3.
¹H-NMR(DMSO-d₆) δ(ppm): 8.95(s,1H), 8.87-8.85(br.-t,1H), 8.50(s,1H), 7.30-7.25(m,6H), 3.77(dt,2H,J=7.0,5.8Hz), 3.12-3.09(m,4H), 2.98-2.91(m,6H), 2.20(s,3H) Example 13: 7-(4-ethylpiperazin- 1-yl)-6-nitro-4-[2-(phenyl)ethylamino]quinazoline (compound 13)

Using the compound obtained in Reference example 6 as starting material, the title compound was obtained according to the same method as Example 4.
¹H-NMR(CDCl₃) δ(ppm): 8.63(s,1H), 8.06(s,1H), 7.37-7.26(m,6H), 5.70-5.69(br.-t,1H), 3.93(dt,2H,J=6.7,6.3Hz), 3.18-3.16 (m,4H), 3.03(t,2H,J=6.7Hz), 2.65-2.62(m,4H), 2.50(q,2H, J=7.0Hz), 1.13(t,3H,J=7.0Hz)

### Example 14: 7-(4-formylpiperazin-1-yl)-6-nitro-4-[2-(phenyl)ethylamino]quinazoline (compound 14)

Using the compound obtained in Reference example 6 as starting material, the title compound was obtained according to the same method as Example 6.
¹H-NMR(CDCl₃) δ(ppm): 8.67(s,1H), 8.17(s,1H), 8.12(s,1H) 7.65-7.24(m,6H), 5.82-5.80(br.-t,1H), 3.99-3.91(m,2H), 3.79-3.75(m,2H), 3.61-3.57 (m,2H), 3.18-3.02(m,6H)

### Example 15: 7-(4-acetylpiperazin-1-yl)-6-nitro-4-[2-(phenyl)ethylamino]quinazoline (compound 15)

Using the compound obtained in Reference example 6 as starting material, the title compound was obtained according to the same method as Example 7.
¹H-NMR(CDCl₃) δ(ppm): 8.66(s,1H), 8.16(s,1H), 7.38-7.24(m,6H), 5.83-5.81(br.-t,1H), 3.98-3.91(m,2H), 3.84-3.80 (m,2H), 3.69-3.65(m,2H), 3.17-3.02(m,6H), 2.15(s,3H)

### Example 16: 7-(4-aminopiperidin-1-yl)-6-nitro-4-[2-(phenyl)ethylamino]quinazoline (compound 16)

Using the compound obtained in Reference example 6 as starting material, the title compound was obtained according to the same method as Example 8.
¹H-NMR(DMSO-d₆) δ(ppm): 8.82(s,1H), 8.59-8.57(br.-t,1H), 8.40(s,1H), 7.37-7.30(m,5H), 7.20(s,1H), 3.73-3.70(m,2H),
3.30-3.27(m,3H), 2.98-2.94(m,5H), 1.85-1.80(m,3H), 1.35-1.32 (m,2H)

### Example 17: 7-(4-aminomethylpiperidin-1-yl)-6-nitro-4-[2-(phenyl)ethylamino]quinazoline (compound 17)

Using the compound obtained in Reference example 6 as starting material, the title compound was obtained according to the same method as Example 9.
¹H-NMR(DMSO-d₆) δ(ppm): 8.79(s,1H), 8.59-8.57(br.-t,1H) 8.33(s,1H), 7.35-7.28(m,5H), 7.20(s,1H), 3.75 (dt,2H, J=7.2,5.7Hz), 3.25-3.22(m,2H), 2.98(t,2H,J=7.2Hz), 2.84-2.80(m,2H), 2.52-2.47(m,2H), 1.78-1.74(m,2H), 1.60-1.55 (m,5H)

### Example 18: 4-(benzylamino)-6-nitro-7-(piperazin-1-yl)quinazoline (compound 18)

Using the compound obtained in Reference example 7 as starting material, the title compound was obtained according to the same method as Example 1.
¹H-NMR(CDCl₃) δ(ppm): 9.05-8.99(m,2H), 8.46(s,1H), 7.39-7.24(m,6H), 4.79(d,2H,J=5.4Hz), 3.14-3.08(m,8H), 2.69-2.63(m,1H)

### Example 19: 4-(benzylamino)-7-(homopiperazin-1-yl)-6-nitroquinazoline (compound 19)

Using the compound obtained in Reference example 7 as starting material, the title compound was obtained according to the same method as Example 2.
¹H-NMR(DMSO-d₆) δ(ppm): 8.57(s,1H), 8.10(s,1H), 7.41-7.28 (m,6H), 5.77-5.75(br.-t,1H), 4.78(d,2H,J=5.5Hz), 3.55-3.49 (m,2H), 3.39-3.35(m,3H), 3.10-3.05(m,2H), 3.00-2.95(m,2H), 2.01-1.93(m,2H)

### Example 20: 4-(benzylamino)-7-(4-methylpiperazin-1-yl)-6-nitroquinazoline (compound 20)

Using the compound obtained in Reference example 7 as starting material, the title compound was obtained according to the same method as Example 3.
¹H-NMR(DMSO-d₆) δ(ppm): 9.00-8.98(br.-t,1H), 8.97(s,1H), 8.47(s,1H), 7.40-7.28(m,6H), 4.80(d,2H,J=5.6Hz), 3.15-3.12(m,4H), 3.00-2.96(m,4H), 2.24(s,3H)

### Example 21: 4-(benzylamino)-7-[4-(2-hydroxyethyl) piperazin-1-yl]-6-nitroquinazoline (compound 21)

Using the compound obtained in Reference example 7 as starting material, the title compound was obtained according to the same method as Example 5.
¹H-NMR(DMSO-d₆) δ(ppm): 9.02-8.99(m,2H), 8.50(s,1H), 7.39-7.28(m,6H), 4.78(d,2H,J=5.4Hz), 4.50(br.-t,1H), 3.50-3.47 (m,2H), 3.08-3.05(m,4H), 2.51-2.44(m,6H)

### Example 22: 4-(benzylamino)-7-(4-formylpiperazin-1-yl)-6-nitroquinazoline (compound 22)

Using the compound obtained in Reference example 7 as starting material, the title compound was obtained according to the same method as Example 6.
¹H-NMR(CDCl₃) δ(ppm): 8.98-8.96(br.-t,1H), 8.93(s,1H), 8.37(s,1H), 7.46-7.37(m,7H), 4.68(d,2H,J=5.5Hz), 3.15-3.11(m,4H), 3.04-3.00(m,4H)

### Example 23: 7-(4-acetylpiperazin-1-yl)-4-(benzylamino)-6-nitroquinazoline (compound 23)

Using the compound obtained in Reference example 7 as starting material, the title compound was obtained according to the same method as Example 7.
¹H-NMR(CDCl₃) δ(ppm): 8.95-8.93(br.-t,1H), 8.91(s,1H), 8.50(s,1H), 7.38-7.29(m,6H), 4.74(d,2H,J=5.6Hz), 3.17-3.12(m,4H), 3.06-3.02(m,4H), 2.23(s,3H)

### Example 24: 7-(4-aminopiperidin-1-yl)-4-(benzylamino)-6-nitroquinazoline (compound 24)

Using the compound obtained in Reference example 7 as starting material, the title compound was obtained according to the same method as Example 8.
¹H-NMR(DMSO-d₆) δ(ppm): 9.02-8.97(m,2H), 8.51(s,1H), 7.42-7.31(m,6H), 4.74(d,2H,J=5.4Hz), 3.30-3.27(m,3H), 2.95-2.90 (m,3H), 1.82-1.78(m,3H), 1.32-1.28(m,2H)

### Example 25: 4-(4-fluorobenzylamino)-6-nitro-7-(piperazin-1-yl)quinazoline (compound 25)

Using the compound obtained in Reference example 8 as starting material, the title compound was obtained according to the same method as Example 1.
¹H-NMR(CDCl₃) δ(ppm): 8.93(s,1H), 8.82(br.-t,1H), 8.51(s,1H), 7.42-7.37(m,2H), 7.22(s,1H), 7.05-6.98(m,2H), 4.78(d,2H, J=5.1Hz), 3.09-3.07(m,4H), 3.04-3.02(m,5H)

### Example 26: 4-(4-fluorobenzylamino)-7-(homopiperazin-1-yl)-6-nitroquinazoline (compound 26)

Using the compound obtained in Reference example 8 as starting material, the title compound was obtained according to the same method as Example 2.
¹H-NMR(CDCl₃) δ(ppm): 8.90(s,1H), 8.79-8.77(br.-t,1H), 8.49(s,1H), 7.37-7.32(m,4H), 7.29(s,1H), 4.76(d,2H, J=5.3Hz), 3.55-3.51(m,2H), 3.47-3.44(m,3H), 3.15-3.09(m,2H), 3.01-2.94(m,2H), 2.08-2.00(m,2H)

### Example 27: 4-(4-fluorobenzylamino)-7-(4-methylpiperazin- 1-yl)-6-nitroquinazoline (compound 27)

Using the compound obtained in Reference example 8 as starting material, the title compound was obtained according to the same method as Example 3.
¹H-NMR(CDCl₃) δ(ppm): 8.96(s,1H), 8.88-8.86(br.-t,1H), 8.47(s,1H), 7.43-7.39(m,3H), 7.08-7.03(m,2H), 4.80(d,2H, J=5.3Hz), 3.12-3.10(m,4H), 3.07-3.05(m,4H), 2.26(s,3H)

### Example 28: 7-(4-ethylpiperazin-1-yl)-4-(4-fluorobenzylamino)-6-nitroquinazoline (compound 28)

Using the compound obtained in Reference example 8 as starting material, the title compound was obtained according to the same method as Example 4.
¹H-NMR(CDCl₃) δ(ppm): 8.92(s,1H), 8.80-8.78(br.-t,1H), 8.45(s,1H), 7.40-7.37(m,3H), 7.10-7.05(m,2H), 4.81(d,2H, J=5.4Hz), 3.19-3.16(m,4H), 3.06(t,2H,J=6.8Hz), 2.71-2.68(m,4H), 1.18(t,3H,J=6.8Hz)

### Example 29: 4-(4-fluorobenzylamino)-7-(4-formylpiperazin-1-yl)-6-nitroquinazoline (compound 29)

Using the compound obtained in Reference example 8 as starting material, the title compound was obtained according to the same method as Example 6.
¹H-NMR(CDCl₃) δ(ppm): 8.95(s,1H), 8.82-8.79(br.-t,1H), 8.50(s,1H), 7.40-7.36(m,3H), 7.23(s,1H), 7.08-7.04(m,2H), 4.74(d,2H, J=5.5Hz), 3.15-3.12(m,4H), 3.09-3.07(m,4H)

### Example 30: 7-(4-aminopiperidin-1-yl)-4-(4-fluorobenzylamino)-6-nitroquinazoline (compound 30)

Using the compound obtained in Reference example 8 as starting material, the title compound was obtained according to the same method as Example 8.
¹H-NMR(DMSO-d₆) δ(ppm): 9.00-8.98(m,2H), 8.49(s,1H), 7.40-7.28(m,4H), 7.15 (s,1H), 4.76(d,2H, J=5.2Hz), 3.33-3.29(m,3H), 2.97-2.92(m,3H), 1.84-1.80(m,3H), 1.34-1.30 (m,2H)

### Example 31: 4-[3,4-(methylenedioxy)benzylamino]-6-nitro-7-(piperazin-1-yl)quinazoline (compound 31)

Using the compound obtained in Reference example 9 as starting material, the title compound was obtained according to the same method as Example 1.
¹H-NMR(DMSO-d₆) δ(ppm): 8.96-8.94(m,2H), 8.46(s,1H), 7.22 (s,1H), 6.91-6.77(m,3H), 5.95(s,2H), 4.68(d,2H, J=5.4Hz), 3.08-3.06(m,4H), 3.03-3.00(m,5H)

### Example 32: 7-(homopiperazin-1-yl)-4-[3,4-(methylenedioxy)benzylamino]-6-nitroquinazoline (compound 32)

Using the compound obtained in Reference example 9 as starting material, the title compound was obtained according to the same method as Example 2.
¹H-NMR(CDCl₃) δ(ppm): 8.59(s,1H), 8.08(s,1H), 7.29(s,1H), 6.88-6.79(m,3H), 5.98(s,2H), 5.75-5.73(br.-t,1H), 4.74(d,2H, J=5.4Hz), 3.54-3.50(m,2H), 3.41-3.37(m,2H), 3.10-3.06(m,3H), 2.97-2.93(m,2H), 1.98-1.90(m,2H)

### Example 33: 7-(4-methylpiperazin-1-yl)-4-[3,4-(methylenedioxy)benzylamino]-6-nitroquinazoline (compound 33)

Using the compound obtained in Reference example 9 as starting material, the title compound was obtained according to the same method as Example 3.
¹H-NMR(DMSO-d₆) δ(ppm): 9.04-9.02(br.-t,1H), 9.00(s,1H), 8.47(s,1H), 7.24(s,1H), 6.94(s,1H), 6.88-6.85(m,2H), 5.97(s,2H), 4.65(d,2H, J=5.9Hz), 3.11-3.06(m,4H), 2.65-2.59(m,4H), 2.32(s,3H)

### Example 34: 7-(4-ethylpiperazin-1-yl)-4-[3,4-(methylenedioxy)benzylamino]-6-nitroquinazoline (compound 34)

Using the compound obtained in Reference example 9 as starting material, the title compound was obtained according to the same method as Example 4.
¹H-NMR(CDCl₃) δ(ppm): 8.63(s,1H), 8.12(s,1H), 7.31(s,1H), 6.90-6.87(m,3H), 6.01(s,2H), 5.81-5.79(br.-t,1H), 4.77(d,2H, J=5.5Hz), 3.22-3.19(m,4H), 3.10(t,2H,J=7.0Hz), 2.83-2.79 (m,4H), 1.20(t,3H,J=7.0Hz)

### Example 35: 7-(4-formylpiperazin-1-yl)-4-[3,4-(methylenedioxy)benzylamino]-6-nitroquinazoline (compound 35)

Using the compound obtained in Reference example 9 as starting material, the title compound was obtained according to the same method as Example 6.
¹H-NMR(CDCl₃) δ(ppm): 8.60(s,1H), 8.15(s,1H), 7.31-7.29(m,2H), 6.88-6.85(m,3H), 6.10(s,2H), 5.79-5.77(br.-t,1H), 4.75(d,2H, J=5.3Hz), 3.23-3.20(m,4H), 2.87-2.83(m,4H)

### Example 36: 7-(4-aminopiperidin-1-yl)-4-[3,4-(methylenedioxy)benzylamino]-6-nitroquinazoline (compound 36)

Using the compound obtained in Reference example 9 as starting material, the title compound was obtained according to the same method as Example 8.
¹H-NMR(DMSO-d₆) δ(ppm): 8.98-8.95(m,2H), 8.47(s,1H), 7.25 (s,1H), 6.89-6.78(m,3H), 5.97(s,2H), 4.70(d,2H, J=5.4Hz), 3.28-3.25(m,3H), 2.96-2.92(m,3H), 1.82-1.77(m,3H), 1.32-1.27(m,2H)

### Example 37: 4-[2-(4-fluorophenyl)ethylamino]-6-nitro-7-(piperazin-1-yl)quinazoline (compound 37)

Using the compound obtained in Reference example 34 as starting material, the title compound was obtained according to the same method as Example 1.
¹H-NMR(DMSO-d₆) δ(ppm): 9.00(s,1H), 8.81-8.79(br.-t,1H), 8.50(s,1H), 7.32(s,1H), 7.30-7.27(m,2H), 7.14-7.08(m,2H) 3.74(dt,2H, J=7.2,5.8Hz), 3.21-3.19(m,5H), 2.95-2.90(m,6H)

### Example 38: 4-[2-(4-fluorophenyl)ethylamino]-7-(homopiperazin-1-yl)-6-nitroquinazoline (compound 38)

Using the compound obtained in Reference example 34 as starting material, the title compound was obtained according to the same method as Example 2.
¹H-NMR(DMSO-d₆) δ(ppm): 8.89(s,1H), 8.72-8.70(br.-t,1H), 8.47(s,1H), 7.33-7.28(m,3H), 7.16-7.10(m,2H) 3.80(dt,2H, J=7.0,5.7Hz), 3.35-3.32(m,2H), 3.07-2.99(m,9H), 2.00-1.97(m,2H)

### Example 39: 4-[2-(4-fluorophenyl)ethylamino]-7-(4-methylpiperazin-1-yl)-6-nitroquinazoline (compound 39)

Using the compound obtained in Reference example 34 as starting material, the title compound was obtained according to the same method as Example 3.
¹H-NMR(DMSO-d₆) δ(ppm): 8.97(s,1H), 8.83-8.80(br.-t,1H), 8.56(s,1H), 7.34(s,1H), 7.33-7.30(m,2H), 7.16-7.13(m,2H) 3.77(dt,2H, J=7.0,5.7Hz), 3.17-3.14(m,4H), 3.02-2.96(m,6H), 2.21(s,3H)

### Example 40: 4-[2-(4-fluorophenyl)ethylamino]-7-(4-formylpiperazin-1-yl)-6-nitroquinazoline (compound 40)

Using the compound obtained in Reference example 34 as starting material, the title compound was obtained according to the same method as Example 6.
¹H-NMR(DMSO-d₆) δ(ppm): 9.01(s,1H), 8.83-8.81(br.-t,1H), 8.47(s,1H), 7.35(s,1H), 7.31-7.28(m,3H), 7.15-7.09(m,2H) 3.76(dt,2H, J=7.3,5.5Hz), 3.19-3.15(m,4H), 2.97-2.92(m,6H)

### Example 41: 7-(4-aminopiperidin-1-yl)-4-[2-(4-fluorophenyl)ethylamino]-6-nitroquinazoline (compound 41)

Using the compound obtained in Reference example 34 as starting material, the title compound was obtained according to the same method as Example 8.
¹H-NMR(DMSO-d₆) δ(ppm): 8.97(s,1H), 8.77-8.75(br.-t,1H), 8.49(s,1H), 7.33(s,1H), 7.33-7.30(m,2H), 7.17-7.14(m,2H) 3.78(dt,2H, J=7.0,5.5Hz), 3.31-3.29(m,3H), 2.95-2.91(m,5H), 1.84-1.80(m,3H), 1.34-1.30(m,2H)

### Example 42: 6-nitro-7-(piperazin-1-yl)-4-[2-(2-thienyl)ethylamino]quinazoline (compound 42)

Using the compound obtained in Reference example 16 as starting material, the title compound was obtained according to the same method as Example 1.
¹H-NMR(CDCl₃) δ(ppm): 8.63(s,1H), 8.12(s,1H), 7.32(s,1H), 7.21(d,1H,J=5.4Hz), 6.99(dd,1H,J=5.4,3.0Hz), 6.89(d,1H, J=3.0Hz), 5.88-5.86(br.-t,1H), 3.96(dt,2H, J=6.3,5.8Hz), 3.26(d,2H,J=6.3Hz), 3.12-3.10(m,4H), 3.07-3.04(m,5H)

### Example 43: 7-(homopiperazin-1-yl)-6-nitro-4-[2-(2-thienyl)ethylamino]quinazoline (compound 43)

Using the compound obtained in Reference example 16 as starting material, the title compound was obtained according to the same method as Example 2.
¹H-NMR(CDCl₃) δ(ppm): 8.67(s,1H), 8.14(s,1H), 7.28(s,1H), 7.19(d,1H,J=5.4Hz), 7.02(dd,1H,J=5.4,3.2Hz), 6.90 (d, 1H,J=3.0Hz), 5.86-5.84(br.-t,1H), 3.92(dt,2H,J=6.2,5.6Hz), 3.50-3.46(m,2H), 3.43-3.39(m,3H), 3.23(d,2H,J=6.2Hz), 3.15-3.12(m,2H), 3.04-3.00(m,2H), 2.05-2.02(m,2H)

### Example 44: 7-(4-methylpiperazin-1-yl)-6-nitro-4-[2-(2-thienyl)ethylamino]quinazoline (compound 44)

Using the compound obtained in Reference example 16 as starting material, the title compound was obtained according to the same method as Example 3.
¹H-NMR(CDCl₃) δ(ppm): 8.69(s,1H), 8.13(s,1H), 7.32(s,1H), 7.20(d,1H,J=5.3Hz), 7.00(dd,1H,J=5.3,3.0Hz), 6.90(d,1H, J=3.0Hz), 5.93-5.91(br.-t,1H), 3.95(dt,2H, J=6.5,5.5Hz), 3.25(d,2H,J=6.5Hz), 3.13-3.10(m,4H), 3.08-3.05(m,4H), 2.40(s,3H)

### Example 45: 7-(4-ethylpiperazin-1-yl)-6-nitro-4-[2-(2-thienyl)ethylamino]quinazoline (compound 45)

Using the compound obtained in Reference example 16 as starting material, the title compound was obtained according to the same method as Example 4.
¹H-NMR(CDCl₃) δ(ppm): 8.72(s,1H), 8.15(s,1H), 7.28(s,1H), 7.18(d,1H,J=5.4Hz), 7.05(dd,1H,J=5.4,3.2Hz), 6.94(d,1H, J=3.2Hz), 5.96-5.94(br.-t,1H), 3.92-3.89(m,2H), 3.31-3.29 (m,2H), 3.15-3.12(m,4H), 3.04-3.01(m,4H), 2.67(q,2H,J=7.1Hz), 1.10(t,3H,J=7.1 Hz)

### Example 46: 7-(4-formylpiperazin-1-yl)-6-nitro-4-[2-(2-thienyl)ethylamino]quinazoline (compound 46)

Using the compound obtained in Reference example 16 as starting material, the title compound was obtained according to the same method as Example 6.
¹H-NMR(CDCl₃) δ(ppm): 8.70(s,1H), 8.15(s,1H), 7.35(s,1H), 7.18-7.20(m,2H), 7.10-6.98(m,2H), 5.94-5.92(br.-t,1H), 3.97(dt,2H,J=6.5,5.5Hz), 3.26(d,2H,J=6.5Hz), 3.14-3.12(m,4H), 3.07-3.03(m,4H)

### Example 47: 7-(4-aminopiperidin-1-yl)-6-nitro-4-[2-(2-thienyl)ethylamino]quinazoline (compound 47)

Using the compound obtained in Reference example 16 as starting material, the title compound was obtained according to the same method as Example 8.
¹H-NMR(CDCl₃) δ(ppm): 8.68(5,1H), 8.16(s,1H), 7.31(s,1H), 7.18(d,1H,J=5.5Hz),
7.05(dd,1H,J=5.5,3.0Hz), 6.93(d,1H, J=3.0Hz), 5.90-5.88(br.-t,1H), 3.94(dt,2H, J=6.3,5.3Hz), 3.35-3.30(m,3H), 3.27(d,2H,J=6.3Hz), 2.98-2.95(m,3H), 1.84-1.80(m,3H), 1.35-1.31(m,2H)

### Example 48: 6-nitro-7-(piperazin-1-yl)-4-[(2-thienyl)methylamino]quinazoline (compound 48)

Using the compound obtained in Reference example 15 as starting material, the title compound was obtained according to the same method as Example 1.
¹H-NMR(CDCl₃) δ(ppm): 8.68(s,1H), 8.18(s,1H), 7.32(s,1H), 7.20(d,1H,J=5.5Hz), 7.07(dd,1H,J=5.5,3.4Hz), 6.91(d,1H, J=3.4Hz), 5.90-5.88(br.-t, 1H), 4.92(d,2H,J=5.3Hz), 3.15-3.12(m,5H), 3.09-3.06(m,4H)

### Example 49: 7-(homopiperazin-1-yl)-6-nitro-4-[(2-thienyl)methylamino]quinazoline (compound 49)

Using the compound obtained in Reference example 15 as starting material, the title compound was obtained according to the same method as Example 2.
¹H-NMR(CDCl₃) δ(ppm): 8.71(s,1H), 8.14(s,1H), 7.29(s,1H), 7.22(d,1H,J=5.4Hz), 7.10(dd,1H,J=5.4,3.2Hz), 6.85(d,1H, J=3.2Hz), 5.87-5.85(br.-t,1H), 4.88(d,2H,J=5.5Hz), 3.60-3.56(m,2H), 3.52-3.48(m,3H), 3.18-3.14(m,2H), 3.06-3.02 (m,2H), 2.00-1.96(m,2H)

### Example 50: 7-(4-methylpiperazin-1-yl)-6-nitro-4-[(2-thienyl)methylamino]quinazoline (compound 50)

Using the compound obtained in Reference example 15 as starting material, the title compound was obtained according to the same method as Example 3.
¹H-NMR(CDCl₃) δ(ppm): 8.66(s,1H), 8.19(s,1H), 7.35(s,1H), 7.18(d,1H,J=5.4Hz), 7.10(dd,1H,J=5.4,3.2Hz), 6.90(d,1H, J=3.2Hz), 5.80-5.78(br.-t,1H), 4.94(d,2H,J=5.7Hz), 3.21-3.18(m,4H), 3.08-3.05 (m,4H), 2.41(s,3H)

### Example 51: 4-[(2-furyl)methylamino]-6-nitro-7-(piperazin-1-yl)quinazoline (compound 51)

Using the compound obtained in Reference example 17 as starting material, the title compound was obtained according to the same method as Example 1.
¹H-NMR(CDCl₃) δ(ppm): 8.66(s,1H), 8.22(s,1H), 7.43(s,1H), 7.33(s,1H), 6.38-6.36(m,2H), 5.93-5.91(br.-t,1H), 4.86 (d,2H,J=5.1Hz), 3.13-3.11(m,4H), 3.07-3.05 (m,5H)

### Example 52: 4-[(2-furyl)methylamino]-7-(homopiperazin-1-yl)-6-nitroquinazoline (compound 52)

Using the compound obtained in Reference example 17 as starting material, the title compound was obtained according to the same method as Example 2.
¹H-NMR(CDCl₃) δ(ppm): 8.68(s,1H), 8.20(s,1H), 7.44(s,1H), 7.31(s,1H), 6.36-6.33(m,2H), 5.96-5.94(br.-t,1H), 4.90 (d,2H,J=5.3Hz), 3.68-3.65(m,2H), 3.62-3.57(m,2H), 3.24-3.20(m,3H), 3.12-3.07 (m,2H), 2.10-2.06(m,2H)

### Example 53: 4-[(2-furyl)methylamino]-7-(4-methylpiperazin-1-yl)-6-nitroquinazoline (compound 53)

Using the compound obtained in Reference example 17 as starting material, the title compound was obtained according to the same method as Example 3.
¹H-NMR(CDCl₃) δ(ppm): 8.70(s,1H), 8.25(s,1H), 7.47(s,1H), 7.30(s,1H), 6.41-6.38(m,2H), 5.96-5.93(br.-t,1H), 4.90 (d,2H,J=5.5Hz), 3.15-3.12(m,4H), 3.07-3.03(m,4H), 2.37(s,3H)

### Example 54: 6-nitro-4-[3-(phenyl)propylamino]-7-(piperazin-1-yl)quinazoline (compound 54)

Using the compound obtained in Reference example 10 as starting material, the title compound was obtained according to the same method as Example 1.
¹H-NMR(CDCl₃) δ(ppm): 8.58(s,1H), 7.79(s,1H), 7.34-7.19(m,6H), 5.49-5.47(br.-t,1H), 3.73(dt,2H,J=6.2,6.0Hz), 3.11-3.09 (m,4H), 3.06-3.02(m,5H), 2.81(t,2H,J=7.4Hz), 2.11 (tt,2H, J=7.4,6.0Hz)

### Example 55: 6-nitro-7-(piperazin-1-yl)-4-[(2-pyridyl)methylamino]quinazoline (compound 55)

Using the compound obtained in Reference example 11 as starting material, the title compound was obtained according to the same method as Example 1.
¹H-NMR(DMSO-d₆) δ(ppm): 8.68-8.65(m,2H), 8.65-8.62(br.-t,1H), 8.55(s,1H), 8.20(s,1H), 7.53-7.49(m,2H), 7.30(s,1H), 5.08(d,2H,J=5.5Hz), 3.10-3.06(m,5H), 2.99-2.96(m,4H)

### Example 56: 6-nitro-7-(piperazin-1-yl)-4-[2-(2-pyridyl)ethylamino]quinazoline (compound 56)

Using the compound obtained in Reference example 12 as starting material, the title compound was obtained according to the same method as Example 1.
¹H-NMR(DMSO-d₆) δ(ppm): 8.93(s,1H), 8.805-8.76(br.-t,1H), 8.52-8.49(m,2H), 7.73-7.67(m,1H), 7.31-7.20(m,3H), 3.89(dt,2H,J=7.0,5.7Hz), 3.16-3.89(m,11H)

### Example 57: 6-nitro-7-(piperazin-1-yl)-4-[2-(3-pyridyl)ethylamino]quinazoline (compound 57)

Using the compound obtained in Reference example 13 as starting material, the title compound was obtained according to the same method as Example 1.
¹H-NMR(DMSO-d₆) δ(ppm): 8.86(s,1H), 8.69-8.67(br.-t,1H), 8.46-8.40(m,3H), 7.69-7.66(m,1H), 7.33-7.29(m,1H), 7.18 (s,1H), 3.77(dt,2H,J=7.1,5.9Hz), 3.03-2.95(m,6H), 2.87-2.83(m,5H)

### Example 58: 6-nitro-7-(piperazin-1-yl)-4-[2-(4-pyridyl)ethylamino]quinazoline (compound 58)

Using the compound obtained in Reference example 14 as starting material, the title compound was obtained according to the same method as Example 1.
¹H-NMR(DMSO-d₆) δ(ppm): 8.81(s,1H), 8.65-8.61(br.-t,1H), 8.47-8.45(m,3H), 7.29-7.27(m,1H), 7.17(s,1H), 3.79(dt,2H, J=7.1,5.7Hz), 3.00-2.96(m,6H), 2.82-2.79(m,5H)

### Example 59: 4-[(1-naphthyl)methylamino]-6-nitro-7-(piperazin-1-yl)quinazoline (compound 59)

Using the compound obtained in Reference example 18 as starting material, the title compound was obtained according to the same method as Example 1.
¹H-NMR(DMSO-d₆) δ(ppm): 9.11-9.09(br.-t,1H), 9.03(s,1H), 8.51(s,1H), 8.16-8.13(m,1H), 7.99-7.96(m,1H), 7.89-7.87 (m,2H), 7.58-7.47(m,4H), 5.23(d,2H,J=4.9Hz), 3.00-3.03 (m,5H), 2.98-2.95(m,4H)

### Example 60: 4-(4-chlorobenzylamino)-6-nitro-7-(piperazin-1-yl)quinazoline (compound 60)

Using the compound obtained in Reference example 19 as starting material, the title compound was obtained according to the same method as Example 1.
¹H-NMR(DMSO-d₆) δ(ppm): 9.13-9.10(br.-t,1H), 8.95(s,1H), 8.46(s,1H), 7.38(m,4H), 7.24(s,1H), 4.74(d,2H,J=5.7Hz), 3.08-3.06(m,4H), 2.95-2.93(m,4H), 2.50(br.-s,1H)

### Example 61: 4-(2-fluorobenzylamino)-6-nitro-7-(piperazin-1-yl)quinazoline (compound 61)

Using the compound obtained in Reference example 20 as starting material, the title compound was obtained according to the same method as Example 1.
¹H-NMR(CDCl₃) δ(ppm): 8.90(s,1H), 8.84-8.82(br.-t,1H), 8.47(s,1H), 7.40-7.36(m,3H), 7.06-7.03(m,2H), 4.80(d,2H, J=5.2Hz), 3.10-3.05(m,5H), 3.02-3.00(m,4H)

### Example 62: 4-(3-fluorobenzylamino)-6-nitro-7-(piperazin-1-yl)quinazoline (compound 62)

Using the compound obtained in Reference example 21 as starting material, the title compound was obtained according to the same method as Example 1.
¹H-NMR(CDCl₃) 6(ppm): 8.92(s,1H), 8.82-8.80(br.-t,1H), 8.50(s,1H), 7.39-7.35(m,3H), 7.10-7.07(m,2H), 4.84(d,2H, J=5.2Hz), 3.11-3.07(m,5H), 3.04-3.00(m,4H)

### Example 63: 6-nitro-7-(piperazin-1-yl)-4-[4-(1,1,1-trifluoiomethyl)benzylamino]quinazoline (compound 63)

Using the compound obtained in Reference example 22 as starting material, the title compound was obtained according to the same method as Example 1.
¹H-NMR(DMSO-d₆) δ(ppm): 9.08-9.06(br.-t,1H), 8.91(s,1H), 8.43(s,1H), 7.63-7.55(m,4H), 7.19(s,1H), 4.85(d,2H, J=5.4Hz), 3.04-3.02(m,4H), 2.92-2.88(m,5H)

### Example 64: 4-(4-methylbenzylamino)-6-nitro-7-(piperazin-1-yl)quinazoline (compound 64)

Using the compound obtained in Reference example 23 as starting material, the title compound was obtained according to the same method as Example 1.
¹H-NMR(DMSO-d₆) δ(ppm): 9.02-8.99(m,2H), 8.46(s,1H), 7.27-7.25(m,3H), 7.14-7.11(m,2H), 4.74(d,2H, J=5.9Hz), 3.12-3.10 (m,4H), 3.04-3.01(m,5H), 2.30(s,3H)

### Example 65: 4-(2-methoxylbenzylamino)-6-nitro-7-(piperazin-1-yl)quinazoline (compound 65)

Using the compound obtained in Reference example 24 as starting material, the title compound was obtained according to the same method as Example 1.
¹H-NMR(DMSO-d₆) δ(ppm): 8.97(s,1H), 8.73-8.71(br.-t,1H), 8.41(s,1H), 7.25-7.17(m,3H), 6.94-6.80(m,2H), 4.74(d,2H, J=5.4Hz), 3.85(s,3H), 3.04-3.02(m,4H), 2.94-2.91(m,5H)

### Example 66: 4-(3-methoxylbenzylamino)-6-nitro-7-(piperazin-1-yl)quinazoline (compound 66)

Using the compound obtained in Reference example 25 as starting material, the title compound was obtained according to the same method as Example 1.
¹H-NMR(DMSO-d₆) δ(ppm): 9.04(t,1H,J=5.4Hz), 8.98(s,1H), 8.46(s,1H), 7.25-7.23(m,2H), 6.95-6.93(m,2H), 6.82-6.79(m,1H), 4.75(d,2H, J=5.4Hz), 3.75(s,3H), 3.14-3.12(m,4H), 3.04-3.01(m,5H)

### Example 67: 4-(4-methoxylbenzylamino)-6-nitro-7-(piperazin-1-yl)quinazoline (compound 67)

Using the compound obtained in Reference example 26 as starting material, the title compound was obtained according to the same method as Example 1.
¹H-NMR(DMSO-d₆) δ(ppm): 9.05-9.03(br.-t,1H), 8.96(s,1H), 8.47(s,1H), 7.31-7.28(m,2H), 7.23(s,1H), 6.90-6.87(m,2H), 4.68(d,2H, J=5.4Hz), 3.72(s,3H), 3.09-3.07(m,4H), 2.97-2.95(m,4H), 2.43(br.-s,1H)

### Example 68: 4-(3,4-dimethoxylbenzylamino)-6-nitro-7-(piperazin-1-yl)quinazoline (compound 68)

Using the compound obtained in Reference example 27 as starting material, the title compound was obtained according to the same method as Example 1.
¹H-NMR(DMSO-d₆) δ(ppm): 8.66(s,1H), 8.20(s,1H), 7.33(s,1H), 6.98-6.86(m,3H), 5.90-5.89(br.-t,1H), 4.78(d,2H, J=5.4Hz), 3.89(s,3H), 3.88(s,3H), 3.12-3.10(m,5H), 3.06-3.04(m,4H)

### Example 69: 6-nitro-7-(piperazin-1-yl)-4-(3,4,5-trimethoxylbenzylamino)quinazoline (compound 69)

Using the compound obtained in Reference example 28 as starting material, the title compound was obtained according to the same method as Example 1.
¹H-NMR(DMSO-d₆) δ(ppm): 8.66(s,1H), 8.25(s,1H), 7.33(s,1H), 6.61(s,2H), 5.98-5.96(br.-t,1H), 4.77(d,2H, J=5.4Hz), 3.86(s,3H), 3.85(s,6H), 3.13-3.11(m,5H), 3.07-3.05(m.4H)

### Example 70: 4-[2-(2-chlorophenyl)ethylamino]-6-nitro-7-(piperazin-1-yl)quinazoline (compound 70)

Using the compound obtained in Reference example 29 as starting material, the title compound was obtained according to the same method as Example 1.
¹H-NMR(DMSO-d₆) δ(ppm): 8.88(s,1H), 8.74-8.70(br.-t,1H), 8.48(s,1H), 7.45-7.22(m,5H), 3.77(dt,2H,J=7.1,5.7Hz), 3.11-3.06(m,6H), 2.97-2.93(m,5H)

### Example 71: 4-[2-(3-chlorophenyl)ethylamino]-6-nitro-7-(piperazin-1-yl)quinazoline (compound 71)

Using the compound obtained in Reference example 30 as starting material, the title compound was obtained according to the same method as Example 1.
¹H-NMR(DMSO-d₆) δ(ppm): 8.86(s,1H), 8.68-8.66(br.-t,1H), 8.49(s,1H), 7.35-7.18(m,5H), 3.75(dt,2H,J=7.0,5.7Hz), 3.01-2.94(m,6H), 2.86-2.75(m,5H)

### Example 72: 4-[2-(4-bromophenyl)ethylamino]-6-nitro-7-(piperazin-1-yl)quinazoline (compound 72)

Using the compound obtained in Reference example 31 as starting material, the title compound was obtained according to the same method as Example 1.
¹H-NMR(DMSO-d₆) δ(ppm): 8.83(s,1H), 8.62-8.60(br.-t,1H), 8.46(s,1H), 7.49-7.46(m,2H), 7.24-7.21(m,2H), 7.16(s,1H), 3.73(dt,2H, J=6.8,5.7Hz), 3.00-2.90(m,6H), 2.82-2.79(m,5H)

### Example 73: 4-[2-(2-fluorophenyl)ethylamino]-6-nitro-7-(piperazin-1-yl)quinazoline (compound 73)

Using the compound obtained in Reference example 32 as starting material, the title compound was obtained according to the same method as Example 1.
¹H-NMR(DMSO-d₆) δ(ppm): 8.88(s,1H), 8.78-8.77(br.-t,1H), 8.48(s,1H), 7.32-7.27(m,3H), 7.13-7.09(m,2H), 3.77-3.75 (m,2H), 3.19-3.17(m,5H), 2.98-2.96(m,6H)

### Example 74: 4-[2-(3-fluorophenyl)ethylamino]-6-nitro-7-(piperazin-1-yl)quinazoline (compound 74)

Using the compound obtained in Reference example 33 as starting material, the title compound was obtained according to the same method as Example 1.
¹H-NMR(DMSO-d₆) δ(ppm): 8.86(s,1H), 8.74-8.72(br.-t,1H), 8.50(s,1H), 7.34-7.30(m,3H), 7.15-7.09(m,2H), 3.77-3.75 (m,2H), 3.20-3.15(m,6H), 3,04-3.08(m,5H)

### Example 75: 4-[2-(4-methylphenyl)ethylamino]-6-nitro-7-(piperazin- 1-yl)quinazoline (compound 75)

Using the compound obtained in Reference example 35 as starting material, the title compound was obtained according to the same method as Example 1.
¹H-NMR(DMSO-d₆) δ(ppm): 8.87(s,1H), 8.68-8.66(br.-t,1H), 8.47(s,1H), 7.18-7.08(m,5H), 3.71 (dt,2H,J=7.0,5.8Hz), 3.03-3.00(m,4H), 2.93-2.87(m,7H), 2.26(s,3H)

### Example 76: 4-[2-(2-methoxyphenyl)ethylamino]-6-nitro-7-(piperazin-1-yl)quinazoline (compound 76)

Using the compound obtained in Reference example 36 as starting material, the title compound was obtained according to the same method as Example 1.
¹H-NMR(DMSO-d₆) δ(ppm): 8.90(s,1H), 8.75-8.73(br.-t,1H), 8.37(s,1H), 7.24-7.17(m,3H), 6.95-6.82(m,2H), 3.85(s,3H), 3.62-3.57(m,2H), 3.12-3.04(m,7H), 3.00-2.96(m,4H)

### Example 77: 4-[2-(3-methoxyphenyl)ethylamino]-6-nitro-7-(piperazin-1-yl)quinazoline (compound 77)

Using the compound obtained in Reference example 37 as starting material, the title compound was obtained according to the same method as Example 1.
¹H-NMR(DMSO-d₆) δ(ppm): 9.00-8.97(br.-t,1H), 8.96(s,1H), 8.43(s,1H), 7.27-7.24(m,2H), 6.98-6.95(m,2H), 6.84-6.80 (m,1H), 3.87(s,3H), 3.58-3.54(m,2H), 3.10-3.04(m,7H), 2.99-2.96(m,4H)

### Example 78: 4-[2-(4-methoxyphenyl)ethylamino]-6-nitro-7-(piperazin-1-yl)quinazoline (compound 78)

Using the compound obtained in Reference example 38 as starting material, the title compound was obtained according to the same method as Example 1.
¹H-NMR(DMSO-d₆) δ(ppm): 8.85(s,1H), 8.65-8.61(br.-t,1H), 8.47(s,1H), 7.26-7.16(m,3H), 6.91-6.84(m,2H), 3.73-3.66 (m,5H), 3.00-2.98(m,4H), 2.97-2.81(m,7H)

### Example 79: 4-[2-(3,4-dimethoxyphenyl)ethylamino]-6-nitro-7-(piperazin-1-yl)quinazoline (compound 79)

Using the compound obtained in Reference example 39 as starting material, the title compound was obtained according to the same method as Example 1.
¹H-NMR(DMSO-d₆) δ(ppm): 8.89(s,1H), 8.65-8.63(br.-t,1H), 8.47(s,1H), 7.18(s,1H), 6.87-6.74(m,4H), 3.70-3.65(m,8H), 3.02-2.87(m,10H)

### Example 80: 4-[2-(4-hydroxyphenyl)ethylamino]-6-nitro-7-(piperazin-1-yl)quinazoline (compound 80)

Using the compound obtained in Reference example 40 as starting material, the title compound was obtained according to the same method as Example 1.
¹H-NMR(DMSO-d₆) δ(ppm): 9.19(s,1H), 8.87(s,1H), 8.67-8.63 (br.-t,1H), 8.47(s,1H), 7.19(s,1H), 7.06-7.03(m,2H), 6.69-6.66(m,2H), 3.67(dt,2H,J=7.0,5.7Hz), 3.05-3.02(m,6H), 2.85-2.80(m,5H)

### Example 81: 4-[2-(4-anilino)ethylamino]-6-nitro-7-(piperazin-1-yl)quinazoline (compound 81)

Using the compound obtained in Reference example 41 as starting material, the title compound was obtained according to the same method as Example 1.
¹H-NMR(DMSO-d₆) δ(ppm): 8.97(s,1H), 8.74-8.72(br.-t,1H), 8.49(s,1H), 7.28(s,1H), 6.92-6.88(m,2H), 6.51-6.48(m,2H), 4.89(br.-s,2H), 3.63(dt,2H,J=7.1,5.8Hz), 3.33-3.14(m,9H), 2.76(t,2H,J=7.1Hz)

### Example 82: 4-(2-methyl-2-phenylethylamino)-6-nitro-7-(piperazin-1-yl)quinazoline (compound 82)

Using the compound obtained in Reference example 42 as starting material, the title compound was obtained according to the same method as Example 1.
¹H-NMR(DMSO-d₆) δ(ppm): 8.62(s,1H), 7.93(s,1H), 7.40-7.28 (m,6H), 5.52-5.53(br.-t,1H), 4.09-4.00(m,1H), 3.66-3.56 (m,1H), 3.23-3.15(m,1H), 3.10-3.08(m,4H), 3.07-3.03(m,5H), 1.89(s,3H)

### Example 83: 4-(2,2-diphenylethylamino)-6-nitro-7-(piperazin-1-yl)quinazoline (compound 83)

Using the compound obtained in Reference example 43 as starting material, the title compound was obtained according to the same method as Example 1.
¹H-NMR(DMSO-d₆) δ(ppm): 8.87(s,1H), 8.71-8.69(br.-t,1H), 8.54(s,1H), 7.35-7.16 (m,11H), 4.58(t,1H,J=7.6Hz), 4.20-4.16(m,2H), 3.18-3.15 (m,5H), 3.14-3.12(m,4H)

### Example 84: 4-(N-methylbenzylamino)-6-nitro-7-(piperazin-1-yl)quinazoline (compound 84)

Using the compound obtained in Reference example 44 as starting material, the title compound was obtained according to the same method as Example 1.
¹H-NMR(CDCl₃) δ(ppm): 9.00-8.97(m,2H), 8.43(s,1H), 7.38-7.32 (m,5H), 4.77(s,2H), 3.12-3.08(m,5H), 3.05(s,3H), 3.00-2.96 (m,4H)

### Example 85: 4-(dibenzylamino)-6-nitro-7-(piperazin-1-yl)quinazoline (compound 85)

Using the compound obtained in Reference example 45 as starting material, the title compound was obtained according to the same method as Example 1.
¹H-NMR(CDCl₃) δ(ppm): 8.88(s,1H), 8.65(s,1H), 8.37(s,1H), 7.40-7.35 (m,10H), 4.80(s,4H), 3.15-3.10(m,5H), 3.02-2:98 (m,4H)

### Example 86: 4-[(1-benzyl-4-piperidyl)amino]-6-nitro-7-(piperazin-1-yl)quinazoline (compound 86)

Using the compound obtained in Reference example 46 as starting material, the title compound was obtained according to the same method as Example 1.
¹H-NMR(CDCl₃) δ(ppm): 8.59(s,1H), 8.19(s,1H), 7.35-7.29 (m,6H), 5.51(d,1H,J=7.8Hz), 4.28-4.25(m,1H), 3.56(s,2H), 3.12-3.09 (m,4H), 3.07-3.04(m,5H), 2.94-2.90(m,2H), 2.28-2.10(m,4H), 1.72-1.68(m,2H)

### Example 87: 4-[[1-(4-methoxybenzyl)-4-piperidyl]amino]-6-nitro-7-(piperazin-1-yl)quinazoline (compound 87)

Using the compound obtained in Reference example 50 as starting material, the title compound was obtained according to the same method as Reference example 5 and Example 1.
¹H-NMR(CDCl₃) δ(ppm): 8.59(s,1H), 8.10(s,1H), 7.31(s,1H), 7.20-7.15(m,2H), 6.92-6.88(m,2H), 5.54(d,1H,J=7.2Hz), 4.51-4.49(m,1H), 3.57(s,2H), 3.30(s,3H), 3.15-3.12(m,4H), 3.10-3.07(m,5H), 2.94-2.92(m,2H), 2.28-2.16(m,4H), 1.67-1.64 (m,2H)

### Example 88: 4-[(6-nitro-7-piperazinylquinazolin-4-yl)amino]piperidinyl-phenylketone (compound 88)

Using the compound obtained in Reference example 52 as starting material, the title compound was obtained according to the same method as Reference example 5 and Example 1.
¹H-NMR(CDCl₃) δ(ppm): 8.58(s,1H), 8.05(s,1H), 7.43-7.37(m,6H), 5.57(d,1H,J=7.3Hz), 4.39-4.37(m,2H), 3.18-3.15(m,4H), 3.12-3.09(m,4H), 2.96-2.94(m,2H), 2.25-2.13(m,4H), 1.62-1.59 (m,2H)

### Example 89: 4-[(6-nitro-7-piperazinylquinazolin-4-yl)amino]piperidinyl-(1,3-dioxaindan-5-yl)ketone (compound 89)

Using the compound obtained in Reference example 54 as starting material, the title compound was obtained according to the same method as Reference example 5 and Example 1.
¹H-NMR(CDCl₃) δ(ppm): 8.57(s,1H), 7.95(s,1H), 7.32(s,1H), 6.90-6.87(m,3H), 5.97(s,2H), 5.60(d,1H,J=7.3Hz), 4.48-4.46(m,1H), 3.16-3.13(m,5H), 3.08-3.05(m,4H), 2.93-2.91 (m,2H), 2.26-2.14(m,4H), 1.68-1.65 (m,2H)

### Example 90: 4-[(6-nitro-7-piperazinylquinazolin-4-yl)amino]piperidinyl-(3-pyridyl)ketone (compound 90)

Using the compound obtained in Reference example 56 as starting material, the title compound was obtained according to the same method as Reference example 5 and Example 1.
¹H-NMR(CDCl₃) δ(ppm): 8.67-8.65(m,2H), 8.57(s,1H), 7.96(s,1H), 7.75-7.72(m,1H), 7.39-7.34(m,2H), 5.48(d,1H,J=7.5Hz), 4.55-4.53(m,1H), 3.15-3.12(m,4H), 3.10-3.07(m,5H), 2.93-2.91 (m,2H), 2.30-2.18(m,4H), 1.70-1.67 (m,2H)

### Example 91: N-phenyl-[4-[(6-nitro-7-piperazinylquinazolin-4-yl)amino]piperidinyl]formamide (compound 91)

Using the compound obtained in Reference example 58 as starting material, the title compound was obtained according to the same method as Reference example 5 and Example 1.
¹H-NMR(CDCl₃) δ(ppm): 8.57(s,1H), 8.10(s,1H), 7.40-7.30(m,6H), 6.48(s,1H), 5.55(d,1H,J=7.0Hz), 4.50-4.48(m,1H), 3.18-3.14 (m,5H), 3.12-3.08(m,4H), 2.97-2.95(m,2H), 2.32-2.22(m,4H), 1.68-1.65(m,2H)

### Example 92: N-(4-fluorophenyl)-[4-[(6-nitro-7-piperazinylquinazolin-4-yl)amino]piperidinyl]formamide (compound 92)

Using the compound obtained in Reference example 60 as starting material, the title compound was obtained according to the same method as Reference example 5 and Example 1.
¹H-NMR(CDCl₃) δ(ppm): 8.60(s,1H), 8.00(s,1H), 7.38-7.32(m,3H), 7.13-7.10(m,2H), 6.52(s,1H), 5.58(d,1H,J=7.2Hz), 4.50-4.48(m,1H), 3.13-3.08(m,5H), 3.06-3.02(m,4H), 2.94-2.92 (m,2H), 2.33-2.21(m,4H), 1.70-1.68(m,2H)

### Example 93: N-(4-methoxyphenyl)-[4-[(6-nitro-7-piperazinylquinazolin-4-yl)amino]piperidinyl]formamide (compound 93)

Using the compound obtained in Reference example 62 as starting material, the title compound was obtained according to the same method as Reference example 5 and Example 1.
¹H-NMR(CDCl₃) δ(ppm): 8.60(s,1H), 8.15(s,1H), 7.35(s,1H), 7.22-7.15(m,3H), 6.95-6.85(m,2H), 5.45(d,1H,J=7.4Hz), 4.40-4.38(m,1H), 3.56(s,3H), 3.13-3.10(m,4H), 3.08-3.05(m,5H), 2.93-2.90(m,2H), 2.35-2.23(m,4H), 1.69-1.67(m,2H)

### Example 94: [4-[(6-nitro-7-piperazinylquinazolin-4-yl)amino]piperidinyl](phenylamino)methane-1-thione (compound 94)

Using the compound obtained in Reference example 64 as starting material, the title compound was obtained according to the same method as Reference example 5 and Example 1.
¹H-NMR(CDCl₃) δ(ppm): 8.60(s,1H), 8.01(s,1H), 7.38-7.32(m,6H), 6.87(s,1H), 5.47(d,1H,J=7.2Hz), 4.48-4.46(m,1H), 3.20-3.16 (m,5H), 3.10-3.06(m,4H), 2.95-2.93(m,2H), 2.28-2.25(m,4H), 1.70-1.68(m,2H)

### Example 95: 4-[(6-nitro-7-piperazinylquinazolin-4-yl)amino]-1-phenylsulfonylpiperidine (compound 95)

Using the compound obtained in Reference example 66 as starting material, the title compound was obtained according to the same method as Reference example 5 and Example 1.
¹H-NMR(CDCl₃) δ(ppm): 8.58(s,1H), 7.95(s,1H), 7.35-7.29(m,6H), 5.65-5.63(br.-d,1H), 4.53-4.50(m,1H), 3.20-3.15 (m,5H), 3.12-3.08(m,4H), 2.98-2.95(m,2H), 2.35-2.23(m,4H), 1.65-1.62(m,2H)

### Example 96: 4-[(6-nitro-7-piperazinylquinazolin-4-yl)amino]-1-(4-methoxyphenyl)sulfonylpiperidine (compound 96)

Using the compound obtained in Reference example 68 as starting material, the title compound was obtained according to the same method as Reference example 5 and Example 1.
¹H-NMR(CDCl₃) δ(ppm): 8.60(s,1H), 7.93(s,1H), 7.22-7.15(m,3H), 6.98-6.90(m,2H), 5.60(d,1H,J=7.2Hz), 4.54-4.52(m,1H), 3.57(s,3H), 3.23-3.18(m,4H), 3.15-3.10(m,5H), 2.96-2.91 (m,2H), 2.40-2.36(m,4H), 1.70-1.67(m,2H)

### Example 97: 4-[(6-nitro-7-piperazinylquinazolin-4-yl)amino]-1-(4-fluorophenyl)sulfonylpiperidine (compound 97)

Using the compound obtained in Reference example 70 as starting material, the title compound was obtained according to the same method as Reference example 5 and Example 1.
¹H-NMR(CDCl₃) δ(ppm): 8.58(s,1H), 7.92(s,1H), 7.39-7.35(m,2H), 7.29(s,1H), 7.18-7.15(m,2H), 5.62(d,1H,J=7.0Hz), 4.60-4.58 (m,1H), 3.20-3.15(m,5H), 3.12-3.09(m,4H), 2.99-2.94(m,2H), 2.42-2.38(m,4H), 1.72-1.68(m,2H)

### Example 98: 6-nitro-4-(4-phenylpiperazinyl)-7-piperazinylquinazoline (compound 98)

Using the compound obtained in Reference example 71 as starting material, the title compound was obtained according to the same method as Example 1.
¹H-NMR(CDCl₃) δ(ppm): 8.66(s,1H), 8.41(s,1H), 7.34-7.29(m,3H), 6.99-6.90(m,3H), 4.06-4.02(m,4H), 3.42-3.39(m,4H), 3.18-3.14(m,5H), 3.09-3.06(m,4H)

### Example 99: 4-[(4-methoxyphenyl)piperazinyl]-6-nitro-7-piperazinylquinazoline (compound 99)

Using 7-chloro-4-[(4-methoxyphenyl)piperazinyl]-6-nitroquinazoline, a compound obtained analogously to Reference example 71, as starting material, the title compound was obtained according to the same method as Example 1.
¹H-NMR(CDCl₃) δ(ppm): 8.57(s,1H), 8.45(s,1H), 7.31(s,1H), 6.95-6.81(m,4H), 4.00-4.04(m,4H), 3.72(s,3H), 3.21-3.25 (m,4H), 3.12-3.14(m,5H), 3.00-2.95(m,4H)

### Example 100: 4-[(2-methoxyphenyl)piperazinyl]-6-nitro-7-piperazinylquinazoline (compound 100)

Using 7-chloro-4-[(2-methoxyphenyl)piperazinyl]-6-nitroquinazoline, a compound obtained analogously to Reference example 71, as starting material, the title compound was obtained according to the same method as Example 1.
¹H-NMR(CDCl₃) δ(ppm): 8.55(s,1H), 8.40(s,1H), 7.42-7.29(m,3H), 7.11-7.08(m,2H), 4.02-3.98(m,4H), 3.64(s,3H), 3.47-3.44 (m,4H), 3.20-3.16(m,5H), 3.10-3.07(m,4H)

### Example 101: 4-[(4-chlorophenyl)piperazinyl]-6-nitro-7-piperazinylquinazoline (compound 101)

Using 7-chloro-4-[(4-chlorophenyl)piperazinyl]-6-nitroquinazoline, a compound obtained analogously to Reference example 71, as starting material, the title compound was obtained according to the same method as Example 1.
¹H-NMR(CDCl₃) δ(ppm): 8.56(s,1H), 8.49(s,1H), 7.34(s,1H), 7.24-7.20(m,2H), 6.96-6.92(m,2H), 4.03-3.99(m,4H), 3.38-3.35(m,4H), 3.18-3.16(m,4H), 3.06-3.02(m,5H)

### Example 102: 4-[(4-fluorophenyl)piperazinyl]-6-nitro-7-piperazinylquinazoline (compound 102)

Using 7-chloro-4-[(4-fluorophenyl)piperazinyl]-6-nitroquinazoline, a compound obtained analogously to Reference example 71, as starting material, the title compound was obtained according to the same method as Example 1.
¹H-NMR(CDCl₃) δ(ppm): 8.60(s,1H), 8.35(s,1H), 7.37-7.30(m,3H), 7.15-7.13(m,2H), 4.10-4.06(m,4H), 3.52-3.48(m,4H), 3.24-3.20(m,5H), 3.12-3.09(m,4H)

### Example 103: 6-nitro-7-piperazinyl-4-[(2-pyridyl) piperazinyl]quinazoline (compound 103)

Using 7-chloro-6-nitro-4-[(2-pyridyl)piperazinyl]quinazoline, a compound obtained analogously to Reference example 71, as starting material, the title compound was obtained according to the same method as Example 1.
¹H-NMR(CDCl₃) δ(ppm): 8.67-8.65(m,2H), 8.57(s,1H), 8.18(s,1H), 7.54-7.49(m,2H), 7.31(s,1H), 4.00-3.96(m,4H), 3.42-3.38 (m,4H), 3.15-3.11(m,5H), 3.02-2.98(m,4H)

### Example 104: 6-nitro-4-(4-benzylpiperazinyl)-7- piperazinylquinazoline (compound 104)

Using 4-(4-benzylpiperazinyl)-7-chloro-6-nitroquinazoline, a compound obtained analogously to Reference example 71, as starting material, the title compound was obtained according to the same method as Example 1.
¹H-NMR(CDCl₃) δ(ppm): 8.68(s,1H), 8.40(s,1H), 7.35-7.30 (m,4H), 7.11-7.08(m,2H). 4.03-4.00(m,4H), 3.55(s,2H), 3.47-3.43 (m,4H), 3.14-3.10(m,5H), 3.06-3.02(m,4H)

### Example 105: 4-(6-nitro-7-piperazinylquinazolin-4-yl)piperazinyl-phenylketone (compound 105)

Using the compound obtained in Reference example 74 as starting material, the title compound was obtained according to the same method as Example 1.
¹H-NMR(CDCl₃) δ(ppm): 8.57(s,1H), 8.43(s,1H), 7.35-7.30 (m,5H), 7.21(s,1H), 4.12-4.08(m,4H), 3.56-3.52(m,4H), 3.24-3.20 (m,5H), 3.12-3.09(m,4H)

### Example 106: 4-(6-nitro-7-piperazinylquinazolin-4-yl)piperazinyl-(4-fluorophenyl)ketone (compound 106)

Using the compound obtained in Reference example 77 as starting material, the title compound was obtained according to the same method as Example 1.
¹H-NMR(CDCl₃) δ(ppm): 8.60(s,1H), 8.49(s,1H), 7.41-7.38 (m,3H), 7.18-7.14(m,2H), 4.08-4.04(m,4H), 3.60-3.56(m,5H), 3.17-3.13 (m,4H), 3.08-3.04(m,4H)

### Example 107: 4-(6-nitro-7-piperazinylquinazolin-4-yl)piperazinyl-(4-methoxyphenyl)ketone (compound 107)

Using the compound obtained in Reference example 80 as starting material, the title compound was obtained according to the same method as Example 1.
¹H-NMR(CDCl₃) δ(ppm): 8.60(s,1H), 8.37(s,1H), 7.38-7.30 (m,3H), 7.12-7.08(m,2H), 4.05-4.01(m,4H), 3.72(s,3H), 3.48-3.47 (m,4H), 3.15-3.10(m,5H), 3.08-3.05(m,4H)

### Example 108: 4-(6-nitro-7-piperazinylquinazolin-4-yl)piperazinyl-1,3-dioxaindan-5-yl-ketone (compound 108)

Using the compound obtained in Reference example 83 as starting material, the title compound was obtained according to the same method as Example 1.
¹H-NMR(CDCl₃) δ(ppm): 8.58(s,1H), 8.21(s,1H), 7.30 (s,1H), 7.10-7.05(m,3H), 5.97(s,2H), 4.08-4.05(m,4H), 3.42-3.38 (m,4H), 3.16-3.12(m,5H), 3.10-3.06(m,4H)

### Example 109: 1-(6-nitro-7-piperazinylquinazolin-4-yl)-4-(phenylsulfonyl)piperazine (compound 109)

Using the compound obtained in Reference example 86 as starting material, the title compound was obtained according to the same method as Example 1.
¹H-NMR(CDCl₃) δ(ppm): 8.57(s,1H), 8.46(s,1H), 7.32-7.28(m,5H), 7.21(s,1H), 4.07-4.03(m,4H), 3.65-3.61(m,4H), 3.20-3.15 (m,5H), 3.07-3.03(m,4H)

### Example 110: 1-(6-nitro-7-piperazinylquinazolin-4-yl)-4-[(4-fluorophenyl)sulfonyl]piperazine (compound 110)

Using the compound obtained in Reference example 89 as starting material, the title compound was obtained according to the same method as Example 1.
¹H-NMR(CDCl₃) δ(ppm): 8.55(s,1H), 8.25(s,1H), 7.40-7.36(m,3H), 7.16-7.12(m,2H), 4.10-4.06(m,4H), 3.48-3.42(m,5H), 3.18-3.14 (m,4H), 3.08-3.04(m,4H)

### Example 111: 4-[2-(4-chlorophenyl)ethylamino]-6-fluoro-7-(piperazin-1-yl)quinazoline (compound 111)

Using the compound obtained in Reference example 93 as starting material, the title compound was obtained according to the same method as Example 1.
¹H-NMR(DMSO-d₆) δ(ppm): 8.35-8.33(br.-t,1H), 8.15(s,1H), 8.05-8.02(m,1H), 7.76-7.73(m,1H), 7.36-7.29(m,4H), 3.68 (dt,2H,J=6.3,5.9Hz), 3.07-3.03(m,5H), 2.98-2.93(m,6H)

### Example 112: 6-fluoro-4-[(4-fluorophenyl)]ethylamino]-7-(piperazin-1-yl)quinazoline (compound 112)

Using the compound obtained in Reference example 94 as starting material, the title compound was obtained according to the same method as Example 1.
¹H-NMR(DMSO-d₆) δ(ppm): 8.76-8.74(br.-t,1H), 8.12(s,1H), 8.06-8.02(m,1H), 7.75-7.70(m,1H), 7.33-7.30(m,2H), 7.16-7.13(m,2H), 3.70-3.68(m,2H), 3.32-3.28(m,5H), 2.97-2.92 (m,6H)

### Example 113: 6-fluoro-4-(4-fluorobenzylamino)-7-(piperazin-1-yl)quinazoline (compound 113)

Using the compound obtained in Reference example 95 as starting material, the title compound was obtained according to the same method as Example 1.
¹H-NMR(DMSO-d₆) δ(ppm): 8.80-8.78(br.-t,1H), 8.16(s,1H), 8.10-8.06(m,1H), 7.79-7.73(m,1H), 7.40-7.38(m,2H), 7.18-7.15(m,2H), 4.80-4.74(m,2H), 3.12-3.08(m,4H), 3.05-3.02(m,5H)

### Example 114: 6-fluoro-4-[3,4-(methylenedioxy)benzylamino]-7-(piperazin-1-yl)quinazoline (compound 114)

Using the compound obtained in Reference example 96 as starting material, the title compound was obtained according to the same method as Example 1.
¹H-NMR(DMSO-d₆) δ(ppm): 8.76-8.74(br.-t,1H), 8.18(s,1H), 8.00-7.96(m,1H), 7.76-7.72(m,1H), 6.95-6.92(m,3H), 5.97(s,2H), 4.82-4.78(m,2H), 3.12-3.08(m,5H), 3.06-3.02 (m,4H)

### Example 115: 6-fluoro-4-[(4-fluoro)anilino]-7-(piperazin-1-yl)quinazoline (compound 115)

Using the compound obtained in Reference example 97 as starting material, the title compound was obtained according to the same method as Example 1.
¹H-NMR(CDCl₃) δ(ppm): 8.65(s,1H), 7.66-7.61(m,2H), 7.47-7.28(m,2H), 7.14-7.08(m,3H), 3.26-3.07(m,9H)

### Example 116: 6-fluoro-4-[(3,4-methylenedioxy)anilino]-7-(piperazin-1-yl)quinazoline (compound 116)

Using the compound obtained in Reference example 98 as starting material, the title compound was obtained according to the same method as Example 1.
¹H-NMR(CDCl₃) δ(ppm): 8.63(s,1H), 7.44-7.26(m,3H), 7.02(brs,1H), 6.94-6.81(m,2H), 6.00(s,2H), 3.26-3.07(m,9H)

### Example 117: 6-fluoro-4-[2-[(4-fluoro)phenyl]ethylamino]-7-(piperazin-1-yl)quinazoline (compound 117)

Using the compound obtained in Reference example 99 as starting material, the title compound was obtained according to the same method as Example 1.
¹H-NMR(CDCl₃) δ(ppm): 8.59(s,1H), 7.22-6.99(m,6H), 5.33 (brt,1H,J=5.1Hz), 3.88(dt,2H,J=5.1,7.0Hz), 3.22-3.06(m,9H), 2.99 (t,2H,J=7.0Hz)

### Example 118: 6-fluoro-4-[2-[(3,4-methylenedioxy)phenyl] ethylamino]-7-(piperazin-1-yl)quinazoline (compound 118)

Using the compound obtained in Reference example 100 as starting material, the title compound was obtained according to the same method as Example 1.
¹H-NMR(CDCl₃) δ(ppm): 8.59(s,1H), 7.25-7.08(m,2H), 6.79-6.67(m,3H), 5.96(s,2H), 5.34-5.30(m,1H), 3.86-3.82(m,2H), 3.22-3.06(m,9H), 2.93(t,2H,J=6.6Hz)

### Example 119: 6-fluoro-4-[3-[(4-fluoro)phenyl]propylamino]-7-(piperazin-1-yl)quinazoline (compound 119)

Using the compound obtained in Reference example 101 as starting material, the title compound was obtained according to the same method as Example 1.
¹H-NMR(CDCl₃) δ(ppm): 8.56(s,1H), 7.25-6.96(m,6H), 5.21 (brt,1H,J=5.7Hz), 3.68(dt,2H,J=5.7,6.6Hz), 3.23-3.06 (m,9H), 2.75(t,2H,J=7.3Hz), 2.05(tt,2H,J=6.6,7.3Hz)

### Example 120: 6-fluoro-4-[3-[(3,4-methylenedioxy)phenyl]propylamino]-7-(piperazin-1-yl)quinazoline (compound 120)

Using the compound obtained in Reference example 102 as starting material, the title compound was obtained according to the same method as Example 1.
¹H-NMR(CDCl₃) δ(ppm): 8.55(s,1H), 7.24-7.20(m,1H), 7.01 (d,1H,J=13.2Hz), 6.77-6.66(m,3H), 5.94(s,2H), 5.20(brt,1H, J=5.4Hz), 3.68(dt,2H,J=5.4,6.3Hz), 3.23-3.06 (m,9H), 2.75 (t,2H,J=7.4Hz), 2.03(tt,2H,J=6.3,7.4Hz)

### Example 121: 4-[(3,4-ethylenedioxy)benzylamino]-6-fluoro-7-(piperazin-1-yl)quinazoline (compound 121)

Using the compound obtained in Reference example 103 as starting material, the title compound was obtained according to the same method as Example 1.
¹H-NMR(CDCl₃) δ(ppm): 8.60(s,1H), 7.24-7.21(m,2H), 6.91-6.86(m,3H), 5.47(brt,1H,J=5.4Hz), 4.71(d,2H,J=5.4Hz), 4.26 (s,4H), 3.23-3.06 (m,9H)

### Example 122: 6-fluoro-4-[[N-methyl-N'-(3,4-methylenedioxybenzyl)]amino]-7-(piperazin-1-yl)quinazoline (compound 122)

Using the compound obtained in Reference example 104 as starting material, the title compound was obtained according to the same method as Example 1.
¹H-NMR(CDCl₃) δ(ppm): 8.60(s,1H), 7.54(d,1H,J=14.6Hz), 7.28- 7.26(m,1H), 6.87-6.83(m,3H), 5.98(s,2H), 4.83(s,2H), 3.31-3.27(m,4H), 3.23(s,3H), 3.15-3.12(m,4H), 2.76-2.72(m,1H)

### Example 123: 4-[(4-fluoro)anilino]-6-nitro-7-(piperazin-1-yl)quinazoline (compound 123)

Using the compound obtained in Reference example 105 as starting material, the title compound was obtained according to the same method as Example 1.
¹H-NMR(CDCl₃) δ(ppm): 8.68(s,1H), 8.42(s,1H), 7.68- 7.63(m,2H), 7.47(s,1H), 7.38(s,1H), 7.17-7.10(m,2H), 3.15-3.06(m,9H)

### Example 124: 4-[(3,4-methylenedioxy)anilino]-6-nitro-7-(piperazin-1-yl)quinazoline (compound 124)

Using the compound obtained in Reference example 106 as starting material, the title compound was obtained according to the same method as Example 1.
¹H-NMR(CDCl₃) δ(ppm): 8.67(s,1H), 8.39(s,1H), 7.45(brs,1H), 7.35-7.31(m,2H), 6.96-6.82(m,2H), 6.01(s,2H), 3.16-3.04 (m,9H)

### Example 125: 4-[2-(3,4-methylenedioxy)phenyl]ethylamino]-6-nitro-7-(piperazin-1-yl)quinazoline (compound 125)

Using the compound obtained in Reference example 107 as starting material, the title compound was obtained according to the same method as Example 1.
¹H-NMR(CDCl₃) δ(ppm): 8.63(s,1H), 8.10(s,1H), 7.31(s,1H), 6.79-6.67(m,3H), 5.96(s,2H), 5.79(brt,1H,J=5.7Hz), 3.88(dt,2H,J=5.7,6.8Hz), 3.11-3.04(m,9H), 2.95(t,2H,J=6.8Hz)

### Example 126: 4-[3-[(4-fluoro)phenyl]propylamino]-6-nitro-7-(piperazin-1-yl)quinazoline (compound 126)

Using the compound obtained in Reference example 108 as starting material, the title compound was obtained according to the same method as Example 1.
¹H-NMR(CDCl₃) δ(ppm): 8.60(s,1H), 8.06(s,1H), 7.30(s,1H), 7.20-7.15(m,2H), 7.01-6.95(m,2H), 5.62(brt,1H,J=5.7Hz), 3.71(dt,2H,J=5.7,6.7Hz), 3.12-3.05(m,9H), 2.76(t,2H, J=7.6Hz), 2.07(tt,2H,J=6.7,7.6Hz)

### Example 127: 4-[3-[(3,4-methylenedioxy)phenyl]propylamino]-6-nitro-7-(piperazin-1-yl)quinazoline (compound 127)

Using the compound obtained in Reference example 109 as starting material, the title compound was obtained according to the same method as Example 1.
¹H-NMR(CDCl₃) δ(ppm): 8.59(s,1H), 7.95(s,1H), 7.29(s,1H), 6.75-6.66(m,3H), 5.92(s,2H), 5.58(brt,1H,J=5.1Hz), 3.71(dt,2H,J=5.1,6.4Hz), 3.11-3.04(m,9H), 2.72(t,2H, J=7.2Hz), 2.05(tt,2H,J=6.4,7.2Hz)

### Example 128: 4-[(3,4-difluoro)benzylamino]-6-nitro-7-(piperazin-1-yl)quinazoline (compound 128)

Using the compound obtained in Reference example 110 as starting material, the title compound was obtained according to the same method as Example 1.
¹H-NMR(DMSO-d₆) δ(ppm): 9.12-9.08(m,1H), 8.93(s,1H), 8.46 (s,1H), 7.47-7.23(m,4H), 4.73(d,2H,J=5.4Hz), 3.05-2.90 (m,9H)

### Example 129: 4-[(4-ethoxycarbonyl)benzylamino]-6-nitro-7-(piperazin-1-yl)quinazoline (compound 129)

Using the compound obtained in Reference example 111 as starting material, the title compound was obtained according to the same method as Example 1.
¹H-NMR(CDCl₃) δ(ppm): 8.64(s,1H), 8.27(s,1H), 8.03(d,2H, J=8.4Hz), 7.44(d,2H,J=8.4Hz), 7.34(s,1H), 6.16-6.12(m,1H), 4.94(d,2H,J=3.8Hz), 4.37(q,2H,J=7.2Hz), 3.14-3.03(m,9H), 1.39(t,3H, J=7.2Hz)

### Example 130: 4-[(3,4-ethylenedioxy)benzylamino]-6-nitro-7-(piperazin-1-yl)quinazoline (compound 130)

Using the compound obtained in Reference example 112 as starting material, the title compound was obtained according to the same method as Example 1.
¹H-NMR(CDCl₃) δ(ppm): 8.64(s,1H), 8.21(s,1H), 7.31(s,1H), 6.91-6.86(m,3H), 5.93(brt,1H,J=5.4Hz), 4.73(d,2H,J=5.4Hz), 4.26(s,4H), 3.11-3.04(m,9H)

### Example 131: 4-[(3,5-di-tert-butyl-4-hydroxy)benzylamino]-6-nitro-7-(piperazin-1-yl)quinazoline (compound 131)

Using the compound obtained in Reference example 113 as starting material, the title compound was obtained according to the same method as Example 1.
¹H-NMR(CDCl₃) δ(ppm): 8.67(s,1H), 8.18(s,1H), 7.32(s,1H), 7.22(s,2H), 5.77-5.74(m,1H), 5.29(s,1H), 4.70(d,2H, J=4.3Hz), 3.12-3.04(m,9H), 1.45(s,18H)

### Example 132: 4-[(3-methoxy-4-propoxy)benzylamino]-6-nitro-7-(piperazin-1-yl)quinazoline (compound 132)

Using the compound obtained in Reference example 114 as starting material, the title compound was obtained according to the same method as Example 1.
¹H-NMR(CDCl₃) δ(ppm): 8.65(s,1H), 8.26(s,1H), 7.31(s,1H), 6.93-6.83(m,3H), 6.10(brt,1H,J=4.9Hz), 4.76(d,2H,J=4.9Hz), 3.97(t,2H,J=6.9Hz), 3.84(s,3H), 3.12-3.03(m,9H), 1.86(dt,2H, J=6.9,7.4Hz), 1.03(t,3H,J=7.4Hz)

### Example 133: 4-[(4-cyclopentyloxy-3-methoxy)benzylamino]-6-nitro-7-(piperazin-1-yl)quinazoline (compound 133)

Using the compound obtained in Reference example 115 as starting material, the title compound was obtained according to the same method as Example 1.
¹H-NMR(CDCl₃) δ(ppm): 8.66(s,1H), 8.26(s,1H), 7.32(s,1H), 6.92-6.83(m,3H), 6.08(brt,1H,J=4.9Hz), 4.76-4.74(m,3H), 3.82(s,3H), 3.12-3.03(m,9H), 1.95-1.78(m,6H), 1.63-1.57 (m,2H)

### Example 134: 6-nitro-4-[(4-nitro)benzylamino]-7- (piperazin-1-yl)quinazoline (compound 134)

Using the compound obtained in Reference example 116 as starting material, the title compound was obtained according to the same method as Example 1.
¹H-NMR(CDCl₃) δ(ppm): 8.62(s,1H), 8.35(s,1H), 8.20(d,2H, J=8.6Hz), 7.55(d,2H,J=8.6Hz), 7.34(s,1H), 6.49(brt,1H, J=5.9Hz), 5.00(d,2H,J=5.9Hz), 3.13-3.03(m,9H)

### Example 135: 4-[[6-chloro-(3,4-methylenedioxy)]benzylamino] -6-nitro-7-(piperazin-1-yl)quinazoline (compound 135)

Using the compound obtained in Reference example 117 as starting material, the title compound was obtained according to the same method as Example 1.
¹H-NMR(CDCl₃) δ(ppm): 8.64(s,1H), 8.21(s,1H), 7.32(s,1H), 6.99(s,1H), 6.88(s,1H), 6.07(brt,1H, J=5.9Hz), 5.98(s,2H), 4.85(d,2H,J=5.9Hz), 3.13-3.03(m,9H)

### Example 136: 4-[[5-methoxy-(3,4-methylenedioxy)] benzylamino]-6-nitro-7-(piperazin-1-yl)quinazoline (compound 136)

Using the compound obtained in Reference example 118 as starting material, the title compound was obtained according to the same method as Example 1.
¹H-NMR(CDCl₃) δ(ppm): 8.65(s,1H), 8.22(s,1H), 7.33(s,1H), 6.59-6.57(m,2H), 5.98(s,2H), 5.92(brt,1H, J=5.4Hz), 4.74(d,2H,J=5.4Hz), 3.91(s,3H), 3.13-3.04(m,9H)

### Example 137: 4-[(4-benzyloxy)benzylamino]-6-nitro-7-(piperazin-1-yl)quinazoline (compound 137)

Using the compound obtained in Reference example 119 as starting material, the title compound was obtained according to the same method as Example 1.
¹H-NMR(CDCl₃) δ(ppm): 8.65(s,1H), 8.18(s,1H), 7.46-7.31(m,8H), 7.01-6.96(m,2H), 5.84(brt,1H, J=5.4Hz), 5.09(s,2H), 4.77(d,2H,J=5.4Hz), 3.13-3.03(m,9H)

### Example 138: 4-[(4-acetamide)benzylamino]-6-nitro-7-(piperazin- 1-yl)quinazoline (compound 138)

Using the compound obtained in Reference example 120 as starting material, the title compound was obtained according to the same method as Example 1.
¹H-NMR(CDCl₃) δ(ppm): 8.64(s,1H), 8.25(s,1H), 7.51-7.48(m,2H), 7.35-7.21(m,4H), 6.10(brt,1H, J=4.9Hz), 4.81(d,2H,J=4.9Hz), 3.13-3.03(m,9H), 2.19(s,3H)

### Example 139: 4-[(4-cyclopropylcarbamoyl)benzylamino]-6-nitro-7-(piperazin-1-yl)quinazoline (compound 139)

Using the compound obtained in Reference example 121 as starting material, the title compound was obtained according to the same method as Example 1.
¹H-NMR(CDCl₃) δ(ppm): 8.61(s,1H), 8.35(s,1H), 7.67(s,1H), 7.44-7.41(m,2H), 7.27-7.24(m,3H), 6.66(brt,1H, J=5.4Hz), 4.76(d,2H,J=5.4Hz), 3.08-3.02(m,9H), 1.57-1.48(m,1H), 1.10-1.04(m,2H), 0.88-0.81(m,2H)

### Example 140: 4-[(3,5-dimethyl-4-hydroxy)benzylamino]-6-nitro-7-(piperazin-1-yl)quinazoline (compound 140)

Using the compound obtained in Reference example 122 as starting material, the title compound was obtained according to the same method as Example 1.
¹H-NMR(DMSO-d₆) δ(ppm): 8.92-8.87(m,2H), 8.45(s,1H), 8.12(s,1H), 7.16(s,1H), 6.90(s,2H), 4.58(d,2H,J=5.4Hz), 2.99-2.79(m,9H), 2.13(s,6H)

### Example 141: 4-[(3-cyclopentyloxy-4-methoxy)benzylamino]-6-nitro-7-(piperazin-1-yl)quinazoline (compound 141)

Using the compound obtained in Reference example 123 as starting material, the title compound was obtained according to the same method as Example 1.
¹H-NMR(CDCl₃) δ(ppm): 8.63(s,1H), 8.26(s,1H), 7.30(s,1H), 6.93-6.85(m,3H), 6.08(brt,1H, J=5.0Hz), 4.77-4.73(m,3H), 3.80(s,3H), 3.14-3.05(m,9H), 2.00-1.83(m,6H), 1.67-1.61 (m,2H)

### Example 142: 4-[(4-methoxy-3-pivaloyloxy)benzylamino]-6-nitro-7-(piperazin-1-yl)quinazoline (compound 142)

Using the compound obtained in Reference example 124 as starting material, the title compound was obtained according to the same method as Example 1.
¹H-NMR(CDCl₃) δ(ppm): 8.64(s,1H), 8.26(s,1H), 7.31(s,1H), 7.24-7.20(m,1H), 7.06-7.05(m,1H), 6.95-6.92(m,1H), 6.15(brt,1H, J=5.4Hz), 4.75(d,2H,J=5.4Hz), 3.81(s,3H), 3.13-3.03(m,9H), 1.36(s,9H)

### Example 143: 4-[(4-hydroxy)benzylamino]-6-nitro-7-(piperazin-1-yl)quinazoline (compound 143)

Using the compound obtained in Reference example 125 as starting material, the title compound was obtained according to the same method as Example 1.
¹H-NMR(DMSO-d₆) δ(ppm): 9.31(s,1H), 9.02-8.93(m,2H), 8.46(s,1H), 7.22(s,1H), 7.17(d,2H,J=8.4Hz), 6.75(d,2H, J=8.4Hz), 4.64(brt,1H, J=5.4Hz), 3.07-2.93(m,9H)

### Example 144: 4-[(4-cyano)benzylamino]-6-nitro-7-(piperazin-1-yl)quinazoline (compound 144)

Using the compound obtained in Reference example 126 as starting material, the title compound was obtained according to the same method as Example 1.
¹H-NMR(CDCl₃) δ(ppm): 8.61(s,1H), 8.34(s,1H), 7.64(d,2H, J=8.9Hz), 7.50(d,2H,J=8.9Hz), 7.33(s,1H), 6.45(brt,1H, J=5.7Hz), 4.96(d,2H,J=5.7Hz), 3.13-3.03(m,9H)

### Example 145: TNF-α production inhibiting test using human PBMC (peripheral blood mononuclear cells)

### Preparation of human PBMC

The blood of a healthy person was taken with a vacuum blood-collecting tube (Nipro neotube) and diluted up to two-fold with physiological saline (Otsuka Pharmaceuticals). To the diluted blood was added 6% dextran T-500 (prepared after dissolution in physiological saline (Otsuka Pharmaceuticals) and sterilization with an autoclave) so as to make a final concentration of 1%. The solution was subjected to tumble mixing and allowed to stand at room temperature for 30 minutes to precipitate the erythrocytes. The supernatant was collected and subjected to centrifugation at 20 °C and at 1,500 rpm for 10 minutes. The cells were suspended in 3 ml of RPMI1640 culture medium (Asahi Technogllas) and overlaid on the solution surface of 3 ml Lymphoprep (NYCOMED). Further, with 1.5 ml of RPMI1640 culture medium being added to the tube which had contained the cell suspension liquid, the cells attached to the tube, which had remained uncollected, were suspended for collection and overlaid on the Lymphoprep previously mentioned. This operation was repeated again. After centrifugation at 20 °C and at 2,000 rpm for 30 minutes, there was collected a fraction of peripheral blood mononuclear cells present in the boundary surface between the Lymphoprep and the RPMI1640 culture medium. The cells were washed twice with 10 ml RPMI1640 culture medium to remove the Lymphoprep and then suspended in a RPMI1640 culture medium added with 10% bovine fetal serum (IRVINE SCIENTIFIC), 100 U/ml penicillin and 100 µg/ml streptomycin (hereinafter referred to as cRPMI1640 culture medium). The number of the cells was measured by Tuerk dying, and the cells, after adjustment to 3 × 10⁶ cells/ml, were planted in 100 µl/well on a 96-well plate (FALCON). The cells were cultured at 37 °C under 5%CO₂ atmosphere for about 5 hours.

### Induction of TNF-α production by LPS stimulus

LPS (E.coli 0111:B4, DIFCO), which had been prepared to 10 mg/ml with water and kept at -20, was diluted to 100 µg/ml with cRPMI1640 culture medium and subjected to sterile filtration. The 100 µg/ml LPS was diluted with cRPMI1640 culture medium to prepare 20 ng/ml LPS. A compound to be tested which was dissolved in DMSO at 1,000-fold concentration was diluted to 500-fold with cRPMI1640 culture medium containing 20 ng/ml LPS and added in 100 µl/well to the cells which had been planted on the previously mentioned 96-well plate (final concentration of LPS: 10 ng/ml). For use as the control group, the solutions were made by diluting DMSO of the same volume as in the test compounds with cRPMI1640 culture medium containing 20 ng/ml LPS, and they were added to the cells. These cells were cultured at 37 °C under 5%CO₂ atmosphere for 16 hours.

### Determination of TNF-α activity (ELISA method)

To the 96-well plate the mouse anti-human TNF-α antibody (prepared to 2 µg/ml with Coating buffer*¹) was added so as to make 100 µl/well and allowed to stand at 4 °C overnight. After 5-times washing with Wash buffer*² (300µl/well), the Blocking buffer*³ was added to the solutions, which were allowed to stand 37 °C for 2 hours. The culture supernatants were diluted up to 5-fold in the plate (supernatant 20 µl being added to 80 µl of cRPMI1640 culture medium). Simultaneously, for using for a calibration curve, a series of dilution of recombinant human TNF-α was prepared arid allowed to stand 37 °C for 1 hour. After 5-times washing with Wash buffer (300 µl /well), biotinylated rabbit anti-human TNF-α antibody (prepared to 0.25 µg/ml with Antibody diluent*⁴) was added so as to make 100 µl /well and allowed to stand at 37 °C for 1 hour. After 5-times washing with Wash buffer (300 µl /well), Streptavidin-Horseradish peroxidase(prepared to 1:4000 with Antibody diluent) was added to make 100 µl /well and allowed to stand at 37 °C for 15 minutes. After 5-times washing with Wash buffer (300 µl /well), TMB microwell peroxidase substrate (KPL) was added to make 100 µl /well and allowed to stand at room temperature for 10 minutes. Stop solution*⁵ was added to make 100 µl /well, absorbance (A450nm)of each well was measured with a microplate reader, and then the data was processed with a Microplate Manager III (BIO-RAD). The human TNF-α activity was determined quantitatively according to the calibration curve from the recombinant TNF-α. The ELISA of human TNF-α mentioned above was conducted using human TNF-α duoset (genzyme). The residual activity of human TNF-α production of the test compound was determined according to the following formula.

Residual activity of human TNF-α production (%)=(human TNF-α activity in culture supernatants of the group added with the compounds)/(human TNF-α activity in culture supernatants of the group not added with the compounds)
* ¹ Coating buffer: 0.1M carbonate, pH 9.4-9.8
* ² Wash buffer: PBS, 0.05% Tween20 (PBS: Takara Shuzo)
* ³ Blocking buffer: 1% bovine serum albumin (BSA), PBS, pH 7.2-7.4 (BSA:SIGMA)
* ⁴ Antibody diluent: 1% BSA, 0.05% Tween20, PBS, pH7.2-7.4
* ⁵ Stop solution: 2NH₂SO₄

From the residual activity of human TNF-α production determined by the above formula, the inhibitory activity IC₅₀ was determined. The results are shown in Table 21.

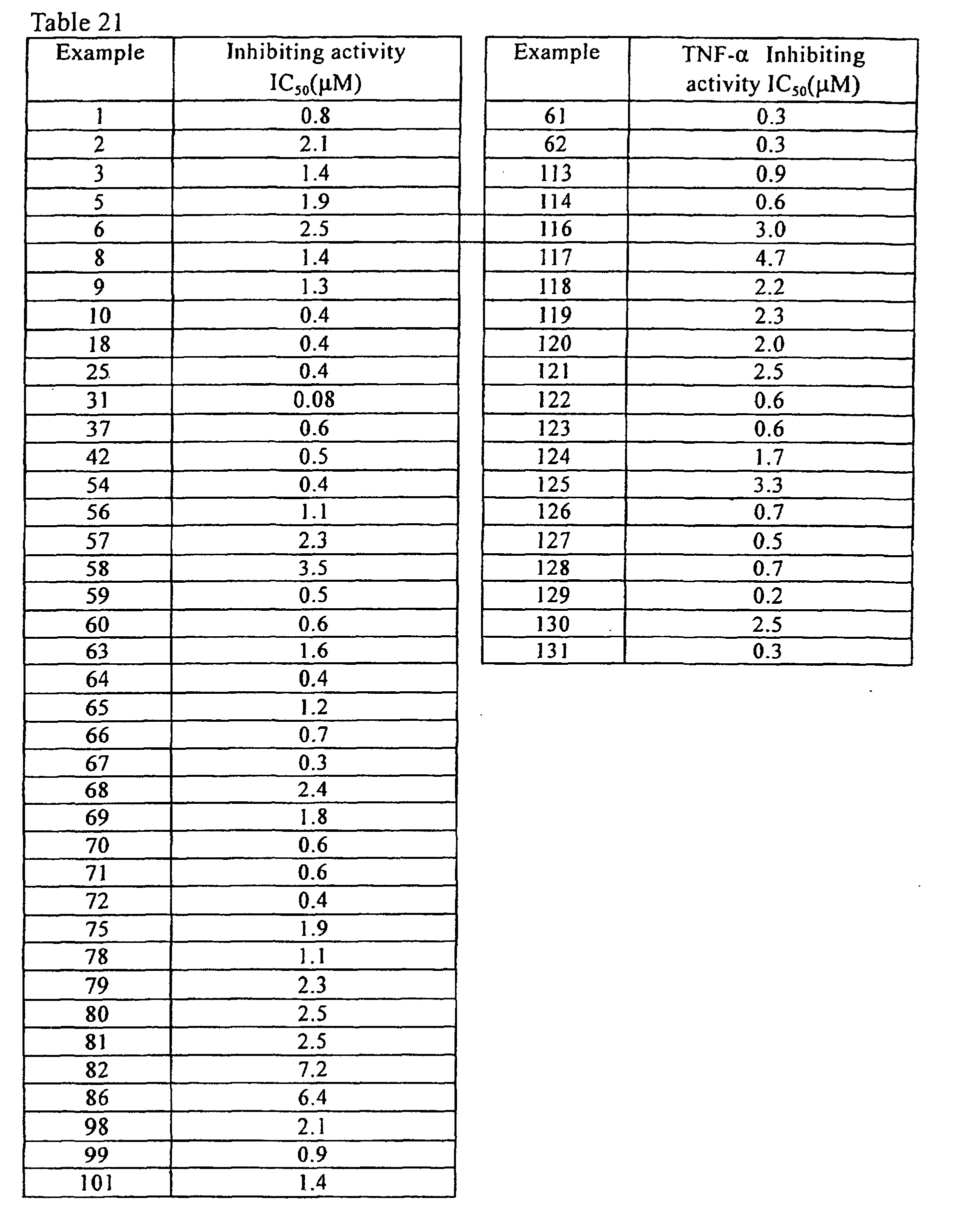

### Example 146: IL-4 and IL-5 production inhibiting test

A 6-weeks-old BALB/c-mouse (Nippon Charles River) was bred preparatively for 1 week and given at the day 0 and day 14 intraperitoneal injection of 100 µl PBS to give immunity which PBS contained 0.1 mg/ml of Ovalbumin(OVA) and 40 mg/ml of aluminum hydroxide. At the day 21 the spleen was taken out from the mouse. The spleen was dissected with a *pincette* in cooled PBS, passed through a mesh and subjected to centrifugation at 4 °C at 1,500 rpm for 5 minutes. The cells was added with 5 ml of 0.2% NaCI to destroy erythrocytes, and then was made isotonic by adding 5 ml of 1.6% of NaCl. After centrifugation at 4 °C, 1,500 rpm for 5 minutes, the cells were washed twice with 10 ml ofRPMI1640 culture medium. The obtained spleen cells were suspended in cRPMI1640 medium, and the number of the cells was measured by Tuerk dying. After being adjusted to 1 × 10⁷ cells/ml, the cells were planted in 100 µl/well on a 96-well plate (FALCON). The cells were cultured at 37 °C under 5% CO₂ atmosphere for about 4 hours.
OVA was prepared to 10 mg/ml with cRPMI1640 culture medium and subjected to sterile filtration. The 10 mg/ml OVA was diluted with cRPMI1640 culture medium to give 1 mg/ml OVA. A compound to be tested which was dissolved in DMSO in at 1,000-fold concentration was diluted up to 500-fold with cRPMI1640 culture medium containing 1 mg/ml OVA and added in 100 µl/well to the cells which had been planted on the previously mentioned 96-well plate (final concentration of OVA: 0.5 mg/ml). As the controls, the solutions were made by diluting DMSO of the same volume as with the test compounds with cRPMI1640 culture medium containing 1 mg/ml OVA, and they were added to the cells. These cells were cultured at 37 °C under 5%CO₂ atmosphere for 3 days.
IL-4 and IL-5 in the culture supernatants after 3 days were quantitatively determined by ELISA method. IL-4 and IL-5 were quantitatively determined using IL-4 Development Kit (Genzyme Techne) and mouse IL-5 MiniKit (ENDOGEN), respectively. The production inhibiting rates of each tested compound against IL-4 are shown in Table 22, those against IL-5 in Table 23.

**Table 22**

| Example | IL-4 inhibiting activity IC₅₀(µM) |
|---|---|
| 48 | 0.1 |
| 51 | 0.1 |
| 131 | 0.2 |
| 31 | 0.01 |
| 129 | 0.1 |
| 61 | 0.2 |
| 62 | 0.1 |

**Table 23**

| Example | IL-5 inhibiting activity IC₅₀(µM) |
|---|---|
| 48 | 0.9 |
| 131 | 1.4 |
| 31 | 0.2 |
| 129 | 1.7 |
| 61 | 2.0 |
| 62 | 1.4 |

### Medicament preparation example 1

Tablets consisting of the following components were made according to the customary method.

**Table 24**

| | Component | Amount(mg/tablet) |
|---|---|---|
| Component 1 | Hydrochloride of | 30 |
| | the compound of Example 1 | |
| Component 2 | Lactose | 124.2 |
| Component 3 | Potato starch | 40 |
| Component 4 | Hydroxypropyl cellulose | 5 |
| Component 5 | Magnesium stearate | 0.8 |
| | Total | 200 mg |

### Medicament preparation example 2

Injection agents consisting of the following components were made according to the customary method.

**Table 25**

| | Component | Amount per vial |
|---|---|---|
| Component 1 | Hydrochloride of | 10 mg |
| | the compound of Example 37 | |
| Component 2 | Purified soybean oil | 180 mg |
| Component 3 | Purified egg yolk lecithin | 25 mg |
| Component 4 | Glycerine for injection use | 50 mg |
| Component 5 | Distilled water for injection use | 2.70 ml |
| | Total | 3.00 ml |

All the publications, patents and patent applications cited herein are incorporated herein by reference in their entirety.

### Industrial Applicability

The quinazoline derivatives of the present invention have TNF-α, IL-4 and IL-5 production inhibiting action, and are useful for prevention or treatment of diseases which arise from the continuous or excess production of TNF-α, IL-4 or IL-5, such as rheumatoid arthritis, septic shock, inflammatory bowel diseases, osteoarthritis, multiple sclerosis, Behcet's disease, systemic lupus erythematodes (SLE), rejection at the time of the bone marrow transplantation, hepatitis, type II diabetes, asthma, allergic dermatitis, allergic rhinitis and the like.

## Claims

1. A quinazoline derivative of the general formula (1) or a pharmaceutically acceptable salt thereof: 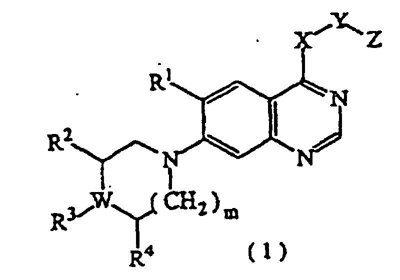 [wherein R¹ represents nitro group or halogen atom; R² and R⁴ represent hydrogen atom, alkyl group having 1 to 4 carbon atoms, carboxyl group, or alkoxycarbonyl group having 2 to 5 carbon atoms; R³ represents hydrogen atom, amino group, unsubstituted or substituted alkyl group having 1 to 4 carbon atoms, alkanoyl group having 1 to 4 carbon atoms or alkoxycarbonyl group having 2 to 5 carbon atoms; W represents carbon atom or nitrogen atom; m represents 0 to 2; X represents a group represented by the following formula (a), (b) or (c): 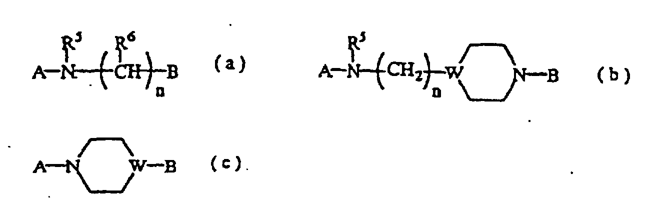 [wherein R⁵ represents hydrogen atom, or unsubstituted or substituted alkyl group having 1 to 4 carbon atoms; R⁶ represents hydrogen atom, unsubstituted or substituted alkyl group having 1 to 4 carbon atoms, hydroxyl group, unsubstituted or substituted aryl group, carboxyl group, or alkoxycarbonyl group having 2 to 5 carbon atoms (if n is two or more, each R⁶ may be the same or different); n represents 0 to 3; W represents carbon atom or nitrogen atom; A represents a single bond to quinazoline ring; and B represents a single bond to Y]; Y represents, if X is represented by the formula (a), single bond or CH₂, if X is represented by the formula (b), single bond, CO, CH₂, CONH, CSNH or SO₂, if X is represented by the formula (c), single bond, CO, CH₂ or SO₂; and Z represents unsubstituted or substituted aryl group, or unsubstituted or substituted heteroaryl group.]

2. A compound according to Claim 1, wherein R¹ in the general formula (1) is nitro group or fluorine atom.

3. A compound according to Claim 1, wherein R² and R⁴ in the general formula (1) are hydrogen atom or alkyl group having 1 to 4 carbon atoms.

4. A compound according to Claim 1, wherein R³ in the general formula (1) R³ is hydrogen atom.

5. A compound according to Claim 1, wherein W in the general formula (1) is nitrogen atom.

6. A compound according to Claim 1, wherein X in the general formula (1) is represented by the formula (a), and R⁵ and R⁶ in the general formula (1) are hydrogen atom.

7. A compound according to Claim 1, wherein n in the formula (a) is 1 or 2.

8. A compound according to Claim 1, wherein X in the general formula (1) is represented by the formula (c).

9. A compound according to Claim 1, wherein Z in the general formula (I) is phenyl, substituted phenyl, thienyl, furyl or pyridyl.

10. A medicament comprising a quinazoline derivative according to Claim 1 or a pharmaceutically acceptable salt thereof as effective ingredient.

11. A medicament for treating the diseases caused by the excess production of TNF-α, comprising a quinazoline derivative according to Claim 1 or a pharmaceutically acceptable salt thereof as effective ingredient.

12. A medicament for treating the diseases caused by the excess production of IL-4, comprising a quinazoline derivative according to Claim 1 or a pharmaceutically acceptable salt thereof as effective ingredient.

13. A medicament for treating the diseases caused by the excess production of IL-5, comprising a quinazoline derivative according to Claim 1 or a pharmaceutically acceptable salt thereof as effective ingredient.
